(19) Europäisches Patentamt
European Patent Office
Office européen des brevets

(11) EP 3 382 037 B1

(12) **EUROPEAN PATENT SPECIFICATION**

(45) Date of publication and mention of the grant of the patent:
**17.02.2021 Bulletin 2021/07**

(51) Int Cl.:
***C12Q 1/6806*** *(2018.01)* ***C12Q 1/6809*** *(2018.01)*
***C12Q 1/6869*** *(2018.01)*

(21) Application number: **18160303.6**

(22) Date of filing: **01.12.2010**

(54) **METHODS FOR DETERMINING FRACTION OF FETAL NUCLEIC ACIDS IN MATERNAL SAMPLES**

VERFAHREN ZUR BESTIMMUNG DER FRAKTION VON FÖTALEN NUKLEINSÄUREN IN MATERNALEN PROBEN

PROCÉDÉS DE DÉTERMINATION DE FRACTION D’ACIDES NUCLÉIQUES FOETAUX DANS D’ÉCHANTILLONS MATERNELS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.01.2010 US 296358 P**
**01.07.2010 US 360837 P**
**26.10.2010 US 455849 P**
**26.10.2010 US 407017 P**

(43) Date of publication of application:
**03.10.2018 Bulletin 2018/40**

(62) Document number(s) of the earlier application(s) in accordance with Art. 76 EPC:
**15181244.3 / 3 006 573**
**10844163.5 / 2 513 339**

(73) Proprietor: **Verinata Health, Inc.**
**San Diego, California 92122 (US)**

(72) Inventors:
- **RAVA, Richard P.**
  **Redwood City, CA California 94062 (US)**
- **CHUU, Yue-Jen**
  **Cupertino, CA California 95014 (US)**
- **CHINNAPPA, Manjula**
  **San Mateo, CA California 94404 (US)**
- **COMSTOCK, David A.**
  **San Francisco, CA California 94107 (US)**
- **HEILEK, Gabrielle**
  **Mountain View, CA California 94043 (US)**
- **HUNKAPILLER, Michael**
  **San Carlos, CA California 94070 (US)**

(74) Representative: **Chapman, Desmond Mark**
**Carpmaels & Ransford LLP**
**One Southampton Row**
**London WC1B 5HA (GB)**

(56) References cited:
**WO-A1-2011/057094 WO-A2-2007/100911**
**WO-A2-2007/147074 US-A1- 2008 050 739**

- **LI YING ET AL: "Size separation of circulatory DNA in maternal plasma permits ready detection of fetal DNA polymorphisms", CLINICAL CHEMISTRY, AMERICAN ASSOCIATION FOR CLINICAL CHEMISTRY, WASHINGTON, DC, vol. 50, no. 6, 1 June 2004 (2004-06-01), pages 1002-1011, XP002510472, ISSN: 0009-9147, DOI: 10.1373/CLINCHEM.2003.029835**
- **CHIU R W K ET AL: "Non-invasive prenatal diagnosis by single molecule counting technologies", TRENDS IN GENETICS, ELSEVIER SCIENCE PUBLISHERS B.V. AMSTERDAM, NL, vol. 25, no. 7, 1 July 2009 (2009-07-01), pages 324-331, XP026301457, ISSN: 0168-9525, DOI: 10.1016/J.TIG.2009.05.004 [retrieved on 2009-06-18]**
- **Ncbi: "NCBI assay ID ss3206919 for rr560681", , 5 September 2001 (2001-09-05), XP055483016, Retrieved from the Internet: URL:https://www.ncbi.nlm.nih.gov/projects/SNP/snp_ss.cgi?subsnp_id=3206919 [retrieved on 2018-06-11]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 382 037 B1

- FAN H C ET AL: "Noninvasive diagnosis of fetal aneuploidy by shotgun sequencing DNA from maternal blood", PROCEEDINGS OF THE NATIONAL ACADEMY OF SCIENCES, NATIONAL ACADEMY OF SCIENCES, US, vol. 105, no. 42, 21 October 2008 (2008-10-21), pages 16266-16271, XP002613056, ISSN: 0027-8424, DOI: 10.1073/PNAS.0808319105 [retrieved on 2008-10-06]
- T. CHU ET AL: "Statistical model for whole genome sequencing and its application to minimally invasive diagnosis of fetal genetic disease", BIOINFORMATICS, vol. 25, no. 10, 23 March 2009 (2009-03-23), pages 1244-1250, XP055018601, ISSN: 1367-4803, DOI: 10.1093/bioinformatics/btp156

**Description**

## FIELD OF THE INVENTION

The present invention relates to methods for determining the fraction of cell-free fetal nucleic acid circulating in a maternal sample.

## BACKGROUND OF THE INVENTION

Invasive prenatal tests are potentially harmful to the mother and to the fetus. Therefore, there is a need for the development of noninvasive prenatal tests. Maternal blood can contain fetal cells (see e.g., U.S. Patent Application Publication No. 20080070792) and cell-free fetal DNA (see e.g., Huang et al. (2008), Methods in Molecular Biology, 444:203-208). While circulating fetal cells present an attractive target for non invasive prenatal diagnostics, particularly for the diagnosis of fetal sex and chromosomal abnormalities by simple karyotyping, the scarcity of intact fetal cells in the maternal circulation (around one cell per ml of maternal blood), low efficiency of enrichment (Bianchi et al., Am J Hum Genet 61:822-829 [1997]) and difficulties with chromosomal analysis associated with abnormally dense nuclei in some cells (Babochkina et al., Haematologica 90:740-745 [2005]), have favored research on cell-free DNA.

The establishment of the concentrations of cell-free fetal DNA (cfDNA) in maternal plasma in healthy pregnant women has formed the platform on which fetal DNA abnormalities in pregnancy-associated disorders can be studied. The finding of a gradual increase in fetal DNA concentration in maternal serum as gestation progresses has been shown to precede complications associated with preterm labor. A five-fold increase in fetal DNA concentration has also been found in the serum obtained from women affected by preeclampsia. Other pregnancy-related disorders that have been linked to an elevated concentration of cfDNA include hyperemesis gravidarum (severe morning sickness), invasive placentation (in which the placenta contacts the maternal bloodstream), intrauterine growth restriction, feto-maternal haemorrhage and polyhydramnios. (Wright C.F. and Burton H., Human Reproduction Update 15(1):139-151 [2009]).

Quantitative analysis of cell free DNA by real-time PCR strategies has also indicated that the concentrations of circulatory fetal DNA are increased in pregnancies with fetal aneuploidies, most notably trisomy 21 (Lo et al., Clin Chem 45:1747-1751 [1999]). However, the fraction of fetal DNA in maternal cell-free plasma DNA is usually determined by comparing the amount of fetal-specific locus (such as the SRY locus on chromosome Y in male pregnancies) to that of a locus on any autosome that is common to both the mother and the fetus by using quantitative real-time PCR (Dahllan et al., Lancet 369:474-481 [2007]; Li et al., Clin Chem 1002-1011 [2004]; Fan et al., Proc Natl Acad Sci 105:16266-16271 [2008]).

WO 2011/057094 teaches fetal genomic analysis from a maternal biological sample. Chiu et al. (Trends in Genetics 2009, vol. 25, no. 7, pages 324-331) teaches non-invasive prenatal diagnosis by single molecule counting technologies.

There is a need for additional methods that would enable the determination of the fraction of fetal nucleic acid in both male and female pregnancies.

The method of the invention fulfills the need in providing the means to determine fetal fraction that is independent of the gender of the fetus. The method can be applied for determining simultaneously the presence or absence of a chromosomal aneuploidy or other copy number variation, and may be used in conjunction with nay known methods that are used to determine aneuploidies in maternal sample.

## SUMMARY OF THE INVENTION

The invention provides a method for determining the fraction of fetal nucleic acids in a maternal sample comprising a mixture of fetal and maternal genomic DNA, as defined in the claims.

In some embodiments, the at least one SNP site is a single SNP site selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In other embodiments, the at least one SNP site is a tandem SNP site selected from tandem SNP pairs rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392- rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672.

Also disclosed herein is a composition for determining the fraction of fetal cfDNA in a maternal sample by massively parallel sequencing the fetal and maternal cfDNA in the maternal sample, *e.g.* a plasma sample, wherein the composition comprises at least one set of primers for amplifying at least one polymorphic nucleic acid in said mixture.

Also disclosed herein is a kit that comprises the composition described above.

## BRIEF DESCRIPTION OF THE DRAWINGS

**Figure 1** is a flowchart of a method 100 for determining the fetal fraction in a maternal test sample comprising a mixture of fetal and maternal nucleic acids using massively parallel sequencing methods or size separation of polymorphic nucleic acid sequences.

**Figure 2** is a bar diagram showing the identification of fetal and maternal polymorphic sequences (SNPs) used to determine fetal fraction in a test sample. The total number of sequence reads (Y-axis) mapped to the SNP sequences identified by rs numbers (X-axis), and the relative level of fetal nucleic acids (*) are shown.

**Figure 3** is a flowchart outlining alternative embodiments of the method for determining fetal fraction by massively parallel sequencing shown in **Figure 1.**

**Figure 4** illustrates STR markers used in the AmpFℓSTR® Identifiler® PCR Amplification Kit.

**Figure 5** illustrates STR markers used in the AmpFℓSTR® MiniFiler® PCR Amplification Kit.

**Figure 6** illustrates the correlation of fetal fraction determined by massively parallel sequencing size separation of polymorphic sequences comprising SNPs and STRs.

**Figure 7** illustrates an embodiment of use of fetal fraction for determining cutoff thresholds for aneuploidy detection.

## DETAILED DESCRIPTION OF THE INVENTION

The invention provides methods for determining the fraction of fetal nucleic acids in a maternal sample comprising a mixture of fetal and maternal nucleic acids. The fraction of fetal nucleic acids can be used in determining the presence or absence of fetal aneuploidy.

The invention is as set out in the appended claims.

Unless otherwise indicated, the practice of the present invention involves conventional techniques commonly used in molecular biology, microbiology, protein purification, protein engineering, protein and DNA sequencing, and recombinant DNA fields, which are within the skill of the art. Such techniques are known to those of skill in the art and are described in numerous standard texts and reference works.

Numeric ranges are inclusive of the numbers defining the range. It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

## DEFINITIONS

As used herein, the singular terms "a", "an," and "the" include the plural reference unless the context clearly indicates otherwise. Unless otherwise indicated, nucleic acids are written left to right in 5' to 3' orientation and amino acid sequences are written left to right in amino to carboxy orientation, respectively.

As used herein, the singular terms "a", "an," and "the" include the plural reference unless the context clearly indicates otherwise. Unless otherwise indicated, nucleic acids are written left to right in 5' to 3' orientation and amino acid sequences are written left to right in amino to carboxy orientation, respectively.

The term "portion" when used in reference to the amount of sequence information of fetal and maternal nucleic acid molecules in a biological sample herein refers to the amount of sequence information of fetal and maternal nucleic acid molecules in a biological sample that in sum amount to less than the sequence information of <1 human genome.

The terms "polynucleotide", "nucleic acid" and "nucleic acid molecules" are used interchangeably and refer to a covalently linked sequence of nucleotides (i.e., ribonucleotides for RNA and deoxyribonucleotides for DNA) in which the 3' position of the pentose of one nucleotide is joined by a phosphodiester group to the 5' position of the pentose of the next, include sequences of any form of nucleic acid, including, but not limited to RNA, DNA and cfDNA molecules. The term "polynucleotide" includes, without limitation, single- and doublestranded polynucleotide.

The term "copy number variation" herein refers to variation in the number of copies of a nucleic acid sequence that is 1 kb or larger present in a test sample in comparison with the copy number of the nucleic acid sequence present in a qualified sample. A "copy number variant" refers to the 1 kb or larger sequence of nucleic acid in which copy-number differences are found by comparison of a sequence of interest in test sample with that present in a qualified sample. Copy number variants/variations include deletions, including microdeletions, insertions, including microinsertions, duplications, multiplications, inversions, translocations and complex multisite variants. CNV encompass chromosomal aneuploidies and partial aneuplodies.

As used herein, the term "fetal fraction" is used interchangeably with "fraction of fetal nucleic acid", which refers to the fraction of fetal nucleic acid in a sample comprising fetal and maternal nucleic acid. Similarly, the term "minor fraction" or "minor component" herein refers to the lesser fraction of the total genetic material that is present in a sample containing genetic material derived from separate sources *e.g.* individuals.

As used herein the term "allele" refers to one specific form of a genetic sequence (such as a gene) within a cell, a sample, an individual or within a population, the specific form differing from other forms of the same gene in the sequence of at least one, and frequently more than one, variant sites within the sequence of the gene. The sequences at these variant sites that differ between different alleles are termed "variants", "polymorphisms", or "mutations". In general, polymorphism is used to refer to variants that have a frequency of at least 1% in a population, while the term mutation is generally used for variants that occur at a frequency of less than 1% in a population. In diploid organisms such as humans, at each autosomal specific chromosomal location or "locus" an individual possesses two alleles, a first inherited from one parent and a second inherited from the other parent, for example one from the mother and one from the father. An individual is "heterozygous" at a locus if it has two different alleles at the locus. An individual is "homozygous" at a locus if it has two identical alleles at that locus.

The term "enrich" herein refers to the process of amplifying polymorphic target nucleic acids contained in a portion of a maternal sample, and combining the amplified product with the remainder of the maternal sample from which the portion was removed.

As used herein, the term "genotyping" refers to the determination of the genetic information an individual carries at one or more positions in the genome. For example, genotyping may comprise the determination of which allele or alleles an individual carries for a single SNP or the determination of which allele or alleles an individual carries for a plurality of SNPs. For example, a particular nucleotide in a genome may be a T in some individuals and a C in other individuals. Those individuals who have a T at the position have the T allele and those who have a C have the C allele. In a diploid organism the individual will have two copies of the sequence containing the polymorphic position so the individual may have a T allele and a C allele or alternatively two copies of the T allele or two copies of the C allele. Those individuals who have two copies of the C allele are homozygous for the C allele, those individuals who have two copies of the T allele are homozygous for the T allele, and those individuals who have one copy of each allele are heterozygous. The alleles are often referred to as the A allele, often the major allele, and the B allele, often the minor allele. The genotypes may be AA (homozygous A), BB (homozygous B) or AB (heterozygous). Genotyping methods generally provide for identification of the sample as AA, BB or AB.

As used herein the term "chromosome" refers to the heredity-bearing gene carrier of a living cell which is derived from chromatin and which comprises DNA and protein components (especially histones). The conventional internationally recognized individual human genome chromosome numbering system is employed herein. The size of an individual chromosome can vary from one type to another with a given multi-chromosomal genome and from one genome to another. In the case of the human genome, the entire DNA mass of a given chromosome is usually greater than about 100,000,000 bp. For example, the size of the entire human genome is about 3.times.10.sup.9 bp. The largest chromosome, chromosome no. 1, contains about 2.4.times.10.sup.8 by while the smallest chromosome, chromosome no. 22, contains about 5.3.times. 10.sup.7 bp.

The term "aneuploidy" herein refers to the occurrence of one or more extra or missing chromosomes.

As used herein the term "chromosomal region" is a portion of a chromosome. The actual physical size or extent of any individual chromosomal region can vary greatly. The term "region" is not necessarily definitive of a particular one or more genes because a region need not take into specific account the particular coding segments (exons) of an individual gene.

As used herein the term "genetic marker" refers to a sequence of DNA that has a specific location on a chromosome that can be measured in a laboratory. The term "genetic marker" can also be used to refer to, e.g., a cDNA and/or an mRNA encoded by a genomic sequence, as well as to that genomic sequence. To be useful, a marker needs to have two or more alleles or variants. Markers can be either direct, that is, located within the gene or locus of interest (i.e., candidate gene), or indirect, that is closely linked with the gene or locus of interest (presumably due to a location which is proximate to, but not inside the gene or locus of interest). Moreover, markers can also include sequences which either do or do not modify the amino acid sequence of a gene.

As used herein, the term "maternal sample" refers to a biological sample obtained from a pregnant subject, and comprises a mixture of fetal and maternal nucleic acids. A "pregnant subject" is not limited to a human being, but may also include other organisms including but not limited to mammals, plants, bacteria or cells derived from any of the above.

The term "whole genome amplification" or "WGA" as used herein generally refers to a method for amplification of a limited DNA sample in a non-specific manner, in order to generate a new sample that is indistinguishable from the original but with a higher DNA concentration. The ideal whole genome amplification technique would amplify a sample up to a microgram level while maintaining the original sequence representation. The DNA of the sample may include an entire genome or a portion thereof. Degenerate oligonucleotide-primed PCR (DOP), primer extension PCR technique (PEP) including modified improved primer extension preamplification (mIPEP), and multiple displacement amplification (MDA), are examples of whole genome amplification methods.

The term "short tandem repeat" or "STR" as used herein refers to a class of polymorphisms that occurs when a pattern of two or more nucleotides are repeated and the repeated sequences are directly adjacent to each other. The pattern can range in length from 2 to 10 base pairs (bp) (for example (CATG)n in a genomic region) and is typically in the non-coding intron region. By examining several STR loci and counting how many repeats of a specific STR sequence there are at a given locus, it is possible to create a unique genetic profile of an individual.

The term "primer," as used herein refers to an isolated oligonucleotide which is capable of acting as a point of initiation of synthesis when placed under conditions in which synthesis of a primer extension product which is complementary to a nucleic acid strand is induced, (i.e., in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer, use of the method, and the parameters used for primer design, as disclosed herein.

The term "primer pair" or "primer set" refers to a set of primers including a 5' "upstream primer" or "forward primer" that hybridizes with the complement of the 5' end of the DNA sequence to be amplified and a 3' "downstream primer" or "reverse primer" that hybridizes with the 3' end of the sequence to be amplified. As will be recognized by those of skill in the art, the terms "upstream" and "downstream" or "forward" and "reverse" are not intended to be limiting, but rather provide illustrative orientation in particular embodiments. A primer pair is said to be "unique" if it can be employed to specifically amplify a particular target nucleotide sequence in a given amplification mixture.

A "polymorphic marker" or "polymorphic site" is a locus at which nucleotide sequence divergence occurs. The locus may be as small as one base pair. Illustrative markers have at least two alleles, each occurring at frequency of greater than 1%, and more typically greater than 10% or 20% of a selected population. A polymorphic site may be as small as one base pair. Polymorphic markers include restriction fragment length polymorphism (RFLPs), variable number of tandem repeats (VNTR's), hypervariable regions, minisatellites, dinucleotide repeats, trinucleotide repeats, tetranucleotide repeats, simple sequence repeats, deletions, and insertion elements such as Alu. The first identified allelic form is arbitrarily designated as the reference form and other allelic forms are designated as alternative or variant alleles. The allelic form occurring most frequently in a selected population is sometimes referred to as the wild type form. Diploid organisms may be homozygous or heterozygous for allelic forms. A diallelic polymorphism has two forms. A triallelic polymorphism has three forms. A polymorphism between two nucleic acids can occur naturally, or be caused by exposure to or contact with chemicals, enzymes, or other agents, or exposure to agents that cause damage to nucleic acids, for example, ultraviolet radiation, mutagens or carcinogens. The terms "polymorphic locus" and "polymorphic site" are herein used interchangeably.

The terms "polymorphic target nucleic acid", "polymorphic sequence", "polymorphic target nucleic acid sequence" and "polymorphic nucleic acid" are used interchangeably herein to refer to a nucleic acid sequence *e.g.* a DNA sequence, that comprises one or more polymorphic sites *e.g* one SNP or a tandem SNP. Polymorphic sequences according to the present technology can be used to specifically differentiate between maternal and non-maternal alleles in the maternal sample comprising a mixture of fetal and maternal nucleic acids.

A "single nucleotide polymorphism" (SNP) occurs at a polymorphic site occupied by a single nucleotide, which is the site of variation between allelic sequences. The site is usually preceded by and followed by highly conserved sequences of the allele (*e.g.*, sequences that vary in less than 1/100 or 1/1000 members of the populations). A SNP usually arises due to substitution of one nucleotide for another at the polymorphic site. A transition is the replacement of one purine by another purine or one pyrimidine by another pyrimidine. A transversion is the replacement of a purine by a pyrimidine or vice versa. SNPs can also arise from a deletion of a nucleotide or an insertion of a nucleotide relative to a reference allele. Single nucleotide polymorphisms (SNPs) are positions at which two alternative bases occur at appreciable frequency (>1%) in the human population, and are the most common type of human genetic variation.

As used herein, the term "short tandem repeat" or "STR" as used herein refers to a class of polymorphisms that occurs when a pattern of two or more nucleotides are repeated and the repeated sequences are directly adjacent to each other. The pattern can range in length from 2 to 10 base pairs (bp) (for example (CATG)n in a genomic region) and is typically in the non-coding intron region. By examining several STR loci and counting how many repeats of a specific STR sequence there are at a given locus, it is possible to create a unique genetic profile of an individual.

As used herein, the term "miniSTR" herein refers to tandem repeat of four or more base pairs that spans less than about 300 base pairs, less than about 250 base airs, less than about 200 base pairs, less than about 150 base pairs, less than about 100 base pairs, less than about 50 base pairs, or less than about 25 base pairs. "miniSTRs" are STRs that are amplifiable from cfDNA templates.

The term "tandem SNPs" herein refers to two or more SNPs that are present within a polymorphic target nucleic acid sequence.

The terms "plurality of polymorphic target nucleic acids", "polymorphic nucleic acids" and "polymorphic sequences" are used interchangeably herein and refer to a number of nucleic acid sequences each comprising at least one polymorphic site *e.g.* one SNP, such that at least 1, 2, 3, 4, 5, 6, 7, 8, 9, 10, 15, 20, 25, 30, or 40 or more different polymorphic sites are amplified from the polymorphic target nucleic acids to identify and/or quantify fetal alleles present in maternal samples comprising fetal and maternal nucleic acids.

As used herein, the term "substantially cell free" encompasses preparations of the desired sample from which components that are normally associated with it are removed. For example, a plasma sample is rendered essentially cell free by removing blood cells *e.g.* red cells, that are normally associated with it. In some embodiments, substantially free samples are processed to remove cells that would otherwise contribute to the desired genetic material that is to be tested for an abnormality

As used herein the term "chromosome" refers to the heredity-bearing gene carrier of a living cell which is derived from chromatin and which comprises DNA and protein components (especially histones). The conventional internationally recognized individual human genome chromosome numbering system is employed herein. The size of an individual chromosome can vary from one type to another with a given multi-chromosomal genome and from one genome to another. In the case of the human genome, the entire DNA mass of a given chromosome is usually greater than about 100,000,000 bp. For example, the size of the entire human genome is about 3.times.10.sup.9 bp. The largest chromosome, chromosome no. 1, contains about 2.4.times.10.sup.8 by while the smallest chromosome, chromosome no. 22, contains about 5.3.times. 10.sup.7 bp.

The term "oligonucleotide" is used to refer to a nucleic acid that is relatively short, generally shorter than 200 nucleotides, more particularly, shorter than 100 nucleotides, most particularly, shorter than 50 nucleotides. Typically, oligonucleotides are single-stranded DNA molecules.

The term "primer" refers to an oligonucleotide that is capable of hybridizing (also termed "annealing") with a nucleic acid and serving as an initiation site for nucleotide (RNA or DNA) polymerization under appropriate conditions (i.e., in the presence of four different nucleoside triphosphates and an agent for polymerization, such as DNA or RNA polymerase or reverse transcriptase) in an appropriate buffer and at a suitable temperature. The appropriate length of a primer depends on the intended use of the primer, but primers are typically at least 7 nucleotides long and, more typically range from 10 to 30 nucleotides, or even more typically from 15 to 30 nucleotides, in length. Other primers can be somewhat longer, *e.g.,* 30 to 50 nucleotides long.

The term "allele call" as used herein, refers to successful characterization of an allele by a given analysis method. If the analysis provides successful characterization of both alleles of a gene locus of a DNA sample, it is said that two allele calls are made. If one allele is characterized while the other allele is not characterized, it is said that one allele call is made. If neither of the two alleles is successfully characterized, no allele calls are made.

The term "allele" as used herein, is any one of a number of viable DNA codings occupying a given locus (position) on a chromosome. Usually alleles are DNA (deoxyribonucleic acid) sequences that code for a gene, but sometimes the term is used to refer to a non-gene sequence. An individual's genotype for that gene is the set of alleles it happens to possess. In a diploid organism, one that has two copies of each chromosome, two alleles make up the individual's genotype.

The term "reaction mixture" as used herein refers to a mixture containing sufficient components to carry out an amplification reaction.

The term "sequence tag density" herein refers to the number of sequence reads that are mapped to a reference genome sequence *e.g.* the sequence tag density for chromosome 21 is the number of sequence reads generated by the sequencing method that are mapped to chromosome 21 of the reference genome. The term "sequence tag density ratio" herein refers to the ratio of the number of sequence tags that are mapped to a chromosome of the reference genome *e.g.* chromosome 21, to the length of the reference genome chromosome 21.

The terms "threshold value" and "qualified threshold value" herein refer to any number that is calculated using a qualifying data set and serves as a limit of diagnosis of a copy number variation *e.g.* an aneuploidy, in an organism. If a threshold is exceeded, a subject can be diagnosed with a copy number variation *e.g.* trisomy 21.

The term "read" refers to a DNA sequence of sufficient length (*e.g.,* at least about 30 bp) that can be used to identify a larger sequence or region, *e.g.*that can be aligned and specifically assigned to a chromosome or genomic region or gene.

The term "sequence tag" is herein used interchangeably with the term "mapped sequence tag" to refer to a sequence read that has been specifically assigned *i.e.* mapped, to a larger sequence *e.g.* a reference genome, by alignment. Mapped sequence tags are uniquely mapped to a reference genome *i.e.* they are assigned to a single location to the reference genome. Tags that can be mapped to more than one location on a reference genome *i.e.* tags that do not map uniquely, are not included in the analysis.

The terms "aligned", "alignment", or "aligning" refer to one or more sequences that are identified as a match in terms of the order of their nucleic acid molecules to a known sequence from a reference genome. Such alignment can be done manually or by a computer algorithm, examples including the Efficient Local Alignment of Nucleotide Data (ELAND) computer program distributed as part of the Illumina Genomics Analysis pipeline. The matching of a sequence read in aligning can be a 100% sequence match or less than 100% (non-perfect match).

The term "reference genome" refers to any particular known genome sequence, whether partial or complete, of any organism or virus which may be used to reference identified sequences from a subject. For example, a reference genome used for human subjects as well as many other organisms is found at the National Center for Biotechnology Information at www.ncbi.nlm.nih.gov. A "genome" refers to the complete genetic information of an organism or virus, expressed in nucleic acid sequences.

The term "artificial target sequences genome" herein refers to a grouping of known sequences that encompass alleles of known polymorphic sites. For example, a "SNP reference genome" is an artificial target sequences genome comprising a grouping of sequences that encompass alleles of known SNPs.

The term "clinically-relevant sequence" herein refers to a nucleic acid sequence that is known or is suspected to be associated or implicated with a genetic or disease condition. Determining the absence or presence of a clinically-relevant sequence can be useful in determining a diagnosis or confirming a diagnosis of a medical condition, or providing a prognosis for the development of a disease.

The term "mixed sample" herein refers to a sample containing a mixture of nucleic acids, which are derived from different genomes.

The term "original maternal sample" herein refers to a biological sample obtained from a pregnant subject *e.g.* a woman, who serves as the source from which a portion is removed to amplify polymorphic target nucleic acids. The "original sample" can be any sample obtained from a pregnant subject, and the processed fractions thereof *e.g.* a purified cfDNA sample extracted from a maternal plasma sample. The term "original maternal sample" herein refers to a biological sample obtained from a pregnant subject *e.g.* a woman, who serves as the source from which a portion is removed to amplify polymorphic target nucleic acids. The "original sample" can be any sample obtained from a pregnant subject, and the processed fractions thereof *e.g.* a purified cfDNA sample extracted from a maternal plasma sample.

The term "biological fluid" herein refers to a liquid taken from a biological source and includes, for example, blood, serum, plasma, sputum, lavage fluid, cerebrospinal fluid, urine, semen, sweat, tears, saliva, and the like. As used herein, the terms "blood," "plasma" and "serum" expressly encompass fractions or processed portions thereof. Similarly, where a sample is taken from a biopsy, swab, smear, etc., the "sample" expressly encompasses a processed fraction or portion derived from the biopsy, swab, smear, etc.

The terms "maternal nucleic acids" and "fetal nucleic acids" herein refer to the nucleic acids of a pregnant female subject and the nucleic acids of the fetus being carried by the pregnant female, respectively.

The term "corresponding to" herein refers to a nucleic acid sequence *e.g.* a gene or a chromosome, that is present in the genome of different subjects, and which does not necessarily have the same sequence in all genomes, but serves to provide the identity rather than the genetic information of a sequence of interest *e.g.* a gene or chromosome.

The term "group of chromosomes" herein refers to two or more chromosomes.

The term "subject" herein refers to a human subject as well as a non-human subject such as a mammal.

## DESCRIPTION

The methods described herein enable the determination of the fraction of the minor fetal nucleic acid component in a sample comprising a mixture of fetal and maternal nucleic acids. In particular, the method enables the determination of the fraction of cfDNA contributed by a fetus to the mixture of fetal and maternal cfDNA in a maternal sample *e.g.* a plasma sample. The difference between the maternal and fetal fraction is determined by the relative contribution of a polymorphic allele derived from the fetal genome to the contribution of the corresponding polymorphic allele derived from the maternal genome. Polymorphic sequences can be used in conjunction with clinically-relevant diagnostic tests as a positive control for the presence of cfDNA in order to highlight false-negative or false-positive results stemming from low levels of cfDNA below the identification limit. The methods described are independent of the gender of the fetus, and are useful across a range of gestational ages.

**Figure 1** provides a flow diagram of an embodiment **100** for determining the fraction of fetal nucleic acids in a maternal biological sample by massively parallel sequencing of PCR-amplified polymorphic target nucleic acids. In step **110** a maternal sample comprising a mixture of fetal and maternal nucleic acids is obtained from a subject. The sample is a maternal sample that is obtained from a pregnant female, for example a pregnant woman. Other maternal samples can be from mammals, for example, cow, horse, dog, or cat. If the subject is a human, the sample can be taken in the first or second trimester of pregnancy. Any maternal biological sample can be used a source of fetal and maternal nucleic acids which are contained in cells or that are "cell-free". In some embodiments, it is advantageous to obtain a maternal sample that comprises cell-free nucleic acids *e.g.* cfDNA. In the claimed invention, the maternal biological sample is a plasma sample.. As used herein, the terms "blood," "plasma" and "serum" expressly encompass fractions or processed portions thereof.

In step **120,** the mixture of fetal and maternal nucleic acids is further processed from the sample fraction *e.g.* plasma, to obtain a sample comprising a purified mixture of fetal and maternal nucleic acids *e.g.* cfDNA. Cell-free nucleic acids, including cell-free DNA, can be obtained by various methods known in the art from biological samples including but not limited to plasma, serum and urine (Fan et al., Proc Natl Acad Sci 105:16266-16271 [2008]; Koide et al., Prenatal Diagnosis 25:604-607 [2005]; Chen et al., Nature Med. 2: 1033-1035 [1996]; Lo et al., Lancet 350: 485-487 [1997). To separate cfDNA from cells, fractionation, centrifugation (e.g., density gradient centrifugation), DNA-specific precipitation, or high-throughput cell sorting and/or separation methods can be used. Examples of methods for processing fluid samples have been previously disclosed, e.g., U.S. Patent Application Nos. 20050282293, 20050224351, and 20050065735. Commercially available kits for manual and automated separation of cfDNA are available (Roche Diagnostics, Indianapolis, IN, Qiagen,Valencia, CA, Macherey-Nagel, Duren, DE). In some instances, it can be advantageous to fragment the nucleic acid molecules in the nucleic acid sample. Fragmentation can be random, or it can be specific, as achieved, for example, using restriction endonuclease digestion. Methods for random fragmentation are well known in the art, and include, for example, limited DNAse digestion, alkali treatment and physical shearing. In the claimed invention, sample nucleic acids are obtained as cfDNA, which is not subjected to fragmentation. In other instances, the sample nucleic acids may be obtained as genomic DNA, which is subjected to fragmentation into fragments of approximately 500 or more base pairs, and to which NGS methods can be readily applied.

In step **130,** a portion of the purified mixture of fetal and maternal cfDNA is used for amplifying a plurality of polymorphic target nucleic acids each comprising a polymorphic site. In some embodiments, the target nucleic acids each comprise a SNP. In other embodiments, each of the target nucleic acids comprises a pair of tandem SNPs. In yet other instances, each the target nucleic acids comprises an STR. Polymorphic sites that are contained in the target nucleic acids include without limitation single nucleotide polymorphisms (SNPs), tandem SNPs, small-scale multi-base deletions or insertions, called IN-DELS (also called deletion insertion polymorphisms or DIPs), Multi-Nucleotide Polymorphisms (MNPs) Short Tandem Repeats (STRs), restriction fragment length polymorphism (RFLP), or a polymorphism comprising any other change of sequence in a chromosome. In some embodiments, the polymorphic sites are located on autosomal chromosomes, thereby enabling the determination of fetal fraction independently of sex of the fetus. Polymorphisms associated with chromosomes other than chromosome 13, 18, 21 and Y can also be used in the methods described herein.

Polymorphisms can be indicative, informative, or both. Indicative polymorphisms indicate the presence of fetal cell-free DNA in a maternal sample. For example, the more there is of a particular genetic sequence, *e.g*. a SNP, the more a method will translate its presence into a particular color intensity, density of color, or some other property which is detectable and measurable and indicative of the presence, absence, and quantity of a particular fragment of DNA and/or particular polymorphism *e.g*. SNP of the embryo. Informative polymorphisms yield information about the fetus - for example, the presence or absence of a disease, genetic abnormality, or any other biological information such as the stage of gestation or gender. With regard to the present invention, the methods are not conducted using all possible SNPs in a genome, but use those which are said to be "informative". "Informative SNPs" in this instance are those which identify differences in the sequence of the mother and the fetus. Any polymorphic site that can be encompassed by the reads generated by the sequencing methods described herein can be used to determine the fetal fraction.

In one embodiment, a portion of the mixture of fetal and maternal nucleic acids in the sample (*i.e.* the cfDNA), is used as template for amplifying target nucleic acids that comprise at least one SNP. In some embodiments, each target nucleic acid comprises a single *i.e.* one SNP. Target nucleic acid sequences comprising SNPs are available from publicly accessible databases including, but not limited to Human SNP Database at world wide web address wi.mit.edu, NCBI dbSNP Home Page at world wide web address ncbi.nlm.nih.gov, world wide web address lifesciences.perkinelmer.com, Applied Biosystems by Life Technologies™ (Carlsbad, CA) at world wide web address appliedbiosystems.com, Celera Human SNP database at world wide web address celera.com, the SNP Database of the Genome Analysis Group (GAN) at world wide web address gan.iarc.fr. In one embodiment, the SNPs chosen for enriching the fetal and maternal cfDNA are selected from the group of 92 individual identification SNPs (IISNPs) described by Pakstis *el al.* (Pakstis et el. Hum Genet 127:315-324 [2010]), which have been shown to have a very small variation in frequency across populations ($F_{st}$ <0.06), and to be highly informative around the world having an average heterozygosity ≥0.4. SNPs that are encompassed by the method of the invention include linked and unlinked SNPs. Other useful SNPs applicable or useful for the methods described herein are disclosed in U.S. Pat. Application Nos. 20080070792, 20090280492, 20080113358, 20080026390, 20080050739, 20080220422, and 20080138809. Each target nucleic acid comprises at least one polymorphic site *e.g.* a single SNP, that differs from that present on another target nucleic acid to generate a panel of polymorphic sites *e.g.* SNPs, that contain a sufficient number of polymorphic sites of which at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 40 or more are informative. For example, a panel of SNPs can be configured to comprise at least one informative SNP. In one embodiment, the SNPs that are targeted for amplification are selected from rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In one embodiment, the panel of SNPs comprises at least 3, at least 5, at least 10, at least 13, at least 15, at least 20, at least 25, at least 30 or more SNPs. In one embodiment, the panel of SNPs comprises rs560681, rs1109037, rs9866013, rs13182883, rs13218440, rs7041158, rs740598, rs10773760, rs4530059, rs7205345, rs8078417, rs576261, and rs2567608. The polymorphic nucleic acids comprising the SNPs can be amplified using exemplary primer pairs provided in Example 3, and disclosed as SEQ ID NOs:57-112.

In other embodiments, each target nucleic acid comprises two or more SNPs *i.e.* each target nucleic acid comprises tandem SNPs. Preferably, each target nucleic acid comprises two tandem SNPs. The tandem SNPs are analyzed as a single unit as short haplotypes, and are provided herein as sets of two SNPs. To identify suitable tandem SNP sequences, the International HapMap Consortium database can be searched (The International HapMap Project, Nature 426:789-796 [2003]). The database is available on the world wide web at hapmap.org. In one embodiment, tandem SNPs that are targeted for amplification are selected from the following sets of tandem pairs of SNPS rs7277033-rs2110153; rs2822654-rs1882882; rs368657-rs376635; rs2822731-rs2822732; rs1475881-rs7275487; rs1735976-rs2827016; rs447340-rs2824097; rs418989- rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102-rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672. The polymorphic nucleic acids comprising the tandem SNPs can be amplified using primer pairs that amplify polymorphic sequences comprising the tandem SNPs. Examples of primer pairs that can be used to amplify the tandem SNPs disclosed herein are SEQ ID NOs:197-310 as provided in Example 8.

In one instance of the disclosure, a portion of the mixture of fetal and maternal nucleic acids in the sample *e.g.* cfDNA, is used as template for amplifying target nucleic acids that comprise at least one STR. In some instances of the disclosure, each target nucleic acid comprises a single *i.e.* one STR. STR loci are found on almost every chromosome in the genome and may be amplified using a variety of polymerase chain reaction (PCR) primers. Tetranucleotide repeats have been preferred among forensic scientists due to their fidelity in PCR amplification, although some tri- and pentanucleotide repeats are also in use. A comprehensive listing of references, facts and sequence information on STRs, published PCR primers, common multiplex systems, and related population data are compiled in STRBase, which may be accessed via the World Wide Web at ibm4.carb.nist.gov:8800/dna/home.htm. Sequence information from GenBank® (http://www2.ncbi.nlm.nih.gov/cgi-bin/genbank) for commonly used STR loci is also accessible through STRBase. Commercial kits available for the analysis of STR loci generally provide all of the necessary reaction components and controls required for amplification. STR multiplex systems allow the simultaneous amplification of multiple nonoverlapping loci in a single reaction, substantially increasing throughput. With multicolor fluorescent detection, even overlapping loci can be multiplexed. The polymorphic nature of tandem repeated DNA sequences that are widespread throughout the human genome have made them important genetic markers for gene mapping studies, linkage analysis, and human identity testing. Because of the high polymorphism of STRs, most individuals will be heterozygous *i.e.* most people will possess two alleles (versions) of each-one inherited from each parent-with a different number of repeats. The PCR products comprising the STRs can be separated and detected using manual, semi-automated or automated methods. Semi-automated systems are gelbased and combine electrophoresis, detection and analysis into one unit. On a semiautomated system, gel assembly and sample loading are still manual processes; however, once samples are loaded onto the gel, electrophoresis, detection and analysis proceed automatically. Data collection occurs in "real time" as fluorescently labeled fragments migrate past the detector at a fixed point and can be viewed as they are collected. As the name implies, capillary electrophoresis is carried out in a microcapillary tube rather than between glass plates. Once samples, gel polymer and buffer are loaded onto the instrument, the capillary is filled with gel polymer and the sample is loaded automatically. Therefore, the non-maternally inherited fetal STR sequence will differ in the number of repeats from the maternal sequence. Amplification of these STR sequences can result in one or two major amplification products corresponding to the maternal alleles (and the maternally inherited fetal allele) and one minor product corresponding to the non-maternally inherited fetal allele. This technique was first reported in 2000 (Pertl et al., Human Genetics 106:45-49 [2002]) and has subsequently been developed using simultaneous identification of multiple different STR regions using real-time PCR (Liu et al., Acta Obset Gyn Scand 86:535-541 [2007]). Various sized PCR amplicons have been used to discern the respective size distributions of circulating fetal and maternal DNA species, and have shown that the fetal DNA molecules in the plasma of pregnant women are generally shorter than maternal DNA molecules (Chan et al., Clin Chem 50:8892 [2004]). Size fractionation of circulating fetal DNA has confirmed that the average length of circulating fetal DNA fragments is <300 bp, while maternal DNA has been estimated to be between about 0.5 and 1 Kb (Li et al., Clin Chem, 50: 1002-1011 [2004]). The disclosure provides a method for determining the fraction of fetal nucleic acid in a maternal sample comprising determining the amount of copies of at least one fetal and one maternal allele at a polymorphic miniSTR site, which can be amplified to generate amplicons that are of lengths about the size of the circulating fetal DNA fragments *e.g.* less than about 250 base pairs. In one instance of the disclosure, the fetal fraction can be determined by a method that comprises sequencing at least a portion of amplified polymorphic target nucleic acids each comprising a miniSTR. Fetal and maternal alleles at an informative STR site are discerned by their different lengths *i.e.* number of repeats, and the fetal fraction can be calculated as a percent ratio of the amount of fetal maternal alleles at that site. The method can use one or a combination of any number of informative miniSTRs to determine the fraction of fetal nucleic acid. For example, any one or a combination of any number of miniSTRs, for example the miniSTRs disclosed in Table 7 and Figures 4 and 5, can be used. In one instance of the disclosure, the fraction of fetal nucleic acid in a maternal sample is performed using a method that includes determining the number of copies of the maternal and fetal nucleic acid present in the maternal sample by amplifying at least one autosomal miniSTR chosen from CSF1PO, FGA, TH01, TPOX, vWA, D3S1358,D5S818, D7S820, D8S1179, D13S317, D16S539, D18S51, D21S11, Penta D, Penta E, D2S1338, D1S1677, D2S441, D4S2364, D10S1248, D14S1434, D22S1045, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D5S2500, D4S2408, D4S2364, D3S4529, D3S3053, D2S1776, D2S441, D1S1677, D1S1627, and D1GATA113. In another instance of the disclosure, the at least one autosomal miniSTR is the group of miniSTRs CSF1PO, FGA, D13S317, D16S539, D18S51, D2S1338, D21S11, D2S1338 and D7S820. In one instance of the disclosure, the method comprises determining the number of copies of at least one fetal and at least one maternal allele at least at one polymorphic miniSTR that is amplified to generate amplicons that are less than about 300 bp, less than about 250 bp, less than about 200 bp, less than about 150 bp, less than about 100 bp, or less than about 50 bp. In another instance of the disclosure, the amplicons that are generated by amplifying the miniSTRs are less than about 300 bp. In another instance of the disclosure, the amplicons that are generated by amplifying the miniSTRs are less than about 250 bp. In another instance of the disclosure, the amplicons that are generated by amplifying the miniSTRs are less than about 200 bp. Amplification of the informative allele includes using miniSTR primers, which allow for the amplification of reduced-size amplicons to detect STR alleles that are less than about 500 bp, less than about 450 bp, less than about 400 bp, less than about 350 bp, less than about 300 base pairs (bp), less than about 250 bp, less than about 200 bp, less than about 150 bp, less than about 100 bp, or less than about 50 bp. The reduced-size amplicons generated using the miniSTR primers are known as miniSTRs that are identified according to the marker name corresponding to the locus to which they have been mapped. In one instance of the disclosure, the miniSTR primers include mini STR primers that have permitted the maximum size reduction in amplicon size for all 13 CODIS STR loci in addition to the D2S1338, Penta D, and pentaE found in commercially available STR kits (Butler et al., J Forensic Sci 48:1054-1064 [2003]), miniSTR loci that are unlinked to the CODIS markers as described by Coble and Butler (Coble and Butler, J Forensic Sci 50:43-53 [2005]), and other minSTRs that have been characterized at NIST. Information regarding the miniSTRs characterized at NIST is accessible via the world wide web at cstl.nist.gov/biotech/strbase/newSTRs.htm. Any one pair or a combination of two or more pairs of miniSTR primers can be used to amplify at least one miniSTR.

In one instance of the disclosure, exemplary primer sets that can be used to amplify STRs in maternal cfDNA samples include the primer sets provided in Example 9 and disclosed as SEQ ID NOs:113-196.

Gender identification (sex-typing) in commonly performed in conjunction with STR typing using PCR products generated from the Amelogenin gene that occurs on both the X- and Y-chromosome. Amelogenin is not an STR locus, but it produces X and Y chromosome specific PCR products. A commonly used PCR primer set first published by Sullivan et al. (1993) (Sullivan et al., BioTechniques 15:637-641 [1993]) targets a 6 bp deletion that occurs on the X-chromosome, which enables amplicons generated from the X- and Y-chromosome to be distinguished from one another when electrophoretic separation is performed to separate STR alleles. Most commercial STR kits utilize the Sullivan et al. (1993) primers or minor modifications. Since females are X,X, only a single peak is observed when testing female DNA whereas males, which possess both X and Y chromosomes, exhibit two peaks with a standard Amelogenin test. In one instance of the disclosure, the method to determine the fraction of fetal nucleic acid in a maternal sample comprises coamplifying Ameleogenin with at least one miniSTR. In another instance of the disclosure, the method does not comprise coamplifying Amelogenin with miniSTR loci.

Amplification of the target nucleic acids in the mixture of fetal and maternal nucleic acid e.g. cfDNA, is accomplished any method that uses PCR or variations of the method including but not limited to digital PCR, real time PCR (RT-PCR), TaqMan PCR System (Applied Biosystems), SNPlex or GenPlex methods, asymmetric PCR, helicase-dependent amplification, hot-start PCR, qPCR, solid phase PCR, and touchdown PCR. Alternatively, replication of target nucleic acid sequences can be obtained by enzyme-independent methods *e.g.* chemical solid-phase synthesis using the phosphoramidites. Amplification of the target sequences is accomplished using primer pairs each capable of amplifying a target nucleic acid sequence comprising the polymorphic site e.g. SNP, in a multiplex PCR reaction. Multiplex PCR reactions include combining at least 2, at least three, at least 3, at least 5, at least 10, at least 15, at least 20, at least 25, at least 30, at least 40 or more sets of primers in the same reaction to quantify the amplified target nucleic acids comprising at least two, at least three, at least 5, at least 10, at least 15, at least 20, at least 25, at least 30, at least 40 or more polymorphic sites in the same sequencing reaction. Any panel of primer sets can be configured to amplify at least one informative polymorphic sequence.

In step **140** of method **100 (Figure 1),** a portion or all of the amplified polymorphic sequences are used to prepare a sequencing library for sequencing in a parallel fashion as described. In one embodiment, the library is prepared for sequencing-by-synthesis using Illumina's reversible terminator-based sequencing chemistry.

In step **140,** sequence information that is needed for determining fetal fraction is obtained using any of the known DNA sequencing methods. In one embodiment, the method described herein employs next generation sequencing technology (NGS) in which clonally amplified DNA templates or single DNA molecules are sequenced in a massively parallel fashion within a flow cell (*e.g.* as described in Volkerding et al. Clin Chem 55:641-658 [2009]; Metzker M Nature Rev 11:31-46 [2010]). In addition to high-throughput sequence information, NGS provides digital quantitative information, in that each sequence read is a countable "sequence tag" representing an individual clonal DNA template or a single DNA molecule. This quantification allows NGS to expand the digital PCR concept of counting cell-free DNA molecules (Fan et al., Proc Natl Acad Sci U S A 105:16266-16271 [2008]; Chiu et al., Proc Natl Acad Sci U S A 2008;105:20458-20463 [2008]). The sequencing technologies of NGS include pyrosequencing, sequencing-by-synthesis with reversible dye terminators, sequencing by oligonucleotide probe ligation and real time sequencing.

Some of the sequencing technologies are available commercially, such as the sequencing-by-hybridization platform from Affymetrix Inc. (Sunnyvale, CA) and the sequencing-by-synthesis platforms from 454 Life Sciences (Bradford, CT), Illumina/Solexa (Hayward, CA) and Helicos Biosciences (Cambridge, MA), and the sequencing-by-ligation platform from Applied Biosystems (Foster City, CA), as described below. In addition to the single molecule sequencing performed using sequencing-by-synthesis of Helicos Biosciences, other single molecule sequencing technologies are encompassed by the method of the invention and include the SMRT™ technology of Pacific Biosciences, the Ion Torrent™ technology, and nanopore sequencing being developed for example, by Oxford Nanopore Technologies.

While the automated Sanger method is considered as a 'first generation' technology, Sanger sequencing including the automated Sanger sequencing, can also be employed by the method of the invention. Additional sequencing methods that comprise the use of developing nucleic acid imaging technologies *e.g.* atomic force microscopy (AFM) or transmission electron microspcopy (TEM), are also encompassed by the method of the invention. Exemplary sequencing technologies are described below.

In one embodiment, the DNA sequencing technology that is used in the method of the invention is the Helicos True Single Molecule Sequencing (tSMS) (*e.g.* as described in Harris T.D. et al., Science 320:106-109 [2008]). In the tSMS technique, a DNA sample is cleaved into strands of approximately 100 to 200 nucleotides, and a poly A sequence is added to the 3' end of each DNA strand. Each strand is labeled by the addition of a fluorescently labeled adenosine nucleotide. The DNA strands are then hybridized to a flow cell, which contains millions of oligo-T capture sites that are immobilized to the flow cell surface. The templates can be at a density of about 100 million templates/$cm^2$. The flow cell is then loaded into an instrument, e.g., HeliScope™ sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are discerned by imaging the flow cell surface. After imaging, a cleavage step removes the fluorescent label, and the process is repeated with other fluorescently labeled nucleotides until the desired read length is achieved. Sequence information is collected with each nucleotide addition step.

In one embodiment, the DNA sequencing technology that is used in the method of the invention is the 454 sequencing (Roche) (*e.g.* as described in Margulies, M. et al. Nature 437:376-380 [2005]). 454 sequencing involves two steps. In the first step, DNA is sheared into fragments of approximately 300-800 base pairs, and the fragments are blunt-ended. Oligonucleotide adaptors are then ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is discerned and analyzed.

In one embodiment, the DNA sequencing technology that is used in the method of the invention is the SOLiD technology (Applied Biosystems). In SOLiD sequencing-by-ligation, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

In one embodiment, the DNA sequencing technology that is used in the method of the invention is the single molecule, real-time (SMRT™) sequencing technology of Pacific Biosciences. In SMRT sequencing, the continuous incorporation of dye-labeled nucleotides is imaged during DNA synthesis. Single DNA polymerase molecules are attached to the bottom surface of individual zero-mode wavelength identifiers (ZMW identifiers) that obtain sequence information while phospolinked nucleotides are being incorporated into the growing primer strand. A ZMW is a confinement structure which enables observation of incorporation of a single nucleotide by DNA polymerase against the background of fluorescent nucleotides that rapidly diffuse in an out of the ZMW (in microseconds). It takes several milliseconds to incorporate a nucleotide into a growing strand. During this time, the fluorescent label is excited and produces a fluorescent signal, and the fluorescent tag is cleaved off. Identification of the corresponding fluorescence of the dye indicates which base was incorporated. The process is repeated.

In one embodiment, the DNA sequencing technology that is used in the method of the invention is nanopore sequencing (e.g. as described in Soni GV and Meller A. Clin Chem 53: 1996-2001 [2007]). Nanopore sequencing DNA analysis techniques are being industrially developed by a number of companies, including Oxford Nanopore Technologies (Oxford, United Kingdom). Nanopore sequencing is a single-molecule sequencing technology whereby a single molecule of DNA is sequenced directly as it passes through a nanopore. A nanopore is a small hole, of the order of 1 nanometer in diameter. Immersion of a nanopore in a conducting fluid and application of a potential (voltage) across it results in a slight electrical current due to conduction of ions through the nanopore. The amount of current which flows is sensitive to the size and shape of the nanopore. As a DNA molecule passes through a nanopore, each nucleotide on the DNA molecule obstructs the nanopore to a different degree, changing the magnitude of the current through the nanopore in different degrees. Thus, this change in the current as the DNA molecule passes through the nanopore represents a reading of the DNA sequence.

In one embodiment, the DNA sequencing technology that is used in the method of the invention is the chemical-sensitive field effect transistor (chemFET) array (e.g., as described in U.S. Patent Application Publication No. 20090026082). In one example of the technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be discerned by a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

In one embodiment, the DNA sequencing technology that is used in the method of the invention is the Halcyon Molecular's method that uses transmission electron microscopy (TEM). The method, termed Individual Molecule Placement Rapid Nano Transfer (IMPRNT), comprises utilizing single atom resolution transmission electron microscope imaging of high-molecular weight (150kb or greater) DNA selectively labeled with heavy atom markers and arranging these molecules on ultra-thin films in ultra-dense (3nm strand-to-strand) parallel arrays with consistent base-to-base spacing. The electron microscope is used to image the molecules on the films to determine the position of the heavy atom markers and to extract base sequence information from the DNA. The method is further described in PCT patent publication WO 2009/046445. The method allows for sequencing complete human genomes in less than ten minutes.

In one embodiment, the DNA sequencing technology is the Ion Torrent single molecule sequencing, which pairs semiconductor technology with a simple sequencing chemistry to directly translate chemically encoded information (A, C, G, T) into digital information (0, 1) on a semiconductor chip. In nature, when a nucleotide is incorporated into a strand of DNA by a polymerase, a hydrogen ion is released as a byproduct. Ion Torrent uses a high-density array of micro-machined wells to perform this biochemical process in a massively parallel way. Each well holds a different DNA molecule. Beneath the wells is an ion-sensitive layer and beneath that an ion sensor. When a nucleotide, for example a C, is added to a DNA template and is then incorporated into a strand of DNA, a hydrogen ion will be released. The charge from that ion will change the pH of the solution, which can be identified by Ion Torrent's ion sensor. The sequencer-essentially the world's smallest solid-state pH meter-calls the base, going directly from chemical information to digital information. The Ion personal Genome Machine (PGM™) sequencer then sequentially floods the chip with one nucleotide after another. If the next nucleotide that floods the chip is not a match. No voltage change will be recorded and no base will be called. If there are two identical bases on the DNA strand, the voltage will be double, and the chip will record two identical bases called. Direct identification allows recordation of nucleotide incorporation in seconds.

Other sequencing methods include digital PCR and sequencing by hybridization. Digital polymerase chain reaction (digital PCR or dPCR) can be used to directly identify and quantify nucleic acids in a sample. Digital PCR can be performed in an emulsion. Individual nucleic acids are separated, e.g., in a microfluidic chamber device, and each nucleic can is individually amplified by PCR. Nucleic acids can be separated such there is an average of approximately 0.5 nucleic acids/well, or not more than one nucleic acid/well. Different probes can be used to distinguish fetal alleles and maternal alleles. Alleles can be enumerated to determine copy number. In sequencing by hybridization, the hybridization comprises contacting the plurality of polynucleotide sequences with a plurality of polynucleotide probes, wherein each of the plurality of polynucleotide probes can be optionally tethered to a substrate. The substrate might be flat surface comprising an array of known nucleotide sequences. The pattern of hybridization to the array can be used to determine the polynucleotide sequences present in the sample. In other embodiments, each probe is tethered to a bead, e.g., a magnetic bead or the like. Hybridization to the beads can be identified and used to identify the plurality of polynucleotide sequences within the sample.

In one embodiment, the method employs massively parallel sequencing of millions of DNA fragments using Illumina's sequencing-by-synthesis and reversible terminator-based sequencing chemistry (*e.g.* as described in Bentley et al., Nature 6:53-59 [2009]). Template DNA can be genomic DNA *e.g.* cfDNA. In some embodiments, genomic DNA from isolated cells is used as the template, and it is fragmented into lengths of several hundred base pairs. In other embodiments, cfDNA is used as the template, and fragmentation is not required as cfDNA exists as short fragments. For example fetal cfDNA circulates in the bloodstream as fragments of <300 bp, and maternal cfDNA has been estimated to circulate as fragments of between about 0.5 and 1 Kb (Li et al., Clin Chem, 50: 1002-1011 [2004]). Illumina's sequencing technology relies on the attachment of fragmented genomic DNA to a planar, optically transparent surface on which oligonucleotide anchors are bound. Template DNA is end-repaired to generate 5'-phosphorylated blunt ends, and the polymerase activity of Klenow fragment is used to add a single A base to the 3' end of the blunt phosphorylated DNA fragments. This addition prepares the DNA fragments for ligation to oligonucleotide adapters, which have an overhang of a single T base at their 3' end to increase ligation efficiency. The adapter oligonucleotides are complementary to the flow-cell anchors. Under limiting-dilution conditions, adapter-modified, single-stranded template DNA is added to the flow cell and immobilized by hybridization to the anchors. Attached DNA fragments are extended and bridge amplified to create an ultra-high density sequencing flow cell with hundreds of millions of clusters, each containing ~1,000 copies of the same template. The cluster amplified DNA molecules are sequenced using a robust four-color DNA sequencing-by-synthesis technology that employs reversible terminators with removable fluorescent dyes. High-sensitivity fluorescence identification is achieved using laser excitation and total internal reflection optics. Short sequence reads of about 20-40 bp *e.g.* 36 bp, are aligned against a repeat-masked reference genome and genetic differences are called using specially developed data analysis pipeline software. After completion of the first read, the templates can be regenerated in situ to enable a second read from the opposite end of the fragments. Thus, either single-end or paired end sequencing of the DNA fragments is used according to the method. Partial sequencing of DNA fragments present in the sample is performed, and sequence tags comprising reads of predetermined length *e.g.* 36 bp, that are mapped to a known reference genome are counted.

The length of the sequence read is associated with the particular sequencing technology. NGS methods provide sequence reads that vary in size from tens to hundreds of base pairs. In some embodiments of the method described herein, the sequence reads are about 20bp, about 25bp, about 30bp, about 35bp, about 40bp, about 45bp, about 50bp, about 55bp, about 60bp, about 65bp, about 70bp, about 75bp, about 80bp, about 85bp, about 90bp, about 95bp, about 100bp, about 110bp, about 120bp, about 130, about 140bp, about 150bp, about 200bp, about 250bp, about 300bp, about 350bp, about 400bp, about 450bp, about 500bp, about 550bp or about 600bp. It is expected that technological advances will enable single-end reads of greater than 500bp enabling for reads of greater than about 1000bp when paired end reads are generated. In one embodiment, the sequence reads are 36bp. Other sequencing methods that can be employed by the method of the invention include the single molecule sequencing methods that can sequence nucleic acids molecules >5000 bp. The massive quantity of sequence output is transferred by an analysis pipeline that transforms primary imaging output from the sequencer into strings of bases. A package of integrated algorithms performs the core primary data transformation steps: image analysis, intensity scoring, base calling, and alignment.

In one embodiment, partial sequencing of amplified target polymorphic nucleic acids is performed, and sequence tags comprising reads of predetermined length *e.g.* 36 bp, that map to a known reference genome are counted. Only sequence reads that uniquely align to a reference genome are counted as sequence tags. In one embodiment, the reference genome is an artificial target sequences genome that comprises the sequences of the polymorphic target nucleic acids *e.g.* SNPs. In one embodiment, the reference genome is an artificial SNP reference genome. In another r embodiment, the reference genome is an artificial STR reference genome. In yet another embodiment, the reference genome is an artificial tandem-STR reference genome. Artificial reference genomes can be compiled using the sequences of the target polymorphic nucleic acids. Artificial reference genomes can comprise polymorphic target sequence each comprising one or more different types of polymorphic sequences. For example, an artificial reference genome can comprise polymorphic sequences comprising SNP alleles and/or STRs. In one embodiment, the reference genome is the human reference genome NCBI36/hg18 sequence, which is available on the world wide web at genome.ucsc.edu/cgi-bin/hgGateway?org=Human&db=hg18&hgsid=166260105). Other sources of public sequence information include GenBank, dbEST, dbSTS, EMBL (the European Molecular Biology Laboratory), and the DDBJ (the DNA Databank of Japan). In another embodiment, the reference genome comprises the human reference genome NCBI36/hg18 sequence and an artificial target sequences genome, which includes the target polymorphic sequences *e.g*. a SNP genome. Mapping of the sequence tags is achieved by comparing the sequence of the mapped tag with the sequence of the reference genome to determine the chromosomal origin of the sequenced nucleic acid (*e.g.* cfDNA) molecule, and specific genetic sequence information is not needed. A number of computer algorithms are available for aligning sequences, including without limitation BLAST (Altschul et al., 1990), BLITZ (MPsrch) (Sturrock & Collins, 1993), FASTA (Person & Lipman, 1988), BOWTIE (Langmead et al., Genome Biology 10:R25.1-R25.10 [2009]), or ELAND (Illumina, Inc., San Diego, CA, USA). In one embodiment, one end of the clonally expanded copies of the plasma cfDNA molecules is sequenced and processed by bioinformatic alignment analysis for the Illumina Genome Analyzer, which uses the Efficient Large-Scale Alignment of Nucleotide Databases (ELAND) software. In embodiments of the method that comprise determining the presence or absence of an aneuploidy and fetal fraction using NGS sequencing methods, analysis of sequencing information for the determination of aneuploidy may allow for a small degree of mismatch (0-2 mismatches per sequence tag) to account for minor polymorphisms that may exist between the reference genome and the genomes in the mixed sample. Analysis of sequencing information for the determination of fetal fraction may allow for a small degree of mismatch depending on the polymorphic sequence. For example, a small degree of mismatch may be allowed if the polymorphic sequence is an STR. In cases when the polymorphic sequence is a SNP, all sequence that match exactly to either of the two alleles at the SNP site are counted first and filtered from the remaining reads, for which a small degree of mismatch may be allowed. Quantification of the number of sequence reads aligning to each chromosome for determining chromosomal aneuploidies can be determined as described herein, or using alternative analyses that employ normalizing the median number of sequence tags for a chromosome of interest to the median number of tags for each of the other autosomes (Fan et al., Proc Natl Acad Sci 105:16266-16271 [2008]), or that compare the number of unique reads aligning to each chromosome to the total number of reads aligning to all chromosomes to derive a percent genomic representation for each chromosome. A "z score" is generated to represent the difference between the percent genomic representation of the chromosome of interest and the mean percent representation for the same chromosome between a euploid control group, divided by the standard deviation (Chiu et al., Clin Chem 56:459-463 [2010]). In another embodiment, the sequencing information can be determined as described in U. S. Provisional Patent Application titled "Normalizing Biological Assays," docket no. 32047-768.101, filed January 19, 2010.

Analysis of sequencing information for the determination of fetal fraction may allow for a small degree of mismatch depending on the polymorphic sequence. For example, a small degree of mismatch may be allowed if the polymorphic sequence is an STR. In cases when the polymorphic sequence is a SNP, all sequences that match exactly to either of the two alleles at the SNP site are counted first and filtered from the remaining reads, for which a small degree of mismatch may be allowed. The present method for determining fetal fraction by sequencing of nucleic acids can be used in combination with other methods.

In step **160,** fetal fraction is determined based on the total number of tags that map to the first allele and the total number of tags that map to second allele at an informative polymorphic site *e.g.* a SNP, contained in a reference genome. For example, the reference genome is an artificial target sequence genome that encompasses the polymorphic sequences that comprise SNPs rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005, and rs530022. In one embodiment, the artificial reference genome includes the polymorphic target sequences of SEQ ID NOs: 1-56 (see Example 3).

In another embodiment, the artificial genome is an artificial target sequence genome that encompasses polymorphic sequences that comprise tandem SNPs rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392- rs4143391; rs1691324- rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031; rs418359-rs2836926; rs7278447-rs7278858; rs385787-rs367001; rs367001-rs386095; rs2837296-rs2837297; and rs2837381-rs4816672.

In another instance of the disclosure, the artificial target genome encompasses polymorphic sequences that comprise STRs selected from CSF1PO, FGA, TH01, TPOX, vWA, D3S1358,D5S818, D7S820, D8S1179, D13S317, D16S539, D18S51, D21S11, PentaD, PentaE, D2S1338, D1S1677, D2S441, D4S2364, D10S1248, D14S1434, D22S1045, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D5S2500, D4S2408, D4S2364, D3S4529, D3S3053, D2S1776, D2S441, D1S1677, D1S1627, and DIGATA113. The composition of the artificial target sequences genome will vary depending on the polymorphic sequences that are used for determining the fetal fraction. Accordingly, an artificial target sequences genome is not limited to the SNP, tandem SNP or STR sequences exemplified herein.

The informative polymorphic site *e.g.* SNP, is identified by the difference in the allelic sequences and the amount of each of the possible alleles. Fetal cfDNA is present at a concentration that is <10% of the maternal cfDNA. Thus, the presence of a minor contribution of an allele to the mixture of fetal and maternal nucleic acids relative to the major contribution of the maternal allele can be assigned to the fetus. Alleles that are derived from the maternal genome are herein referred to as major alleles, and alleles that are derived from the fetal genome are herein referred to as minor alleles. Alleles that are represented by similar levels of mapped sequence tags represent maternal alleles. The results of an exemplary multiplex amplification of target nucleic acids comprising SNPs and derived from a maternal plasma sample is shown in **Figure 2.** Informative SNPs are discerned from the single nucleotide change at a polymorphic site, and fetal alleles are discerned by their relative minor contribution to the mixture of fetal and maternal nucleic acids in the sample when compared to the major contribution to the mixture by the maternal nucleic acids. Accordingly, the relative abundance of fetal cfDNA in the maternal sample is determined as a parameter of the total number of unique sequence tags mapped to the target nucleic acid sequence on a reference genome for each of the two alleles of the predetermined polymorphic site. In one embodiment, the fraction of fetal nucleic acids in the mixture of fetal and maternal nucleic acids is calculated for each of the informative allele ($allele_x$) as follows:

$$\%\ \text{fetal fraction allele}_x = ((\textstyle\sum \text{Fetal sequence tags for allele}_x) / (\sum \text{Maternal sequence tags for allele}_x)) \times 100$$

and fetal fraction for the sample is calculated as the average of the fetal fraction of all of the informative alleles. Optionally, the fraction of fetal nucleic acids in the mixture of fetal and maternal nucleic acids is calculated for each of the informative allele ($allele_x$) as follows:

$$\%\ \text{fetal fraction allele}_x = ((2\ \text{X} \textstyle\sum \text{Fetal sequence tags for allele}_x) / (\sum \text{Maternal sequence tags for allele}_x)) \times 100,$$

to compensate for the presence of 2 fetal alleles, one being masked by the maternal background.

The percent fetal fraction is calculated for at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 40 or more informative alleles. In one embodiment, the fetal fraction is the average fetal fraction determined for at least 3 informative alleles.

**Figure 3,** shows a flowchart of alternate methods whereby fetal fraction can be determined from amplified target nucleic acids that have been combined with unamplified fetal and maternal cfDNA sample to allow for the simultaneous determination of fetal fraction and the presence or absence of fetal aneuploidy by enriching the maternal sample comprising fetal and maternal nucleic acids for polymorphic target nucleic acids. In one embodiment, the sample that is enriched is the plasma fraction of a blood sample (a). For example, a portion of an original maternal plasma sample is used for amplifying target nucleic acid sequences. Subsequently, some or all of the amplified product is combined with the remaining unamplified original plasma sample thereby enriching it (see Example 7). In another embodiment, the sample that is enriched is the sample of purified cfDNA that is extracted from plasma (b). For example, enrichment comprises amplifying the target nucleic acids that are contained in a portion of an original sample of purified mixture of fetal and maternal nucleic acids *e.g.* cfDNA that has been purified from a maternal plasma sample, and subsequently combining some or all of the amplified product with the remaining unamplified original purified sample (see Example 6). In yet another embodiment, the sample that is enriched is a sequencing library sample prepared from a purified mixture of fetal and maternal nucleic acids (c). For example, enrichment comprises amplifying the target nucleic acids that are contained in a portion of an original sample of purified mixture of fetal and maternal nucleic acids *e.g.* cfDNA that has been purified from a maternal plasma sample, preparing a first sequencing library of unamplified nucleic acid sequences, preparing a second sequencing library of amplified polymorphic target nucleic acids, and subsequently combining some or all of the second sequencing library with some or all of the first sequencing library (see Example 5). The amount of amplified product that is used to enrich the original sample is selected to obtain sufficient sequencing information for determining both the presence or absence of aneuploidy and the fetal fraction from the same sequencing run. At least about 3%, at least about 5%, at least about 7%, at least about 10%, at least about 15%, at least about 20%, at least about 25%, at least about 30% or more of the total number of sequence tags obtained from sequencing are mapped to determine the fetal fraction. Sequencing of the library generated following any one of the methods depicted in **Figure 3,** provides sequence tags derived from the amplified target nucleic acids and tags derived from the original unamplified maternal sample. Fetal fraction is calculated from the number of tags mapped to an artificial reference genome, and the presence or absence of aneuploidy is determined from the number of tags that map to the subject genome *e.g.* human genome.

An alternative method for determining fetal fraction from amplified polymorphic target nucleic acids at step **130** of **Figure 1,** uses size separation of amplified polymorphic target nucleic acids comprising STRs (step **150** of **Figure 1).** As described above, the polymorphic character of a STR locus is due to variation in the number of tandemly repeated units between alleles. Because of the high polymorphism of the STRs most individuals will be heterozygous for STRs. Amplification of an STR will result in one or two PCR products in most samples. In samples obtained from pregnant women *e.g.* plasma samples, amplification of an STR will result in one or two major PCR products, which correspond to the one or two maternal alleles including one fetal maternally-inherited allele, and a third paternally-inherited fetal allele that is detected at an informative STR.

The STRs that are targeted for amplification are miniSTRs as described herein that are less than about 300 base pairs and that are amplified in a multiplex PCR reaction, which allows the simultaneous amplification of multiple loci in a single reaction. The primers are labeled with different fluorescent dyes each emitting fluorescence at a different wavelength *e.g.* 6FAM™, VIC™, NED™, and PET™ , and the number of repeat units for each fluorescently tagged STR in the resulting PCR products is detected following their separation and accurate sizing that is achieved by slab or capillary electrophoresis. In one instance, capillary electrophoresis is used, and it can be performed in microfabricated channels or capillary arrays. Alternatively, methods utilizing mass spectrometry and microarray technology are used. Multiplex STR analysis can be performed to determine fetal fraction using commercially-available kits *e.g.* AmpFℓSTR® Identifiler® PCR Amplification Kit (**Figure 4**) and AmpFℓSTR® MiniFiler® PCR Amplification Kit (**Figure 5**) (Applied Biosystems, Foster City, Calif.). The AmpFℓSTR® MiniFiler® PCR Amplification Kit was designed to amplify as miniSTRs eight of the largest sized loci in the AmpFℓSTR® Identifiler® PCR Amplification Kit. Together with the gender-identification locus Amelogenin, the nine-locus multiplex enables simultaneous amplification of loci of cfDNA samples (see Example 10).

In one instance of the disclosure, multiplex STR analysis for determining fetal fraction is performed by amplifying polymorphic target nucleic acids present in a maternal plasma sample that each comprise a miniSTR selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. In another instance of the disclosure, multiplex STR analysis for determining fetal fraction is performed by amplifying polymorphic target nucleic acids present in a maternal plasma sample for the panel of miniSTRs: CSF1PO, D13S317, D16S539, D18S51, D21S11, D2S1338, D7S820 and FGA. The miniSTRs can be located on the same or on different chromosomes. The method is a fetal gender-independent method. Therefore, in some instances of the disclosure, the miniSTRs are located on chromosomes other than the Y chromosome. In other instances of the disclosure, the miniSTRs are located on chromosomes other than chromosomes 13, 18, 21 or X *i.e.* chromosomes that might be involved in an aneuploidy.

Samples of maternal plasma often contain less than 100 pg of cfDNA. The low copy number DNA samples can fall below the sensitivity limitations of STR analysis methods. The intractable samples can be made amenable by increasing the number of starting cfDNA available for subsequent STR analysis by a whole genome amplification strategy. In one embodiment, the mixture of fetal and maternal nucleic acids can be preamplified before alleles are detected or quantified. For example, template cell-free DNA can be amplified by PCR. The nucleic acid can be amplified for about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 cycles. Nucleic acid can be amplified for about 1-10 cycles, about 1-20 cycles, about 1-30 cycles, about 1-40 cycles, about 5-15 cycles, about 5-20 cycles, about 5-30 cycles, about 5-40 cycles, about 10-15 cycles, about 10-20 cycles, about 10-30 cycles, about 10-40 cycles, about 20-30 cycles, about 20-40 cycles, or about 30-40 cycles. The amount of template nucleic acid that can be amplified can about 10-1000 pg, 25-1000 pg, 50-1000 pg, 100-1000, pg, 200-1000 pg, 300-1000 pg, 400-1000 pg, 500-1000 pg, 600-1000, pg, 700-1000 pg, or 800-1000 pg. Following preamplification, the nucleic acids can be diluted before alleles are detected or quantified. Preamplification can be used to increase the detection sensitivity of alleles in a sample, for example, a maternal sample (Example 11). In another embodiment, genotyping a polymorphism need not require a pre-amplification step. Any PCR-based amplification method can be used to preamplify the cfDNA. Amplification methods include but are not limited to whole genome amplification strategies including methods such as primer extension preamplification, degenerate oligonucleotide-primed PCR (DOP-PCR), low fragments from low quantities of DOP-PCR, improved primer extension preamplification PCR (IPEP PCR), and modified improved primer extension preamplification (mIPEP). Thus, in one embodiment, the method that is used for determining the fetal fraction in a maternal sample *e.g.* plasma sample, comprises preamplifying the mixture of fetal and maternal nucleic acids present in the plasma cfDNA sample using a whole genome amplification method, amplifying a plurality of polymorphic nucleic acids in said mixture of fetal and maternal nucleic acids, wherein each of said at least one polymorphic nucleic acid comprises an STR; determining the amount of fetal and maternal STR alleles at least one polymorphic nucleic acid; and calculating the fetal fraction from the amount of fetal and maternal STR alleles. Following preamplification, multiplex STR analysis for determining fetal fraction is performed by amplifying polymorphic target nucleic acids present in a maternal plasma sample that each comprise a miniSTR selected from CSF1PO, FGA, TH01, vWA, D3S1358, D5S818, D7S820, D8S1179, D13S317, D16S539, D18S51, D2S1338, Penta D, Penta E, D22S1045, D20S1082, D20S482, D18S853, D17S1301, D17S974, D14S1434, D12ATA63, D11S4463, D10S1435, D10S1248, D9S2157, D9S1122, D8S1115, D6S1017, D6S474, D5S2500, D4S2408, D4S2364, D3S4529, D3S3053, D2S1776, D2S441, D1S1677, D1S1627, and D1GATA113. Alternatively, multiplex STR analysis for determining fetal fraction is performed by amplifying polymorphic target nucleic acids for the panel of miniSTRs: CSF1PO, D13S317, D16S539, D18S51, D21S11, D2S1338, D7S820 andFGA.

**Applications**

Methods described herein are applicable to diagnosis or prognosis of various disease conditions including, but not limited to, cancer, genetic disorders and infection. The fetal fraction of nucleic acid in a maternal sample can be used for determining a chromosomal abnormality. Examples of chromosomal abnormalities include, for example, aneuploidy, monosomy, trisomy, duplication, inversion, deletion, polyploidy, deletion of a part of a chromosome, addition, addition of a part of chromosome, insertion, a fragment of a chromosome, a region of a chromosome, chromosomal rearrangement, and translocation. For example, aneuploidy can refer to the occurrence of one or more extra or missing chromosomes in a sample.

Examples of fetal conditions that can be determined using the methods of the provided invention include, for example, Angleman syndrome (15q11.2-q13), cri-du-chat syndrome (5p-), DiGeorge syndrome and Velo-cardiofacial syndrome (22q11.2), Miller-Dieker syndrome (17 p13.3), Prader-Willi syndrome (15q11.2-q13), retinoblastoma (13q14), Smith-Magenis syndrome (17 p11.2), trisomy 13, trisomy 16, trisomy 18, trisomy 21 (Down's syndrome), triploidy, Williams syndrome (7q 11.23), and Wolf-Hirschhom syndrome (4p-). Examples of sex chromosome abnormalities that can be detected by methods described herein include, but are not limited to, Kallman syndrome (Xp22.3), steroid sulfate deficiency (STS) (Xp22.3), X-linked ichthyosis (Xp22.3), Klinefelter syndrome (XXY), fragile X syndrome, Turner syndrome metafemales or trisomy X, and monosomy X.

In one embodiment, fetal fraction information can be used to set thresholds and estimate minimum sample size in aneuploidy detection. Such use is described in Example 7 below. Fetal fraction information can be used in conjunction with sequencing information. For example, nucleic acids from a cell-free sample, for example a maternal plasma or serum sample, can be used to enumerate sequences in a sample. Sequences can be enumerated using any of the sequencing techniques described above. Knowledge of fetal fraction can be used to set "cutoff' thresholds to call "aneuploidy," "normal," or "marginal/no call" (uncertain) states. Then, calculations can be performed to estimate the minimum number of sequences required to achieve adequate sensitivity (i.e. probability of correctly identifying an aneuploidy state).

The determination of fetal fraction according to the method of the invention can be practiced in combination with any method used to determine the presence of absence of fetal aneuploidy in a maternal plasma sample. In addition to the method described herein for the determination of aneuploidy, the determination of fetal fraction by massively parallel sequencing can be used in conjunction with other methods for determining fetal aneuploidy, for example, according to the methods described in U.S. U.S. Patent Application Publication Nos. US 2007/0202525A1; US2010/0112575A1, US 2009/0087847A1; US2009/0029377A1; US 2008/0220422A1; US2008/0138809A1, US2008/0153090A1, and US Patent 7,645,576. The methods can also be combined with assays for determining other prenatal conditions associated with the mother and/or the fetus. For example, the method can be used in conjunction with prenatal analyses, for example, as described in U. S. Patent Application Publication Nos. US2010/0112590A1, US2009/0162842A1, US2007/0207466A1, and US2001/0051341A1.

The methods described can be applied to determine the fraction of any one population of nucleic acids in a mixture of nucleic acids contributed by different genomes. In addition to determining the fraction contributed to a sample by two individuals e.g. the different genomes are contributed by the fetus and the mother carrying the fetus, the methods can be used to determine the fraction of a genome in a mixture derived from two different cells of from one individual *e.g.* the genomes are contributed to the sample by aneuploid cancerous cells and normal euploid cells from the same subject.

## Compositions and Kits

The present disclosure is also directed to compositions and kits or reagent systems useful for practicing the methods described herein.

The compositions disclosed herein can be included in kits for massively parallel sequencing mixtures of fetal and maternal nucleic acid molecules *e.g.* cfDNA, present in a maternal sample *e.g.* a plasma sample. The kits comprise a composition comprising at least one set of primers for amplifying at least one polymorphic target nucleic acid in said fetal and maternal nucleic acid molecules. Polymorphic target nucleic acids can comprise without limitation single nucleotide polymorphisms (SNPs), tandem SNPs, small-scale multi-base deletions or insertions, called IN-DELS (also called deletion insertion polymorphisms or DIPs), Multi-Nucleotide Polymorphisms (MNPs) Short Tandem Repeats (STRs), restriction fragment length polymorphism (RFLP), or a polymorphism comprising any other change of sequence in a chromosome. Sequencing methods utilizing the compositions are NGS methods of single nucleic acid molecules or clonally amplified nucleic acid molecules as described herein. The massively parallel sequencing methods of NGS include pyrosequencing, sequencing by synthesis with reversible dye terminators, real-time sequencing, or sequencing by oligonucleotide probe ligation.

In one instance, the compositions includes primers for amplifying polymorphic target nucleic acids that each comprise at least one SNP. In one instance, the at least one SNP is selected from SNPs rs560681, rs1109037, rs9866013, rs13182883, rs13218440, rs7041158, rs740598, rs10773760, rs4530059, rs7205345, rs8078417, rs576261, rs2567608, rs430046, rs9951171, rs338882, rs10776839, rs9905977, rs1277284, rs258684, rs1347696, rs508485, rs9788670, rs8137254, rs3143, rs2182957, rs3739005,and rs530022. Exemplary corresponding sets of primers for amplifying the SNPs are provided as SEQ ID NOs:57-112.

In another instance, the composition includes primers for amplifying polymorphic target nucleic acids that each comprise at least one tandem SNP. In one instance, the composition includes primers for amplifying tandem SNPs. In one instance, the composition includes primers for amplifying the tandem SNPs disclosed herein, and the composition comprises the corresponding exemplary primers of SEQ ID NOS:197-310.

Kits can contain a reagent combination including the elements required to conduct an assay according to the methods disclosed herein. The reagent system is presented in a commercially packaged form, as a composition or admixture where the compatibility of the reagents will allow, in a test device configuration, or more typically as a test kit, i.e., a packaged combination of one or more containers, devices, or the like holding the necessary reagents, and preferably including written instructions for the performance of assays. The kit disclosed herein may be adapted for any configuration of assay and may include compositions for performing any of the various assay formats described herein. Kits for determining fetal fraction comprise compositions including primer sets for amplifying polymorphic nucleic acids present in a maternal sample as described and, where applicable, reagents for purifying cfDNA, are disclosed herein. In one instance, a kit designed to allow quantification of fetal and maternal polymorphic sequences *e.g.* STRs and/or SNPs and/or tandem SNPs, in a cfDNA plasma sample, includes at least one set of allele specific oligonucleotides specific for a selected SNP and/or region of tandem repeats. Preferably, the kit includes a plurality of primer sets to amplify a panel of polymorphic sequences. A kit can comprise other reagents and/or information for genotyping or quantifying alleles in a sample (e.g., buffers, nucleotides, instructions). The kits also include a plurality of containers of appropriate buffers and reagents.

## Example 1

### Determination of Fetal Fraction using Massively Parallel Sequencing: Sample Processing and cfDNA Extraction

Peripheral blood samples were collected from pregnant women in their first or second trimester of pregnancy and who were deemed at risk for fetal aneuploidy. Informed consent was obtained from each participant prior to the blood draw. Blood was collected before amniocentesis or chorionic villus sampling. Karyotype analysis was performed using the chorionic villus or amniocentesis samples to confirm fetal karyotype.

Peripheral blood drawn from each subject was collected in ACD tubes. One tube of blood sample (approximately 6-9 mL/tube) was transferred into one 15-mL low speed centrifuge tube. Blood was centrifuged at 2640 rpm, 4°C for 10 min using Beckman Allegra 6 R centrifuge and rotor model GA 3.8.

For cell-free plasma extraction, the upper plasma layer was transferred to a 15-ml high speed centrifuge tube and centrifuged at 16000 x g, 4°C for 10 min using Beckman Coulter Avanti J-E centrifuge, and JA-14 rotor. The two centrifugation steps were performed within 72 h after blood collection. Cell-free plasma comprising cfDNA was stored at -80°C and thawed only once before amplification of plasma cfDNA or for purification of cfDNA.

Purified cell-free DNA (cfDNA) was extracted from cell-free plasma using the QIAamp Blood DNA Mini kit (Qiagen) essentially according to the manufacturer's instruction. One milliliter of buffer AL and 100 μl of Protease solution were added to 1 ml of plasma. The mixture was incubated for 15 minutes at 56°C. One milliliter of 100% ethanol was added to the plasma digest. The resulting mixture was transferred to QIAamp mini columns that were assembled with VacValves and VacConnectors provided in the QIAvac 24 Plus column assembly (Qiagen). Vacuum was applied to the samples, and the cfDNA retained on the column filters was washed under vacuum with 750μl of buffer AW1, followed by a second wash with 750μl of buffer AW24. The column was centrifuged at 14,000 RPM for 5 minutes to remove any residual buffer from the filter. The cfDNA was eluted with buffer AE by centrifugation at 14,000 RPM, and the concentration determined using Qubit™ Quantitation Platform (Invitrogen).

## Example 2

### Determination of Fetal Fraction using Massively Parallel Sequencing: Preparation of Sequencing Libraries, Sequencing, and Analysis of Sequencing Data

#### a. Preparation of Sequencing Libraries

All sequencing libraries *i.e.* target, primary and enriched libraries, were prepared from approximately 2 ng of purified cfDNA that was extracted from maternal plasma. Library preparation was performed using reagents of the NEBNext™ DNA Sample Prep DNA Reagent Set 1 (Part No. E6000L; New England Biolabs, Ipswich, MA) for Illumina® as follows. Because cell-free plasma DNA is fragmented in nature, no further fragmentation by nebulization or sonication was done on the plasma DNA samples. The overhangs of approximately 2 ng purified cfDNA fragments contained in 40μl were converted into phosphorylated blunt ends according to the NEBNext® End Repair Module by incubating in a 1.5ml microfuge tube the cfDNA with 5μl 10X phosphorylation buffer, 2μl deoxynucleotide solution mix (10 mM each dNTP), 1μl of a 1:5 dilution of DNA Polymerase I, 1μl T4 DNA Polymerase and 1μl T4 Polynucleotide Kinase provided in the NEBNext™ DNA Sample Prep DNA Reagent Set 1 for 15 minutes at 20°C. The enzymes were then heat inactivated by incubating the reaction mixture at 75°C for 5 minutes. The mixture was cooled to 4°C, and dA tailing of the blunt-ended DNA was accomplished using 10μl of the dA-tailing master mix containing the Klenow fragment (3' to 5' exo minus) (NEBNext™ DNA Sample Prep DNA Reagent Set 1), and incubating for 15 minutes at 37°C. Subsequently, the Klenow fragment was heat inactivated by incubating the reaction mixture at 75°C for 5 minutes. Following the inactivation of the Klenow fragment, 1μl of a 1:5 dilution of Illumina Genomic Adaptor Oligo Mix (Part No. 1000521; Illumina Inc., Hayward, CA) was used to ligate the Illumina adaptors (Non-Index Y-Adaptors) to the dA-tailed DNA using 4μl of the T4 DNA ligase provided in the NEBNext™ DNA Sample Prep DNA Reagent Set 1, by incubating the reaction mixture for 15 minutes at 25°C. The mixture was cooled to 4°C, and the adaptor-ligated cfDNA was purified from unligated adaptors, adaptor dimers, and other reagents using magnetic beads provided in the Agencourt AMPure XP PCR purification system (Part No. A63881; Beckman Coulter Genomics, Danvers, MA). Eighteen cycles of PCR were performed to selectively enrich adaptor-ligated cfDNA using Phusion ® High-Fidelity Master Mix (Finnzymes, Woburn, MA) and Illumina's PCR primers complementary to the adaptors (Part No. 1000537 and 1000537). The adaptor-ligated DNA was subjected to PCR (98°C for 30 seconds; 18 cycles of 98°C for 10 seconds, 65°C for 30 seconds, and 72°C for 30 seconds; final extension at 72°C for 5 minutes, and hold at 4°C) using Illumina Genomic PCR Primers (Part Nos. 100537 and 1000538) and the Phusion HF PCR Master Mix provided in the NEBNext™ DNA Sample Prep DNA Reagent Set 1, according to the manufacturer's instructions. The amplified product was purified using the Agencourt AMPure XP PCR purification system (Agencourt Bioscience Corporation, Beverly, MA) according to the manufacturer's instructions available at www.beckmangenomics.com/products/AMPureXPProtocol_000387v001.pdf. The purified amplified product was eluted in 40μl of Qiagen EB Buffer, and the concentration and size distribution of the amplified libraries was analyzed using the Agilent DNA 1000 Kit for the 2100 Bioanalyzer (Agilent technologies Inc., Santa Clara, CA).

**b. Sequencing**

Sequencing of library DNA was performed using the Genome Analyzer II (Illumina Inc., San Diego, CA, USA) according to standard manufacturer protocols. Copies of the protocol for whole genome sequencing using Illumina/Solexa technology may be found at BioTechniques.RTM. Protocol Guide 2007 Published December 2006: p 29, and on the world wide web at biotechniques.com/default.asp?page=protocol&subsection=article display&id=1 12378. The DNA library was diluted to InM and denatured. Library DNA (5pM) was subjected to cluster amplification according to the procedure described in Illumina's Cluster Station User Guide and Cluster Station Operations Guide, available on the world wide web at illumina.com/systems/genome analyzer/cluster_station.ilmn. The amplified DNA was sequenced using Illumina's Genome Analyzer II to obtain single-end reads of 36bp. Only about 30 bp of random sequence information are needed to identify a sequence as belonging to a specific human chromosome. Longer sequences can uniquely identify more particular targets. In the present case, a large number of 36 bp reads were obtained, covering approximately 10% of the genome.

**c. Analysis of sequencing data for the determination of fetal fraction**

Upon completion of sequencing of the sample, the Illumina "Sequencer Control Software" transferred image and base call files to a Unix server running the Illumina "Genome Analyzer Pipeline" software version 1.51. the 36bp reads were aligned to an artificial reference genome *e.g.* a SNP genome, using the BOWTIE program. The artificial reference genome was identified as the grouping of the polymorphic DNA sequences that encompass the alleles comprised in the polymorphic target sequences. For example, the artificial reference genome is a SNP genome comprising SEQ ID NOs: 1-56. Only reads that mapped uniquely to the artificial genome were used for the analysis of fetal fraction. Reads that matched perfectly to the SNP genome were counted as tags and filtered. Of the remaining reads, only reads having one or two mismatches were counted as tags and included in the analysis. Tags mapped to each of the polymorphic alleles were counted, and the fetal fraction was determined as a percent of the ratio of the number of tags mapped to the major allele *i.e.* maternal allele, and the number of tags mapped to the minor allele *i.e.* fetal allele.

**Example 3**

**Selection of Autosomal SNPs for the Determination of Fetal Fraction**

A set of 28 autosomal SNPs were selected from a list of 92 SNPs (Pakstis et al., Hum Genet 127:315-324 [2010]) and from Applied Biosystems by Life Technologies™ (Carlsbad, CA) at world wide web address appliedbiosystems.com, and validated for use in multiplexed PCR amplification. Primers were designed to hybridize to a sequence close to the SNPs site on the cfDNA to ensure that it be included in the 36 bp read generated from the massively parallel sequencing on the Illumina Analyzer GII, and to generate amplicons of sufficient length to undergo bridge-amplification during cluster formation. Thus, primers were designed to generate amplicons that were at least 110 bp, which when combined with the universal adaptors (Illumina Inc., San Diego, CA) used for cluster amplification, resulted in DNA molecules of at least 200bp. Primer sequences were identified, and primer sets *i.e.* forward and reverse primers, were synthesized by Integrated DNA Technologies (San Diego, CA), and stored as a 1μM solution to be used for amplifying polymorphic target sequences as described in Examples 4-7. Table 1 provides the RefSNP (rs) accession ID numbers, the primers used for amplifying the target cfDNA sequence, and the sequences of the amplicons comprising the possible SNP alleles that would be generated using the primers. The SNPs given in Table 1 were used for the simultaneous amplification of 13 target sequences in a multiplexed assay. The panel provided in Table 1 is an exemplary SNP panel. Fewer or more SNPs can be employed to enrich the fetal and maternal DNA for polymorphic target nucleic acids. Additional SNPs that can be used include the SNPs given in Table 2. The SNP alleles are shown in bold and are underlined. Other additional SNPs that can be used to determine fetal fraction according to the present method include rs315791, rs3780962, rs1410059, rs279844, rs38882, rs9951171, rs214955, rs6444724, rs2503107, rs1019029, rs1413212, rs1031825, rs891700, rs1005533, rs2831700, rs354439, rs1979255, rs1454361, rs8037429, and rs1490413, which have been analyzed for determining fetal fraction by TaqMan PCR, and are disclosed in US Provisional applications 61/296,358 and 61/360,837.

**TABLE 1**

| SNP Panel for the Determination of Fetal Fraction | | | | | |
|---|---|---|---|---|---|
| **SNP ID** | **Chr** | **Amplicon: Allele 1** | **Amplicon: Allele 2** | **Forward Primer Sequence, name and SEQ ID NO:** | **Reverse Primer Sequence, name and SEQ ID NO:** |
| rs560681 | 1 | CACATGCACAGCCAGCAACCCTGTCAGCAGGAGTTCCCACCAGTTTCTTTCTGAGAACATCTGTTCAGGTTTCTCTCCATCTCT**A**TTTACTCAGGTCACAGGACCTTGGGG (SEQ ID NO:1) | CACATGCACAGCCAGCAACCCTGTCAGCAGGAGTTCCCACCAGTTTCTTTCTGAGAACATCTGTTCAGGTTTCTCTCCATCTCT**G**TTTACTCAGGTCACAGGACCTTGGGG (SEQ ID NO:2) | CACATGCACAGCCAGCAACCC (rs560681_C1_1_F; SEQ ID NO:57) | CCCCAAGGTCCTGTGACCTGAGT (rs560681_C1_1_R; SEQ ID NO:58) |
| rs1109037 | 2 | TGAGGAAGTGAGGCTCAGAGGGTAAGAAACTTTGTCACAGAGCTGGTGGTGAGGGTGGAGATTTTACACTCCCTGCCTCCCACACCAGTTTCTCC**A**GAGTGGAAAGACTTTCATCTCGCACTGGCA (SEQ ID NO:3) | TGAGGAAGTGAGGCTCAGAGGGTAAGAAACTTTGTCACAGAGCTGGTGGTGAGGGTGGAGATTTTACACTCCCTGCCTCCCACACCAGTTTCTCC**G**GAGTGGAAAGACTTTCATCTCGCACTGGCA (SEQ ID NO:4) | TGAGGAAGTGAGGCTCAGAGGGT (rs110937_C2_1_F ; SEQ ID NO:59) | TGCCAGTGCGAGATGAAAGTCTTT (rs110937_C2_1_R; SEQ ID NO:60) |

(continued)

| SNP Panel for the Determination of Fetal Fraction | | | | | |
|---|---|---|---|---|---|
| SNP ID | Chr | Amplicon: Allele 1 | Amplicon: Allele 2 | Forward Primer Sequence, name and SEQ ID NO: | Reverse Primer Sequence, name and SEQ ID NO: |
| rs9866013 | 3 | GTGCCTTCAGAACCTTTGAGATCTGATTCTATTTTTAAAGCTTCTTAGAAGAGAGATTGCAAAGTGGGTTGTTTCTCTAGCCAGACAGGGCAGG**C**AAATAGGGGTGGCTGGTGGGATGGGA (SEQ ID NO:5) | GTGCCTTCAGAACCTTTGAGATCTGATTCTATTTTTAAAGCTTCTTAGAAGAGAGATTGCAAAGTGGGTTGTTTCTCTAGCCAGACAGGGCAGG**T**AAATAGGGGTGGCTGGTGGGATGGGA (SEQ ID NO:6) | GTGCCTTCAGAACCTTTGAGATCTGAT (rs9866013_C3_1_F; SEQ ID NO:61) | TCCCATCCCACCAGCCACCC (rs9866013_C3_1_R; SEQ ID NO:62) |
| rs13182883 | 5 | AGGTGTGTCTCTCTTTTGTGAGGGGAGGGGTCCCTTCTGGCCTAGTAGAGGGCCTGGCCTGCAGTGAGCATTCAAATCCTC**A**AGGAACAGGGTGGGGAGGTGGGACAAAGG (SEQ ID NO:7) | AGGTGTGTCTCTCTTTTGTGAGGGGAGGGGTCCCTTCTGGCCTAGTAGAGGGCCTGGCCTGCAGTGAGCATTCAAATCCTC**G**AGGAACAGGGTGGGGAGGTGGGACAAAGG (SEQ ID NO:8) | AGGTGTGTCTCTCTTTTGTGAGGGG (rs13182883_C5_1_F; SEQ ID NO:63) | CCTTTGTCCCACCTCCCCACC (rs13182883_C5_1_R; SEQ ID NO:64) |

(continued)

| SNP Panel for the Determination of Fetal Fraction | | | | | |
|---|---|---|---|---|---|
| SNP ID | Chr | Amplicon: Allele 1 | Amplicon: Allele 2 | Forward Primer Sequence, name and SEQ ID NO: | Reverse Primer Sequence, name and SEQ ID NO: |
| rs13218440 | 6 | CCTCGCCTACTGTGCTGTTTCTAACCATCATGCTTTTCCCTGAATCTCTTGAGTCTTTTTCTGCTGTGGACTGAAACTTGATCCTGAGATTCACCTCTAGTCCCTCTG**A**GCAGCCTCCTGGAATACTCAGCTGGGATGG (SEQ ID NO:9) | CCTCGCCTACTGTGCTGTTTCTAACCATCATGCTTTTCCCTGAATCTCTTGAGTCTTTTTCTGCTGTGGACTGAAACTTGATCCTGAGATTCACCTCTAGTCCCTCTG**G**GCAGCCTCCTGGAATACTCAGCTGGGATGG (SEQ ID NO:10) | CCTCGCCTACTGTGCTGTTTCTAACC (rs13218440_C6_1_F; SEQ ID NO:65) | CCATCCCAGCTGAGTATTCCAGGAG (rs13218440_C6_1_R; SEQ ID NO:66) |
| rs7041158 | 9 | AATTGCAATGGTGAGAGGTTGATGGTAAAATCAAACGGAACTTGTTATTTTGTCATTCTGATGGACTGGAACTGAGGATTTTCAATTTCCT**C**TCCAACCCAAGACACTTCTCACTGG (SEQ ID NO:11) | AATTGCAATGGTGAGAGGTTGATGGTAAAATCAAACGGAACTTGTTATTTTGTCATTCTGATGGACTGGAACTGAGGATTTTCAATTTCCT**T**TCCAACCCAAGACACTTCTCACTGG (SEQ ID NO:12) | AATTGCAATGGTGAGAGGTTGATGGT (SEQ ID NO:67) | CCAGTGAGAAGTGTCTTGGGTTGG (SEQ ID NO:68) |

(continued)

| SNP Panel for the Determination of Fetal Fraction | | | | | |
|---|---|---|---|---|---|
| **SNP ID** | **Chr** | **Amplicon: Allele 1** | **Amplicon: Allele 2** | **Forward Primer Sequence, name and SEQ ID NO:** | **Reverse Primer Sequence, name and SEQ ID NO:** |
| rs740598 | 10 | GAAATGCCTTCTCAGGTAATGGAAGGTTATCCAAATATTTTTCGTAAGTATTTCAAATAGCAATGGCTCGTCTATGGTTAGTCTC**A**CAGCCACATTCTCAGAACTGCTCAAACC (SEQ ID NO:13) | GAAATGCCTTCTCAGGTAATGGAAGGTTATCCAAATATTTTTCGTAAGTATTTCAAATAGCAATGGCTCGTCTATGGTTAGTCTC**G**CAGCCACATTCTCAGAACTGCTCAAACC (SEQ ID NO:14) | GAAATGCCTTCTCAGGTAATGGAAGGT (SEQ ID NO:69) | GGTTTGAGCAGTTCTGAGAATGTGGCT (SEQ ID NO:70) |
| rs10773760 | 12 | ACCCAAAACACTGGAGGGGCCTCTTCTCATTTTCGGTAGACTGCAAGTGTTAGCCGTCGGGACCAGCTTCTGTCTGGAAGTTCGTCAAATTGCAGTTA**A**GTCCAAGTATGCCACATAGCAGATAAGGG (SEQ ID NO:15) | ACCCAAAACACTGGAGGGGCCTCTTCTCATTTTCGGTAGACTGCAAGTGTTAGCCGTCGGGACCAGCTTCTGTCTGGAAGTTCGTCAAATTGCAGTTA**G**GTCCAAGTATGCCACATAGCAGATAAGGG (SEQ ID NO:16) | ACCCAAAACACTGGAGGGGCCT (SEQ ID NO:71) | CCCTTATCTGCTATGTGGCATACTTGG (SEQ ID NO:72) |

(continued)

| SNP Panel for the Determination of Fetal Fraction | | | | | |
|---|---|---|---|---|---|
| SNP ID | Chr | Amplicon: Allele 1 | Amplicon: Allele 2 | Forward Primer Sequence, name and SEQ ID NO: | Reverse Primer Sequence, name and SEQ ID NO: |
| rs4530059 | 14 | GCACCAGAATTTAAACAACGCTGACAATAAATATGCAGTCGATGATGACTTCCCAGAGCTCCAGAAGCAACTCCAGCACAC**A**GAGAGGCGCTGATGTGCCTGTCAGGTGC (SEQ ID NO:17) | GCACCAGAATTTAAACAACGCTGACAATAAATATGCAGTCGATGATGACTTCCCAGAGCTCCAGAAGCAACTCCAGCACAC**G**GAGAGGCGCTGATGTGCCTGTCAGGTGC (SEQ ID NO:18) | GCACCAGAATTTAAACAACGCTGACAA (SEQ ID NO:73) | GCACCTGACAGGCACATCAGCG (SEQ ID NO:74) |
| rs7205345 | 16 | TGACTGTATACCCCAGGTGCACCCTTGGGTCATCTCTATCATAGAACTTATCTCACAGAGTATAAGAGCTGATTTCTGTGTCTGCCT**C**TCACACTAGACTTCCACATCCTTAGTGC (SEQ ID NO:19) | TGACTGTATACCCCAGGTGCACCCTTGGGTCATCTCTATCATAGAACTTATCTCACAGAGTATAAGAGCTGATTTCTGTGTCTGCCT**G**TCACACTAGACTTCCACATCCTTAGTGC (SEQ ID NO:20) | TGACTGTATACCCCAGGTGCACCC (SEQ ID NO:75) | GCACTAAGGATGTGGAAGTCTAGTGTG (SEQ ID NO:76) |

(continued)

| SNP Panel for the Determination of Fetal Fraction | | | | | |
|---|---|---|---|---|---|
| **SNP ID** | **Chr** | **Amplicon: Allele 1** | **Amplicon: Allele 2** | **Forward Primer Sequence, name and SEQ ID NO:** | **Reverse Primer Sequence, name and SEQ ID NO:** |
| rs8078417 | 17 | TGTACGTGGTCACCA GGGGACGCCTGGCG CTGCGAGGGAGGCC CCGAGCCTCGTGCCC CCGTGAAGCTTCAG CTCCCCTCCC**<u>C</u>**GGCT GTCCTTGAGGCTCTT CTCACACT (SEQ ID NO:21) | TGTACGTGGTCACCA GGGGACGCCTGGCG CTGCGAGGGAGGCC CCGAGCCTCGTGCCC CCGTGAAGCTTCAG CTCCCCTCCC**<u>T</u>**GGCT GTCCTTGAGGCTCTT CTCACACT (SEQ ID NO:22) | TGTACGTGG TCACCAGG GGACG (SEQ ID NO:77) | AGTGTGAGAAG AGCCTCAAGGA CAGC (SEQ ID NO:78) |
| rs576261 | 19 | CAGTGGACCCTGCT GCACCTTTCCTCCCC TCCCATCAACCTCTT TTGTGCCTCCCCCTC CGTGTACCACCTTCT CTGTCACCA**<u>A</u>**CCCTG GCCTCACAACTCTCT CCTTTGCCAC (SEQ ID NO:23) | CAGTGGACCCTGCT GCACCTTTCCTCCCC TCCCATCAACCTCTT TTGTGCCTCCCCCTC CGTGTACCACCTTCT CTGTCACCA**<u>C</u>**CCCTG GCCTCACAACTCTCT CCTTTGCCAC (SEQ ID NO:24) | CAGTGGAC CCTGCTGCA CCTT (SEQ ID NO:79) | GTGGCAAAGGA GAGAGTTGTGA GG (SEQ ID NO:80) |

(continued)

| SNP Panel for the Determination of Fetal Fraction | | | | | |
|---|---|---|---|---|---|
| SNP ID | Chr | Amplicon: Allele 1 | Amplicon: Allele 2 | Forward Primer Sequence, name and SEQ ID NO: | Reverse Primer Sequence, name and SEQ ID NO: |
| rs2567608 | 20 | CAGTGGCATAGTAG TCCAGGGGCTCCTCC TCAGCACCTCCAGC ACCTTCCAGGAGGC AGCAGCGCAGGCAG AGAACCCGCTGGAA G**A**ATCGGCGGAAGT TGTCGGAGAGG (SEQ ID NO:25) | CAGTGGCATAGTAG TCCAGGGGCTCCTCC TCAGCACCTCCAGC ACCTTCCAGGAGGC AGCAGCGCAGGCAG AGAACCCGCTGGAA G**G**ATCGGCGGAAGT TGTCGGAGAGG (SEQ ID NO:26) | CAGTGGCA TAGTAGTCC AGGGGCT (SEQ ID NO:81) | CCTCTCCGACA ACTTCCGCCG (SEQ ID NO:82) |

TABLE 2 Additional SNPs for the Determination of Fetal Fraction

| SNP ID | Chr | Amplicon: Allele 1 | Amplicon: Allele 2 | Forward Primer Sequence, name and SEQ ID NO: | Reverse Primer Sequence, name and SEQ ID NO: |
|---|---|---|---|---|---|
| rs430046 | 16 | AGGTCTGGGGGCCGCTGAATGCCAAGCTGGGAATCTTAAATGTTAAGGAACAAGGTCATACAATGAATGGTGTGATGTAAAAGCTTGGGAGGTGATTT**C**TGAGGGTAGGTGCTGGGTTTAATGGGAGGA (SEQ ID NO:27) | AGGTCTGGGGGCCGCTGAATGCCAAGCTGGGAATCTTAAATGTTAAGGAACAAGGTCATACAATGAATGGTGTGATGTAAAAGCTTGGGAGGTGATTT**T**TGAGGGTAGGTGCTGGGTTTAATGGGAGGA (SEQ ID NO:28) | AGGTCTGGGGGCCGCTGAAT (rs430046_C1_1_F; SEQ ID NO:83) | TCCTCCCATTAAACCCAGCACCT (rs430046_C1_1_R; SEQ ID NO:84) |
| rs995 1171 | 18 | ACGGTTCTGTCCTGTAGGGGAGAAAAGTCCTCGTTGTTCCTCTGGGATGCAACATGAGAGAGCAGCACACTGAGGCTTTATGG**A**TTGCCCTGCCACAAGTGAACAGG (SEQ ID NO:29) | ACGGTTCTGTCCTGTAGGGGAGAAAAGTCCTCGTTGTTCCTCTGGGATGCAACATGAGAGAGCAGCACACTGAGGCTTTATGG**G**TTGCCCTGCCACAAGTGAACAGG (SEQ ID NO:30) | ACGGTTCTGTCCTGTAGGGGAGA (rs9951171_C1_1_F; SEQ ID NO:85) | CCTGTTCACTTGTGGCAGGGCA (rs9951171_C1_1_R; SEQ ID NO:86) |

(continued)

| SNP ID | Chr | Amplicon: Allele 1 | Amplicon: Allele 2 | Forward Primer Sequence, name and SEQ ID NO: | Reverse Primer Sequence, name and SEQ ID NO: |
|---|---|---|---|---|---|
| rs338882 | 5 | GCGCAGTCAGATGGGCGTGCTGGCGTCTGTCTTCTCTCTCTCCTGCTCTCTGGCTTCATTTTTCTCTCCTTCTGTCTCACCTTCTTTCGTGTGCCTGTGCA**C**ACACACGTTTGGGACAAGGGCTGGA (SEQ ID NO:31) | GCGCAGTCAGATGGGCGTGCTGGCGTCTGTCTTCTCTCTCTCCTGCTCTCTGGCTTCATTTTTCTCTCCTTCTGTCTCACCTTCTTTCGTGTGCCTGTGCA**T**ACACACGTTTGGGACAAGGGCTGGA (SEQ ID NO:32) | GCGCAGTCAGATGGGCGTGC (rs338882_C1_1_F; SEQ ID NO:87) | TCCAGCCCTTGTCCCAAACGTGT (rs338882_C1_1_R; SEQ ID NO:88) |
| rs10776839 | 9 | GCCGGACCTGCGAAATCCCAAAATGCCAAACATTCCCGCCTCACATGATCCCAGAGAGAGGGGACCCAGTGTTCCCAGCTTGCAGCTGAGGAGCCCGAG**G**TTGCCGTCAGATCAGAGCCCCAGTTGCCCG (SEQ ID NO:33) | GCCGGACCTGCGAAATCCCAAAATGCCAAACATTCCCGCCTCACATGATCCCAGAGAGAGGGGACCCAGTGTTCCCAGCTTGCAGCTGAGGAGCCCGAG**T**TTGCCGTCAGATCAGAGCCCCAGTTGCCCG (SEQ ID NO:34) | GCCGGACCTGCGAAATCCCAA (rs10776839C1_1_F; SEQ ID NO:89) | CGGGCAACTGGGGCTCTGATC (rs10776839_C1_1_R; SEQ ID NO:90) |

(continued)

| SNP ID | Chr | Amplicon: Allele 1 | Amplicon: Allele 2 | Forward Primer Sequence, name and SEQ ID NO: | Reverse Primer Sequence, name and SEQ ID NO: |
|---|---|---|---|---|---|
| rs9905977 | 17 | AGCAGCCTCCCTCG ACTAGCTCACACTA CGATAAGGAAAAT TCATGAGCTGGTGT CCAAGGAGGGCTG GGTGACTCGTGGCT CAGTCAGC**A**TCAAG ATTCCTTTCGTCTTT CCCCTCTGCC (SEQ ID NO:35) | AGCAGCCTCCCTCGA CTAGCTCACACTACG ATAAGGAAAATTCAT GAGCTGGTGTCCAAG GAGGGCTGGGTGACT CGTGGCTCAGTCAGC **G**TCAAGATTCCTTTC GTCTTTCCCCTCTGCC (SEQ ID NO:36) | AGCAGCCT CCCTCGACT AGCT (rs9905977_C 1_1_F; SEQ ID NO:91) | GGCAGAGGGGA AAGACGAAAGG A (rs9905977_C1_1_ R; SEQ ID NO:92) |
| rs1277284 | 4 | TGGCATTGCCTGTA ATATACATAGCCAT GGTTTTTTATAGGC AATTTAAGATGAAT AGCTTCTAAACTAT AGATAAGTTTCATT ACCCCAGGAAGCT | TGGCATTGCCTGTAA TATACATAGCCATGG TTTTTTATAGGCAATT TAAGATGAATAGCTT CTAAACTATAGATAA GTTTCATTACCCCAG GAAGCTGAACTATAG | TGGCATTGC CTGTAATAT ACATAG (rs1277284_C 4_1_F; SEQ ID NO:93) | AAGCACCATTC TAATGATTTTG G (rs1277284_C4_1_ R; SEQ ID NO:94) |
| | | GAACTATAGCTACT TT**A**CCCAAAATCAT TAGAATGGTGCTT (SEQ ID NO:37) | CTACTTT**C**CCCAAAA TCATTAGAATGGTGC TT (SEQ ID NO:38) | | |

(continued)

| SNP ID | Chr | Amplicon: Allele 1 | Amplicon: Allele 2 | Forward Primer Sequence, name and SEQ ID NO: | Reverse Primer Sequence, name and SEQ ID NO: |
|---|---|---|---|---|---|
| rs258684 | 7 | ATGAAGCCTTCCACCAACTGCCTGTATGACTCATCTGGGGACTTCTGCTCTATACTCAAAGTGGCTTAGTCACTGCCAATGTATTTCCATATGAGGGACG**A**TGATTACTAAGGAAATATAGAAACAACAACTGATC (SEQ ID NO:39) | ATGAAGCCTTCCACCAACTGCCTGTATGACTCATCTGGGGACTTCTGCTCTATACTCAAAGTGGCTTAGTCACTGCCAATGTATTTCCATATGAGGGACG**G**TGATTACTAAGGAAATATAGAAACAACAACTGATC (SEQ ID NO:40) | ATGAAGCCTTCCACCAACTG (rs258684_C7_1_F; SEQ ID NO:95) | GATCAGTTGTTGTTTCTATATTTCCTT (rs258684_C7_1_R; SEQ ID NO:96) |
| rs1347696 | 8 | ACAACAGAATCAGGTGATTGGAGAAAAGATCACAGGCCTAGGCACCCAAGGCTTGAAGGATGAAAGAATGAAAGATGGACGGAA**C**AAAATTAGGACCTTAATTCTTTGTTCAGTTCAG (SEQ ID NO:41) | ACAACAGAATCAGGTGATTGGAGAAAAGATCACAGGCCTAGGCACCCAAGGCTTGAAGGATGAAAGAATGAAAGATGGACGGAA**G**AAAATTAGGACCTTAATTCTTTGTTCAGTTCAG (SEQ ID NO:42) | ACAACAGAATCAGGTGATTGGA (rs1347696_C8_4_F; SEQ ID NO:97) | CTGAACTGAACAAAGAATTAAGGTC (rs1347696_C8_4_F; SEQ ID NO:98) |

(continued)

| SNP ID | Chr | Amplicon: Allele 1 | Amplicon: Allele 2 | Forward Primer Sequence, name and SEQ ID NO: | Reverse Primer Sequence, name and SEQ ID NO: |
|---|---|---|---|---|---|
| rs508485 | 11 | TTGGGGTAAATTTTCATTGTCATATGTGGAATTTAAATATACCATCATCTACAAAGAATTCCACAGAGTTAAATATCTTAAGTTAAACACTTAAAATAAGTGTTTGCGTGATATTTTGATGA**C**AGATAAACAGAGTCTAATTCCCACCCC (SEQ ID NO:43) | TTGGGGTAAATTTTCATTGTCATATGTGGAATTTAAATATACCATCATCTACAAAGAATTCCACAGAGTTAAATATCTTAAGTTAAACACTTAAAATAAGTGTTTGCGTGATATTTTGATGA**T**AGATAAACAGAGTCTAATTCCCACCCC (SEQ ID NO:44) | TTGGGGTAAATTTTCATTGTCA (rs508485_C11_1_F; SEQ ID NO:99) | GGGGTGGGAATTAGACTCTG (rs508485_C11_1_R; SEQ ID NO100) |
| rs9788670 | 15 | TGCAATTCAAATCAGGAAGTATGACCAAAAGACAGAGATCTTTTTTGGATGATCCCTAGCCTAGCAATGCCTGGCAGCCATGCAGGTGCAATGTCAACCTTAAATAATGTATTGCAAA**C**TCAGAGCTGACAAACCTCGATGTTGC (SEQ ID NO:45) | TGCAATTCAAATCAGGAAGTATGACCAAAAGACAGAGATCTTTTTTGGATGATCCCTAGCCTAGCAATGCCTGGCAGCCATGCAGGTGCAATGTCAACCTTAAATAATGTATTGCAAA**T**TCAGAGCTGACAAACCTCGATGTTGC (SEQ ID NO:46) | TGCAATTCAAATCAGGAAGTATG (rs9788670_c15_2_F; SEQ ID NO:101) | GCAACATCGAGGTTTGTCAG (rs9788670_c15_2_R; SEQ ID NO:102) |

(continued)

| SNP ID | Chr | Amplicon: Allele 1 | Amplicon: Allele 2 | Forward Primer Sequence, name and SEQ ID NO: | Reverse Primer Sequence, name and SEQ ID NO: |
|---|---|---|---|---|---|
| rs8137254 | 22 | CTGTGCTCTGCGAA TAGCTGCAGAAGTA ACTTGGGGACCCAA AATAAAGCAGAAT GCTAATGTCAAGTC CTGAGAACCAAGC CCTGGGACTCTGGT GCCATTT**C**GGATTC TCCATGAGCATGGT (SEQ ID NO:47) | CTGTGCTCTGCGAAT AGCTGCAGAAGTAAC TTGGGGACCCAAAAT AAAGCAGAATGCTAA TGTCAAGTCCTGAGA ACCAAGCCCTGGGAC TCTGGTGCCATTT**T**G GATTCTCCATGAGCA TGGT (SEQ ID NO:48) | CTGTGCTCT GCGAATAG CTG (rs8137254_c2 2_2_F: SEQ ID NO:103) | ACCATGCTCAT GGAGAATCC (rs8137254_c22_2 _R; SEQ ID NO:104) |
| rs3143 | 19 | TTTTTCCAGCCAAC TCAAGGCCAAAAA AAATTTCTTAATAT AGTTATTATGCGAG GGGAGGGGAAGCA AAGGAGCACAGGT AGTCCACAGAATA **A**GACACAAGAAAC CTCAAGCTGTG (SEQ ID NO:49) | TTTTTCCAGCCAACTC AAGGCCAAAAAAAT TTCTTAATATAGTTAT TATGCGAGGGGAGGG GAAGCAAAGGAGCA CAGGTAGTCCACAGA ATA**G**GACACAAGAA ACCTCAAGCTGTG (SEQ ID NO:50) | TTTTTCCAG CCAACTCA AGG (rs3143_c19_2 _F: SEQ ID NO:105) | CACAGCTTGAG GTTTCTTGTG (rs3143_c19_2_R; SEQ ID NO:106) |

(continued)

| SNP ID | Chr | Amplicon: Allele 1 | Amplicon: Allele 2 | Forward Primer Sequence, name and SEQ ID NO: | Reverse Primer Sequence, name and SEQ ID NO: |
|---|---|---|---|---|---|
| rs2182957 | 13 | TCTTCTCGTCCCCTAAGCAAACAACATCCGCTTGCTTCTGTCTGTGTAACCACAGTGAATGGGTGTGCACGCTTG**A**TGGGCCTCTGAGCCCCTGTTGCACAAACCAGAAA (SEQ ID NO:51) | TCTTCTCGTCCCCTAAGCAAACAACATCCGCTTGCTTCTGTCTGTGTAACCACAGTGAATGGGTGTGCACGCTTG**G**TGGGCCTCTGAGCCCCTGTTGCACAAACCAGAAA (SEQ ID NO:52) | TCTTCTCGTCCCCTAAGCAA (rs2182957_c13_1_F: SEQ ID NO:107) | TTTCTGGTTTGTGCAACAGG (rs2182957_c13_1_R; SEQ ID NO:108) |
| rs3739005 | 2 | CACATGGGGGCATTAAGAATCGCCCAGGGAGGAGGAGGGAGAACGCGTGCTTTTCACATTTGCATTTGAATTTT**C**GAGTTCCCAGGATGTGTTTTTGTGCTCATCGATGT (SEQ ID NO:53) | CACATGGGGGCATTAAGAATCGCCCAGGGAGGAGGAGGGAGAACGCGTGCTTTTCACATTTGCATTTGAATTTT**T**GAGTTCCCAGGATGTGTTTTTGTGCTCATCGATGT (SEQ ID NO:54) | CACATGGGGGCATTAAGAAT (rs3739005_c2_2_F; SEQ ID NO:109) | ACATCGATGAGCACAAAAACAC (rs3739005_c2_2_R; SEQ ID NO:110) |

(continued)

| SNP ID | Chr | Amplicon: Allele 1 | Amplicon: Allele 2 | Forward Primer Sequence, name and SEQ ID NO: | Reverse Primer Sequence, name and SEQ ID NO: |
|---|---|---|---|---|---|
| rs530022 | 1 | GGGCTCTGAGGTGTGTGAAATAAAAACAAATGTCCATGTCTGTCCTTTTATGGCATTTTGGGACTTTACATTTCAAACATTTCAGACATGTATCACAACACGA**A**GGAATAACAGTTCCAGGGATATCT (SEQ ID NO:55) | GGGCTCTGAGGTGTGTGAAATAAAAACAAATGTCCATGTCTGTCCTTTTATGGCATTTTGGGACTTTACATTTCAAACATTTCAGACATGTATCACAACACGA**G**GGAATAACAGTTCCAGGGATATCT (SEQ ID NO:56) | GGGCTCTGAGGTGTGTGAAA (rs530022_c1_2_F; SEQ ID NO:111) | AGATATCCCTGGAACTGTTATTCC (rs530022_c1_2_R; SEQ ID NO:112) |

**Example 4**

**Determination of Fetal Fraction by Massively Parallel Sequencing of a Target Library**

To determine the fraction of fetal cfDNA in a maternal sample, target polymorphic nucleic acid sequences each comprising a SNP were amplified and used for preparing a target library for sequencing in a massively parallel fashion.

cfDNA was extracted as described in Example 1. A target sequencing library was prepared as follows. cfDNA contained in 5μl of purified cfDNA was amplified in a reaction volume of 50μl containing 7.5μl of a 1μM primer mix (Table 1), 10μl of NEB 5X Mastermix and 27 μl water. Thermal cycling was performed with the Gene Amp9700 (Applied Biosystems) using the following cycling conditions: incubating at 95°C for 1 minute, followed by 20-30 cycles at 95°C for 20 seconds, 68°C for 1 minute, and 68°C for 30s, which was followed by a final incubation at 68°C for 5 minutes. A final hold at 4°C was added until the samples were removed for combining with the unamplified portion of the purified cfDNA sample. The amplified product was purified using the Agencourt AMPure XP PCR purification system (Part No. A63881; Beckman Coulter Genomics, Danvers, MA). A final hold at 4°C was added until the samples were removed for preparing the target library. The amplified product was analyzed with a 2100 Bioanalyzer (Agilent Technologies, Sunnyvale, CA), and the concentration of amplified product determined. A sequencing library of amplified target nucleic acids was prepared as described in Example 2, and was sequenced in a massively parallel fashion using sequencing-by-synthesis with reversible dye terminators and according to the Illumina protocol (BioTechniques.RTM. Protocol Guide 2007 Published December 2006: p 29, and on the world wide web at biotechniques.com/default.asp?page=protocol&subsection=article_display&id=112378). Analysis and counting of tags mapped to a reference genome consisting of 26 sequences (13 pairs each representing two alleles) comprising a SNP *i.e.* SEQ ID NO: 1-26 was performed as described.

Table 3 provides the tag counts obtained from sequencing the target library, and the calculated fetal fraction derived from sequencing data.

**TABLE 3 Determination of Fetal Fraction by Massively Parallel Sequencing of a Library of Polymorphic Nucleic Acids**

| SNP | SNP TAG COUNTS | Fetal Fraction (%) |
|---|---|---|
| rs10773760.1\|Chr.12\|length=128\|allele=A | 236590 | 1.98 |
| rs10773760.2\|Chr.12\|length=128\|allele=G | 4680 | |
| rs13182883.11Chr.5\|length=111\|allele=A | 3607 | 4.99 |
| rs13182883,2\|Chr.5\|length=111\|allele=G | 72347 | |
| rs4530059.1\|Chr.14\|length=110\|allele=A | 3698 | 1.54 |
| rs4530059.1\|Chr.14\|length=110\|allele=G | 239801 | |
| rs8078417.1\|Chr.17\|length=110\|allele=C | 1E+06 | 3.66 |
| rs8078417.2\|Chr.17\|length=110\|allele=T | 50565 | |
| **Fetal Fraction (Mean±S.D.) = 12.4±6.6** | | |

The results show that polymorphic nucleic acid sequences each comprising at least one SNP can be amplified from cfDNA derived from a maternal plasma sample to construct a library that can be sequenced in a massively parallel fashion to determine the fraction of fetal nucleic acids in the maternal sample.

**Example 5**

**Determination of Fetal Fraction Following Enrichment of Fetal and Maternal Nucleic Acids in a cfDNA Sequencing Library Sample.**

To enrich the fetal and maternal cfDNA contained in a primary sequencing library constructed using purified fetal and maternal cfDNA, a portion of a purified cfDNA sample was used for amplifying polymorphic target nucleic acid sequences, and for preparing a sequencing library of amplified polymorphic target nucleic acids, which was used to enrich the fetal and maternal nucleic acid sequences comprised in the primary library.

The method corresponds to workflow 3 diagrammed in Figure 3. A target sequencing library was prepared from a portion of the purified cfDNA as described in Example 2. A primary sequencing library was prepared using the remaining portion of the purified cfDNA as described in Example 2. Enrichment of the primary library for the amplified polymorphic nucleic acids comprised in the target library was obtained by diluting the primary and the target sequencing libraries to 10nM, and combining the target library with the primary library at a ratio of 1:9 to provide an enriched sequencing library. Sequencing of the enriched library and analysis of the sequencing data was performed as described in Example 2.

Table 4 provides the number of sequence tags that mapped to the SNP genome for the informative SNPs identified from sequencing an enriched library derived from plasma samples of pregnant women each carrying a T21, a T13, a T18 and a monosomy X fetus, respectively. Fetal fraction was calculated as follows:

$$\% \text{ fetal fraction allele}_x = ((\sum \text{Fetal sequence tags for allele}_x) / (\sum \text{Maternal sequence tags for allele}_x)) \times 100$$

Table 4 also provides the number of the sequence tags mapped to the human reference genome. Tags mapped to the human reference genome were used to determine the presence or absence of aneuploidy using the same plasma sample that was utilized for determining the corresponding fetal fraction. Method for using sequence tags counts for determining aneuploidy are described in U.S. Provisional Applications 61/407,017 and 61/455,849778.

**TABLE 4 Determination of Fetal Fraction by Massively Parallel Sequencing of an Enriched Library of Polymorphic Nucleic Acids**

| Sample ID (karyotype) | SNP | SNP TAG COUNTS | FETAL FRACTION (%) |
|---|---|---|---|
| 11409 (47, XY+21) | rs13182883.1\|Chr. 5\|length=111\|allele=A | 261 | 4.41 |
| | rs13182883.2\|Chr. 5\|length=111\|allele=G | 5918 | |
| | rs740598.1\|Chr. 10\|length=114\|allele=A | 5545 | 7.30 |
| | rs740598.2\|Chr. 10\|length=114\|allele=G | 405 | |
| | rs8078417.1\|Chr. 17\|length=110\|allele=C | 8189 | 6.74 |
| | rs8078417.2\|Chr. 17\|length=110\|allele=T | 121470 | |
| | rs576261.1\|Chr. 19\|length=114\|allele=A | 58342 | 7.62 |
| | rs576261.2\|Chr. 19\|length=114\|allele=C | 4443 | |
| **Fetal Fraction (Mean±S.D.) = 6.5±1.5** | | | |

(continued)

| Sample ID | | | |
|---|---|---|---|
| 95133 (47, XX+18) | rs1109037.1\|Chr. 2\|length=126\|allele=A | 12229 | 2.15 |
| | rs1109037.2\|Chr. 2\|length=126\|allele=G | 263 | |
| | rs13218440.1\|Chr. 6\|length=139\|allele=A | 55949 | 3.09 |
| | rs13218440.2\|Chr. 6\|length=139\|allele=G | 1729 | |
| | rs7041158.1\|Chr. 9\|length=117\|allele=C | 7281 | 4.12 |
| | rs7041158.2\|Chr. 9\|length=117\|allele=T | 300 | |
| | rs7205345.1\|Chr. 16\|length=116\|allele=C | 53999 | 2.14 |
| | rs7205345.2\|Chr. 16\|length=116\|allele=G | 1154 | |
| **Fetal Fraction (Mean±S.D.) = 2.9±0.9** | | | |
| **Sample ID** | | | |
| 51236 (46,XY+13) | rs13218440.1\|Chr. 6\|length=139\|allele=A | 1119 | 1.65 |
| | rs13218440.2\|Chr. 6\|length=139\|allele=G | 67756 | |
| | rs560681.1\|Chr. 1\|length=111\|allele=A | 14123 | 5.18 |
| | rs560681.2\|Chr. 1\|length=111\|allele=G | 732 | |
| | rs7205345.1\|Chr. 16\|length=116\|allele=C | 18176 | 1.63 |
| | rs7205345.2\|Chr. 16\|length=116\|allele=G | 296 | |
| | rs9866013.1\|Chr. 3\|length=121\|allele=C | 117 | 2.33 |
| | rs9866013.2\|Chr. 3\|length=121\|allele=T | 5024 | |
| **Fetal Fraction (Mean±S.D.) = 2.7±1.7** | | | |

(continued)

| Sample ID | | | |
|---|---|---|---|
| 54430 (45,XO) | rs1109037.1\|Chr. 2\|length=126\|allele=A | 19841 | 1.80 |
| | rs1109037.2\|Chr. 2\|length=126\|allele=G | 357 | |
| | rs9866013.1\|Chr. 3\|length=121\|allele=C | 12931 | 3.81 |
| | rs9866013.2\|Chr. 3\|length=121\|allele=T | 493 | |
| | rs7041158.1\|Chr. 9\|length=117\|allele=C | 2800 | 4.25 |
| | rs7041158.2\|Chr. 9\|length=117\|allele=T | 119 | |
| | rs740598.1\|Chr. 10\|length=114\|allele=A | 12903 | 4.87 |
| | rs740598.2\|Chr. 10\|length=114\|allele=G | 628 | |
| | rs10773760.1\|Chr. 12\|length=128\|allele=A | 46324 | 4.65 |
| | rs10773760.2\|Chr. 12\|length=128\|allele=G | 2154 | |
| **Fetal Fraction (Mean±S.D.) = 3.9±1.2** | | | |

**Example 6**

**Determination of Fetal Fraction by Massively Parallel Sequencing: Enrichment of Fetal and Maternal Nucleic Acids for Polymorphic Nucleic Acids in a Purified cfDNA Sample.**

To enrich the fetal and maternal cfDNA contained in a purified sample of cfDNA extracted from a maternal plasma sample, a portion of the purified cfDNA was used for amplifying polymorphic target nucleic acid sequences each comprising one SNP chosen from the panel of SNPs given in Table 5.

The method corresponds to workflow 2 diagrammed in Figure 3. Cell-free plasma was obtained from a maternal blood sample, and cfDNA was purified from the plasma sample as described in Example 1. The final concentration was determined to be 92.8pg/μl. cfDNA contained in 5μl of purified cfDNA was amplified in a reaction volume of 50μl containing 7.5μl of a luM primer mix (Table 1), 10μl of NEB 5X Mastermix and 27 μl water. Thermal cycling was performed with the Gene Amp9700 (Applied Biosystems). Using the following cycling conditions: incubating at 95°C for 1 minute, followed by 30 cycles at 95°C for 20 seconds, 68°C for 1 minute, and 68°C for 30s, which was followed by a final incubation at 68°C for 5 minutes. A final hold at 4°C was added until the samples were removed for combining with the unamplified portion of the purified cfDNA sample. The amplified product was purified using the Agencourt AMPure XP PCR purification system (Part No. A63881; Beckman Coulter Genomics, Danvers, MA), and the concentration quantified using the Nanodrop 2000 (Thermo Scientific, Wilmington, DE). The purified amplification product was diluted 1:10 in water and 0.9 μl (371pg) added to 40μl of purified cfDNA sample to obtain a 10% spike. The enriched fetal and maternal cfDNA present in the purified cfDNA sample was used for preparing a sequencing library, and was sequenced as described in Example 2.

Table 5 provides the tag counts obtained for each of chromosomes 21, 18, 13, X and Y *i.e.* sequence tag density, and the tag counts obtained for the informative polymorphic sequences contained in the SNP reference genome *.i.e.* SNP tag density. The data show that sequencing information can be obtained from sequencing a single library constructed from a purified maternal cfDNA sample that has been enriched for sequences comprising SNPs to simultaneously determine the presence or absence of aneuploidy and the fetal fraction. The presence or absence of aneuploidy was determined using the number of tags mapped to chromosomes as described in U.S. Provisional Applications 61/407,017 and 61/455,849. In the example given, the data show that the fraction of fetal DNA in plasma sample AFR105 was quantifiable from the sequencing results of five informative SNPs and determined to be 3.84%. Sequence tag densities are provided for chromosomes 21, 13, 18, X and Y.

The example shows that the enrichment protocol provides the requisite tag counts for determining aneuploidy and fetal fraction from a single sequencing process.

**TABLE 5**

| **Determination of Fetal Fraction by Massively Parallel Sequencing: Enrichment of Fetal and Maternal Nucleic Acids for Polymorphic Nucleic Acids in a Purified cfDNA sample** | | | | | |
|---|---|---|---|---|---|
| **Aneuploidy** | | | | | |
| | Chromosome 21 | Chromosome 18 | Chromosome 13 | Chromosome X | Chromosome Y |
| Sequence Tag Density | 178763 | 359529 | 388204 | 572330 | 2219 |
| Karyotype | Unaffected | Unaffected | Unaffected | Unaffected | Unaffected |
| **Fetal Fraction** | | | | | |
| **SNP** | | **SNP TAG DENSITY** | | **FETAL FRACTION (%)** | |
| rs10773760.1\|Chr. 12\|length=128\|allele=A | | 18903 | | 2.81 | |
| rs10773760.2\|Chr. 12\|length=128\|allele=G | | 532 | | | |
| rs1109037.1\|Chr. 2\|length=126\|allele=A | | 347 | | 5.43 | |
| rs1109037.2\|Chr. 2\|length=126\|allele=G | | 6394 | | | |
| rs2567608.1\|Chr. 20\|length=110\|allele=A | | 94503 | | 1.74 | |
| rs2567608.2\|Chr. 20\|length=110\|allele=G | | 1649 | | | |
| rs7041158.1\|Chr. 9\|length=117\|allele=C | | 107 | | 5.61 | |
| rs7041158.2\|Chr. 9\|length=117\|allele=T | | 6 | | | |
| rs8078417.1\|Chr. 17\|length=110\|allele=C | | 162668 | | 3.61 | |
| rs8078417.2\|Chr. 17\|length=110\|allele=T | | 5877 | | | |
| **Fetal Fraction (Mean±S.D.) = 3.8±1.7** | | | | | |

### Example 7

### Determination of Fetal Fraction by Massively Parallel Sequencing: Enrichment of Fetal and Maternal Nucleic Acids for Polymorphic Nucleic Acids in a Plasma Sample.

To enrich the fetal and maternal cfDNA contained in an original plasma sample derived from a pregnant woman, a portion the original plasma sample was used for amplifying polymorphic target nucleic acid sequences each comprising one SNP chosen from the panel of SNPs given in Table 1, and a portion of the amplified product was combined with the remaining original plasma sample.

The method corresponds to workflow 2 diagrammed in Figure 3. cfDNA contained in 15μl of cell-free plasma was amplified in a reaction volume of 50μl containing 9ul of a 1 μM mixture of primers (15 plexTable 1), 1μl of Phusion

blood DNA polymerase, 25ul of the 2X Phusion blood PCR buffer containing deoxynucleotide triphosphates (dNTPs: dATP, dCTP, dGTP and dTTP). Thermal cycling was performed with the Gene Amp9700 (Applied Biosystems) using the following cycling conditions: incubating at 95°C for 3 minutes, followed by 35 cycles at 95°C for 20 seconds, 55°C for 30s, and 70°C for 1 minute, which was followed by a final incubation at 68°C for 5 minutes. A final hold at 4°C was added until the samples were removed for combining with the unamplified portion of the cell-free plasma. The amplified product was diluted 1:2 with water and analyzed using the Bioanalyzer. An additional 3 μl of amplified product was diluted with 11.85 μl of water to obtain a final concentration of 2ng/μl. 2.2μl of the diluted amplified product was combined with the remaining plasma sample. The enriched fetal and maternal cfDNA present in the plasma sample was purified as described in Example 1, and used for preparing a sequencing library. Sequencing and analysis of the sequencing data was performed as described in Example 2.

The results are given in Table 6. In the example given, the data show that the fraction of fetal DNA in plasma sample SAC2517 was quantifiable from the sequencing results of one informative SNP and determined to be 9.5%. In the example given, sample SAC2517 was shown by karyotyping to be unaffected for aneuploidies of chromosomes 21, 13, 18, X and Y. Sequence tag densities are provided for chromosomes 21, 13, 18, X and Y. The presence or absence of aneuploidy was determined using tag counts as described in U.S. Provisional Applications 61/407,017 and 61/455,849.

The example demonstrates that enriching the mixture of fetal and maternal cfDNA present in a plasma sample for nucleic acid sequences that comprise at least one informative SNP can be used to provide the requisite sequence and SNP tag counts for determining aneuploidy and fetal fraction from a single sequencing process by massively parallel sequencing a library prepared from cfDNA contained in a plasma sample that is enriched for polymorphic nucleic acids.

**TABLE 6**

| **Determination of Fetal Fraction by Massively Parallel Sequencing: Enrichment of Fetal and Maternal Nucleic Acids for Polymorphic Nucleic Acids Comprising a SNP in a Plasma Sample** | | | | | |
|---|---|---|---|---|---|
| **Aneuploidy** | | | | | |
| | Chromosome 21 | Chromosome 18 | Chromosome 13 | Chromosome X | Chromosome Y |
| Sequence Tag Density | 183851 | 400582 | 470526 | 714055 | 2449 |
| Karyotype | Unaffected | Unaffected | Unaffected | Unaffected | Unaffected |
| **Fetal Fraction** | | | | | |
| **SNP** | | **TAG COUNTS** | **FETAL FRACTION (%)** | | |
| rs10773760.1\|Chr. 12\|length=128\|allele=A | | 8536 | 9.5 | | |
| rs10773760.2\|Chr. 12\|length=128\|allele=G | | 89924 | | | |

## Example 8

### Determination of Fetal Fraction by Massively Parallel Sequencing of Samples Comprising Amplified Polymorphic Sequences: Tandem SNPs

To determine the fraction of fetal cfDNA in a maternal sample, target polymorphic nucleic acid sequences each comprising a pair of tandem SNPs are amplified and used for preparing a target library for sequencing in a massively parallel fashion. Pairs of tandem SNPs can be selected from rs7277033-rs2110153; rs2822654-rs1882882; rs368657-rs376635; rs2822731-rs2822732; rs1475881-rs7275487; rs1735976-rs2827016; rs447340-rs2824097; rs418989-rs13047336; rs987980- rs987981; rs4143392- rs4143391; rs1691324-rs13050434; rs11909758-rs9980111; rs2826842-rs232414; rs1980969-rs1980970; rs9978999-rs9979175; rs1034346-rs12481852; rs7509629-rs2828358; rs4817013-rs7277036; rs9981121-rs2829696; rs455921-rs2898102; rs2898102- rs458848; rs961301-rs2830208; rs2174536-rs458076; rs11088023-rs11088024; rs1011734-rs1011733; rs2831244-rs9789838; rs8132769-rs2831440; rs8134080-rs2831524; rs4817219-rs4817220; rs2250911-rs2250997; rs2831899-rs2831900; rs2831902-rs2831903; rs11088086-rs2251447; rs2832040-rs11088088; rs2832141-rs2246777; rs2832959 -rs9980934; rs2833734-rs2833735; rs933121-rs933122; rs2834140-rs12626953; rs2834485-rs3453; rs9974986-rs2834703; rs2776266-rs2835001; rs1984014-rs1984015; rs7281674-rs2835316; rs13047304-rs13047322; rs2835545-rs4816551; rs2835735-rs2835736; rs13047608-rs2835826; rs2836550-rs2212596; rs2836660-rs2836661; rs465612-rs8131220; rs9980072-rs8130031;

rs418359-rs2836926; rs7278447-rs7278858; rs385787-rs367001; rs367001-rs386095; rs2837296-rs2837297; and rs2837381-rs4816672. The primers used for amplifying the target sequences comprising the tandem SNPs are designed to encompass both SNP sites. For example, the forward primer is designed to encompass the first SNP, and the reverse primer is designed to encompass the second of the tandem SNP pair *i.e.* each of the SNP sites in the tandem pair is encompassed within the 36 bp generated by the sequencing method. Paired-end sequencing can be used to identify all sequences encompassing the tandem SNP sites. Exemplary sets of primers that are used to amplify the tandem SNPs disclosed herein are rs7277033-rs2110153_F: TCCTGGAAACAAAAGTATT (SEQ ID NO:197) and rs7277033-rs2110153_R: AACCTTACAACAAAGCTAGAA (SEQ ID NO:198), set rs2822654-rs1882882_F: ACTAAGCCTTGGGGATCCAG (SEQ ID NO:199) and rs2822654-rs1882882_R: TGCTGTGGAAATACTAAAAGG (SEQ ID NO:200), set rs368657-rs376635_F:CTCCAGAGGTAATCCTGTGA (SEQ ID NO:201) and rs368657-rs376635_R:TGGTGTGAGATGGTATCTAGG (SEQ ID NO:202), rs2822731-rs2822732_F:GTATAATCCATGAATCTTGTTT (SEQ ID NO:203) and rs2822731-rs2822732_R:TTCAAATTGTATATAAGAGAGT (SEQ ID NO:204), rs1475881-rs7275487_F:GCAGGAAAGTTATTTTTAAT (SEQ ID NO:205) and rs1475881-rs7275487_R:TGCTTGAGAAAGCTAACACTT (SEQ ID NO:206), rs1735976-rs2827016F:CAGTGTTTGGAAATTGTCTG (SEQ ID NO:207) and rs1735976-rs2827016_R:GGCACTGGGAGATTATTGTA (SEQ ID NO:208), rs447349-rs2824097_F:TCCTGTTGTTAAGTACACAT (SEQ ID NO:209) and rs447349-rs2824097_R:GGGCCGTAATTACTTTTG (SEQ ID NO:210), rs418989-rs13047336_F:ACTCAGTAGGCACTTTGTGTC (SEQ ID NO:211) and rs418989-rs13047336_R:TCTTCCACCACACCAATC (SEQ ID NO:212), rs987980-rs987981_F:TGGCTTTTCAAAGGTAAAA (SEQ ID NO:213) and rs987980-rs987981_R: GCAACGTTAACATCTGAATTT (SEQ ID NO:214), rs4143392- rs4143391_F: rs4143392- rs4143391 (SEQ ID NO:215) and rs4143392- rs4143391_R:ATTTTATATGTCATGATCTAAG (SEQ ID NO:216), rs1691324-rs13050434_F: AGAGATTACAGGTGTGAGC (SEQ ID NO:217) and rs1691324- rs13050434_R: ATGATCCTCAACTGCCTCT (SEQ ID NO:218), rs11909758-rs9980111_F: TGAAACTCAAAAGAGAAAAG (SEQ ID NO:219) and rs11909758-rs9980111_R: ACAGATTTCTACTTAAAATT (SEQ ID NO:220), rs2826842-rs232414_F: TGAAACTCAAAAGAGAAAAG (SEQ ID NO:221) and rs2826842-rs232414_R: ACAGATTTCTACTTAAAATT (SEQ ID NO:22), rs2826842-rs232414_F: GCAAAGGGGTACTCTATGTA (SEQ ID NO:223) and rs2826842-rs232414_R: TATCGGGTCATCTTGTTAAA (SEQ ID NO:224), rs1980969-rs1980970_F: TCTAACAAAGCTCTGTCCAAAA (SEQ ID NO:225) and rs1980969-rs1980970_R: CCACACTGAATAACTGGAACA (SEQ ID NO:226), rs9978999-rs9979175_F: GCAAGCAAGCTCTCTACCTTC (SEQ ID NO:227) and rs9978999-rs9979175_R: TGTTCTTCCAAAATTCACATGC (SEQ ID NO:228), rs1034346-rs12481852_F: ATTTCACTATTCCTTCATTTT (SEQ ID NO:229) and rs1034346-rs12481852_R: TAATTGTTGCACACTAAATTAC (SEQ ID NO:230), rs4817013-rs7277036_F: AAAAAGCCACAGAAATCAGTC (SEQ ID NO:231) and rs4817013-rs7277036_R: TTCTTATATCTCACTGGGCATT (SEQ ID NO:232), rs9981121-rs2829696_F: GGATGGTAGAAGAGAAGAAAGG (SEQ ID NO:233) and rs9981121-rs2829696_R: GGATGGTAGAAGAGAAGAAAGG (SEQ ID NO:234), rs455921-rs2898102_F: TGCAAAGATGCAGAACCAAC (SEQ ID NO:235) and rs455921-rs2898102_R: TTTTGTTCCTTGTCCTGGCTGA (SEQ ID NO:236), rs2898102- rs458848_F: TGCAAAGATGCAGAACCAAC (SEQ ID NO:237) and rs2898102- rs458848_R: GCCTCCAGCTCTATCCAAGTT (SEQ ID NO:238), rs961301-rs2830208_F: CCTTAATATCTTCCCATGTCCA (SEQ ID NO:239) and rs961301-rs2830208_R: ATTGTTAGTGCCTCTTCTGCTT (SEQ ID NO:240), rs2174536-rs458076_F: GAGAAGTGAGGTCAGCAGCT (SEQ ID NO:241) and rs2174536-rs458076_R: TTTCTAAATTTCCATTGAACAG (SEQ ID NO:242), rs11088023-rs11088024_F: GAAATTGGCAATCTGATTCT (SEQ ID NO:243) and rs11088023-rs11088024_R: CAACTTGTCCTTTATTGATGT (SEQ ID NO:244), rs1011734-rs1011733_F: CTATGTTGATAAAACATTGAAA (SEQ ID NO:245) and rs1011734-rs1011733_R: GCCTGTCTGGAATATAGTTT (SEQ ID NO:246), rs2831244-rs9789838_F: CAGGGCATATAATCTAAGCTGT (SEQ ID NO:247) and rs2831244-rs9789838_R: CAATGACTCTGAGTTGAGCAC (SEQ ID NO:248), rs8132769-rs2831440_F: ACTCTCTCCCTCCCCTCT (SEQ ID NO:249) and rs8132769-rs2831440_R: TATGGCCCCAAAACTATTCT (SEQ ID NO:250), rs8134080-rs2831524_F: ACAAGTACTGGGCAGATTGA (SEQ ID NO:251) and rs8134080-rs2831524_R: GCCAGGTTTAGCTTTCAAGT (SEQ ID NO:252), rs4817219-rs4817220_F: TTTTATATCAGGAGAAACACTG (SEQ ID NO:253) and rs4817219-rs4817220_R: CCAGAATTTTGGAGGTTTAAT (SEQ ID NO:254), rs2250911-rs2250997_F: TGTCATTCCTCCTTTATCTCCA (SEQ ID NO:255) and rs2250911-rs2250997_R: TTCTTTTGCCTCTCCCAAAG (SEQ ID NO:256), rs2831899-rs2831900_F: ACCCTGGCACAGTGTTGACT (SEQ ID NO:257) and rs2831899-rs2831900_R: TGGGCCTGAGTTGAGAAGAT (SEQ ID NO:258), rs2831902-rs2831903_F: AATTTGTAAGTATGTGCAACG (SEQ ID NO:259) and rs2831902-rs2831903_R: TTTTTCCCATTTCCAACTCT (SEQ ID NO:260), rs11088086-rs2251447_F: AAAAGATGAGACAGGCAGGT (SEQ ID NO:261) and rs11088086-rs2251447 _R: ACCCCTGTGAATCTCAAAAT (SEQ ID NO:262), rs2832040-rs11088088_F: GCACTTGCTTCTATTGTTTGT (SEQ ID NO:263) and rs2832040-rs11088088_R: CCCTTCCTCTCTTCCATTCT (SEQ ID NO:264), rs2832141-rs2246777_F: AGCACTGCAGGTA (SEQ ID NO:265) and rs2832141-rs2246777_R: ACAGATACCAAAGAACTGCAA (SEQ ID NO:266), rs2832959 - rs9980934_F: TGGACACCTTTCAACTTAGA (SEQ ID NO:267) and rs2832959 -rs9980934_R: GAACAGTAATGTTGAACTTTTT (SEQ ID NO:268), rs2833734-rs2833735_F: TCTTGCAAAAAGCTTAGCACA (SEQ ID NO:269) and rs2833734-rs2833735_R: AAAAAGATCTCAAAGGGTCCA (SEQ ID NO:270), rs933121-rs933122_F: GCTTTTGCTGAACATCAAGT (SEQ ID NO:271) and rs933121-rs933122_R: CCTTCCAGCAGCATAGTCT (SEQ ID NO:272), rs2834140-rs12626953_F: AAATCCAGGATGTGCAGT (SEQ ID NO:273) and rs2834140-rs12626953_R: ATGATGAGGTCAGTGGTGT (SEQ ID NO:274), rs2834485-rs3453_F: CATCACAGATCATAGTAAATGG (SEQ ID NO:275) and rs2834485-rs3453_R: AATTATTATTTTGCAGGCAAT (SEQ ID NO:276), rs9974986-rs2834703_F: CATGAGGCAAACACCTTTCC (SEQ ID NO:277) and rs9974986-rs2834703_R: GCTGGACTCAGGATAAAGAACA (SEQ ID NO:278), rs2776266-rs2835001_F: TGGAAGCCTGAGCTGACTAA (SEQ ID NO:279) and rs2776266-rs2835001_R:CCTTCTTTTCCCCCAGAATC (SEQ ID NO:280), rs1984014-rs1984015-F:TAGGAGAACAGAAGATCAGAG (SEQ ID NO:281) and rs1984014-rs1984015_R:AAAGACTATTGCTAAATGCTTG (SEQ ID NO:282), rs7281674-rs2835316_F: TAAGCGTAGGGCTGTGTGTG (SEQ ID NO:283) and rs7281674-rs2835316_R: GGACGGATAGACTCCAGAAGG (SEQ ID NO:284), rs13047304-rs13047322_F: GAATGACCTTGGCACTTTTATCA (SEQ ID NO:285) and rs13047304-rs13047322_R: AAGGATAGAGATATACAGATGAATGGA (SEQ ID NO:286), rs2835735-rs2835736_F: CATGCACCGCGCAAATAC (SEQ ID NO:287) and rs2835735-rs2835736_R: ATGCCTCACCCACAAACAC (SEQ ID NO:288), rs13047608-rs2835826_F: TCCAAGCCCTTCTCACTCAC (SEQ ID NO:289) and rs13047608-rs2835826_R: CTGGGACGGTGACATTTTCT (SEQ ID NO:290), rs2836550-rs2212596_F: CCCAGGAAGAGTGGAAAGATT (SEQ ID NO:291) and rs2836550-rs2212596_R: TTAGCTTGCATGTACCTGTGT (SEQ ID NO:292), rs2836660-rs2836661_F: AGCTAGATGGGGTGAATTTT (SEQ ID NO:293) and_R: TGGGCTGAGGGGAGATTC (SEQ ID NO:294), rs465612-rs8131220_F: ATCAAGCTAATTAATGTTATCT (SEQ ID NO:295) and rs465612-rs8131220_R: AATGAATAAGGTCCTCAGAG (SEQ ID NO:296), rs9980072-rs8130031_F:TTTAATCTGATCATTGCCCTA (SEQ ID NO:297) and rs9980072-rs8130031_R: AGCTGTGGGTGACCTTGA (SEQ ID NO:298), rs418359-rs2836926_F: TGTCCCACCATTGTGTATTA (SEQ ID NO:299) and rs418359-rs2836926_R: TCAGACTTGAAGTCCAGGAT (SEQ ID NO:300), rs7278447-rs7278858_F: GCTTCAGGGGTGTTAGTTTT (SEQ ID NO:301) and rs7278447-rs7278858_R: CTTTGTGAAAAGTCGTCCAG (SEQ ID NO:302), rs385787-rs367001_F:CCATCATGGAAAGCATGG (SEQ ID NO:303) and rs385787-rs367001_R: TCATCTCCATGACTGCACTA (SEQ ID NO:304), rs367001-rs386095_F: GAGATGACGGAGTAGCTCAT (SEQ ID NO:305) and rs367001-rs386095_R: CCCAGCTGCACTGTCTAC (SEQ ID NO:306), rs2837296-rs2837297_F: TCTTGTTCCAATCACAGGAC (SEQ ID NO:307) and rs2837296-rs2837297_R: ATGCTGTTAGCTGAAGCTCT (SEQ ID NO:308), and rs2837381-rs4816672_F: TGAAAGCTCCTAAAGCAGAG (SEQ ID NO:309) and rs2837381-rs4816672_R:TTGAAGAGATGTGCTATCAT (SEQ ID N0:310). Polynucleotide sequences e.g. GC clamp sequences, can be included to ensure specific hybridization of AT-rich primers (Ghanta et al., PLOS ONE 5(10): doi10.1371/joumal.pone.0013184 [2010], available on the world wide web at plosone.org). An example of a GC clamp sequence that can be included either 5' of the forward primer or 3' of the reverse primer is GCCGCCTGCAGCCCGCGCCCCCCGTGCCCCCGCCCCGCCGCCGGCCCGGGCGCC (SEQ ID NO:311). Polymorphic sequences can be used alone or in combination with unamplified cfDNA to determine either fetal fraction or the presence or absence of aneuploidy and fetal fraction in a maternal sample as described for polymorphic SNP sequences. Sample preparation and enrichment of cfDNA sequencing library, a purified cfDNA sample, and a plasma sample is performed according to the method described in Examples 5, 6, and 7, respectively. All sequencing libraries are prepared as described in Example 2a., and sequencing is performed as described in Example 2b. Analysis of the sequencing data for the determination of fetal aneuploidy is performed as described in Example 5. Concomitant to the analysis for determining aneuploidy, the sequencing data is analyzed to determine the fetal fraction as follows. Following the transfer of the image and base call files to the Unix server running the Illumina "Genome Analyzer Pipeline" software version 1.51 as described in Example 3a., the 36bp reads are aligned to a 'tandem SNP genome' using the BOWTIE program. The tandem SNP genome is identified as the grouping of the DNA sequences that encompass the alleles of the 58 tandem SNP pairs disclosed above. Only reads that mapped uniquely to the tandem SNP genome are used for the analysis of fetal fraction. Reads that match perfectly to the tandem SNP genome are counted as tags and filtered. Of the remaining reads, only reads having one or two mismatches are counted as tags and included in the analysis. Tags mapped to each of the tandem SNP alleles are counted, and the fetal fraction is determined essentially as described in Example 6 above but accounting for tags mapped to the two tandem SNP alleles x and y present on each of the amplified polymorphic target nucleic acid sequences that are amplified to enrich the samples *i.e.*

$$\%\ \text{fetal fraction allele}_{x+y} = ((\sum \text{Fetal sequence tags for allele}_{x+y}) / (\sum \text{Maternal sequence tags for allele}_{x+y})) \times 100$$

Only informative tandem SNPs are used to determine the fetal fraction.

Optionally, the fraction of fetal nucleic acids in the mixture of fetal and maternal nucleic acids is calculated for each of the informative allele ($\text{allele}_x+_y$) as follows:

$$\%\ \text{fetal fraction allele}_{x+y} = ((2 \text{ X } \sum \text{Fetal sequence tags for allele}_{x+y}) / (\sum \text{Maternal sequence tags for allele}_{x+y})) \times 100,$$

to compensate for the presence of 2 sets of tandem fetal alleles, one being masked by the maternal background.

The percent fetal fraction is calculated for at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, or more informative sets of tandem alleles. In one embodiment, the fetal fraction is the average fetal fraction determined for at least 3 informative sets of tandem alleles.

**Example 9**

**Determination of Fetal Fraction by Massively Parallel Sequencing of Samples Comprising Amplified Polymorphic Sequences: STRs**

To determine the fraction of fetal cfDNA in a maternal sample, target polymorphic nucleic acid sequences each comprising an STR are amplified and used for preparing a target library for sequencing in a massively parallel fashion.

Peripheral blood samples are obtained from pregnant subjects, and cfDNA is purified from the plasma fraction as described in Examples 1 and 2 STRs that are amplified are chosen from the codis and non-codis STRs disclosed in Table 7, and amplification of the polymorphic STR sequences is obtained using the corresponding sets of primers provided. For example, the STRs listed in Table 7 are amplified using the corresponding primers (SEQ ID NOs: 113-197), and the amplified product is used to generate a target sequencing library. The STR target sequencing library is prepared as described for the preparation of the SNP target library as described in Example 8. STRs CSF1PO, D13S317, D16S539, D18S51, D21S11, D2S1338D7S820, and FGA have been analyzed previously for determining fetal fraction, and are disclosed in US Provisional applications 61/296,358 and 61/360,837.

**TABLE 7 CODIS and NON-CODIS miniSTRs**

| STR Locus (Marker Name) | Chromosome Location | Size Range (bp) | GenBank Accession | Primer Sequences (Forward/Reverse) |
|---|---|---|---|---|
| **Codis miniS TR loci*** | | | | |
| CSF1PO | 5q33.1 | 89-129 | X14720 | ACAGTAACTGCCTTCATAGATAG(CSF1PO_F; SEQ ID NO:113)<br>GTGTCAGACCCTGTTCTAAGTA(CSF1PO_R; SEQ ID NO:114) |
| FGA | 4q31.3 | 125-281 | M64982 | AAATAAAATTAGGCATATTTACAAGC(FGA_F; SEQ ID NO:115)<br>GCTGAGTGATTTGTCTGTAATTG(FGA_R; SEQ ID NO:116) |
| TH01 | 11p15.5 | 51-98 | D00269 | CCTGTTCCTCCCTTATTTCCC(TH01_F; SEQ ID NO:117)<br>GGGAACACAGACTCCATGGTG(TH01_R; SEQ ID NO:118) |
| TPOX | 2p25.3 | 65-101 | M68651 | CTTAGGGAACCCTCACTGAATG(TPOX_F; SEQ ID NO:119)<br>GTCCTTGTCAGCGTTTATTTGC(TPOX_R; SEQ ID NO:120) |
| vWA | 12p13.31 | 88-148 | M25858 | AATAATCAGTATGTGACTTGGATTGA(vWA_F; SEQ ID NO:121)<br>ATAGGATGGATGGATAGATGGA(vWA_R; SEQ ID NO:122) |
| D3S1358 | 3p21.31 | 72-120 | NT_005997 | CAGAGCAAGACCCTGTCTCAT(D3S1358_F; SEQ ID NO:123)<br>TCAACAGAGGCTTGCATGTAT(D3S1358_R; SEQ ID NO:124) |

(continued)

| STR Locus (Marker Name) | Chromosome Location | Size Range (bp) | GenBank Accession | Primer Sequences (Forward/Reverse) |
|---|---|---|---|---|
| **Codis miniS TR loci*** | | | | |
| D5S818 | 5q23.2 | 81-117 | AC008512 | GGGTGATTTTCCTCTTTGGT(D5S818_F; SEQ ID NO:125)<br>AACATTTGTATCTTTATCTGTATCCTTAT (D5S818_R; SEQ ID NO:126) |
| D7S820 | 7q21.11 | 136-176 | AC004848 | GAACACTTGTCATAGTTTAGAACGAAC ( D7S820 F; SEQ ID NO: 127)<br>TCATTGACAGAATTGCACCA(D7S820_R; SEQ ID NO: 128) |
| D8S1179 | 8q24.13 | 86-134 | AF216671 | TTTGTATTTCATGTGTACATTCGTATC(D 7S820 F; SEQ ID NO: 129)<br>ACCTATCCTGTAGATTATTTTCACTGTG ( D7S820 _R; SEQ ID NO: 130) |
| D13S317 | 13q31.1 | 88-132 | AL353628 | TCTGACCCATCTAACGCCTA(D13S317_F; SEQ ID NO: 131)<br>CAGACAGAAAGATAGATAGATGATTGA ( D13S317 R; SEQ ID NO: 132) |
| D16S539 | 16q24.1 | 81-121 | AC024591 | ATACAGACAGACAGACAGGTG(D 16S539 _F; SEQ ID NO: 133)<br>GCATGTATCTATCATCCATCTCT(D 16S53 9_R; SEQ ID NO: 134) |
| D18S51 | 18q21.33 | 113-193 | AP001534 | TGAGTGACAAATTGAGACCTT(D18S51_F ; SEQ ID NO: 135)<br>GTCTTACAATAACAGTTGCTACTATT(D1 8S51_R; SEQ ID NO: 136) |
| D21S11 | 21q21.1 | 153-221 | AP000433 | ATTCCCCAAGTGAATTGC(D21S11_F; SEQ ID NO:137)<br>GGTAGATAGACTGGATAGATAGACGA(D 21S11_R; SEQ ID NO:138) |
| D2S1338 | 2q35 | 90-142 | AC01036 | TGGAAACAGAAATGGCTTGG(D2S1338_F ; SEQ ID NO:139)<br>GATTGCAGGAGGGAAGGAAG(D2S1338_ R; SEQ ID NO:140) |
| Penta D | 21q22.3 | 94-167 | AP001752 | GAGCAAGACACCATCTCAAGAA(Penta D_F; SEQ ID NO:141)<br>GAAATTTTACATTTATGTTTATGATTCTC T (Penta D_R; SEQ ID NO:142) |
| Penta E | 15q26.2 | 80-175 | AC027004 | GGCGACTGAGCAAGACTC(Penta E_F; SEQ ID NO:143)<br>GGTTATTAATTGAGAAAACTCCTTACA( Penta E_R; SEQ ID NO:144) |
| **Non-Codis miniSTR loci*** | | | | |
| D22S1045 | 22q12.3 | 82-115 | AL022314 (17) | ATTTTCCCCGATGATAGTAGTCT(D22S10 45_F; SEQ ID NO: 145) |

(continued)

| Non-Codis miniSTR loci* | | | | |
|---|---|---|---|---|
| | | | | GCGAATGTATGATTGGCAATATTTTT(D2 2S1045 R; SEQ ID NO: 146) |
| D20S1082 | 20q13.2 | 73 - 101 | AL158015 | ACATGTATCCCAGAACTTAAAGTAAAC ( D20S1082 F; SEQ ID NO:147) GCAGAAGGGAAAATTGAAGCTG(D20S 10 82_R; SEQ ID NO: 148) |
| D20S482 | 20pl3 | 85 - 126 | AL121781 (14) | CAGAGACACCGAACCAATAAGA(D20S482_F; SEQ ID NO:149) GCCACATGAATCAATTCCTATAATAAA ( D20S482 R; SEQ ID NO: 150) |
| D18S853 | 18p11.31 | 82 - 104 | AP005130 (11) | GCACATGTACCCTAAAACTTAAAAT(D18 S853_F; SEQ ID NO:151) GTCAACCAAAACTCAACAAGTAGTAA(D 18S853_ R; SEQ ID NO:152) |
| D17S1301 | 17q25.1 | 114 - 139 | AC016888 (12) | AAGATGAAATTGCCATGTAAAAATA(D1 7S1301_F; SEQ ID NO:153) GTGTGTATAACAAAATTCCTATGATGG ( D17S1301_R; SEQ ID NO:154) |
| D17S974 | 17p13.1 | 114 - 139 | AC034303 (10) | GCACCCAAAACTGAATGTCATA(D 17S974_F; SEQ ID NO:155) GGTGAGAGTGAGACCCTGTC(D 17S974 R ; SEQ ID NO: 156) |
| D14S1434 | 14q32.13 | 70 - 98 | AL121612 (13) | TGTAATAACTCTACGACTGTCTGTCTG(D 14S1434_F; SEQ ID NO:157) GAATAGGAGGTGGATGGATGG(D 14S 143 4_R; SEQ ID NO:158) |
| D12ATA63 | 12q23.3 | 76 - 106 | AC009771 (13) | GAGCGAGACCCTGTCTCAAG(D 12ATA63 _F; SEQ ID NO:159) GGAAAAGACATAGGATAGCAATTT(D12 ATA63 R; SEQ ID NO:160) |
| D11S4463 | 11q25 | 88 - 116 | AP002806 (14) | TCTGGATTGATCTGTCTGTCC(D 11S4463_ F; SEQ ID NO: 161) GAATTAAATACCATCTGAGCACTGAA(D 11S4463_R; SEQ ID NO:162) |
| D10S1435 | 10p15.3 | 82 - 139 | AL354747 (11) | TGTTATAATGCATTGAGTTTTATTCTG(D 10S1435_ F; SEQ ID NO:163) GCCTGTCTCAAAAATAAAGAGATAGAC A (D10S1435_R; SEQ ID NO:164) |
| D10S1248 | 10q26.3 | 79 - 123 | AL391869 (13) | TTAATGAATTGAACAAATGAGTGAG(D1 OS1248_ F; SEQ ID NO:165) |
| | | | | GCAACTCTGGTTGTATTGTCTTCAT(D10S 1248_R; SEQ ID NO:166) |
| D9S2157 | 9q34.2 | 71 - 107 | AL162417 (10) | CAAAGCGAGACTCTGTCTCAA(D9S2157_ F; SEQ ID NO:167) GAAAATGCTATCCTCTTTGGTATAAAT ( D9S2157 R; SEQ ID NO:168) |

(continued)

| Non-Codis miniSTR loci* | | | | |
|---|---|---|---|---|
| D9S1122 | 9q21.2 | 93 - 125 | AL161789 (12) | GGGTATTTCAAGATAACTGTAGATAGG ( D9S1122 F; SEQ ID NO:168) GCTTCTGAAAGCTTCTAGTTTACC(D9S 11 22_R; SEQ ID NO:170) |
| D8S1115 | 8p11.21 | 63 - 96 | AC090739 (9) | TCCACATCCTCACCAACAC(D8S1115_F; SEQ ID NO:171) GCCTAGGAAGGCTACTGTCAA(D8S1115-R; SEQ ID NO: 172) |
| D6S1017 | 6p21.1 | 81 - 110 | AL035588 (10) | CCACCCGTCCATTTAGGC(D6S1017_ F; SEQ ID NO:173) GTGAAAAAGTAGATATAATGGTTGGTG ( D6S1017 R; SEQ ID NO:174) |
| D6S474 | 6q21 | 107 - 136 | AL357514 (17) | GGTTTTCCAAGAGATAGACCAATTA(D6S 474_F; SEQ ID NO:175) GTCCTCTCATAAATCCCTACTCATATC(D 6S474 R; SEQ ID NO:176) |
| D5S2500 | 5q11.2 | 85 - 126 | AC008791 (17) | CTGTTGGTACATAATAGGTAGGTAGGT ( D5S2500 F; SEQ ID NO:177) GTCGTGGGCCCCATAAA TC(D5S2500_ _R; SEQ ID NO:178) |
| D4S2408 | 4p15.1 | 85 - 109 | AC110763 (9) | AAGGTACATAACAGTTCAATAGAAAGC ( D4S2408 F; SEQ ID NO:179) GTGAAATGACTGAAAAATAGTAACCA(D 4S2408 R; SEQ ID NO:180) |
| D4S2364 | 4q22.3 | 67 - 83 | AC022317 (9) | CTAGGAGATCATGTGGGTATGATT(D4S2 364U_F; SEQ ID NO:181) GCAGTGAATAAATGAACGAATGGA(D4S 2364_R; SEQ ID NO: 182) |
| D3S4529 | 3p12.1 | 111 - 139 | AC117452 (13) | CCCAAAATTACTTGAGCCAAT(D3 S452_F ; SEQ ID NO:183) GAGACAAAATGAAGAAACAGACAG(D3S 452_R; SEQ ID NO:184) |
| D3S3053 | 3q26.31 | 84 - 108 | AC069259 (9) | TCTTTGCTCTCATGAAT AGATCAGT(D3 S 3053_F; SEQ ID NO:185) |
| | | | | GTTTGTGATAATGAACCCACTCAG(D3 S3 053_R; SEQ ID NO:186) |
| D2S1776 | 2q24.3 | 127 - 161 | AC009475 (11) | TGAACACAGATGTTAAGTGTGTATATG ( D2S1776 F; SEQ ID NO:187) GTCTGAGGTGGACAGTTATGAAA(D2S 17 76_R; SEQ ID NO:188) |
| D2S441 | 2p14 | 78 - 110 | AC079112 (12) | CTGTGGCTCATCTATGAAAACTT(D2S441 _F; SEQ ID NO:189) GAAGTGGCTGTGGTGTT A TGAT(D2S441_ R; SEQ ID NO:190) |
| D1S1677 | 1q23.3 | 81 - 117 | AL513307 (15) | TTCTGTTGGTATAGAGCAGTGTTT(D 1S16 77_F; SEQ ID NO:191) GTGACAGGAAGGACGGAATG(D 1S 1677_ R; SEQ ID NO: 192) |

(continued)

| Non-Codis miniSTR loci* | | | | |
|---|---|---|---|---|
| D1S1627 | 1p21.1 | 81 - 100 | AC093119 (13) | CATGAGGTTTGCAAATACTATCTTAAC ( D1S1627 F; SEQ ID NO:193) GTTTTAATTTTCTCCAAATCTCCA(D 1S16 27_R; SEQ ID NO:194) |
| D1GATA113 | 1p36.23 | 81 - 105 | Z97987 (11) | TCTTAGCCTAGATAGATACTTGCTTCC(D 1GATA113 F; SEQ ID NO:195) GTCAACCTTTGAGGCTATAGGAA(DIGA TA113 R; SEQ ID NO: 196) |
| *(Butler et al., J Forensic Sci 5:1054-1064; Hill et al., Poster #44- 17th International Symposium on Human Identification - 2006) | | | | |

Sequencing of the library enriched for polymorphic STR sequences is performed using a NGS technology *e.g.* sequencing by synthesis. Sequence reads of lengths that encompass the STRs *e.g.* miniSTRs of at least 100 bp, to a reference STR genome consisting of the polymorphic sequences which were amplified in the sample. Informative STR alleles are identified by differences in the length of the repeats, and the number of STR sequence tags are counted, and used to determine the fetal fraction. Optionally, amplification of the polymorphic STR sequences is performed to enrich a plasma sample, a purified cfDNA sample or a cfDNA sequencing library sample, as described in Examples 5, 6, and 7, respectively.

## Example 10

### Determination of Fetal Fraction by Capillary Electrophoresis of Polymorphic Sequences Comprising STRs

To determine fetal fraction in maternal samples comprising fetal and maternal cfDNA, peripheral blood samples were collected from volunteer pregnant women carrying either male or female fetuses. Peripheral blood samples were obtained and processed to provide purified cfDNA as described in Example 1

Ten microliters of cfDNA samples were analyzed using the AmpF1STR® MiniFiler™ PCR amplification kit (Applied Biosystems, Foster City, CA) according to the manufacturer's instructions. Briefly, cfDNA contained in 10 μl was amplified in a reaction volume of 25 μl containing 5μL fluorescently labeled primers (AmpF/STR® MiniFiler™ Primer Set), and the AmF/STR® MiniFiler™ Master Mix, which includes AmpliTaq Gold® DNA polymerase and associated buffer, salt (1.5 mM MgC12), and 200 μM deoxynucleotide triphosphates (dNTPs: dATP, dCTP, dGTP and dTTP). The fluorescently labeled primers are forward primers that are labeled with 6FAM™, VIC™, NED™, and PET™ dyes. Thermal cycling was performed with the Gene Amp9700 (Applied Biosystems) using the following cycling conditions: incubating at 95°C for 10 minutes, followed by 30 cycles at 94°C for 20 seconds, 59°C for 2 minute, and 72°C for 1 minute, which was followed by a final incubation at 60°C for 45 minutes. A final hold at 4°C was added until the samples were removed for analysis. The amplified product was prepared by diluting 1ul of amplified product in 8.7ul Hi-DiTM formamide (Applied Biosystems) and 0.3 μl GeneScanTM-500 LIZ_internal size standard (Applied Biosystems), and analyzed with an ABI PRISM3130xl Genetic Analyzer (Applied Biosystems) using Data Collection HID G5 POP4 (Applied Biosystems), and a 36-cm capillary array. All genotyping was performed with GeneMapper_ID v3.2 software (Applied Biosystems) using manufacturer provided allelic ladders and bins and panels.

All genotyping measurement were performed on the Applied Biosystems 3130xl Genetic Analyzer, using a ±0.5-nt "window" around the size obtained for each allele to allow for detection and correct assignment of alleles. Any sample allele whose size was outside the ±0.5-nt window was determined to be OL *i.e.* "Off Ladder". OL alleles are alleles of a size that is not represented in the AnmF/STR® MiniFiler™ Allelic Ladder or an allele that does not correspond to an allelic ladder, but whose size is just outside a window because of measurement error. The minimum peak height threshold of >50 RFU was set based on validation experiments performed to avoid typing when stochastic effects are likely to interfere with accurate interpretation of mixtures. The calculation of fetal fraction is based on averaging all informative markers. Informative markers are identified by the presence of peaks on the electropherogram that fall within the parameters of preset bins for the STRs that are analyzed.

Calculations of fetal fraction were performed using the average peak height for major and minor alleles at every STR locus determined from triplicate injections. The rules applied to the calculation are:

1. off-ladder allele (OL) data for alleles are not included in the calculation; and

2. only peak heights derived from >50 RFU (relative fluorescence units) are included in the calculation
3. if only one bin is present the marker is deemed non-informative; and
4. if a second bin is called but the peaks of the first and second bins are within 50-70% of their relative fluorescence units (RFU) in peak height, the minority fraction is not measured and the marker is deemed not informative.

The fraction of the minor allele for any given informative marker is calculated by dividing the peak height of the minor component by the sum of the peak height for the major component, and expressed as a percent was first calculated for each informative locus as

$$\textbf{fetal fraction} = (\sum \textbf{peak height of minor allele} / \sum \textbf{peak height of major allele(s)}) \times 100,$$

The fetal fraction for a sample comprising two or more informative STRs, would be calculated as the average of the fetal fractions calculated for the two or more informative markers.

Table 8 provides the data obtained from analyzing cfDNA of a subject pregnant with a male fetus.

**TABLE 8 Fetal Fraction Determined in cfDNA of a Pregnant Subject by Analysis of STRs**

| STR | Allele 1 | Allele 2 | Allele 3 | Allele 1 Height | Allele 2 Height | Allele 3 Height | Fetal Fraction | Fetal Fraction (Mean/STR) |
|---|---|---|---|---|---|---|---|---|
| AMEL | X | Y | | 3599 | 106 | | 2.9 | |
| AMEL | X | Y | | 3602 | 110 | | 3.1 | |
| AMEL | X | Y | | 3652 | 109 | | 3.0 | 3.0 |
| CSF1PO | 11 | 12 | | 2870 | 2730 | | | |
| CSF1PO | 11 | 12 | | 2924 | 2762 | | | |
| CSF1PO | 11 | 12 | | 2953 | 2786 | | | |
| D13S317 | 11 | 12 | | 2621 | 2588 | | | |
| D13S317 | 11 | 12 | | 2680 | 2619 | | | |
| D13S317 | 11 | 12 | | 2717 | 2659 | | | |
| D16S539 | 9 | 11 | | 1056 | 1416 | | | |
| D16S539 | 9 | 11 | | 1038 | 1394 | | | |
| D16S539 | 9 | 11 | | 1072 | 1437 | | | |
| D18S51 | 13 | 15 | | 2026 | 1555 | | | |
| D18S51 | 13 | 15 | | 2006 | 1557 | | | |
| D18S51 | 13 | 15 | | 2050 | 1578 | | | |
| D21S11 | 28 | 31.2 | | 2450 | 61 | | 2.5 | |
| D21S11 | 28 | 31.2 | | 2472 | 62 | | 2.5 | |
| D21S11 | 28 | 31.2 | | 2508 | 67 | | 2.7 | 2.6 |
| D2S1338 | 20 | 23 | | 3417 | 3017 | | | |
| D2S1338 | 20 | 23 | | 3407 | 3020 | | | |
| D2S1338 | 20 | 23 | | 3493 | 3055 | | | |
| D7S820 | 9 | 12 | 13 | 2373 | 178 | 1123 | 5.1 | |
| D7S820 | 9 | 12 | 13 | 2411 | 181 | 1140 | 5.1 | |
| D7S820 | 9 | 12 | 13 | 2441 | 182 | 1156 | 5.1 | 5.1 |
| FGA | 17.2 | 22 | 25 | 68 | 1140 | 896 | 3.3 | |
| FGA | 17.2 | 22 | 25 | 68 | 1144 | 909 | 3.1 | |

(continued)

| STR | Allele 1 | Allele 2 | Allele 3 | Allele 1 Height | Allele 2 Height | Allele 3 Height | Fetal Fraction | Fetal Fraction (Mean/STR) |
|---|---|---|---|---|---|---|---|---|
| FGA | 17.2 | 22 | 25 | 68 | 1151 | 925 | 3.3 | 3.2 |
| **Fetal Fraction = 3.5** | | | | | | | | |

The results show that cfDNA can be used for determining the presence or absence of fetal DNA as indicated by the detection of a minor component at one or more STR alleles, for determining the percent fetal fraction, and for determining fetal gender as indicated by the presence or absence of the Amelogenin allele.

**Example 11**

**Preamplification of cfDNA for Determining Fetal Fraction by Capillary Electrophoresis of Polymorphic Sequences Comprising STRs**

To improve the sensitivity of the STR assay in detecting and quantifying the STR alleles in the minor contributor of the cfDNA sample, the number of starting genomes in the artificial samples was increased by a modified whole genome amplification strategy.

Peripheral blood samples were collected and processed as described in Example 2. Cell-free DNA was extracted from 1 ml cell-free plasma using the Roche MagNA Pure Compact Nucleic Acid Isolation Kit I - Large Volume (Roche Applied Science, IN) using the MagNA Pure Compact Instrument, and eluted in 50 μl of elution buffer. Ten microliters of the extracted cfDNA were used to quantify the cfDNA, and the remainder was stored (see storage instructions WI0035 Clinical Sample Storage). The concentration of the plasma extracted cfDNA was determined by fluorescence-based quantitation with UV absorbance measurements using the Qubit™ Quantitation Platform (Invitrogen).

The concentration of cfDNA quantified in plasma samples prepared using the MagnaPure Nucleic Acid Isolation Kit I from 16 pregnant subjects was determined to range between 20 and 100 pg/μl. As the fetal component of plasma cfDNA is known to contribute 3-10% of the total plasma cfDNA, artificial plasma samples were created by spiking aliquots of cfDNA derived from plasma of female volunteer subjects with cfDNA extracted from plasma of male volunteer subjects to mimic the ratios of fetal to maternal cfDNA found in the pregnant subjects. Artificial samples were created to contain 200-1000pg of extracted female cfDNA that was spiked with 45-150 pg of extracted male cfDNA in a total volume of 10 μl. Each artificial sample was spiked to contain 3%, 5% and 10% male cfDNA.

Artificial samples having concentrations of total cfDNA of less than approximately 50 pg/μl, were preamplified using the modified improved primer extension amplification PCR (mIPEP) amplification according to the method of Hanson and Ballantyne, (Hanson and Ballantyne, Analytical Biochem 346:246-257 [2005]) as follows. Ten microliters of spiked plasma cfDNA were amplified in a 25 μl reaction volume containing ImM dNTPs, 2.5mM $MgCl_2$ (Applied Biosystems), IX Expand High Fidelity Buffer (No.3), 10.5U Expand High Fidelity Enzyme Mix (Roche Diagnostics), and 40 μM PEP primer (5'-NNNNNNNNNNNNNNN-3', Qiagen). The amplification was performed in a GeneAmp PCR System 9700 Thermocycler under the following conditions: (1) 20 and 30 cycles of 94°C for 1 minute, 37°C for 2 minutes, and 0.1°C/s ramp to 55°C for 4 minutes. The amplification product was purified using a Qiagen column. The concentration of the amplification product was determined using the Qubit™ Quantitation Platform as described above. STR analysis was performed as described in Example 9 above, except that only peak heights >100 RFU were included in the calculations.

The results are shown in Tables 9, 10 and 11. The results provided in Table 9 show that the cfDNA contained in 10μl cfDNA of artificial samples ART23 and ART24 having a starting concentration of cfDNA of 46.2 and 50.2 pg/μl, respectively, was amplified by approximately 5 and 10 fold following 20 and 30 cycles of PCR amplification, respectively.

These data indicate that a pre-amplification of cfDNA using the mIPEP method provided enhanced levels of total cfDNA rendering the level of the minor component more amenable to the STR analysis.

**TABLE 9 Preamplification with mIPEP**

| SAMPLE | cfDNA without mIPEP (pg/μl) | cfDNA with mIPEP:20 PCR cycles (pg/50μl) | cfDNA with mIPEP:30 PCR cycles (pg/50μl) |
|---|---|---|---|
| ART23 | 46.2 | 2265 | 4125 |
| ART24 | 50.2 | 2085 | 3875 |

Table 10 shows triplicate measurements profiling 9 loci of the cfDNA of spiked samples ART23 and ART24 following the mIPEP procedure with 20 and 30 cycles of amplification, as described above.

The data in Table 11 indicate that pre-amplification of cfDNA enables the detection and quantification of the minor component at most loci tested in artificially mixed samples having a starting cfDNA concentration that would otherwise not permit an accurate analysis of the minor STR alleles.

**TABLE 10 mIPEP Preamplification and Detection of Minor Component**

| | | ART23 (453pg) mIPEP amplified 20 cycles | ART23 (82 5pg) mIPEP amplified 20 cycles | ART23 (462pg) Extracted unamplified cfDNA | | ART24 (417pg) mIPEP amplified 30 cycles | ART24 (775pg) mIPEP amplifie d 30 cycles | ART24 (502pg) Extracted unamplified cfDNA |
|---|---|---|---|---|---|---|---|---|
| **STR Locus** | **Allele** | **Allele Height** | **Allele Height** | **Allele Height** | **Allele** | **Allele Height** | **Allele Height** | **Allele Height** |
| AMEL | X/Y | 291/95 | 397/170 | 535/832 | X/Y | 695/359 | 1878/114 8 | 1564/1959 |
| AMEL | X/Y | 425/147 | 428/188 | 675/1048 | X/Y | 1216/619 | 1551/954 | 1573/1943 |
| AMEL | X/Y | 267/94 | 455/203 | 664/1043 | X/Y | 718/363 | 1479/924 | 1621/2024 |
| **CSF1PO** | 10/11 | 800/979 | 725/1009 | 1429/1325 | 11/12 | 2029/1317 | 4159/231 7 | 2990/3083 |
| **CSF1PO** | 10/11 | 1147/1432 | 789/1102 | 1779/1650 | 11/12 | 3449/2223 | **3460/113 /1890** | 2996/3118 |
| **CSF1PO** | 10/11 | 729/906 | 831/1162 | 1783/1657 | 11/12 | 2006/1309 | 3362/1840 | 3072/3183 |
| **D13S317** | 12 | 743 | 515 | 1229 | 11 | 955 | 1490 | 3634 |
| **D13S317** | 12 | 1079 | 563 | 1534 | 11 | 1631 | 1198 | 3631 |
| **D13S317** | 12 | 668 | 583 | 1520 | 11 | 968 | 1170 | 3795 |
| **D16S539** | 9/10 | 239/140 | 370/466 | 835/676 | 10/11 | 513/512 | 1173/1472 | 1678/973 |
| **D16S539** | 9/10 | 347/203 | 64*(OL)/3 91/489 | 1046/864 | 10/11 | 859/870 | **973/1212** | 1730/999 |
| **D16S539** | 9/10 | 227/134 | 441/515 | 1055/860 | 10/11 | 530/513 | 960/1183 | 1784/1044 |
| **D18S51** | 14/15 | 359/464 | 363/220 | 785/541 | 12/18 | 1044/576 | 1840/786 | 2559/1507 |
| | | 512/645 | 391/226 | 999/672 | 12/18 | 1769/994 | 1511/643 | 2565/1469 |
| | | 313/402 | 409/245 | 994/685 | 12/18 | 1033/567 | 1496/631 | 2643/1523 |
| **D21S11** | 29/32 | 103/104 | 114/173 | 605/413 | 31.2 | 381 | 661 | 3276 |
| | | 149/153 | 130/182 | 759/523 | 31.2 | 650 | 536 | 3028 |
| | | 85/86 | 131/196 | 760/525 | 31.2 | 380 | 520 | 3282 |
| **D2S1338** | 18/20 | 572/383 | 428/363 | 1116/1013 | 19/20 | 1066/433 | 2315/124 3 | 2962/2968 |
| | | 827/553 | 454/386 | 1428/1279 | 19/20 | 1821/757 | 1901/101 | 2942/2942 |
| | | 530/351 | 482/408 | 1431/1275 | 19/20 | 1063/444 | 1859/101 2 | 3072/3067 |

(continued)

| | | ART23 (453pg) mIPEP amplified 20 cycles | ART23 (82 5pg) mIPEP amplified 20 cycles | ART23 (462pg) Extracted unamplified cfDNA | | ART24 (417pg) mIPEP amplified 30 cycles | ART24 (775pg) mIPEP amplifie d 30 cycles | ART24 (502pg) Extracted unamplified cfDNA |
|---|---|---|---|---|---|---|---|---|
| **D7S820** | 11/12 | 262/167 | 149/270 | 557/627 | 11/12 | 256/138 | 520/322 | 1550/1548 |
| | | 62/366/23 1 | 162/292 | 699/775 | 11/12 | 448/236 | 419/258 | 1484/1466 |
| | | 224/146 | 169/307 | 689/779 | 11/12 | 253/141 | 406/250 | 1579/1573 |
| **FGA** | 21/23 | 263/146 | 181/88 | 596/365 | 22/24 | 228/244 | 375/429 | 1272/1064 |
| | | 384/215 | 191/92 | 762/450 | 22/24 | 409/425 | 303/345 | 1221/1023 |
| | | 230/136 | 202/102 | 749/456 | 22/24 | 232/250 | 297/348 | 1298/1087 |
| *"OL" means "Off Ladder measurement" | | | | | | | | |

**TABLE 11 Fetal Fraction Determined in a Sample Following Preamplification Using mIPEP**

| STR marker | Allele 1/ Height | Allele 2/ Height | Allele 3/ Height | Allele 4/ Height | Percent minor fraction/STR - minor >100 RFU | Percent minor fraction/STR - minor <100 RFU |
|---|---|---|---|---|---|---|
| Amelogenin | X/2799 | Y/207 | | | | |
| Amelogenin | X/2751 | Y/198 | | | | |
| Amelogenin | X/3109 | Y/232 | | | | |
| | **X/2886** | **Y/212** | | | 7 | |
| CSF1PO | 10/2377 | 11/1869 | 12/508 | | | |
| CSF1PO | 10/2299 | 11/1814 | 12/498 | | | |
| CSF1PO | 10/2616 | 11/206 | 12/562 | | | |
| | **10/2431** | **11/1917** | **12/523** | | **12** | |
| D13S317 | 10/1232 | 11/1600 | 13/186 | | | |
| D13S317 | 10/1208 | 11/1548 | 13/182 | | | |
| D13S317 | 10/1386 | 11/1758 | 13/212 | | | |
| | **10/1275** | **11/1635** | **13/193** | | **12** | |
| D16S539 | 11/757 | 12/933 | | | | |
| D16S539 | 11/729 | 12/885 | | | | |
| D16S539 | 11/836 | 12/1031 | | | | |
| | **11/774** | **12/950** | | | **12** | |
| D18S51 | OL/80 | 14/3137 | 15/371 | | | |
| D18S51 | 11/73 | 14/3082 | 15/362 | | | |
| D18S51 | OL/83 | 14/3488 | 15/413 | | | |
| | **OL** | **14/3236** | **15/382** | | | |
| D21S11 | 29/953 | 30/941 | | | | |
| D21S11 | 29/921 | 30/908 | | | | |
| D21S11 | 29/1046 | 30/1045 | | | | |
| | **29/973** | **30/965** | | | | |
| D2S1338 | 17/461 | 18/366 | 20/2280 | 24/1760 | | |
| D2S1338 | 17/460 | 18/360 | 20/2240 | 24/1712 | | |
| D2S1338 | 17/508 | 18/409 | 20/2563 | 24/1971 | | |
| | **17/476** | **18/378** | **20/2361** | **24/1814** | **20** | |
| D7S820 | 8/1409 | 9/60 | 12/1059 | | | |
| D7S820 | 8/1380 | 9/60 | 12/1036 | | | |
| D7S820 | 8/1561 | 9/69 | 12/1166 | | | |
| | **8/1450** | **9/63** | **12/1087** | | | **2** |
| FGA | 19/825 | 21/850 | 25/279 | | | |
| FGA | 19/807 | 21/841 | 25/265 | | | |
| FGA | 19/913 | 21/958 | 25/306 | | | |
| | **19/848** | **21/883** | **25/283** | | **16** | |

(continued)

| STR marker | Allele 1/ Height | Allele 2/ Height | Allele 3/ Height | Allele 4/ Height | Percent minor fraction/STR - minor >100 RFU | Percent minor fraction/STR - minor <100 RFU |
|---|---|---|---|---|---|---|
| % fetal fraction >100RFU for minor allele → 12 | | | | | **12** | |
| % fetal fraction including <100RFU for minor allele → 11 | | | | | | **11** |

**Example 12**

**Correlation of Fetal Fraction Determined by Analysis of Fetal and Maternal SNPs and STRs**

To verify that the calculated fetal fraction *i.e.* fraction of minor nucleic acid component, determined using the SNP and STR assay as described in the preceding Examples provided an accurate measurement of the fetal fraction, the percent fetal fraction of cfDNA in the plasma from the same pregnant subjects was compared.

Peripheral blood samples were obtained from 48 volunteer subjects, 24 of the subjects were pregnant with male fetuses, and 24 were pregnant with female fetuses. cfDNA was prepared as described in Example 1, Fetal fraction using SNPs was determined by massively parallel sequencing by synthesis as described in Example 5, and fetal fraction using STRs was determined using capillary electrophoresis as described in Example 10

The results shown in **Figure 6** indicate that a positive correlation exists between the fraction determined using the STR assay and the fraction determined using the SNP sequencing. These data further validate the use of polymorphic sequences comprising STRs or SNPs for determining the fraction of fetal cfDNA in a plasma sample.

**Example 13**

**Use of Fetal Fraction to Set Thresholds and Estimate Minimum Sample Size in Aneuploidy Detection**

Counts of sequence matches to different chromosomes are manipulated to generate a score which will vary with chromosomal copy number that can be interpreted to identify chromosomal amplification or deletion. For example, such a score could be generated by comparing the relative amount of a sequence tags on a chromosome undergoing copy number changes to a chromosome known to be a euploid. Examples of scores that can be used to identify amplification or deletion include but are not limited to: counts for the chromosome of interest divided by counts of another chromosome from the same experimental run, the counts for the chromosome of interest divided by the total number of counts from the experimental run, comparison of counts from the sample of interest versus a separate control sample. Without loss of generality, it can be assumed that scores will increase as copy number increases. Knowledge of fetal fraction can be used to set "cutoff thresholds to call "aneuploidy", "normal", or "marginal" (uncertain) states. Then, calculations are performed to estimate the minimum number of sequences required to achieve adequate sensitivity (i.e. probability of correctly identifying an aneuploidy state).

**Figure 7** is a plot of two different populations of scores. The x-axis is score and the y-axis is frequency. Scores on samples of chromosomes without aneuploidy can have a distribution shown in **Figure 7A. Figure 7B** illustrates a hypothetical distribution of a population of scores on samples with an amplified chromosome. Without loss of generality, the graphs and equations show the case of a univariate score where the aneuploidy condition represents an amplification of copy number. Multivariate cases and/or reduction/deletion abnormalities are simple extensions or rearrangements of the given descriptions and are intend to fall within the scope of this art.

The amount of "overlap" between the populations can determine how well normal and aneuploidy cases can be discriminated. In general, increasing fetal fraction, ff, increases discrimination power by separating the two population centers (by moving "C2," the "Center of Aneuploidy Scores", and increasing "d," causing the populations to overlap less. Furthermore, an increase in the absolute value of the magnitude, m, (for example having four copies of the chromosome instead of a trisomy) of the amplification will also increase separation of population centers leading to higher power (i.e. higher probability of correctly identifying aneuploidy states).

Increasing the number of sequences generated, N, reduces standard deviations "sdevA" and/or "sdevB," the spread of the two populations of scores, which also causes the populations to overlap less.

**Setting Thresholds and Estimating Sample Size**

The following procedure can be used to set "c", the critical value for calling "aneuploidy", "normal", or "marginal" (uncertain) states. Without loss of generality, one sided statistical tests are used below.

First, an acceptable false positive rate, FP (sometimes also called "type I error" or "specificity"), is decided, which is the probability of a false positive or falsely calling aneuploidy. For example, FP can be at least, or about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, or 0.1.

Second, the value of "c" can be determined by solving the equation: FP = integral from c to infinity of (f1(x)dx).

$$\mathrm{FP} = \int_{c}^{\infty} f1(x)dx \qquad \text{(Equation 1)}$$

Once a critical value, c, has been determined, the minimum number sequences required to achieve a certain TP = True positive rate can be estimated. The true positive rate can be, for example, about 0.5, 0.6, 0.7, 0.8, or 0.9. In one embodiment, the true positive rate can be 0.8. In other words, N is the minimum number of sequences required to identify aneuploidy 100*TP percent of the time. N = minimum number such that TP = integral from c to infinity of f2(x,ff)dx > 0.8. N is determined by solving

$$\min_N \; s.t. \{TB \geq \int_{c}^{\infty} f2(x,N)dx\} \qquad \text{(Equation 2)}$$

In classical statistical tests f1 and f2 are often F, non-central F distributions (a special case of t and non-central t distributions) although that is not a necessary condition for this application.

**Setting "Levels" of Thresholds to Give More Control of Errors**

Thresholds can also be set in stages using the above methods. For example, a threshold can be set for high confidence calling of "aneuploidy", say ca, using FP 0.001 and a "marginal" threshold, say cb, using FP 0.05. In this case if Score, S:

(S > ca) then call "Trisomy"
(cb > S <= ca) then call "Marginal"
(S < cb) then call "Normal"

**Some Trivial Generalizations Falling Within Scope of this Art**

Different values for thresholds such as TP, FP, etc can be used. Procedures can be run in any order. For example, one can start with N and solve for c, etc. Distributions can depend on ff so that f1(x,N,ff), f2(x,N,ff), and/or other variables. The above integral equations can be solved by reference to tables or by iterative computer methods. A non-centrality parameter can be estimated and power can be read from standard statistical tables. Statistical power and sample sizes may be derived from calculation or estimation of expected mean squares. Closed form theoretical distributions such as f, t, non-central t, normal, etc. or estimates (kernel or other) can be used to model the distributions f1, f2. Empirical threshold setting and parameter selection using Receiver Operator Characteristic Curves (ROC) can be used and collated with fetal fraction. Various estimates of distribution spread (variance, mean absolute deviation, inter quartile range, etc.) may be used. Various estimates of distribution center (mean, median, etc.) can be used. Two sided as opposed to one sided statistical tests can be used. The simple hypothesis test can be reformulated as linear or non-linear regression. Combinatorial methods, simulation (e.g., monte carlo), maximization (e.g., expectation maximization), iterative, or other methods can be used independently or in conjunction with the above to establish statistical power or thresholds.

## Claims

**1.** A method for determining the fraction of fetal nucleic acids in a maternal sample comprising a mixture of fetal and maternal genomic DNA, wherein the maternal sample is a plasma sample and wherein said fetal and maternal genomic DNA is cell-free DNA (cfDNA), said method comprising:

(a) amplifying a plurality of polymorphic target nucleic acids in said mixture, each of said plurality of polymorphic target nucleic acids comprising at least one single nucleotide polymorphism (SNP) site, such that at least 40 different SNP sites are amplified from the polymorphic target nucleic acids, wherein at least one SNP site is informative, and wherein said plurality of polymorphic target nucleic acids are located on a plurality of different chromosomes;
(b) performing massively parallel sequencing of at least a portion of the amplified product obtained in (a), wherein said sequencing comprises providing a plurality of sequence tags; and
(c) based on said sequencing, determining said fraction, wherein:

(i) said fraction is determined based on the total number of tags that map to a first allele and the total number of tags that map to a second allele at an informative SNP site contained in a reference genome; and
(ii) the informative SNP site is identified by the difference in the allelic sequences and the amount of each of the possible alleles.

**2.** The method of Claim 1, wherein each of said plurality of polymorphic target nucleic acids comprises a single SNP site.

**3.** The method of Claim 1, wherein the reference genome is an artificial target sequences genome that comprises the sequences of the polymorphic target nucleic acids.

**4.** The method of Claim 3, wherein the reference genome is an artificial SNP reference genome.

**5.** The method of Claim 1, wherein the amplification of the plurality of polymorphic target nucleic acids is accomplished using primer pairs each capable of amplifying a target nucleic acid sequence comprising a SNP site in a multiplex PCR reaction.

**6.** The method of Claim 5, wherein the method comprises combining at least 2, at least 3, at least 5, at least 10, at least 15, at least 20, at least 25, at least 30, at least 40 or more sets of primers in the same reaction to quantify the amplified target nucleic acids comprising at least two, at least three, at least 5, at least 10, at least 15, at least 20, at least 25, at least 30, at least 40 or more SNP sites in the same sequencing reaction.

**7.** The method of Claim 1, wherein the percent fetal fraction is calculated for at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 40 or more informative alleles.

**8.** The method of Claim 7, wherein the fetal fraction is the average fetal fraction determined for at least 3 informative alleles.

**9.** The method of Claim 1, wherein said sequencing is: (i) sequencing-by-synthesis with reversible dye terminators; (ii) sequencing-by-ligation; or (iii) single molecule sequencing.

**10.** The method of Claim 1, wherein said sequencing comprises an amplification.

**11.** The method of Claim 1, wherein said sequencing is sequencing by hybridization, wherein the hybridization comprises contacting the plurality of polynucleotide sequences with a plurality of polynucleotide probes, wherein each of the plurality of polynucleotide probes can be optionally tethered to a substrate, and wherein the pattern of hybridization to the polynucleotide probes can be used to determine the polynucleotide sequences present in the sample.

**12.** The method of Claim 11, wherein the substrate is a flat surface comprising an array of known nucleotide sequences.

**13.** The method of Claim 1, wherein said method is a fetal gender-independent method.

**14.** The method of Claim 1, wherein said plurality of polymorphic target nucleic acids contains a sufficient number of SNP sites such that at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 40 or more are informative.

**15.** The method of Claim 1, wherein:

(i) the plurality of polymorphic nucleic acids are located on chromosomes 1-22; or
(ii) the plurality of polymorphic nucleic acids are located on a plurality of different chromosomes other than chromosomes 13, 18, 21, X or Y.

## Patentansprüche

**1.** Verfahren zur Bestimmung der Fraktion von fetalen Nucleinsäuren in einer maternalen Probe, umfassend eine Mischung aus fetaler und maternaler genomer DNA, wobei die maternale Probe eine Plasmaprobe ist und wobei die fetale und maternale genome DNA zellfreie DNA (cfDNA) ist, wobei das Verfahren umfasst:

(a) Amplifizieren einer Vielzahl von polymorphen Zielnucleinsäuren in der Mischung, wobei jede der Vielzahl von polymorphen Zielnucleinsäuren mindestens eine Einzelnucleotidpolymorphismus(SNP)-Stelle umfasst, sodass mindestens 40 verschiedene SNP-Stellen aus den polymorphen Zielnucleinsäuren amplifiziert werden, wobei mindestens eine SNP-Stelle informativ ist und wobei sich die Vielzahl von polymorphen Zielnucleinsäuren auf einer Vielzahl von verschiedenen Chromosomen befindet;
(b) Durchführen von massiver Parallelsequenzierung von mindestens einem Teil des in (a) erhaltenen amplifizierten Produkts, wobei das Sequenzieren das Bereitstellen einer Vielzahl von Sequenz-Tags umfasst; und
(c) basierend auf der Sequenzierung, Bestimmen der Fraktion, wobei:

(i) die Fraktion bestimmt wird basierend auf der Gesamtzahl der Tags, die einem ersten Allel, und der Gesamtzahl der Tags, die einem zweiten Allel, an einer informativen SNP-Stelle, die in einem Referenzgenom enthalten ist, zugeordnet sind; und
(ii) die informative SNP-Stelle durch den Unterschied in den Allelsequenzen und der Menge jedes der möglichen Allele identifiziert wird.

**2.** Verfahren nach Anspruch 1, wobei jede der Vielzahl von polymorphen Zielnucleinsäuren eine einzige SNP-Stelle umfasst.

**3.** Verfahren nach Anspruch 1, wobei das Referenzgenom ein künstliches Zielsequenzgenom ist, das die Sequenzen der polymorphen Zielnucleinsäuren umfasst.

**4.** Verfahren nach Anspruch 3, wobei das Referenzgenom ein künstliches SNP-Referenzgenom ist.

**5.** Verfahren nach Anspruch 1, wobei das Amplifizieren der mehreren polymorphen Zielnucleinsäuren unter Verwendung von Primerpaaren erzielt wird, die jeweils in der Lage sind, eine Zielnucleinsäuresequenz, die eine SNP-Stelle umfasst, in einer Multiplex-PCR-Reaktion zu amplifizieren.

**6.** Verfahren nach Anspruch 5, wobei das Verfahren des Kombinieren von mindestens 2, mindestens 3, mindestens 5, mindestens 10, mindestens 15, mindestens 20, mindestens 25, mindestens 30, mindestens 40 oder mehr Primersätzen in derselben Reaktion zum Quantifizieren der amplifizierten Zielnucleinsäuren umfasst, umfassend mindestens zwei, mindestens drei, mindestens 5, mindestens 10, mindestens 15, mindestens 20, mindestens 25, mindestens 30, mindestens 40 oder mehr SNP-Stellen in derselben Sequenzierungsreaktion.

**7.** Verfahren nach Anspruch 1, wobei der prozentuale fetale Anteil für mindestens 2, mindestens 3, mindestens 4, mindestens 5, mindestens 6, mindestens 7, mindestens 8, mindestens 9, bei mindestens 10, mindestens 11, mindestens 12, mindestens 13, mindestens 14, mindestens 15, mindestens 16, mindestens 17, mindestens 18, mindestens 19, mindestens 20, mindestens 25, mindestens 30 mindestens 40 oder mehr informative Allele berechnet wird.

**8.** Verfahren nach Anspruch 7, wobei die fetale Fraktion die für mindestens 3 informative Allele bestimmte durchschnittliche fetale Fraktion ist.

**9.** Verfahren nach Anspruch 1, wobei die Sequenzierung Folgendes ist: (i) Sequenzierung durch Synthese mit reversiblen Farbstoffterminatoren; (ii) Sequenzierung durch Ligation; oder (iii) Einzelmolekülsequenzierung.

10. Verfahren nach Anspruch 1, wobei die Sequenzierung eine Amplifikation umfasst.

11. Verfahren nach Anspruch 1, wobei die Sequenzierung Sequenzierung durch Hybridisierung ist, wobei die Hybridisierung das Kontaktieren der Vielzahl von Polynucleotidsequenzen mit einer Vielzahl von Polynucleotidsonden umfasst, wobei jede der Vielzahl von Polynucleotidsonden wahlweise an ein Substrat gebunden sein kann, und wobei das Muster der Hybridisierung an die Polynucleotidsonden verwendet werden kann, um die in der Probe vorhandenen Polynucleotidsequenzen zu bestimmen.

12. Verfahren nach Anspruch 11, wobei das Substrat eine flache Oberfläche ist, die eine Anordnung von bekannten Nucleotidsequenzen umfasst.

13. Verfahren nach Anspruch 1, wobei das Verfahren ein vom Geschlecht des Fetus unabhängiges Verfahren ist.

14. Verfahren nach Anspruch 1, wobei die Vielzahl von polymorphen Zielnucleinsäuren eine ausreichende Anzahl von SNP-Stellen aufweist, sodass mindestens 2, mindestens 3, mindestens 4, mindestens 5, mindestens 6, mindestens 7, mindestens 8, mindestens 9, mindestens 10, mindestens 11, mindestens 12, mindestens 13, mindestens 14, mindestens 15, mindestens 16, mindestens 17, mindestens 18, mindestens 19, mindestens 20, mindestens 25, mindestens 30, mindestens 40 oder mehr informativ sind.

15. Verfahren nach Anspruch 1, wobei:

(i) die Vielzahl polymorpher Nucleinsäuren sich auf den Chromosomen 1-22 befindet; oder
(ii) die Vielzahl polymorpher Nucleinsäuren sich auf anderen Chromosomen als den Chromosomen 13, 18, 21, X oder Y befindet.

**Revendications**

1. Procédé de détermination de la fraction d’acides nucléiques fœtaux dans un échantillon de sang maternel comprenant un mélange d’ADN génomique fœtal et maternel, dans lequel l’échantillon maternel est un échantillon de plasma et dans lequel ledit ADN génomique fœtal et maternel est de l’ADN sans cellules (ADNcf), ledit procédé comprenant :

(a) l’amplification d’une pluralité d’acides nucléiques cibles polymorphes dans ledit mélange, chacun de ladite pluralité d’acides nucléiques cibles polymorphes comprenant au moins un site de polymorphisme d’un seul nucléotide (SNP), de sorte qu’au moins 40 SNP différents sont amplifiés à partir des acides nucléiques cibles polymorphes, dans lequel au moins un site SNP est informatif, et dans lequel ladite pluralité d’acides nucléiques cibles polymorphes sont situés sur une pluralité de chromosomes différents ;
(b) la conduite d’un séquençage massif en parallèle d’au moins une partie du produit amplifié obtenu dans (a), ledit séquençage comprenant la fourniture d’une pluralité d’étiquettes de séquence ; et
(c) sur la base dudit séquençage, la détermination de ladite fraction, où :

(i) ladite fraction est déterminée sur la base du nombre total d’étiquettes qui correspondent à un premier allèle et le nombre total d’étiquettes qui correspondent à un deuxième allèle à un site SNP informatif contenu dans un génome de référence ; et
(ii) le site SNP informatif est identifié par la différence de séquences alléliques et la quantité de chacun des allèles possibles.

2. Procédé selon la revendication 1, dans lequel chacun de ladite pluralité d’acides nucléiques cibles polymorphes comprend un seul site SNP.

3. Procédé selon la revendication 1, dans lequel le génome de référence est un génome de séquences cibles artificielles qui comprend les séquences des acides nucléiques cibles polymorphes.

4. Procédé selon la revendication 3, dans lequel le génome de référence est un génome de référence SNP artificiel.

5. Procédé selon la revendication 1, dans lequel l’amplification de la pluralité d’acides nucléiques cibles polymorphes est effectuée au moyen de paires d’amorces dont chacune permet d’amplifier une séquence d’acide nucléique cible comprenant un site SNP dans une réaction PCR multiplexe.

6. Procédé selon la revendication 5, le procédé comprenant la combinaison d'au moins 2, au moins 3, au moins 5, au moins 10, au moins 15, au moins 20, au moins 25, au moins 30, au moins 40 ensembles d'amorces ou plus dans la même réaction pour quantifier les acides nucléiques cibles amplifiés comprenant au moins deux, au moins trois, au moins 5, au moins 10, au moins 15, au moins 20, au moins 25, au moins 30, au moins 40 sites SNP ou plus dans la même réaction de séquençage.

7. Procédé selon la revendication 1, dans lequel la fraction fœtale en pourcentage est calculée pour au moins 2, au moins 3, au moins 4, au moins 5, au moins 6, au moins 7, au moins 8, au moins 9, au moins 10, au moins 11, au moins 12, au moins 13, au moins 14, au moins 15, au moins 16, au moins 17, au moins 18, au moins 19, au moins 20, au moins 25, au moins 30, au moins 40 allèles informatifs ou plus.

8. Procédé selon la revendication 7, dans lequel la fraction fœtale est la fraction fœtale moyenne déterminée pour au moins 3 allèles informatifs.

9. Procédé selon la revendication 1, dans lequel ledit séquençage est: (i) un séquençage par synthèse avec des terminateurs de colorant réversible ; (ii) un séquençage par ligature ; or (iii) un séquençage de molécule unique.

10. Procédé selon la revendication 1, dans lequel ledit séquençage comprend une amplification.

11. Procédé selon la revendication 1, dans lequel ledit séquençage est un séquençage par hybridation, où l'hybridation comprend la mise en contact de la pluralité de séquences polynucléotidiques avec une pluralité de sondes polynucléotidiques, dans lequel chacune de la pluralité de sondes polynucléotidiques peut facultativement être attachée à un substrat, et dans lequel le motif d'hybridation aux sondes polynucléotidiques peut être utilisé pour déterminer les séquences polynucléotidiques présentes dans l'échantillon.

12. Procédé selon la revendication 11, dans lequel le substrat est une surface plate comprenant une matrice de séquences nucléotidiques connues.

13. Procédé selon la revendication 1, dans lequel ledit procédé est un procédé indépendant du sexe fœtal.

14. Procédé selon la revendication 1, dans lequel ladite pluralité d'acides nucléiques cibles polymorphes contient un nombre suffisant de sites SNP de sorte qu'au moins 2, au moins 3, au moins 4, au moins 5, au moins 6, au moins 7, au moins 8, au moins 9, au moins 10, au moins 11, au moins 12, au moins 13, au moins 14, au moins 15, au moins 16, au moins 17, au moins 18, au moins 19, au moins 20, au moins 25, au moins 30, au moins 40 ou plus sont informatifs.

15. Procédé selon la revendication 1, dans lequel :

(i) la pluralité d'acides nucléiques cibles polymorphes sont situés sur les chromosomes 1-22 ; ou
(ii) la pluralité d'acides nucléiques polymorphes sont situés sur une pluralité de chromosomes différents autres que les chromosomes 13, 18, 21, X ou Y.

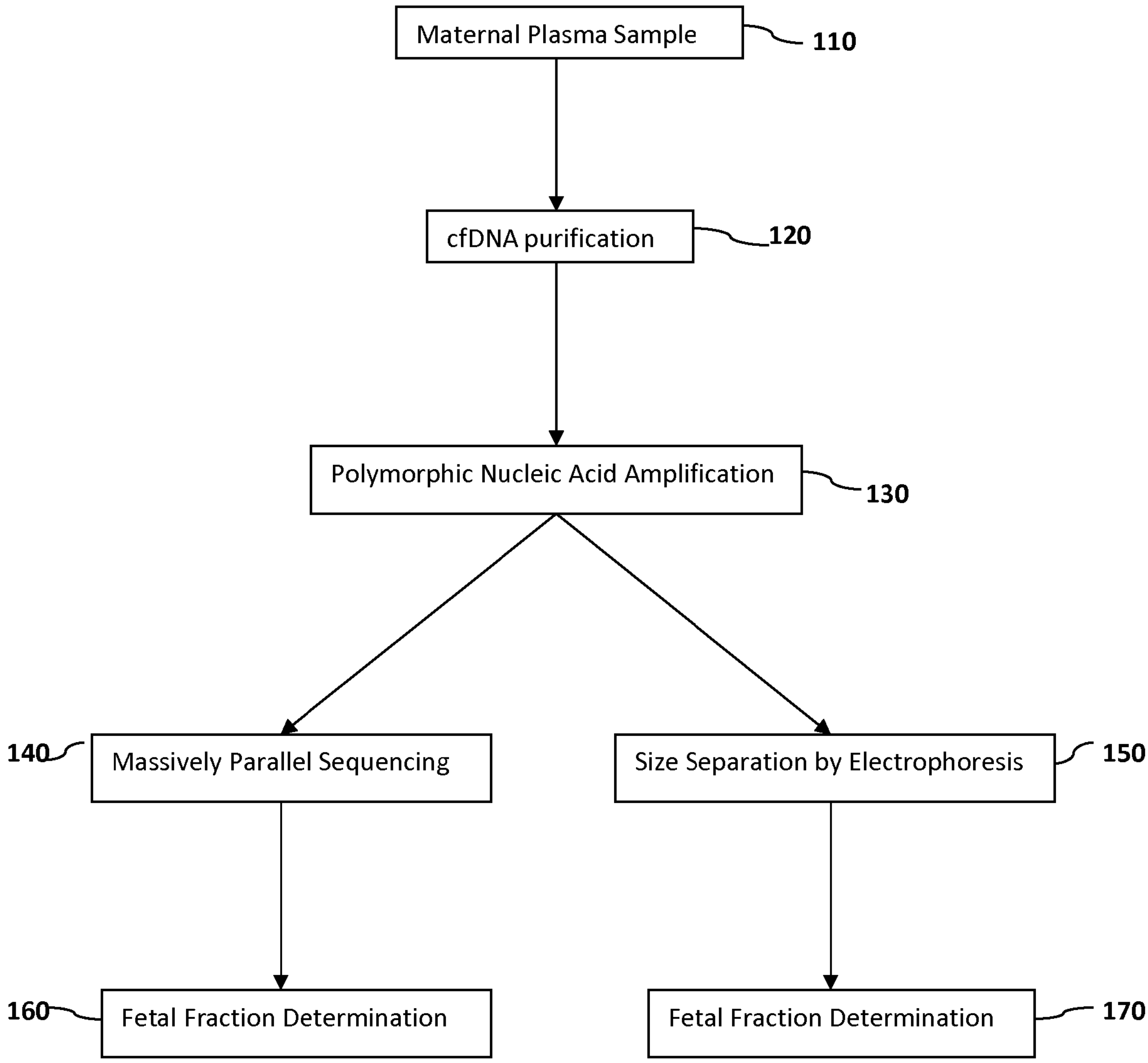
Maternal Plasma Sample
110
cfDNA purification
120
Polymorphic Nucleic Acid Amplification
130
140
Massively Parallel Sequencing
Size Separation by Electrophoresis
150
160
Fetal Fraction Determination
Fetal Fraction Determination
170

FIGURE 1

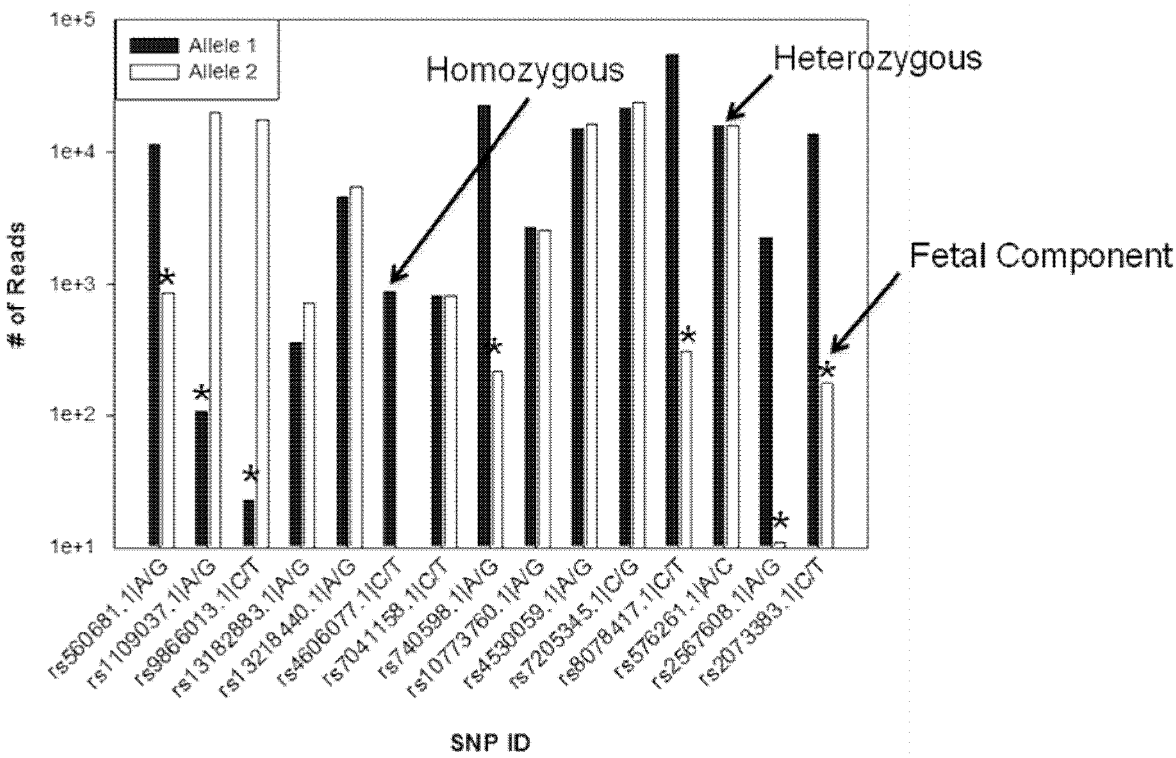
1e+5
1e+4
1e+3
1e+2
1e+1
# of Reads
Allele 1
Allele 2
Homozygous
Heterozygous
Fetal Component
*
rs560681.1|A/G
rs1109037.1|A/G
rs9866013.1|C/T
rs13182883.1|A/G
rs13218440.1|A/G
rs4606077.1|C/T
rs7041158.1|C/T
rs740598.1|A/G
rs10773760.1|A/G
rs4530059.1|A/G
rs7205345.1|C/G
rs8078417.1|C/T
rs576261.1|A/C
rs2567608.1|A/G
rs2073383.1|C/T
SNP ID

FIGURE 2

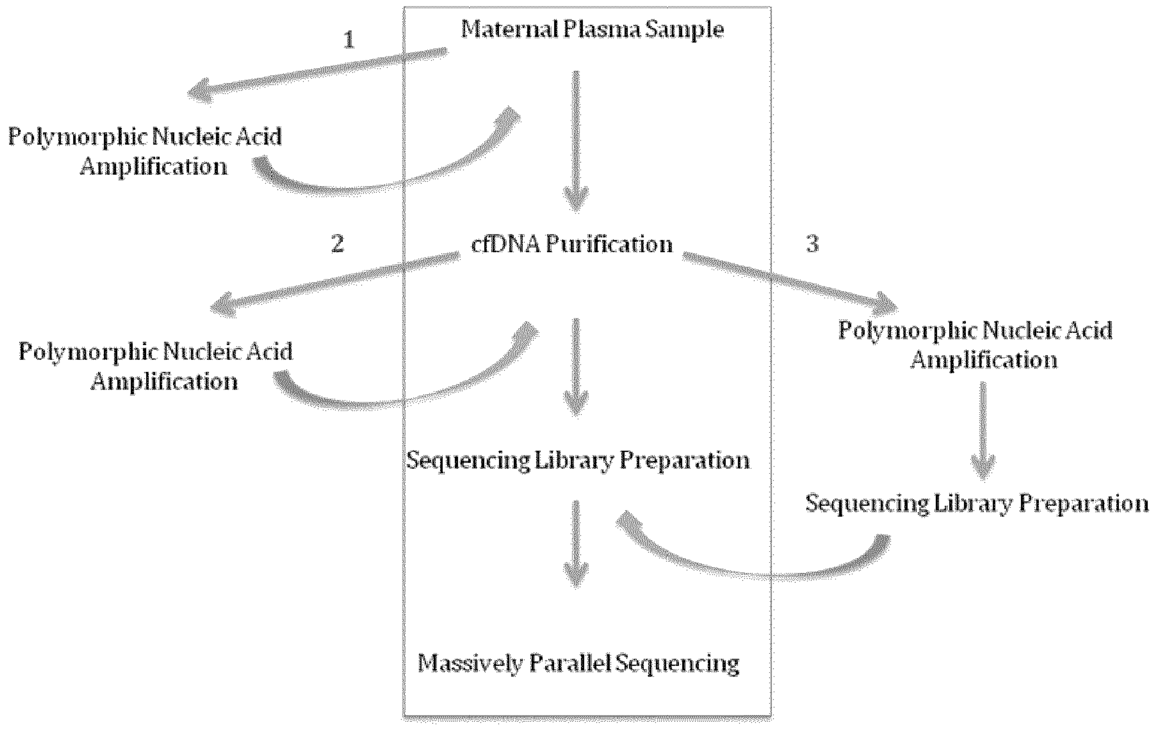
1
Maternal Plasma Sample
Polymorphic Nucleic Acid Amplification
2
cfDNA Purification
3
Polymorphic Nucleic Acid Amplification
Polymorphic Nucleic Acid Amplification
Sequencing Library Preparation
Sequencing Library Preparation
Massively Parallel Sequencing

FIGURE 3

| **Amplified Loci: Locus Designation** | **Chromosome Location** | **Alleles Included in Identifiler Allelic Ladder** | **Dye Label** | **Control DNA 9947A** |
|---|---|---|---|---|
| D8S1179 | 8 | 8, 9 10, 11, 12, 13, 14, 15, 16, 17, 18, 19 | 6-FAM | 13a |
| D21S11 | 21q11.2-q21 | 24, 24.2, 25, 26, 27, 28, 28.2, 29, 29.2, 30, 30.2, 31, 31.2, 32, 32.2, 33, 33.2, 34, 34.2, 35, 35.2, 36, 37, 38 | | 30b |
| D7S820 | 7q11.21-22 | 6, 7, 8, 9, 10, 11, 12, 13, 14, 15 | | 10, 11 |
| CSF1PO | 5q33.3-34 | 6, 7, 8, 9, 10, 11, 12, 13, 14, 15 | | 10, 12 |
| D3S1358 | 3p | 12, 13, 14, 15, 16, 17, 18, 19 | VIC | 14, 15 |
| TH01 | 11p15.5 | 4, 5, 6, 7, 8, 9, 9.3, 10, 11, 13.3 | | 8, 9.3 |
| D13S317 | 13q22-31 | 8, 9, 10, 11, 12, 13, 14, 15 | | 11c |
| D16S539 | 16q24-qter | 5, 8, 9, 10, 11, 12,13, 14, 15 | | 11, 12 |
| D2S1338 | 2q35-37.1 | 15, 16, 17, 18, 19, 20, 21, 22, 23, 24, 25, 26, 27, 28 | | 19, 23 |
| D19S433 | 19q12-13.1 | 9, 10, 11, 12, 12.2, 13, 13.2, 14, 14.2, 15, 15.2, 16, 16.2, 17, 17.2 | NED | 14, 15 |
| vWA | 12p12-pter | 11,12, 13, 14, 15, 16, 17, 18, 19, 20, 21, 22, 23, 24 | | 17, 18 |
| TPOX | 2p23-2per | 6, 7, 8, 9, 10, 11, 12, 13 | | 8d |
| D18S51 | 18q21.3 | 7, 9, 10, 10.2, 11, 12, 13, 13.2, 14, 14.2, 15, 16, 17, 18, 19, 20, 21, 22, 23, 24, 25, 26, 27 | | 15, 19 |
| Amelogenin | X: p22.1-22.3<br>Y: p11.2 | X, Y | PET | X |
| D5S818 | 5q21-31 | 7, 8, 9, 10, 11, 12, 13, 14, 15, 16 | | 11e |
| FGA | 4q28 | 17, 18, 19, 20, 21, 22, 23, 24, 25, 26, 26.2, 27, 28, 29, 30, 30.2, 31.2, 32.2, 33.2, 42.2, 43.2, 44.2, 45.2, 46.2, 47.2, 48.2, 50.2, 51.2 | | 23, 24 |

## FIGURE 4

| Amplified Loci: Locus Designation | Chromosome Location | Alleles Included in Identifiler Allelic Ladder | Dye Label | Control DNA 9947A |
|---|---|---|---|---|
| D8S1179 | 8 | 8, 9 10, 11, 12, 13, 14, 15, 16, 17, 18, 19 | 6-FAM | 13a |
| D21S11 | 21q11.2-q21 | 24, 24.2, 25, 26, 27, 28, 28.2, 29, 29.2, 30, 30.2, 31, 31.2, 32, 32.2, 33, 33.2, 34, 34.2, 35, 35.2, 36, 37, 38 | | 30b |
| D7S820 | 7q11.21-22 | 6, 7, 8, 9, 10, 11, 12, 13, 14, 15 | | 10, 11 |
| CSF1PO | 5q33.3-34 | 6, 7, 8, 9, 10, 11, 12, 13, 14, 15 | | 10, 12 |
| D3S1358 | 3p | 12, 13, 14, 15, 16, 17, 18, 19 | VIC | 14, 15 |
| TH01 | 11p15.5 | 4, 5, 6, 7, 8, 9, 9.3, 10, 11, 13.3 | | 8, 9.3 |
| D13S317 | 13q22-31 | 8, 9, 10, 11, 12, 13, 14, 15 | | 11c |
| D16S539 | 16q24-qter | 5, 8, 9, 10, 11, 12,13, 14, 15 | | 11, 12 |
| D2S1338 | 2q35-37.1 | 15, 16, 17, 18, 19, 20, 21, 22, 23, 24, 25, 26, 27, 28 | | 19, 23 |
| D19S433 | 19q12-13.1 | 9, 10, 11, 12, 12.2, 13, 13.2, 14, 14.2, 15, 15.2, 16, 16.2, 17, 17.2 | NED | 14, 15 |
| vWA | 12p12-pter | 11,12, 13, 14, 15, 16, 17, 18, 19, 20, 21, 22, 23, 24 | | 17, 18 |
| TPOX | 2p23-2per | 6, 7, 8, 9, 10, 11, 12, 13 | | 8d |
| D18S51 | 18q21.3 | 7, 9, 10, 10.2, 11, 12, 13, 13.2, 14, 14.2, 15, 16, 17, 18, 19, 20, 21, 22, 23, 24, 25, 26, 27 | | 15, 19 |
| Amelogenin | X: p22.1-22.3<br>Y: p11.2 | X, Y | PET | X |
| D5S818 | 5q21-31 | 7, 8, 9, 10, 11, 12, 13, 14, 15, 16 | | 11e |
| FGA | 4q28 | 17, 18, 19, 20, 21, 22, 23, 24, 25, 26, 26.2, 27, 28, 29, 30, 30.2, 31.2, 32.2, 33.2, 42.2, 43.2, 44.2, 45.2, 46.2, 47.2, 48.2, 50.2, 51.2 | | 23, 24 |

**FIGURE 5**

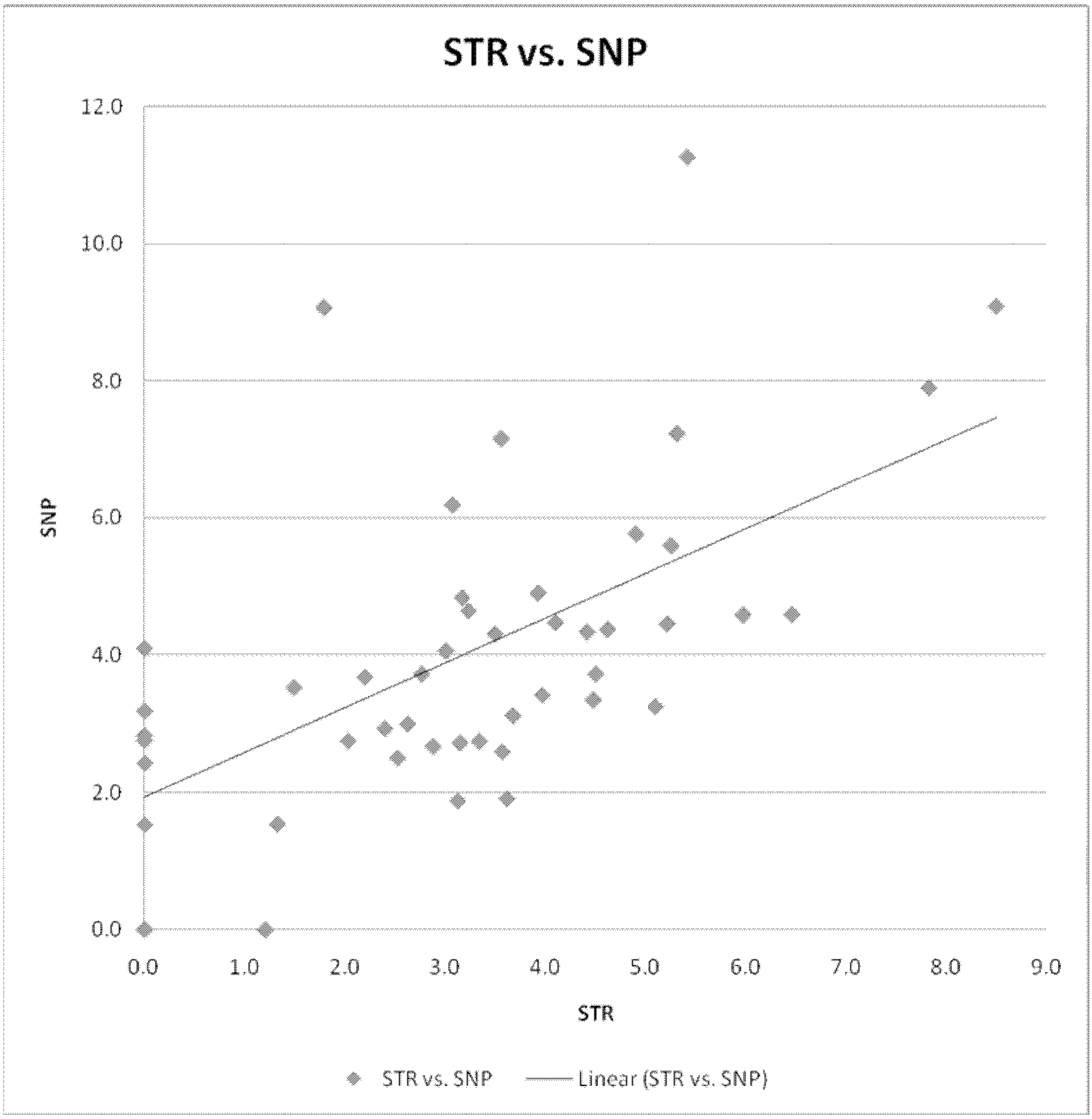
STR vs. SNP
12.0
10.0
8.0
6.0
4.0
2.0
0.0
SNP
0.0
1.0
2.0
3.0
4.0
5.0
6.0
7.0
8.0
9.0
STR
STR vs. SNP
Linear (STR vs. SNP)

FIGURE 6

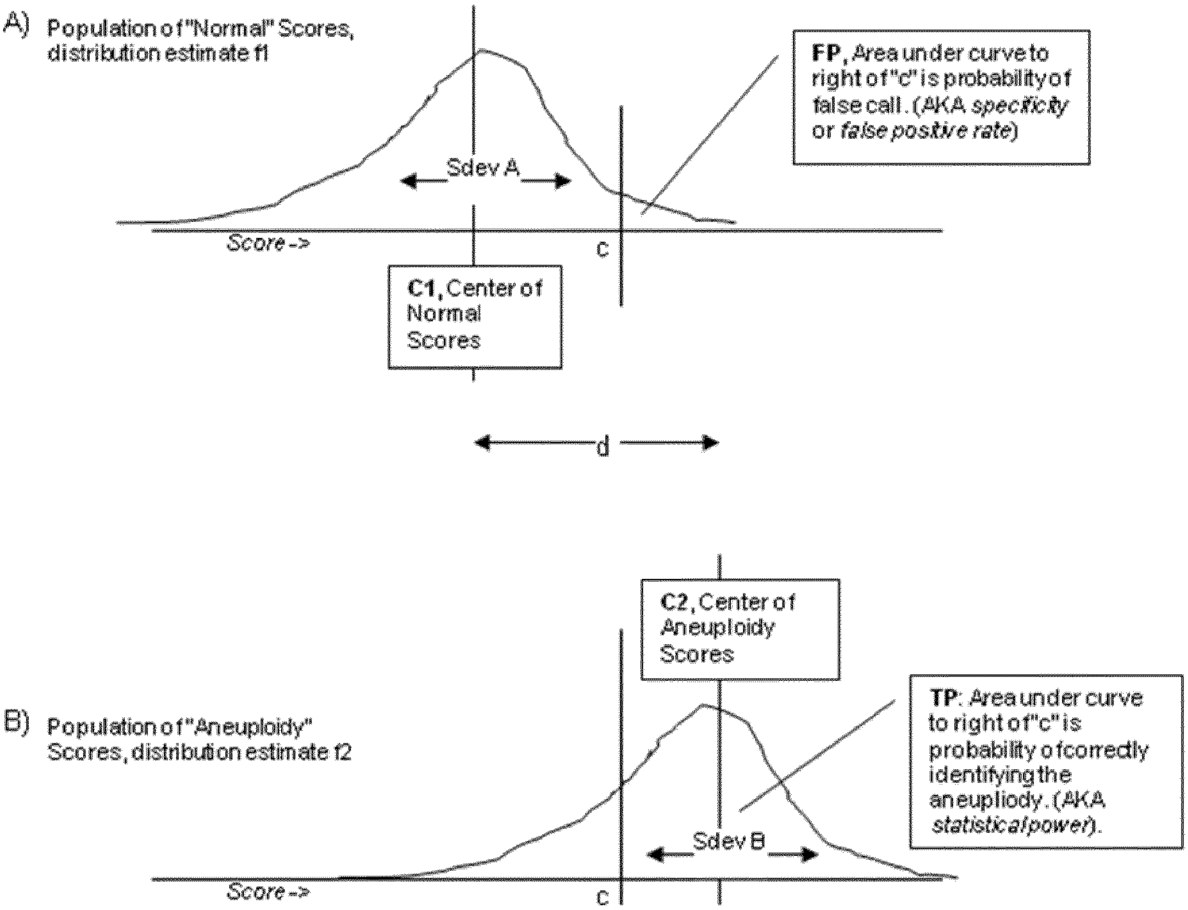
A) Population of "Normal" Scores, distribution estimate f1
FP, Area under curve to right of "c" is probability of false call. (AKA specificity or false positive rate)
Sdev A
Score ->
c
C1, Center of Normal Scores
d
C2, Center of Aneuploidy Scores
B) Population of "Aneuploidy" Scores, distribution estimate f2
TP: Area under curve to right of "c" is probability of correctly identifying the aneupliody. (AKA statistical power).
Sdev B
Score ->
c

**FIGURE 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080070792
- WO 2011057094 A
- US 20050282293 A
- US 20050224351 A
- US 20050065735 A
- US 20080070792 A
- US 20090280492 A
- US 20080113358 A
- US 20080026390 A
- US 20080050739 A
- US 20080220422 A
- US 20080138809 A
- US 20090026082
- WO 2009046445 PCT
- US 20070202525 A1
- US 20100112575 A1
- US 20090087847 A1
- US 20090029377 A1
- US 20080220422 A1
- US 20080138809 A1
- US 20080153090 A1
- US 7645576 B
- US 20100112590 A1
- US 20090162842 A1
- US 20070207466 A1
- US 20010051341 A1
- US 61296358
- US 61360837 B
- US 61407017
- US 61455849778 A
- US 61455849 B
- US 2010058606 W
- US 61407017 B
- US 61296358 B


### Non-patent literature cited in the description


- **HUANG et al.** *Methods in Molecular Biology,* 2008, vol. 444, 203-208
- **BIANCHI et al.** *Am J Hum Genet,* 1997, vol. 61, 822-829
- **BABOCHKINA et al.** *Haematologica,* 2005, vol. 90, 740-745
- **WRIGHT C.F. ; BURTON H.** *Human Reproduction Update,* 2009, vol. 15 (1), 139-151
- **LO et al.** *Clin Chem,* 1999, vol. 45, 1747-1751
- **DAHLLAN et al.** *Lancet,* 2007, vol. 369, 474-481
- **LI et al.** *Clin Chem,* 2004, 1002-1011
- **FAN et al.** *Proc Natl Acad Sci,* 2008, vol. 105, 16266-16271
- **CHIU et al.** *Trends in Genetics,* 2009, vol. 25 (7), 324-331
- **KOIDE et al.** *Prenatal Diagnosis,* 2005, vol. 25, 604-607
- **CHEN et al.** *Nature Med.,* 1996, vol. 2, 1033-1035
- **LO et al.** *Lancet,* 1997, vol. 350, 485-487
- **PAKSTIS.** *Hum Genet,* 2010, vol. 127, 315-324
- The International HapMap Project. *Nature,* 2003, vol. 426, 789-796
- **PERTL et al.** *Human Genetics,* 2002, vol. 106, 45-49
- **LIU et al.** *Acta Obset Gyn Scand,* 2007, vol. 86, 535-541
- **CHAN et al.** *Clin Chem,* 2004, vol. 50, 8892
- **LI et al.** *Clin Chem,* 2004, vol. 50, 1002-1011
- **BUTLER et al.** *J Forensic Sci,* 2003, vol. 48, 1054-1064
- **COBLE ; BUTLER.** *J Forensic Sci,* 2005, vol. 50, 43-53
- **SULLIVAN et al.** *BioTechniques,* 1993, vol. 15, 637-641
- **VOLKERDING et al.** *Clin Chem,* 2009, vol. 55, 641-658
- **METZKER M.** *Nature Rev,* 2010, vol. 11, 31-46
- **FAN et al.** *Proc Natl Acad Sci U S A,* 2008, vol. 105, 16266-16271
- **CHIU et al.** *Proc Natl Acad Sci U S A,* 2008, vol. 105, 20458-20463
- **HARRIS T.D. et al.** *Science,* 2008, vol. 320, 106-109
- **MARGULIES, M. et al.** *Nature,* 2005, vol. 437, 376-380
- **SONI GV ; MELLER A.** *Clin Chem,* 2007, vol. 53, 1996-2001
- **BENTLEY et al.** *Nature,* 2009, vol. 6, 53-59
- **LANGMEAD et al.** *Genome Biology,* 2009, vol. 10, R25.1-R25.10

- **CHIU et al.** *Clin Chem,* 2010, vol. 56, 459-463
- **PAKSTIS et al.** *Hum Genet,* 2010, vol. 127, 315-324
- *BioTechniques.RTM. Protocol Guide 2007,* December 2006, 29
- **GHANTA et al.** *PLOS ONE,* 2010, vol. 5 (10
- **BUTLER et al.** *J Forensic Sci,* vol. 5, 1054-1064
- **HILL et al.** *Poster #44- 17th International Symposium on Human Identification,* 2006
- **HANSON ; BALLANTYNE.** *Hanson and Ballantyne, Analytical Biochem,* 2005, vol. 346, 246-257

SEQUENCE LISTING

<110> VERINATA HEALTH, INC.

<120> METHODS FOR DETERMINING FRACTION OF FETAL NUCLEIC ACIDS IN MATERNAL SAMPLES

<130> P072639EP

<140> PCT/US2010/058606
<141> 2010-12-01

<150> 61/455,849
<151> 2010-10-26

<150> 61/407,017
<151> 2010-10-26

<150> 61/360,837
<151> 2010-07-01

<150> 61/296,358
<151> 2010-01-19

<160> 427

<170> Patent In version 3.5

<210> 1
<211> 111
<212> DNA
<213> Homo sapiens

<400> 1

**cacatgcaca gccagcaacc ctgtcagcag gagttcccac cagtttcttt ctgagaacat 60**

**ctgttcaggt ttctctccat ctctatttac tcaggtcaca ggaccttggg g 111**

<210> 2
<211> 111
<212> DNA
<213> Homo sapiens

<400> 2

**cacatgcaca gccagcaacc ctgtcagcag gagttcccac cagtttcttt ctgagaacat 60**

**ctgttcaggt ttctctccat ctctgtttac tcaggtcaca ggaccttggg g 111**

<210> 3
<211> 126
<212> DNA
<213> Homo sapiens

<400> 3

**tgaggaagtg aggctcagag ggtaagaaac tttgtcacag agctggtggt gagggtggag 60**

**attttacact ccctgcctcc cacaccagtt tctccagagt ggaaagactt tcatctcgca 120**

**ctggca 126**

<210> 4
<211> 126

<212> DNA
<213> Homo sapiens

<400> 4

tgaggaagtg aggctcagag ggtaagaaac tttgtcacag agctggtggt gagggtggag 60

attttacact ccctgcctcc cacaccagtt tctccggagt ggaaagactt tcatctcgca 120

ctggca 126

<210> 5
<211> 121
<212> DNA
<213> Homo sapiens

<400> 5

gtgccttcag aacctttgag atctgattct atttttaaag cttcttagaa gagagattgc 60

aaagtgggtt gtttctctag ccagacaggg caggcaaata ggggtggctg gtgggatggg 120

a 121

<210> 6
<211> 121
<212> DNA
<213> Homo sapiens

<400> 6

gtgccttcag aacctttgag atctgattct atttttaaag cttcttagaa gagagattgc 60

aaagtgggtt gtttctctag ccagacaggg caggtaaata ggggtggctg gtgggatggg 120

a 121

<210> 7
<211> 111
<212> DNA
<213> Homo sapiens

<400> 7

aggtgtgtct ctcttttgtg aggggagggg tcccttctgg cctagtagag ggcctggcct 60

gcagtgagca ttcaaatcct caaggaacag ggtggggagg tgggacaaag g 111

<210> 8
<211> 111
<212> DNA
<213> Homo sapiens

<400> 8

aggtgtgtct ctcttttgtg aggggagggg tcccttctgg cctagtagag ggcctggcct 60

gcagtgagca ttcaaatcct cgaggaacag ggtggggagg tgggacaaag g 111

<210> 9
<211> 139
<212> DNA
<213> Homo sapiens

<400> 9

cctcgcctac tgtgctgttt ctaaccatca tgcttttccc tgaatctctt gagtcttttt 60

ctgctgtgga ctgaaacttg atcctgagat tcacctctag tccctctgag cagcctcctg 120

gaatactcag ctgggatgg 139

<210> 10
<211> 139
<212> DNA
<213> Homo sapiens

<400> 10

cctcgcctac tgtgctgttt ctaaccatca tgcttttccc tgaatctctt gagtcttttt 60

ctgctgtgga ctgaaacttg atcctgagat tcacctctag tccctctggg cagcctcctg 120

gaatactcag ctgggatgg 139

<210> 11
<211> 117
<212> DNA
<213> Homo sapiens

<400> 11

aattgcaatg gtgagaggtt gatggtaaaa tcaaacggaa cttgttattt tgtcattctg 60

atggactgga actgaggatt ttcaatttcc tctccaaccc aagacacttc tcactgg 117

<210> 12
<211> 117
<212> DNA
<213> Homo sapiens

<400> 12

aattgcaatg gtgagaggtt gatggtaaaa tcaaacggaa cttgttattt tgtcattctg 60

atggactgga actgaggatt ttcaatttcc tttccaaccc aagacacttc tcactgg 117

<210> 13
<211> 114
<212> DNA

<213> Homo sapiens

<400> 13

```
gaaatgcctt ctcaggtaat ggaaggttat ccaaatattt ttcgtaagta tttcaaatag        60
caatggctcg tctatggtta gtctcacagc cacattctca gaactgctca aacc             114
```

<210> 14
<211> 114
<212> DNA
<213> Homo sapiens

<400> 14

```
gaaatgcctt ctcaggtaat ggaaggttat ccaaatattt ttcgtaagta tttcaaatag        60
caatggctcg tctatggtta gtctcgcagc cacattctca gaactgctca aacc             114
```

<210> 15
<211> 128
<212> DNA
<213> Homo sapiens

<400> 15

```
acccaaaaca ctggaggggc ctcttctcat tttcggtaga ctgcaagtgt tagccgtcgg        60
gaccagcttc tgtctggaag ttcgtcaaat tgcagttaag tccaagtatg ccacatagca       120
gataaggg                                                                128
```

<210> 16
<211> 128
<212> DNA
<213> Homo sapiens

<400> 16

```
acccaaaaca ctggaggggc ctcttctcat tttcggtaga ctgcaagtgt tagccgtcgg        60
gaccagcttc tgtctggaag ttcgtcaaat tgcagttagg tccaagtatg ccacatagca       120
gataaggg                                                                128
```

<210> 17
<211> 110
<212> DNA
<213> Homo sapiens

<400> 17

```
gcaccagaat ttaaacaacg ctgacaataa atatgcagtc gatgatgact tcccagagct        60
ccagaagcaa ctccagcaca cagagaggcg ctgatgtgcc tgtcaggtgc                  110
```

<210> 18
<211> 110
<212> DNA
<213> Homo sapiens

<400> 18

gcaccagaat ttaaacaacg ctgacaataa atatgcagtc gatgatgact tcccagagct 60

ccagaagcaa ctccagcaca cggagaggcg ctgatgtgcc tgtcaggtgc 110

<210> 19
<211> 116
<212> DNA
<213> Homo sapiens

<400> 19

tgactgtata ccccaggtgc acccttgggt catctctatc atagaactta tctcacagag 60

tataagagct gatttctgtg tctgcctctc acactagact tccacatcct tagtgc 116

<210> 20
<211> 116
<212> DNA
<213> Homo sapiens

<400> 20

tgactgtata ccccaggtgc acccttgggt catctctatc atagaactta tctcacagag 60

tataagagct gatttctgtg tctgcctgtc acactagact tccacatcct tagtgc 116

<210> 21
<211> 110
<212> DNA
<213> Homo sapiens

<400> 21

tgtacgtggt caccagggga cgcctggcgc tgcgagggag gccccgagcc tcgtgccccc 60

gtgaagcttc agctcccctc cccggctgtc cttgaggctc ttctcacact 110

<210> 22
<211> 110
<212> DNA
<213> Homo sapiens

<400> 22

tgtacgtggt caccagggga cgcctggcgc tgcgagggag gccccgagcc tcgtgccccc 60

gtgaagcttc agctcccctc cctggctgtc cttgaggctc ttctcacact 110

<210> 23
<211> 114
<212> DNA
<213> Homo sapiens

<400> 23

cagtggaccc tgctgcacct ttcctcccct cccatcaacc tcttttgtgc ctccccctcc 60

gtgtaccacc ttctctgtca ccaaccctgg cctcacaact ctctcctttg ccac 114

<210> 24
<211> 114
<212> DNA
<213> Homo sapiens

<400> 24

cagtggaccc tgctgcacct ttcctcccct cccatcaacc tcttttgtgc ctccccctcc 60

gtgtaccacc ttctctgtca ccacccctgg cctcacaact ctctcctttg ccac 114

<210> 25
<211> 110
<212> DNA
<213> Homo sapiens

<400> 25

cagtggcata gtagtccagg ggctcctcct cagcacctcc agcaccttcc aggaggcagc 60

agcgcaggca gagaacccgc tggaagaatc ggcggaagtt gtcggagagg 110

<210> 26
<211> 110
<212> DNA
<213> Homo sapiens

<400> 26

cagtggcata gtagtccagg ggctcctcct cagcacctcc agcaccttcc aggaggcagc 60

agcgcaggca gagaacccgc tggaaggatc ggcggaagtt gtcggagagg 110

<210> 27
<211> 129
<212> DNA
<213> Homo sapiens

<400> 27

```
aggtctgggg gccgctgaat gccaagctgg gaatcttaaa tgttaaggaa caaggtcata      60
caatgaatgg tgtgatgtaa aagcttggga ggtgatttct gagggtaggt gctgggttta     120
atgggagga                                                             129
```

<210> 28
<211> 129
<212> DNA
<213> Homo sapiens

<400> 28

```
aggtctgggg gccgctgaat gccaagctgg gaatcttaaa tgttaaggaa caaggtcata      60
caatgaatgg tgtgatgtaa aagcttggga ggtgattttt gagggtaggt gctgggttta     120
atgggagga                                                             129
```

<210> 29
<211> 107
<212> DNA
<213> Homo sapiens

<400> 29

```
acggttctgt cctgtagggg agaaaagtcc tcgttgttcc tctgggatgc aacatgagag      60
agcagcacac tgaggcttta tggattgccc tgccacaagt gaacagg                   107
```

<210> 30
<211> 107
<212> DNA
<213> Homo sapiens

<400> 30

```
acggttctgt cctgtagggg agaaaagtcc tcgttgttcc tctgggatgc aacatgagag      60
agcagcacac tgaggcttta tgggttgccc tgccacaagt gaacagg                   107
```

<210> 31
<211> 127
<212> DNA
<213> Homo sapiens

<400> 31

```
gcgcagtcag atgggcgtgc tggcgtctgt cttctctctc tcctgctctc tggcttcatt      60
tttctctcct tctgtctcac cttctttcgt gtgcctgtgc acacacacgt ttgggacaag     120
ggctgga                                                               127
```

<210> 32
<211> 127
<212> DNA
<213> Homo sapiens

<400> 32

gcgcagtcag atgggcgtgc tggcgtctgt cttctctctc tcctgctctc tggcttcatt 60

tttctctcct tctgtctcac cttctttcgt gtgcctgtgc atacacacgt ttgggacaag 120

ggctgga 127

<210> 33
<211> 130
<212> DNA
<213> Homo sapiens

<400> 33

gccggacctg cgaaatccca aaatgccaaa cattcccgcc tcacatgatc ccagagagag 60

gggacccagt gttcccagct tgcagctgag gagcccgagg ttgccgtcag atcagagccc 120

cagttgcccg 130

<210> 34
<211> 130
<212> DNA
<213> Homo sapiens

<400> 34

gccggacctg cgaaatccca aaatgccaaa cattcccgcc tcacatgatc ccagagagag 60

gggacccagt gttcccagct tgcagctgag gagcccgagt ttgccgtcag atcagagccc 120

cagttgcccg 130

<210> 35
<211> 121
<212> DNA
<213> Homo sapiens

<400> 35

agcagcctcc ctcgactagc tcacactacg ataaggaaaa ttcatgagct ggtgtccaag 60

gagggctggg tgactcgtgg ctcagtcagc atcaagattc ctttcgtctt tcccctctgc 120

c 121

<210> 36
<211> 121
<212> DNA
<213> Homo sapiens

<400> 36

agcagcctcc ctcgactagc tcacactacg ataaggaaaa ttcatgagct ggtgtccaag 60

gagggctggg tgactcgtgg ctcagtcagc gtcaagattc ctttcgtctt tcccctctgc 120

c 121

<210> 37
<211> 138
<212> DNA
<213> Homo sapiens

<400> 37

tggcattgcc tgtaatatac atagccatgg ttttttatag gcaatttaag atgaatagct 60

tctaaactat agataagttt cattacccca ggaagctgaa ctatagctac tttacccaaa 120

atcattagaa tggtgctt 138

<210> 38
<211> 138
<212> DNA
<213> Homo sapiens

<400> 38

tggcattgcc tgtaatatac atagccatgg ttttttatag gcaatttaag atgaatagct 60

tctaaactat agataagttt cattacccca ggaagctgaa ctatagctac tttccccaaa 120

atcattagaa tggtgctt 138

<210> 39
<211> 136
<212> DNA
<213> Homo sapiens

<400> 39

atgaagcctt ccaccaactg cctgtatgac tcatctgggg acttctgctc tatactcaaa 60

gtggcttagt cactgccaat gtatttccat atgagggacg atgattacta aggaaatata 120

gaaacaacaa ctgatc 136

<210> 40
<211> 136
<212> DNA
<213> Homo sapiens

<400> 40

atgaagcctt ccaccaactg cctgtatgac tcatctgggg acttctgctc tatactcaaa 60

gtggcttagt cactgccaat gtatttccat atgagggacg gtgattacta aggaaatata 120

gaaacaacaa ctgatc 136

<210> 41
<211> 118
<212> DNA
<213> Homo sapiens

<400> 41

acaacagaat caggtgattg gagaaaagat cacaggccta ggcacccaag gcttgaagga 60

tgaaagaatg aaagatggac ggaacaaaat taggacctta attctttgtt cagttcag 118

<210> 42
<211> 118
<212> DNA
<213> Homo sapiens

<400> 42

acaacagaat caggtgattg gagaaaagat cacaggccta ggcacccaag gcttgaagga 60

tgaaagaatg aaagatggac ggaagaaaat taggacctta attctttgtt cagttcag 118

<210> 43
<211> 150
<212> DNA
<213> Homo sapiens

<400> 43

ttggggtaaa ttttcattgt catatgtgga atttaaatat accatcatct acaaagaatt 60

ccacagagtt aaatatctta agttaaacac ttaaaataag tgtttgcgtg atattttgat 120

gacagataaa cagagtctaa ttcccacccc 150

<210> 44
<211> 150
<212> DNA
<213> Homo sapiens

<400> 44

ttggggtaaa ttttcattgt catatgtgga atttaaatat accatcatct acaaagaatt 60

ccacagagtt aaatatctta agttaaacac ttaaaataag tgtttgcgtg atattttgat 120

gatagataaa cagagtctaa ttcccacccc 150

<210> 45
<211> 145
<212> DNA
<213> Homo sapiens

<400> 45

tgcaattcaa atcaggaagt atgaccaaaa gacagagatc ttttttggat gatccctagc 60

ctagcaatgc ctggcagcca tgcaggtgca atgtcaacct taaataatgt attgcaaact 120

cagagctgac aaacctcgat gttgc 145

<210> 46
<211> 145
<212> DNA
<213> Homo sapiens

<400> 46

tgcaattcaa atcaggaagt atgaccaaaa gacagagatc ttttttggat gatccctagc 60

ctagcaatgc ctggcagcca tgcaggtgca atgtcaacct taaataatgt attgcaaatt 120

cagagctgac aaacctcgat gttgc 145

<210> 47
<211> 124
<212> DNA
<213> Homo sapiens

<400> 47

ctgtgctctg cgaatagctg cagaagtaac ttggggaccc aaaataaagc agaatgctaa 60

tgtcaagtcc tgagaaccaa gccctgggac tctggtgcca tttcggattc tccatgagca 120

tggt 124

<210> 48
<211> 124
<212> DNA
<213> Homo sapiens

<400> 48

ctgtgctctg cgaatagctg cagaagtaac ttggggaccc aaaataaagc agaatgctaa 60

tgtcaagtcc tgagaaccaa gccctgggac tctggtgcca ttttggattc tccatgagca 120

tggt 124

<210> 49
<211> 118
<212> DNA
<213> Homo sapiens

<400> 49

tttttccagc caactcaagg ccaaaaaaaa tttcttaata tagttattat gcgaggggag 60

gggaagcaaa ggagcacagg tagtccacag aataagacac aagaaacctc aagctgtg 118

<210> 50
<211> 118
<212> DNA
<213> Homo sapiens

<400> 50

tttttccagc caactcaagg ccaaaaaaaa tttcttaata tagttattat gcgaggggag 60

gggaagcaaa ggagcacagg tagtccacag aataggacac aagaaacctc aagctgtg 118

<210> 51
<211> 110
<212> DNA
<213> Homo sapiens

<400> 51

tcttctcgtc ccctaagcaa acaacatccg cttgcttctg tctgtgtaac cacagtgaat 60

gggtgtgcac gcttgatggg cctctgagcc cctgttgcac aaaccagaaa 110

<210> 52
<211> 110
<212> DNA
<213> Homo sapiens

<400> 52

tcttctcgtc ccctaagcaa acaacatccg cttgcttctg tctgtgtaac cacagtgaat 60

gggtgtgcac gcttggtggg cctctgagcc cctgttgcac aaaccagaaa 110

<210> 53
<211> 110
<212> DNA
<213> Homo sapiens

<400> 53

cacatggggg cattaagaat cgcccaggga ggaggaggga gaacgcgtgc ttttcacatt 60

tgcatttgaa ttttcgagtt cccaggatgt gtttttgtgc tcatcgatgt 110

<210> 54
<211> 110
<212> DNA
<213> Homo sapiens

<400> 54

cacatggggg cattaagaat cgcccaggga ggaggaggga gaacgcgtgc ttttcacatt 60

tgcatttgaa tttttgagtt cccaggatgt gtttttgtgc tcatcgatgt 110

<210> 55
<211> 128
<212> DNA
<213> Homo sapiens

<400> 55

gggctctgag gtgtgtgaaa taaaaacaaa tgtccatgtc tgtcctttta tggcattttg 60

ggactttaca tttcaaacat ttcagacatg tatcacaaca cgaaggaata acagttccag 120

ggatatct 128

<210> 56
<211> 128
<212> DNA
<213> Homo sapiens

<400> 56

gggctctgag gtgtgtgaaa taaaaacaaa tgtccatgtc tgtcctttta tggcattttg 60

ggactttaca tttcaaacat ttcagacatg tatcacaaca cgagggaata acagttccag 120

ggatatct 128

<210> 57
<211> 21
<212> DNA
<213> Artificial Sequence

<220>
<223> Description of Artificial Sequence: Synthetic primer

<400> 57
cacatgcaca gccagcaacc c 21

<210> 58
<211> 23
<212> DNA
<213> Artificial Sequence

<220>
<223> Description of Artificial Sequence: Synthetic primer

<400> 58
ccccaaggtc ctgtgacctg agt 23

<210> 59
<211> 23
<212> DNA
<213> Artificial Sequence

<220>
<223> Description of Artificial Sequence: Synthetic primer

<400> 59
tgaggaagtg aggctcagag ggt 23

<210> 60
<211> 24
<212> DNA
<213> Artificial Sequence

<220>
<223> Description of Artificial Sequence: Synthetic primer

<400> 60
tgccagtgcg agatgaaagt cttt 24

<210> 61
<211> 27
<212> DNA
<213> Artificial Sequence

<220>
<223> Description of Artificial Sequence: Synthetic primer

<400> 61
gtgccttcag aacctttgag atctgat 27

<210> 62
<211> 20
<212> DNA
<213> Artificial Sequence

<220>
<223> Description of Artificial Sequence: Synthetic primer

<400> 62
tcccatccca ccagccaccc 20

<210> 63
<211> 25
<212> DNA
<213> Artificial Sequence

<220>
<223> Description of Artificial Sequence: Synthetic primer

<400> 63
aggtgtgtct ctcttttgtg agggg 25

<210> 64
<211> 21
<212> DNA
<213> Artificial Sequence

<220>
<223> Description of Artificial Sequence: Synthetic primer

<400> 64
cctttgtccc acctccccac c 21

<210> 65
<211> 26
<212> DNA
<213> Artificial Sequence

<220>
<223> Description of Artificial Sequence: Synthetic primer

<400> 65
cctcgcctac tgtgctgttt ctaacc 26

<210> 66
<211> 25
<212> DNA
<213> Artificial Sequence

<220>
<223> Description of Artificial Sequence: Synthetic primer

<400> 66
ccatcccagc tgagtattcc aggag 25

<210> 67
<211> 26
<212> DNA
<213> Artificial Sequence

<220>
<223> Description of Artificial Sequence: Synthetic primer

<400> 67
aattgcaatg gtgagaggtt gatggt 26

<210> 68
<211> 24
<212> DNA
<213> Artificial Sequence

<220>
<223> Description of Artificial Sequence: Synthetic primer

<400> 68
ccagtgagaa gtgtcttggg ttgg 24

<210> 69
<211> 27
<212> DNA
<213> Artificial Sequence

<220>
<223> Description of Artificial Sequence: Synthetic primer

<400> 69
gaaatgcctt ctcaggtaat ggaaggt 27

<210> 70
<211> 27
<212> DNA
<213> Artificial Sequence

<220>
<223> Description of Artificial Sequence: Synthetic primer

<400> 70
ggtttgagca gttctgagaa tgtggct 27

<210> 71
<211> 22
<212> DNA
<213> Artificial Sequence

<220>
<223> Description of Artificial Sequence: Synthetic primer

<400> 71
acccaaaaca ctggaggggc ct 22

<210> 72
<211> 27
<212> DNA
<213> Artificial Sequence

<220>
<223> Description of Artificial Sequence: Synthetic primer

<400> 72
cccttatctg ctatgtggca tacttgg 27

<210> 73
<211> 27
<212> DNA
<213> Artificial Sequence

<220>
<223> Description of Artificial Sequence: Synthetic primer

<400> 73
gcaccagaat ttaaacaacg ctgacaa 27

<210> 74
<211> 22
<212> DNA
<213> Artificial Sequence

<220>
<223> Description of Artificial Sequence: Synthetic primer

<400> 74
gcacctgaca ggcacatcag cg 22

<210> 75
<211> 24
<212> DNA

<213> Artificial Sequence

<220>
<223> Description of Artificial Sequence: Synthetic primer

<400> 75
tgactgtata ccccaggtgc accc 24

<210> 76
<211> 27
<212> DNA
<213> Artificial Sequence

<220>
<223> Description of Artificial Sequence: Synthetic primer

<400> 76
gcactaagga tgtggaagtc tagtgtg 27

<210> 77
<211> 22
<212> DNA
<213> Artificial Sequence

<220>
<223> Description of Artificial Sequence: Synthetic primer

<400> 77
tgtacgtggt caccagggga cg 22

<210> 78
<211> 26
<212> DNA
<213> Artificial Sequence

<220>
<223> Description of Artificial Sequence: Synthetic primer

<400> 78
agtgtgagaa gagcctcaag gacagc 26

<210> 79
<211> 21
<212> DNA
<213> Artificial Sequence

<220>
<223> Description of Artificial Sequence: Synthetic primer

<400> 79
cagtggaccc tgctgcacct t 21

<210> 80
<211> 24
<212> DNA
<213> Artificial Sequence

<220>

<223> Description of Artificial Sequence: Synthetic primer

<400> 80
gtggcaaagg agagagttgt gagg 24

<210> 81
<211> 24
<212> DNA
<213> Artificial Sequence

<220>
<223> Description of Artificial Sequence: Synthetic primer

<400> 81
cagtggcata gtagtccagg ggct 24

<210> 82
<211> 21
<212> DNA
<213> Artificial Sequence

<220>
<223> Description of Artificial Sequence: Synthetic primer

<400> 82
cctctccgac aacttccgcc g 21

<210> 83
<211> 20
<212> DNA
<213> Artificial Sequence

<220>
<223> Description of Artificial Sequence: Synthetic primer

<400> 83
aggtctgggg gccgctgaat 20

<210> 84
<211> 23
<212> DNA
<213> Artificial Sequence

<220>
<223> Description of Artificial Sequence: Synthetic primer

<400> 84
tcctcccatt aaacccagca cct 23

<210> 85
<211> 23
<212> DNA
<213> Artificial Sequence

<220>
<223> Description of Artificial Sequence: Synthetic primer

<400> 85

acggttctgt cctgtagggg aga 23

<210> 86
<211> 22
<212> DNA
<213> Artificial Sequence

<220>
<223> Description of Artificial Sequence: Synthetic primer

<400> 86
cctgttcact tgtggcaggg ca 22

<210> 87
<211> 20
<212> DNA
<213> Artificial Sequence

<220>
<223> Description of Artificial Sequence: Synthetic primer

<400> 87
gcgcagtcag atgggcgtgc 20

<210> 88
<211> 23
<212> DNA
<213> Artificial Sequence

<220>
<223> Description of Artificial Sequence: Synthetic primer

<400> 88
tccagccctt gtcccaaacg tgt 23

<210> 89
<211> 21
<212> DNA
<213> Artificial Sequence

<220>
<223> Description of Artificial Sequence: Synthetic primer

<400> 89
gccggacctg cgaaatccca a 21

<210> 90
<211> 21
<212> DNA
<213> Artificial Sequence

<220>
<223> Description of Artificial Sequence: Synthetic primer

<400> 90
cgggcaactg gggctctgat c 21

<210> 91

<211> 21
<212> DNA
<213> Artificial Sequence

<220>
<223> Description of Artificial Sequence: Synthetic primer

<400> 91
agcagcctcc ctcgactagc t 21

<210> 92
<211> 23
<212> DNA
<213> Artificial Sequence

<220>
<223> Description of Artificial Sequence: Synthetic primer

<400> 92
ggcagagggg aaagacgaaa gga 23

<210> 93
<211> 24
<212> DNA
<213> Artificial Sequence

<220>
<223> Description of Artificial Sequence: Synthetic primer

<400> 93
tggcattgcc tgtaatatac atag 24

<210> 94
<211> 23
<212> DNA
<213> Artificial Sequence

<220>
<223> Description of Artificial Sequence: Synthetic primer

<400> 94
aagcaccatt ctaatgattt tgg 23

<210> 95
<211> 20
<212> DNA
<213> Artificial Sequence

<220>
<223> Description of Artificial Sequence: Synthetic primer

<400> 95
atgaagcctt ccaccaactg 20

<210> 96
<211> 27
<212> DNA
<213> Artificial Sequence

<220>
<223> Description of Artificial Sequence: Synthetic primer

<400> 96
gatcagttgt tgtttctata tttcctt 27

<210> 97
<211> 22
<212> DNA
<213> Artificial Sequence

<220>
<223> Description of Artificial Sequence: Synthetic primer

<400> 97
acaacagaat caggtgattg ga 22

<210> 98
<211> 25
<212> DNA
<213> Artificial Sequence

<220>
<223> Description of Artificial Sequence: Synthetic primer

<400> 98
ctgaactgaa caaagaatta aggtc 25

<210> 99
<211> 22
<212> DNA
<213> Artificial Sequence

<220>
<223> Description of Artificial Sequence: Synthetic primer

<400> 99
ttggggtaaa ttttcattgt ca 22

<210> 100
<211> 20
<212> DNA
<213> Artificial Sequence

<220>
<223> Description of Artificial Sequence: Synthetic primer

<400> 100
ggggtgggaa ttagactctg 20

<210> 101
<211> 23
<212> DNA
<213> Artificial Sequence

<220>
<223> Description of Artificial Sequence: Synthetic primer

<400> 101
tgcaattcaa atcaggaagt atg 23

<210> 102
<211> 20
<212> DNA
<213> Artificial Sequence

<220>
<223> Description of Artificial Sequence: Synthetic primer

<400> 102
gcaacatcga ggtttgtcag 20

<210> 103
<211> 20
<212> DNA
<213> Artificial Sequence

<220>
<223> Description of Artificial Sequence: Synthetic primer

<400> 103
ctgtgctctg cgaatagctg 20

<210> 104
<211> 20
<212> DNA
<213> Artificial Sequence

<220>
<223> Description of Artificial Sequence: Synthetic primer

<400> 104
accatgctca tggagaatcc 20

<210> 105
<211> 20
<212> DNA
<213> Artificial Sequence

<220>
<223> Description of Artificial Sequence: Synthetic primer

<400> 105
tttttccagc caactcaagg 20

<210> 106
<211> 21
<212> DNA
<213> Artificial Sequence

<220>
<223> Description of Artificial Sequence: Synthetic primer

<400> 106
cacagcttga ggtttcttgt g 21

<210> 107
<211> 20
<212> DNA
<213> Artificial Sequence

<220>
<223> Description of Artificial Sequence: Synthetic primer

<400> 107
tcttctcgtc ccctaagcaa 20

<210> 108
<211> 20
<212> DNA
<213> Artificial Sequence

<220>
<223> Description of Artificial Sequence: Synthetic primer

<400> 108
tttctggttt gtgcaacagg 20

<210> 109
<211> 20
<212> DNA
<213> Artificial Sequence

<220>
<223> Description of Artificial Sequence: Synthetic primer

<400> 109
cacatggggg cattaagaat 20

<210> 110
<211> 22
<212> DNA
<213> Artificial Sequence

<220>
<223> Description of Artificial Sequence: Synthetic primer

<400> 110
acatcgatga gcacaaaaac ac 22

<210> 111
<211> 20
<212> DNA
<213> Artificial Sequence

<220>
<223> Description of Artificial Sequence: Synthetic primer

<400> 111
gggctctgag gtgtgtgaaa 20

<210> 112
<211> 24
<212> DNA

<213> Artificial Sequence

<220>
<223> Description of Artificial Sequence: Synthetic primer

<400> 112
agatatccct ggaactgtta ttcc 24

<210> 113
<211> 23
<212> DNA
<213> Artificial Sequence

<220>
<223> Description of Artificial Sequence: Synthetic primer

<400> 113
acagtaactg ccttcataga tag 23

<210> 114
<211> 22
<212> DNA
<213> Artificial Sequence

<220>
<223> Description of Artificial Sequence: Synthetic primer

<400> 114
gtgtcagacc ctgttctaag ta 22

<210> 115
<211> 26
<212> DNA
<213> Artificial Sequence

<220>
<223> Description of Artificial Sequence: Synthetic primer

<400> 115
aaataaaatt aggcatattt acaagc 26

<210> 116
<211> 23
<212> DNA
<213> Artificial Sequence

<220>
<223> Description of Artificial Sequence: Synthetic primer

<400> 116
gctgagtgat ttgtctgtaa ttg 23

<210> 117
<211> 21
<212> DNA
<213> Artificial Sequence

<220>

<223> Description of Artificial Sequence: Synthetic primer

<400> 117
cctgttcctc ccttatttcc c 21

<210> 118
<211> 21
<212> DNA
<213> Artificial Sequence

<220>
<223> Description of Artificial Sequence: Synthetic primer

<400> 118
gggaacacag actccatggt g 21

<210> 119
<211> 22
<212> DNA
<213> Artificial Sequence

<220>
<223> Description of Artificial Sequence: Synthetic primer

<400> 119
cttagggaac cctcactgaa tg 22

<210> 120
<211> 22
<212> DNA
<213> Artificial Sequence

<220>
<223> Description of Artificial Sequence: Synthetic primer

<400> 120
gtccttgtca gcgtttattt gc 22

<210> 121
<211> 26
<212> DNA
<213> Artificial Sequence

<220>
<223> Description of Artificial Sequence: Synthetic primer

<400> 121
aataatcagt atgtgacttg gattga 26

<210> 122
<211> 22
<212> DNA
<213> Artificial Sequence

<220>
<223> Description of Artificial Sequence: Synthetic primer

<400> 122

ataggatgga tggatagatg ga 22

<210> 123
<211> 21
<212> DNA
<213> Artificial Sequence

<220>
<223> Description of Artificial Sequence: Synthetic primer

<400> 123
cagagcaaga ccctgtctca t 21

<210> 124
<211> 21
<212> DNA
<213> Artificial Sequence

<220>
<223> Description of Artificial Sequence: Synthetic primer

<400> 124
tcaacagagg cttgcatgta t 21

<210> 125
<211> 20
<212> DNA
<213> Artificial Sequence

<220>
<223> Description of Artificial Sequence: Synthetic primer

<400> 125
gggtgatttt cctctttggt 20

<210> 126
<211> 33
<212> DNA
<213> Artificial Sequence

<220>
<223> Description of Artificial Sequence: Synthetic primer

<400> 126
aacatttgta tctttatctg tatccttatt tat 33

<210> 127
<211> 27
<212> DNA
<213> Artificial Sequence

<220>
<223> Description of Artificial Sequence: Synthetic primer

<400> 127
gaacacttgt catagtttag aacgaac 27

<210> 128

<211> 20
<212> DNA
<213> Artificial Sequence

<220>
<223> Description of Artificial Sequence: Synthetic primer

<400> 128
tcattgacag aattgcacca 20

<210> 129
<211> 27
<212> DNA
<213> Artificial Sequence

<220>
<223> Description of Artificial Sequence: Synthetic primer

<400> 129
tttgtatttc atgtgtacat tcgtatc 27

<210> 130
<211> 28
<212> DNA
<213> Artificial Sequence

<220>
<223> Description of Artificial Sequence: Synthetic primer

<400> 130
acctatcctg tagattattt tcactgtg 28

<210> 131
<211> 20
<212> DNA
<213> Artificial Sequence

<220>
<223> Description of Artificial Sequence: Synthetic primer

<400> 131
tctgacccat ctaacgccta 20

<210> 132
<211> 27
<212> DNA
<213> Artificial Sequence

<220>
<223> Description of Artificial Sequence: Synthetic primer

<400> 132
cagacagaaa gatagataga tgattga 27

<210> 133
<211> 21
<212> DNA
<213> Artificial Sequence

<220>
<223> Description of Artificial Sequence: Synthetic primer

<400> 133
atacagacag acagacaggt g 21

<210> 134
<211> 23
<212> DNA
<213> Artificial Sequence

<220>
<223> Description of Artificial Sequence: Synthetic primer

<400> 134
gcatgtatct atcatccatc tct 23

<210> 135
<211> 21
<212> DNA
<213> Artificial Sequence

<220>
<223> Description of Artificial Sequence: Synthetic primer

<400> 135
tgagtgacaa attgagacct t 21

<210> 136
<211> 26
<212> DNA
<213> Artificial Sequence

<220>
<223> Description of Artificial Sequence: Synthetic primer

<400> 136
gtcttacaat aacagttgct actatt 26

<210> 137
<211> 18
<212> DNA
<213> Artificial Sequence

<220>
<223> Description of Artificial Sequence: Synthetic primer

<400> 137
attccccaag tgaattgc 18

<210> 138
<211> 26
<212> DNA
<213> Artificial Sequence

<220>
<223> Description of Artificial Sequence: Synthetic primer

<400> 138
ggtagataga ctggatagat agacga 26

<210> 139
<211> 20
<212> DNA
<213> Artificial Sequence

<220>
<223> Description of Artificial Sequence: Synthetic primer

<400> 139
tggaaacaga aatggcttgg 20

<210> 140
<211> 20
<212> DNA
<213> Artificial Sequence

<220>
<223> Description of Artificial Sequence: Synthetic primer

<400> 140
gattgcagga gggaaggaag 20

<210> 141
<211> 22
<212> DNA
<213> Artificial Sequence

<220>
<223> Description of Artificial Sequence: Synthetic primer

<400> 141
gagcaagaca ccatctcaag aa 22

<210> 142
<211> 30
<212> DNA
<213> Artificial Sequence

<220>
<223> Description of Artificial Sequence: Synthetic primer

<400> 142
gaaattttac atttatgttt atgattctct 30

<210> 143
<211> 18
<212> DNA
<213> Artificial Sequence

<220>
<223> Description of Artificial Sequence: Synthetic primer

<400> 143
ggcgactgag caagactc 18

<210> 144
<211> 27
<212> DNA
<213> Artificial Sequence

<220>
<223> Description of Artificial Sequence: Synthetic primer

<400> 144
ggttattaat tgagaaaact ccttaca 27

<210> 145
<211> 23
<212> DNA
<213> Artificial Sequence

<220>
<223> Description of Artificial Sequence: Synthetic primer

<400> 145
attttccccg atgatagtag tct 23

<210> 146
<211> 26
<212> DNA
<213> Artificial Sequence

<220>
<223> Description of Artificial Sequence: Synthetic primer

<400> 146
gcgaatgtat gattggcaat attttt 26

<210> 147
<211> 27
<212> DNA
<213> Artificial Sequence

<220>
<223> Description of Artificial Sequence: Synthetic primer

<400> 147
acatgtatcc cagaacttaa agtaaac 27

<210> 148
<211> 22
<212> DNA
<213> Artificial Sequence

<220>
<223> Description of Artificial Sequence: Synthetic primer

<400> 148
gcagaaggga aaattgaagc tg 22

<210> 149
<211> 22
<212> DNA

<213> Artificial Sequence

<220>
<223> Description of Artificial Sequence: Synthetic primer

<400> 149
cagagacacc gaaccaataa ga 22

<210> 150
<211> 27
<212> DNA
<213> Artificial Sequence

<220>
<223> Description of Artificial Sequence: Synthetic primer

<400> 150
gccacatgaa tcaattccta taataaa 27

<210> 151
<211> 25
<212> DNA
<213> Artificial Sequence

<220>
<223> Description of Artificial Sequence: Synthetic primer

<400> 151
gcacatgtac cctaaaactt aaaat 25

<210> 152
<211> 26
<212> DNA
<213> Artificial Sequence

<220>
<223> Description of Artificial Sequence: Synthetic primer

<400> 152
gtcaaccaaa actcaacaag tagtaa 26

<210> 153
<211> 25
<212> DNA
<213> Artificial Sequence

<220>
<223> Description of Artificial Sequence: Synthetic primer

<400> 153
aagatgaaat tgccatgtaa aaata 25

<210> 154
<211> 27
<212> DNA
<213> Artificial Sequence

<220>

<223> Description of Artificial Sequence: Synthetic primer

<400> 154
gtgtgtataa caaaattcct atgatgg 27

<210> 155
<211> 22
<212> DNA
<213> Artificial Sequence

<220>
<223> Description of Artificial Sequence: Synthetic primer

<400> 155
gcacccaaaa ctgaatgtca ta 22

<210> 156
<211> 20
<212> DNA
<213> Artificial Sequence

<220>
<223> Description of Artificial Sequence: Synthetic primer

<400> 156
ggtgagagtg agaccctgtc 20

<210> 157
<211> 27
<212> DNA
<213> Artificial Sequence

<220>
<223> Description of Artificial Sequence: Synthetic primer

<400> 157
tgtaataact ctacgactgt ctgtctg 27

<210> 158
<211> 21
<212> DNA
<213> Artificial Sequence

<220>
<223> Description of Artificial Sequence: Synthetic primer

<400> 158
gaataggagg tggatggatg g 21

<210> 159
<211> 20
<212> DNA
<213> Artificial Sequence

<220>
<223> Description of Artificial Sequence: Synthetic primer

<400> 159

gagcgagacc ctgtctcaag 20

<210> 160
<211> 24
<212> DNA
<213> Artificial Sequence

<220>
<223> Description of Artificial Sequence: Synthetic primer

<400> 160
ggaaaagaca taggatagca attt 24

<210> 161
<211> 21
<212> DNA
<213> Artificial Sequence

<220>
<223> Description of Artificial Sequence: Synthetic primer

<400> 161
tctggattga tctgtctgtc c 21

<210> 162
<211> 26
<212> DNA
<213> Artificial Sequence

<220>
<223> Description of Artificial Sequence: Synthetic primer

<400> 162
gaattaaata ccatctgagc actgaa 26

<210> 163
<211> 27
<212> DNA
<213> Artificial Sequence

<220>
<223> Description of Artificial Sequence: Synthetic primer

<400> 163
tgttataatg cattgagttt tattctg 27

<210> 164
<211> 28
<212> DNA
<213> Artificial Sequence

<220>
<223> Description of Artificial Sequence: Synthetic primer

<400> 164
gcctgtctca aaaataaaga gatagaca 28

<210> 165

<211> 25
<212> DNA
<213> Artificial Sequence

<220>
<223> Description of Artificial Sequence: Synthetic primer

<400> 165
ttaatgaatt gaacaaatga gtgag 25

<210> 166
<211> 25
<212> DNA
<213> Artificial Sequence

<220>
<223> Description of Artificial Sequence: Synthetic primer

<400> 166
gcaactctgg ttgtattgtc ttcat 25

<210> 167
<211> 21
<212> DNA
<213> Artificial Sequence

<220>
<223> Description of Artificial Sequence: Synthetic primer

<400> 167
caaagcgaga ctctgtctca a 21

<210> 168
<211> 27
<212> DNA
<213> Artificial Sequence

<220>
<223> Description of Artificial Sequence: Synthetic primer

<400> 168
gaaaatgcta tcctctttgg tataaat 27

<210> 169
<211> 27
<212> DNA
<213> Artificial Sequence

<220>
<223> Description of Artificial Sequence: Synthetic primer

<400> 169
gggtatttca agataactgt agatagg 27

<210> 170
<211> 24
<212> DNA
<213> Artificial Sequence

<220>
<223> Description of Artificial Sequence: Synthetic primer

<400> 170
gcttctgaaa gcttctagtt tacc 24

<210> 171
<211> 19
<212> DNA
<213> Artificial Sequence

<220>
<223> Description of Artificial Sequence: Synthetic primer

<400> 171
tccacatcct caccaacac 19

<210> 172
<211> 21
<212> DNA
<213> Artificial Sequence

<220>
<223> Description of Artificial Sequence: Synthetic primer

<400> 172
gcctaggaag gctactgtca a 21

<210> 173
<211> 18
<212> DNA
<213> Artificial Sequence

<220>
<223> Description of Artificial Sequence: Synthetic primer

<400> 173
ccacccgtcc atttaggc 18

<210> 174
<211> 27
<212> DNA
<213> Artificial Sequence

<220>
<223> Description of Artificial Sequence: Synthetic primer

<400> 174
gtgaaaaagt agatataatg gttggtg 27

<210> 175
<211> 25
<212> DNA
<213> Artificial Sequence

<220>
<223> Description of Artificial Sequence: Synthetic primer

<400> 175
ggttttccaa gagatagacc aatta 25

<210> 176
<211> 27
<212> DNA
<213> Artificial Sequence

<220>
<223> Description of Artificial Sequence: Synthetic primer

<400> 176
gtcctctcat aaatccctac tcatatc 27

<210> 177
<211> 27
<212> DNA
<213> Artificial Sequence

<220>
<223> Description of Artificial Sequence: Synthetic primer

<400> 177
ctgttggtac ataataggta ggtaggt 27

<210> 178
<211> 19
<212> DNA
<213> Artificial Sequence

<220>
<223> Description of Artificial Sequence: Synthetic primer

<400> 178
gtcgtgggcc ccataaatc 19

<210> 179
<211> 27
<212> DNA
<213> Artificial Sequence

<220>
<223> Description of Artificial Sequence: Synthetic primer

<400> 179
aaggtacata acagttcaat agaaagc 27

<210> 180
<211> 26
<212> DNA
<213> Artificial Sequence

<220>
<223> Description of Artificial Sequence: Synthetic primer

<400> 180
gtgaaatgac tgaaaaatag taacca 26

<210> 181
<211> 24
<212> DNA
<213> Artificial Sequence

<220>
<223> Description of Artificial Sequence: Synthetic primer

<400> 181
ctaggagatc atgtgggtat gatt 24

<210> 182
<211> 24
<212> DNA
<213> Artificial Sequence

<220>
<223> Description of Artificial Sequence: Synthetic primer

<400> 182
gcagtgaata aatgaacgaa tgga 24

<210> 183
<211> 21
<212> DNA
<213> Artificial Sequence

<220>
<223> Description of Artificial Sequence: Synthetic primer

<400> 183
cccaaaatta cttgagccaa t 21

<210> 184
<211> 24
<212> DNA
<213> Artificial Sequence

<220>
<223> Description of Artificial Sequence: Synthetic primer

<400> 184
gagacaaaat gaagaaacag acag 24

<210> 185
<211> 25
<212> DNA
<213> Artificial Sequence

<220>
<223> Description of Artificial Sequence: Synthetic primer

<400> 185
tctttgctct catgaataga tcagt 25

<210> 186
<211> 24
<212> DNA

<213> Artificial Sequence

<220>
<223> Description of Artificial Sequence: Synthetic primer

<400> 186
gtttgtgata atgaacccac tcag 24

<210> 187
<211> 27
<212> DNA
<213> Artificial Sequence

<220>
<223> Description of Artificial Sequence: Synthetic primer

<400> 187
tgaacacaga tgttaagtgt gtatatg 27

<210> 188
<211> 23
<212> DNA
<213> Artificial Sequence

<220>
<223> Description of Artificial Sequence: Synthetic primer

<400> 188
gtctgaggtg gacagttatg aaa 23

<210> 189
<211> 23
<212> DNA
<213> Artificial Sequence

<220>
<223> Description of Artificial Sequence: Synthetic primer

<400> 189
ctgtggctca tctatgaaaa ctt 23

<210> 190
<211> 22
<212> DNA
<213> Artificial Sequence

<220>
<223> Description of Artificial Sequence: Synthetic primer

<400> 190
gaagtggctg tggtgttatg at 22

<210> 191
<211> 24
<212> DNA
<213> Artificial Sequence

<220>

<223> Description of Artificial Sequence: Synthetic primer

<400> 191
ttctgttggt atagagcagt gttt 24

<210> 192
<211> 20
<212> DNA
<213> Artificial Sequence

<220>
<223> Description of Artificial Sequence: Synthetic primer

<400> 192
gtgacaggaa ggacggaatg 20

<210> 193
<211> 27
<212> DNA
<213> Artificial Sequence

<220>
<223> Description of Artificial Sequence: Synthetic primer

<400> 193
catgaggttt gcaaatacta tcttaac 27

<210> 194
<211> 24
<212> DNA
<213> Artificial Sequence

<220>
<223> Description of Artificial Sequence: Synthetic primer

<400> 194
gttttaattt tctccaaatc tcca 24

<210> 195
<211> 27
<212> DNA
<213> Artificial Sequence

<220>
<223> Description of Artificial Sequence: Synthetic primer

<400> 195
tcttagccta gatagatact tgcttcc 27

<210> 196
<211> 23
<212> DNA
<213> Artificial Sequence

<220>
<223> Description of Artificial Sequence: Synthetic primer

<400> 196

gtcaaccttt gaggctatag gaa 23

<210> 197
<211> 19
<212> DNA
<213> Artificial Sequence

<220>
<223> Description of Artificial Sequence: Synthetic primer

<400> 197
tcctggaaac aaaagtatt 19

<210> 198
<211> 21
<212> DNA
<213> Artificial Sequence

<220>
<223> Description of Artificial Sequence: Synthetic primer

<400> 198
aaccttacaa caaagctaga a 21

<210> 199
<211> 20
<212> DNA
<213> Artificial Sequence

<220>
<223> Description of Artificial Sequence: Synthetic primer

<400> 199
actaagcctt ggggatccag 20

<210> 200
<211> 21
<212> DNA
<213> Artificial Sequence

<220>
<223> Description of Artificial Sequence: Synthetic primer

<400> 200
tgctgtggaa atactaaaag g 21

<210> 201
<211> 20
<212> DNA
<213> Artificial Sequence

<220>
<223> Description of Artificial Sequence: Synthetic primer

<400> 201
ctccagaggt aatcctgtga 20

<210> 202

<211> 21
<212> DNA
<213> Artificial Sequence

<220>
<223> Description of Artificial Sequence: Synthetic primer

<400> 202
tggtgtgaga tggtatctag g 21

<210> 203
<211> 22
<212> DNA
<213> Artificial Sequence

<220>
<223> Description of Artificial Sequence: Synthetic primer

<400> 203
gtataatcca tgaatcttgt tt 22

<210> 204
<211> 22
<212> DNA
<213> Artificial Sequence

<220>
<223> Description of Artificial Sequence: Synthetic primer

<400> 204
ttcaaattgt atataagaga gt 22

<210> 205
<211> 20
<212> DNA
<213> Artificial Sequence

<220>
<223> Description of Artificial Sequence: Synthetic primer

<400> 205
gcaggaaagt tatttttaat 20

<210> 206
<211> 21
<212> DNA
<213> Artificial Sequence

<220>
<223> Description of Artificial Sequence: Synthetic primer

<400> 206
tgcttgagaa agctaacact t 21

<210> 207
<211> 20
<212> DNA
<213> Artificial Sequence

<220>
<223> Description of Artificial Sequence: Synthetic primer

<400> 207
cagtgtttgg aaattgtctg 20

<210> 208
<211> 20
<212> DNA
<213> Artificial Sequence

<220>
<223> Description of Artificial Sequence: Synthetic primer

<400> 208
ggcactggga gattattgta 20

<210> 209
<211> 20
<212> DNA
<213> Artificial Sequence

<220>
<223> Description of Artificial Sequence: Synthetic primer

<400> 209
tcctgttgtt aagtacacat 20

<210> 210
<211> 18
<212> DNA
<213> Artificial Sequence

<220>
<223> Description of Artificial Sequence: Synthetic primer

<400> 210
gggccgtaat tacttttg 18

<210> 211
<211> 21
<212> DNA
<213> Artificial Sequence

<220>
<223> Description of Artificial Sequence: Synthetic primer

<400> 211
actcagtagg cactttgtgt c 21

<210> 212
<211> 18
<212> DNA
<213> Artificial Sequence

<220>
<223> Description of Artificial Sequence: Synthetic primer

<400> 212
tcttccacca caccaatc 18

<210> 213
<211> 19
<212> DNA
<213> Artificial Sequence

<220>
<223> Description of Artificial Sequence: Synthetic primer

<400> 213
tggcttttca aaggtaaaa 19

<210> 214
<211> 21
<212> DNA
<213> Artificial Sequence

<220>
<223> Description of Artificial Sequence: Synthetic primer

<400> 214
gcaacgttaa catctgaatt t 21

<210> 215

<400> 215
000

<210> 216
<211> 22
<212> DNA
<213> Artificial Sequence

<220>
<223> Description of Artificial Sequence: Synthetic primer

<400> 216
attttatatg tcatgatcta ag 22

<210> 217
<211> 19
<212> DNA
<213> Artificial Sequence

<220>
<223> Description of Artificial Sequence: Synthetic primer

<400> 217
agagattaca ggtgtgagc 19

<210> 218
<211> 19
<212> DNA
<213> Artificial Sequence

<220>

<223> Description of Artificial Sequence: Synthetic primer

<400> 218
atgatcctca actgcctct 19

<210> 219
<211> 20
<212> DNA
<213> Artificial Sequence

<220>
<223> Description of Artificial Sequence: Synthetic primer

<400> 219
tgaaactcaa aagagaaaag 20

<210> 220
<211> 20
<212> DNA
<213> Artificial Sequence

<220>
<223> Description of Artificial Sequence: Synthetic primer

<400> 220
acagatttct acttaaaatt 20

<210> 221
<211> 20
<212> DNA
<213> Artificial Sequence

<220>
<223> Description of Artificial Sequence: Synthetic primer

<400> 221
tgaaactcaa aagagaaaag 20

<210> 222
<211> 20
<212> DNA
<213> Artificial Sequence

<220>
<223> Description of Artificial Sequence: Synthetic primer

<400> 222
acagatttct acttaaaatt 20

<210> 223
<211> 20
<212> DNA
<213> Artificial Sequence

<220>
<223> Description of Artificial Sequence: Synthetic primer

<400> 223

gcaaaggggt actctatgta 20

<210> 224
<211> 20
<212> DNA
<213> Artificial Sequence

<220>
<223> Description of Artificial Sequence: Synthetic primer

<400> 224
tatcgggtca tcttgttaaa 20

<210> 225
<211> 22
<212> DNA
<213> Artificial Sequence

<220>
<223> Description of Artificial Sequence: Synthetic primer

<400> 225
tctaacaaag ctctgtccaa aa 22

<210> 226
<211> 21
<212> DNA
<213> Artificial Sequence

<220>
<223> Description of Artificial Sequence: Synthetic primer

<400> 226
ccacactgaa taactggaac a 21

<210> 227
<211> 21
<212> DNA
<213> Artificial Sequence

<220>
<223> Description of Artificial Sequence: Synthetic primer

<400> 227
gcaagcaagc tctctacctt c 21

<210> 228
<211> 22
<212> DNA
<213> Artificial Sequence

<220>
<223> Description of Artificial Sequence: Synthetic primer

<400> 228
tgttcttcca aaattcacat gc 22

<210> 229

<211> 21
<212> DNA
<213> Artificial Sequence

<220>
<223> Description of Artificial Sequence: Synthetic primer

<400> 229
atttcactat tccttcattt t 21

<210> 230
<211> 22
<212> DNA
<213> Artificial Sequence

<220>
<223> Description of Artificial Sequence: Synthetic primer

<400> 230
taattgttgc acactaaatt ac 22

<210> 231
<211> 21
<212> DNA
<213> Artificial Sequence

<220>
<223> Description of Artificial Sequence: Synthetic primer

<400> 231
aaaaagccac agaaatcagt c 21

<210> 232
<211> 22
<212> DNA
<213> Artificial Sequence

<220>
<223> Description of Artificial Sequence: Synthetic primer

<400> 232
ttcttatatc tcactgggca tt 22

<210> 233
<211> 22
<212> DNA
<213> Artificial Sequence

<220>
<223> Description of Artificial Sequence: Synthetic primer

<400> 233
ggatggtaga agagaagaaa gg 22

<210> 234
<211> 22
<212> DNA
<213> Artificial Sequence

<220>
<223> Description of Artificial Sequence: Synthetic primer

<400> 234
ggatggtaga agagaagaaa gg 22

<210> 235
<211> 20
<212> DNA
<213> Artificial Sequence

<220>
<223> Description of Artificial Sequence: Synthetic primer

<400> 235
tgcaaagatg cagaaccaac 20

<210> 236
<211> 22
<212> DNA
<213> Artificial Sequence

<220>
<223> Description of Artificial Sequence: Synthetic primer

<400> 236
ttttgttcct tgtcctggct ga 22

<210> 237
<211> 20
<212> DNA
<213> Artificial Sequence

<220>
<223> Description of Artificial Sequence: Synthetic primer

<400> 237
tgcaaagatg cagaaccaac 20

<210> 238
<211> 21
<212> DNA
<213> Artificial Sequence

<220>
<223> Description of Artificial Sequence: Synthetic primer

<400> 238
gcctccagct ctatccaagt t 21

<210> 239
<211> 22
<212> DNA
<213> Artificial Sequence

<220>
<223> Description of Artificial Sequence: Synthetic primer

<400> 239
ccttaatatc ttcccatgtc ca 22

<210> 240
<211> 22
<212> DNA
<213> Artificial Sequence

<220>
<223> Description of Artificial Sequence: Synthetic primer

<400> 240
attgttagtg cctcttctgc tt 22

<210> 241
<211> 20
<212> DNA
<213> Artificial Sequence

<220>
<223> Description of Artificial Sequence: Synthetic primer

<400> 241
gagaagtgag gtcagcagct 20

<210> 242
<211> 22
<212> DNA
<213> Artificial Sequence

<220>
<223> Description of Artificial Sequence: Synthetic primer

<400> 242
tttctaaatt tccattgaac ag 22

<210> 243
<211> 20
<212> DNA
<213> Artificial Sequence

<220>
<223> Description of Artificial Sequence: Synthetic primer

<400> 243
gaaattggca atctgattct 20

<210> 244
<211> 21
<212> DNA
<213> Artificial Sequence

<220>
<223> Description of Artificial Sequence: Synthetic primer

<400> 244
caacttgtcc tttattgatg t 21

<210> 245
<211> 22
<212> DNA
<213> Artificial Sequence

<220>
<223> Description of Artificial Sequence: Synthetic primer

<400> 245
ctatgttgat aaaacattga aa 22

<210> 246
<211> 20
<212> DNA
<213> Artificial Sequence

<220>
<223> Description of Artificial Sequence: Synthetic primer

<400> 246
gcctgtctgg aatatagttt 20

<210> 247
<211> 22
<212> DNA
<213> Artificial Sequence

<220>
<223> Description of Artificial Sequence: Synthetic primer

<400> 247
cagggcatat aatctaagct gt 22

<210> 248
<211> 21
<212> DNA
<213> Artificial Sequence

<220>
<223> Description of Artificial Sequence: Synthetic primer

<400> 248
caatgactct gagttgagca c 21

<210> 249
<211> 18
<212> DNA
<213> Artificial Sequence

<220>
<223> Description of Artificial Sequence: Synthetic primer

<400> 249
actctctccc tcccctct 18

<210> 250
<211> 20
<212> DNA

<213> Artificial Sequence

<220>
<223> Description of Artificial Sequence: Synthetic primer

<400> 250
tatggcccca aaactattct 20

<210> 251
<211> 20
<212> DNA
<213> Artificial Sequence

<220>
<223> Description of Artificial Sequence: Synthetic primer

<400> 251
acaagtactg ggcagattga 20

<210> 252
<211> 20
<212> DNA
<213> Artificial Sequence

<220>
<223> Description of Artificial Sequence: Synthetic primer

<400> 252
gccaggttta gctttcaagt 20

<210> 253
<211> 22
<212> DNA
<213> Artificial Sequence

<220>
<223> Description of Artificial Sequence: Synthetic primer

<400> 253
ttttatatca ggagaaacac tg 22

<210> 254
<211> 21
<212> DNA
<213> Artificial Sequence

<220>
<223> Description of Artificial Sequence: Synthetic primer

<400> 254
ccagaatttt ggaggtttaa t 21

<210> 255
<211> 22
<212> DNA
<213> Artificial Sequence

<220>

<223> Description of Artificial Sequence: Synthetic primer

<400> 255
tgtcattcct cctttatctc ca 22

<210> 256
<211> 20
<212> DNA
<213> Artificial Sequence

<220>
<223> Description of Artificial Sequence: Synthetic primer

<400> 256
ttcttttgcc tctcccaaag 20

<210> 257
<211> 20
<212> DNA
<213> Artificial Sequence

<220>
<223> Description of Artificial Sequence: Synthetic primer

<400> 257
accctggcac agtgttgact 20

<210> 258
<211> 20
<212> DNA
<213> Artificial Sequence

<220>
<223> Description of Artificial Sequence: Synthetic primer

<400> 258
tgggcctgag ttgagaagat 20

<210> 259
<211> 21
<212> DNA
<213> Artificial Sequence

<220>
<223> Description of Artificial Sequence: Synthetic primer

<400> 259
aatttgtaag tatgtgcaac g 21

<210> 260
<211> 20
<212> DNA
<213> Artificial Sequence

<220>
<223> Description of Artificial Sequence: Synthetic primer

<400> 260

tttttcccat ttccaactct 20

<210> 261
<211> 20
<212> DNA
<213> Artificial Sequence

<220>
<223> Description of Artificial Sequence: Synthetic primer

<400> 261
aaaagatgag acaggcaggt 20

<210> 262
<211> 20
<212> DNA
<213> Artificial Sequence

<220>
<223> Description of Artificial Sequence: Synthetic primer

<400> 262
acccctgtga atctcaaaat 20

<210> 263
<211> 21
<212> DNA
<213> Artificial Sequence

<220>
<223> Description of Artificial Sequence: Synthetic primer

<400> 263
gcacttgctt ctattgtttg t 21

<210> 264
<211> 20
<212> DNA
<213> Artificial Sequence

<220>
<223> Description of Artificial Sequence: Synthetic primer

<400> 264
cccttcctct cttccattct 20

<210> 265
<211> 13
<212> DNA
<213> Artificial Sequence

<220>
<223> Description of Artificial Sequence: Synthetic primer

<400> 265
agcactgcag gta 13

<210> 266

<211> 21
<212> DNA
<213> Artificial Sequence

<220>
<223> Description of Artificial Sequence: Synthetic primer

<400> 266
acagatacca aagaactgca a 21

<210> 267
<211> 20
<212> DNA
<213> Artificial Sequence

<220>
<223> Description of Artificial Sequence: Synthetic primer

<400> 267
tggacacctt tcaacttaga 20

<210> 268
<211> 22
<212> DNA
<213> Artificial Sequence

<220>
<223> Description of Artificial Sequence: Synthetic primer

<400> 268
gaacagtaat gttgaacttt tt 22

<210> 269
<211> 21
<212> DNA
<213> Artificial Sequence

<220>
<223> Description of Artificial Sequence: Synthetic primer

<400> 269
tcttgcaaaa agcttagcac a 21

<210> 270
<211> 21
<212> DNA
<213> Artificial Sequence

<220>
<223> Description of Artificial Sequence: Synthetic primer

<400> 270
aaaaagatct caaagggtcc a 21

<210> 271
<211> 20
<212> DNA
<213> Artificial Sequence

<220>
<223> Description of Artificial Sequence: Synthetic primer

<400> 271
gcttttgctg aacatcaagt 20

<210> 272
<211> 19
<212> DNA
<213> Artificial Sequence

<220>
<223> Description of Artificial Sequence: Synthetic primer

<400> 272
ccttccagca gcatagtct 19

<210> 273
<211> 18
<212> DNA
<213> Artificial Sequence

<220>
<223> Description of Artificial Sequence: Synthetic primer

<400> 273
aaatccagga tgtgcagt 18

<210> 274
<211> 19
<212> DNA
<213> Artificial Sequence

<220>
<223> Description of Artificial Sequence: Synthetic primer

<400> 274
atgatgaggt cagtggtgt 19

<210> 275
<211> 22
<212> DNA
<213> Artificial Sequence

<220>
<223> Description of Artificial Sequence: Synthetic primer

<400> 275
catcacagat catagtaaat gg 22

<210> 276
<211> 21
<212> DNA
<213> Artificial Sequence

<220>
<223> Description of Artificial Sequence: Synthetic primer

<400> 276
aattattatt ttgcaggcaa t 21

<210> 277
<211> 20
<212> DNA
<213> Artificial Sequence

<220>
<223> Description of Artificial Sequence: Synthetic primer

<400> 277
catgaggcaa acacctttcc 20

<210> 278
<211> 22
<212> DNA
<213> Artificial Sequence

<220>
<223> Description of Artificial Sequence: Synthetic primer

<400> 278
gctggactca ggataaagaa ca 22

<210> 279
<211> 20
<212> DNA
<213> Artificial Sequence

<220>
<223> Description of Artificial Sequence: Synthetic primer

<400> 279
tggaagcctg agctgactaa 20

<210> 280
<211> 20
<212> DNA
<213> Artificial Sequence

<220>
<223> Description of Artificial Sequence: Synthetic primer

<400> 280
ccttcttttc ccccagaatc 20

<210> 281
<211> 21
<212> DNA
<213> Artificial Sequence

<220>
<223> Description of Artificial Sequence: Synthetic primer

<400> 281
taggagaaca gaagatcaga g 21

<210> 282
<211> 22
<212> DNA
<213> Artificial Sequence

<220>
<223> Description of Artificial Sequence: Synthetic primer

<400> 282
aaagactatt gctaaatgct tg 22

<210> 283
<211> 20
<212> DNA
<213> Artificial Sequence

<220>
<223> Description of Artificial Sequence: Synthetic primer

<400> 283
taagcgtagg gctgtgtgtg 20

<210> 284
<211> 21
<212> DNA
<213> Artificial Sequence

<220>
<223> Description of Artificial Sequence: Synthetic primer

<400> 284
ggacggatag actccagaag g 21

<210> 285
<211> 23
<212> DNA
<213> Artificial Sequence

<220>
<223> Description of Artificial Sequence: Synthetic primer

<400> 285
gaatgacctt ggcactttta tca 23

<210> 286
<211> 27
<212> DNA
<213> Artificial Sequence

<220>
<223> Description of Artificial Sequence: Synthetic primer

<400> 286
aaggatagag atatacagat gaatgga 27

<210> 287
<211> 18
<212> DNA

<213> Artificial Sequence

<220>
<223> Description of Artificial Sequence: Synthetic primer

<400> 287
catgcaccgc gcaaatac 18

<210> 288
<211> 19
<212> DNA
<213> Artificial Sequence

<220>
<223> Description of Artificial Sequence: Synthetic primer

<400> 288
atgcctcacc cacaaacac 19

<210> 289
<211> 20
<212> DNA
<213> Artificial Sequence

<220>
<223> Description of Artificial Sequence: Synthetic primer

<400> 289
tccaagccct tctcactcac 20

<210> 290
<211> 20
<212> DNA
<213> Artificial Sequence

<220>
<223> Description of Artificial Sequence: Synthetic primer

<400> 290
ctgggacggt gacattttct 20

<210> 291
<211> 21
<212> DNA
<213> Artificial Sequence

<220>
<223> Description of Artificial Sequence: Synthetic primer

<400> 291
cccaggaaga gtggaaagat t 21

<210> 292
<211> 21
<212> DNA
<213> Artificial Sequence

<220>

<223> Description of Artificial Sequence: Synthetic primer

<400> 292
ttagcttgca tgtacctgtg t 21

<210> 293
<211> 20
<212> DNA
<213> Artificial Sequence

<220>
<223> Description of Artificial Sequence: Synthetic primer

<400> 293
agctagatgg ggtgaatttt 20

<210> 294
<211> 18
<212> DNA
<213> Artificial Sequence

<220>
<223> Description of Artificial Sequence: Synthetic primer

<400> 294
tgggctgagg ggagattc 18

<210> 295
<211> 22
<212> DNA
<213> Artificial Sequence

<220>
<223> Description of Artificial Sequence: Synthetic primer

<400> 295
atcaagctaa ttaatgttat ct 22

<210> 296
<211> 20
<212> DNA
<213> Artificial Sequence

<220>
<223> Description of Artificial Sequence: Synthetic primer

<400> 296
aatgaataag gtcctcagag 20

<210> 297
<211> 21
<212> DNA
<213> Artificial Sequence

<220>
<223> Description of Artificial Sequence: Synthetic primer

<400> 297

tttaatctga tcattgccct a 21

<210> 298
<211> 18
<212> DNA
<213> Artificial Sequence

<220>
<223> Description of Artificial Sequence: Synthetic primer

<400> 298
agctgtgggt gaccttga 18

<210> 299
<211> 20
<212> DNA
<213> Artificial Sequence

<220>
<223> Description of Artificial Sequence: Synthetic primer

<400> 299
tgtcccacca ttgtgtatta 20

<210> 300
<211> 20
<212> DNA
<213> Artificial Sequence

<220>
<223> Description of Artificial Sequence: Synthetic primer

<400> 300
tcagacttga agtccaggat 20

<210> 301
<211> 20
<212> DNA
<213> Artificial Sequence

<220>
<223> Description of Artificial Sequence: Synthetic primer

<400> 301
gcttcagggg tgttagtttt 20

<210> 302
<211> 20
<212> DNA
<213> Artificial Sequence

<220>
<223> Description of Artificial Sequence: Synthetic primer

<400> 302
ctttgtgaaa agtcgtccag 20

<210> 303

<211> 18
<212> DNA
<213> Artificial Sequence

<220>
<223> Description of Artificial Sequence: Synthetic primer

<400> 303
ccatcatgga aagcatgg 18

<210> 304
<211> 20
<212> DNA
<213> Artificial Sequence

<220>
<223> Description of Artificial Sequence: Synthetic primer

<400> 304
tcatctccat gactgcacta 20

<210> 305
<211> 20
<212> DNA
<213> Artificial Sequence

<220>
<223> Description of Artificial Sequence: Synthetic primer

<400> 305
gagatgacgg agtagctcat 20

<210> 306
<211> 18
<212> DNA
<213> Artificial Sequence

<220>
<223> Description of Artificial Sequence: Synthetic primer

<400> 306
cccagctgca ctgtctac 18

<210> 307
<211> 20
<212> DNA
<213> Artificial Sequence

<220>
<223> Description of Artificial Sequence: Synthetic primer

<400> 307
tcttgttcca atcacaggac 20

<210> 308
<211> 20
<212> DNA
<213> Artificial Sequence

<220>
<223> Description of Artificial Sequence: Synthetic primer

<400> 308
atgctgttag ctgaagctct 20

<210> 309
<211> 20
<212> DNA
<213> Artificial Sequence

<220>
<223> Description of Artificial Sequence: Synthetic primer

<400> 309
tgaaagctcc taaagcagag 20

<210> 310
<211> 20
<212> DNA
<213> Artificial Sequence

<220>
<223> Description of Artificial Sequence: Synthetic primer

<400> 310
ttgaagagat gtgctatcat 20

<210> 311
<211> 54
<212> DNA
<213> Artificial Sequence

<220>
<223> Description of Artificial Sequence: Synthetic primer

<400> 311
gccgcctgca gcccgcgccc cccgtgcccc cgccccgccg ccggcccggg cgcc 54

<210> 312
<211> 601
<212> DNA
<213> Homo sapiens

<400> 312

catagtgaca ggtatatgcc caactaactg tggaaaacag ttctttcttt caaccttact 60

catcaccctc acggtctgtt tatgaggctc tcctccacca gccagaaagg atgacgtgcc 120

atacctgcaa aacttataca gcatcaacag aatgaatctt tccaacaagc cgaaacattg 180

agtattgtgg cacagaatat gccccaccca ttactcaatc tagatatcct tttattccac 240

cgtctcatga ttttcttttt cctggaaaac aaaagtattt ctttcatagc ccagctagca 300

ygataaatca gcgagtcaga attctagctt tgttgtaagg ttttgcgaat atctgatcct 360

cttattttgt acttttctat ttcctaggca aatctgagta tttcacccag ttttccttaa 420

ctaggcattg aaaactcagt ttttttctta caaaccttca tgtcttcctg ctcatttgca 480

cagtcttatc ttgcacctcc tataaaatgg agaaacttga cattaaaacg taatttttat 540

tacattttga gggattccca gagaattttt ccccaatctc cttaggtagg gacttcttta 600

c 601

<210> 313
<211> 601
<212> DNA
<213> Homo sapiens

<400> 313

gtgggaacta tagtaaagaa gtccctacct aaggagattg gggaaaaatt ctctgggaat 60

ccctcaaaat gtaataaaaa ttacgtttta atgtcaagtt tctccatttt ataggaggtg 120

caagataaga ctgtgcaaat gagcaggaag acatgaaggt ttgtaagaaa aaaactgagt 180

tttcaatgcc tagttaagga aaactgggtg aaatactcag atttgcctag gaaatagaaa 240

agtacaaaat aagaggatca gatattcgca aaaccttaca acaaagctag aattctgact 300

ygctgattta tcgtgctagc tgggctatga aagaaatact tttgttttcc aggaaaaaga 360

aaatcatgag acggtggaat aaaaggatat ctagattgag taatgggtgg ggcatattct 420

gtgccacaat actcaatgtt tcggcttgtt ggaaagattc attctgttga tgctgtataa 480

gttttgcagg tatggcacgt catcctttct ggctggtgga ggagagcctc ataaacagac 540

cgtgagggtg atgagtaagg ttgaaagaaa gaactgtttt ccacagttag ttgggcatat 600

a 601

<210> 314
<211> 650
<212> DNA
<213> Homo sapiens

<400> 314

```
tttattggtc ctgactggta caaatactga taaaaaggat tttaagatca tattcatact      60
tttggggaat gagagccaca attaattaac aatgtctgcc atgagattgg atgcaagagt     120
atggcactca tactattcct acttctgtct aattacacta tttgtttctg tgtgcaaaaa     180
tctttggtag gtggtggatg tgcccaagac acagggaaga aaaagaagta aacagggaag     240
tacaacacag actctgaaat ggggcatcat ggaagacgga gctttgtcgt cttggtcttt     300
gctgtatatt cacttcctac aacagtgcta aataccttgt ggatgcttaa atatattaaa     360
tgaatgcata aatgaaaaga gtaaataaag agtgtatatg aaagtatgta gataaaattc     420
ttcactaagc cttggggatc cagctgcttm aggactaaga ccgtatctag ctccttttag     480
tatttccaca gcatgccatg gagatacatg tttctgatta tatatgatac atggaaatta     540
tatgttgttg aatgagtgat tgagtaaatg tgtactaggg cagctaatca taaatatttc     600
tactattgct aaaatgactg gatttatcca ttccttctga gagtttatac                650
```

<210> 315
<211> 626
<212> DNA
<213> Homo sapiens

<400> 315

```
ctgcttaagg actaagaccr tatctagctc cttttagtat ttccacagca tgccatggag      60
atacatgttt ctgattatat atgatacatg gaaattatat gttgttgaat gagtgattga     120
gtaaatgtgt actagggcag ctaatcataa atatttctac tattgctaaa atgactggat     180
ttatccattc cttctgagag tttatactga ttgcttatat tgtatcaaat accgtaactg     240
agggcaatgt ttactcaaac taatagcacc attcaaattt atgcaaacaa taacactata     300
tctttaaaat gttttcacta aaagctgcat aaagagtgta ttcaacaaca atagaataat     360
tttacaatct tttttcttgc ttaatggcca tttgtgcctt ctgacatgct gctagccatt     420
caaaggtcac actaccttga agttgaagat caagacaaat gattagactc ataaaagaca     480
aatcacgtct ttctggacag gtgattatta ataattaatt agcatttaaa catgtattat     540
ttaagttctt tttaagttat aaagtctttg atttgctaaa cagtttaaat aatgaataaa     600
acataaaata ataatagtta ccattt                                          626
```

<210> 316
<211> 1113
<212> DNA
<213> Homo sapiens

<400> 316

```
caagagctgc atctcactcc aatttttctt ctccctataa ccttatctag attcccagtt        60
gagggaaccg atgacctaat tcctctcagt ttaaatgcaa cacaggagca aattccaaat       120
atctatgctg gtcttgctgg gattgcagaa ccccagggtg gttatcctcc tccagaggta       180
atcctgtgat cagcactaac rccacatacc agccctttca tcagcttgtt ggagaagcat       240
ctttacttcc caccaagcag tgacctagat accatctcac accagttaga atcaggatca       300
ttaaaaagtc aagaaaaaac agatgctgaa gaggatgtgg agaaatagga atgcttttac       360
actgttagtg ggaatgtaaa ttagttcaac cattgtcaaa gacagtgtgg cgatccctca       420
cagatctaga accagaaata ccatttgacc cagcaatccc attactgggt ctatacccaa       480
aggattataa attactctac tataaagaca catgcacaca tatgtttatt gcagcaccat       540
tcacaatagc aaagaattgc aaccaaccct aatgcccatc aatgacagac tggataaaga       600
aaatctggca catatacacc atggaatact acgcagccat aaaaaaggat gagtttatgt       660
cctttacagg gacatggatg aagctggaaa ccatcattct cagcaaacta acacaggaac       720
agaaaaccaa acacatgttc tcactcacaa gtgggagttg aacaatgaga acacatggac       780
acagggaggg gaacatcaca caccactgct tgtcaggggg tgggggggcta ggggaaggat       840
agcattagga gaaataccta atgtagatga aggggttgatg ggtgcagcaa accaccatgg       900
catgtgtata cctgtgtaac aaacctccat gttctgcacg tgtatcccag aacttaaagt       960
acaatacaaa aaaaaaaaaa agtgtaatcc agtttacatt ttcaaggtca aagtgggtac      1020
aatgctatct atcttgggct aagaagagaa aaggaaaaat tcttgcttta aatcttagaa      1080
gtctggtttt tttccctgtt ttgtacccca tcc                                   1113
```

<210> 317
<211> 1113
<212> DNA
<213> Homo sapiens

<400> 317

ttcccagttg agggaaccga tgacctaatt cctctcagtt taaatgcaac acaggagcaa 60

attccaaata tctatgctgg tcttgctggg attgcagaac cccagggtgg ttatcctcct 120

ccagaggtaa tcctgtgatc agcactaacg ccacatacca gccctttcat cagcttgttg 180

gagaagcatc tttacttccc rccaagcagt gacctagata ccatctcaca ccagttagaa 240

tcaggatcat taaaaagtca agaaaaaaca gatgctgaag aggatgtgga gaaataggaa 300

tgcttttaca ctgttagtgg gaatgtaaat tagttcaacc attgtcaaag acagtgtggc 360

gatccctcac agatctagaa ccagaaatac catttgaccc agcaatccca ttactgggtc 420

tatacccaaa ggattataaa ttactctact ataaagacac atgcacacat atgtttattg 480

cagcaccatt cacaatagca aagaattgca accaacccta atgcccatca atgacagact 540

ggataaagaa aatctggcac atatacacca tggaatacta cgcagccata aaaaaggatg 600

agtttatgtc ctttacaggg acatggatga agctggaaac catcattctc agcaaactaa 660

cacaggaaca gaaaaccaaa cacatgttct cactcacaag tgggagttga acaatgagaa 720

cacatggaca cagggagggg aacatcacac accactgctt gtcaggggggt gggggggctag 780

gggaaggata gcattaggag aaatacctaa tgtagatgaa gggttgatgg gtgcagcaaa 840

ccaccatggc atgtgtatac ctgtgtaaca aacctccatg ttctgcacgt gtatcccaga 900

acttaaagta caatacaaaa aaaaaaaaaa gtgtaatcca gtttacattt tcaaggtcaa 960

agtgggtaca atgctatcta tcttgggcta agaagagaaa aggaaaaatt cttgctttaa 1020

atcttagaag tctggttttt ttccctgttt tgtaccccat cctcttggtc tctctagata 1080

tatttaagac tcacatagga cttgtctttt cta 1113

<210> 318
<211> 1001
<212> DNA
<213> Homo sapiens

<400> 318

tcatcaacta aatagttgat gaggggaaat tgttctgtat atgttcatac ttcagctaat 60

caattaaaaa tgatgaaata ataagattac cattttgcaa acccctaatg caatgttgga 120
tccaggcaat gatcatcaat ggccactaaa atcacacaaa aggagataac cagaatatgt 180
gctttgtgat ggaagcatta aatacaacta atgagatatt gtttataaga aagaaaggaa 240
gcaagaaagc aatcacacca agctctgtat ctagctacca catttaagga aaaaaagaga 300
cagaagagca tgttaaatgt taccaagaag atacagtcag tcggaaaaaa tacagacaag 360
aaaatacaga gcaaaacaac ccagcttctt cagcaaatca atataaaaaa attttaagaa 420
agagttaaag tataaactga gagacttcag aaacatatta tccaagtata atccatgaat 480
cttgtttaaa tatagatcaa rtaaaccact ataccaaaaa catcaaaaga caactgggta 540
aattttttaa atgactagct atttgatgtt aaggaagtaa tgttactctc ttatatacaa 600
tttgaaataa tctagcgagg agcagcaaat gtgcggctat gaggaagaaa cacaattggc 660
cattcttgaa tcattagctg gatggtggct atatgggggt agattttact actctctaat 720
tttacatata tttaaaatgt tccataataa attgttgagt tatcaaaaga aatatttcta 780
tataatagct aaaattattt ataaaagtta gtggtctcat aactttattt atttatttac 840
ttattttgag accgagtctc cctctgttat gcaggctgga gtgcagtggc tccatctcgg 900
ctcactgcaa acttcacctc ctggattgaa gcgattctcc tgcctcagcc cccccgagta 960
gctgggatta caggcttgca cccccacgcc cagctaattt t 1001

<210> 319
<211> 601
<212> DNA
<213> Homo sapiens

<400> 319

agctaccaca tttaaggaaa aaaagagaca gaagagcatg ttaaatgtta ccaagaagat 60
acagtcagtc ggaaaaaata cagacaagaa aatacagagc aaaacaaccc agcttcttca 120
gcaaatcaat ataaaaaaat tttaagaaag agttaaagta taaactgaga gacttcagaa 180
acatattatc caagtataat ccatgaatct tgtttaaata tagatcaaat aaaccactat 240
accaaaaaca tcaaaagaca actgggtaaa ttttttaaat gactagctat ttgatgttaa 300
rgaagtaatg ttactctctt atatacaatt tgaaataatc tagcgaggag cagcaaatgt 360
gcggctatga ggaagaaaca caattggcca ttcttgaatc attagctgga tggtggctat 420
atgggggtag attttactac tctctaattt tacatatatt taaaatgttc cataataaat 480
tgttgagtta tcaaaagaaa tatttctata taatagctaa aattatttat aaaagttagt 540
ggtctcataa ctttatttat ttatttactt attttgagac cgagtctccc tctgttatgc 600
a 601

<210> 320
<211> 601
<212> DNA
<213> Homo sapiens

<400> 320

```
ccaactgatc taattagata aacttagtca atatatttga atcccacatt ccagcagcta      60
ttttctccat ttgcttttat tgctgtttgt ggtgagtttg atatataatt ttaaggtgtt     120
aacatcccta acttatgtat gggtacagct cataaatacg aacctgtgtc atgcaactca     180
tatatgactg tgttcaaaat aatgtgtatt agactgtaaa acgattttaa tattttaaat     240
aactttcctg catttgtcgg tttcagcagg aaagttattt ttaataactt ccctgtattt     300
sttggtttca gtattaatta atctcattaa tgctaaactt tgtgatccta ggttaaaaaa     360
catattcaag atagcttcag aatgtttggt atacaaatag gtctggctaa atataagtgt     420
tagctttctc aagcatctaa atgctggcgg gcttttaaaa aaccagggct ttaaggagaa     480
aacacctgct ctgtggtttt gtagcagata tgaagtattc aaatttctta ataaatagaa     540
aaagaaatat ataacagaaa caggttgcac ttgtctttct cattaagcag gtggttagta     600
c                                                                     601
```

<210> 321
<211> 501
<212> DNA
<213> Homo sapiens

<400> 321

```
agctcataaa tacgaacctg tgtcatgcaa ctcatatatg actgtgttca aaataatgtg      60
tattagactg taaaacgatt ttaatatttt aaataacttt cctgcatttg tcggtttcag     120
caggaaagtt atttttaata acttccctgt atttgttggt ttcagtatta attaatctca     180
ttaatgctaa actttgtgat cctaggttaa aaaacatatt caagatagct tcagaatgtt     240
tggtatacaa rtaggtctgg ctaaatataa gtgttagctt tctcaagcat ctaaatgctg     300
gcgggctttt aaaaaaccag ggctttaagg agaaaacacc tgctctgtgg ttttgtagca     360
gatatgaagt attcaaattt cttaataaat agaaaaagaa atatataaca gaaacaggtt     420
gcacttgtct ttctcattaa gcaggtggtt agtaccatta tttgcattct catagcctta     480
atatacattt tccttctcta g                                               501
```

<210> 322
<211> 601
<212> DNA
<213> Homo sapiens

<400> 322

```
ttttgagttt ctactttagt gtcttagtgc tttctcgata tgggagaatt catgtcctcc        60
attcagaagt atgcactaag taagaggtat catgtctggt tcttgattag gtactaatct       120
tgaaatatta tcctacaata ggttagagca cgtatatctc ctgataatat attgaatatg       180
atagatttaa ataattggtt aactaaatac taaagcaaat tgctgcacgt atcatttatt       240
attcattgtg tagaaagtgc ctgactcagt gtttggaaat tgtctgactt ttcctcatat       300
rtagtgtggt ttcatgttat tgtatataag acctgacatg aactctgttt acaataatct       360
cccagtgcca taaagaccat aataaataat ataaccaatt ggtttcttta tgctgtcatt       420
tattagggca tatggcatta gtggaggatt accttgtatt acccatagtg cttagagtat       480
gaatcacaca tgcaccttga aggaaaagag gtgcaatgta ataagaaacc agatattgaa       540
aatgcaagtt ttgttatgtt attctgggta tgttaacctt tattcctgcc ctccatatgc       600
a                                                                       601
```

<210> 323
<211> 501
<212> DNA
<213> Homo sapiens

<400> 323

```
aagaggtatc atgtctggtt cttgattagg tactaatctt gaaatactat cctacagtag        60
gttagagcac gtatatctcc tgataatata ttgaatatga tagatttaaa taattggtta       120
actaaatact aaagcaaatt gctgcacgta tcatttatta ttcattgtgt agaaagtgcc       180
tgactcagtg tttggaaatt gtctgacttt tcctcatata tagtgtggtt tcatgttatt       240
gtatataaga mctgacatga accctgttta caataatctc ccagtgccat aaagaccata       300
ataaataata taaccaattg gtttctttat gctgtcattt attagggcat atggcattag       360
tggaggatta ccttgtatta cccatagtgc ttagagtatg aatcacacat gcaccttgaa       420
ggaaaagagg tgcaatgtaa taagaaacca gatattgaaa atgcaagttt tgttatgtta       480
ttctgggtat gttaaccttt a                                                 501
```

<210> 324
<211> 854
<212> DNA
<213> Homo sapiens

<400> 324

tttcagcact gagagccaga gtggaattgt ctccttcatt gccactgcct tcacgttttg 60
tgtgtcgtat ctgttttgtg atcactgaga cccaagaacc cccgacttgc cgacatacta 120
tgtggccccg agagaggact tgagctctct gggtttcatc attaccatca attaaataaa 180
caggacagta gcttcttcct tggattgtta atttaaggct ctggataata catgtaaccg 240
ccttatgata gagcagaatt gtaagtaggc tcatggtaga atcgttcaat gacatttccc 300
tttcctttgg gagaaacaga aattcacagg tctaattctt ttcctattaa tagttcctgr 360

ccattattcc agaactgtcc taaaggaatt ctttctcctt aaggacacca cctcccagga 420
gggtatttaa agatttgcac aggccgggca cggtggctca tgcttgtaat cccagcagtt 480
tgggaggcca aggcgggtgg atcacttgtg ctcaggggtt caagaccggc ctggccaaca 540
tggtgaaacc ctatctctac taaaaacaca aaagttagct gggcctggct atgcatgcct 600
gtaattccag ctactcggga ggctgaggct ggagaatagc ttgaaccagg gaggtggaga 660
taacagtgag ctgagatgcc actatgacac tccagcctgg gtgacagagc aagactctct 720
ctcaaaaaaa aaaaaagatt tttatagtcc agtattcaac gttcatagta cacctttctt 780
atcctagtaa atcttctttt atcaaggtat atgatcccat atagtagtta actcttactc 840
ttactttatg acaa 854

<210> 325
<211> 501
<212> DNA
<213> Homo sapiens

<400> 325

aaatacttac tattaaatat gagaaactgt ggtgtttatc ggtaagatcc acgaaggaag 60
aagttttaaa gaaaaatact ttaaccgtgg aaaaaaaaaa ctttaatgtc tattatcgaa 120
taggggccgt aattactttt gcaaaataaa aaaacaaaca agactagcta tagtgtaaat 180
gtaatctgta tgctttttaa tgaaacaatt aagtaggttg cccatttaca attagcctga 240
ttttctcctg ygtggtatta tgtgtactta acaacaggac ccagtggaaa ttcactcatt 300
taacaaagtc tgcctacatg gtttcaaata tgggcctaac ttgaaaattc agtcataatt 360
aaatctaagg actaaaacaa atctgtataa aaagattctg ctaaataagg gaaaattcaa 420
gtctagggct acattctgaa agatattgaa gtagaacctc tgcagcaaga ctaggcttgg 480
aaagtgcggg gaggagggaa a 501

<210> 326
<211> 601
<212> DNA

<213> Homo sapiens

<400> 326

```
ccacatcaga aacatgagga aattctacat ggtaaaaaca gcaacaacca aaaaatactt      60
aaagtcaaca aaccaggaaa agacatctct gaatatagga atgccaaacc tttaacacaa     120
taaaacacag attatatttc agaaggctat attatatgtg tataccaaca tcaatatgtc     180
cagagtagct gcacagagtt ccatatttta gtctttataa gttcccctcc tcaccctact     240
cagtaggcac tttgtgtcta gaaacttctg tgtcaacagt tttccctctc tctggaattc     300
mtcaggacag aagtgattgg tgtggtggaa gagggttgtg ctaagagtga agttatatga     360
aagtaggatg gaggttagca agtagttaaa gtccagaaag gcaataaggt gttaaggaag     420
aacttttcca ttttacaggt ctgagcaagc aggaaatcaa ctctacaaac tttgaaactt     480
ggtaaatatg aaaacattct caataccatt tgtcatttaa taaatacaaa ttatactatt     540
ttactgcttg catctagaag tttgtcaaag atctcgtctt aattattcat tgtgtcggcg     600
a                                                                     601
```

<210> 327
<211> 601
<212> DNA
<213> Homo sapiens

<400> 327

```
gacgagatct ttgacaaact tctagatgca agcagtaaaa tagtataatt tgtatttatt      60
aaatgacaaa tggtattgag aatgttttca tatttaccaa gtttcaaagt ttgtagagtt     120
gatttcctgc ttgctcagac ctgtaaaatg gaaaagttct tccttaacac cttattgcct     180
ttctggactt taactacttg ctaacctcca tcctactttc atataacttc actcttagca     240
caaccctctt ccaccacacc aatcacttct gtcctgatga attccagaga gagggaaaac     300
ygttgacaca gaagtttcta gacacaaagt gcctactgag tagggtgagg aggggaactt     360
ataaagacta aaatatggaa ctctgtgcag ctactctgga catattgatg ttggtataca     420
catataatat agccttctga aatataatct gtgttttatt gtgttaaagg tttggcattc     480
ctatattcag agatgtcttt tcctggtttg ttgactttaa gtattttttg gttgttgctg     540
tttttaccat gtagaatttc ctcatgtttc tgatgtggaa agtataagaa tatcagccag     600
a                                                                     601
```

<210> 328
<211> 811
<212> DNA
<213> Homo sapiens

<400> 328

```
taaataatct ctaattagta taatgggtgt tcttagtgca gtgggtactt ttaaagtgct      60
ttgtggcttt tgatgaaaat tgtcttagta tttaaaactt tttcttaccc aatttttttgt    120
tcccatcgaa ttagcaatgc tgtaaagaaa ggcatcttat tccatttttt gttgctataa     180
aggaatactt gaggctgggt aatttataaa gatgaaaagt ttatttggct cgcaattctg     240
gatggctgga aggttaagta ctgggccaca gcatctggtg gggcctcga gctgcttcta      300
gtcataatgg aaggtgaagg gtgtaaagat catgtgacaa gggaggaaag aagagaagga     360
aggaggtgct ggttctttct atcaaccaat tcgcaagaga actaatagag aaagaactca     420
cttagccctg tgggaacaca ttaatctatt cataagggat ctggctgtat gatacaaaca     480
cctcccatta ggccccacct ccaaattgta tcccattggg gatcaaattt caaaaagaga     540

tttggaagga acaaacaaac catatctaag ccatagtaaa aggaatggct tttcaaaggt     600
aaaatttact ragtgtatta atattttacc aatttccagc caggagagta tgaatgttgc     660
attattacat tgctttgaaa caaagcatta gtcttaattc agaagtttaa attcagatgt     720
taacgttgca tatttaataa tgcacaacca gtactaaaat cctcattgaa atgacaaata     780
attttatttc gaatccctta tagaggttca c                                    811
```

<210> 329
<211> 811
<212> DNA
<213> Homo sapiens

<400> 329

```
tgtcttagta tttaaaactt tttcttaccc aatttttttgt tcccatcgaa ttagcaatgc        60
tgtaaagaaa ggcatcttat tccatttttt gttgctataa aggaatactt gaggctgggt       120
aatttataaa gatgaaaagt ttatttggct cgcaattctg gatggctgga aggttaagta       180
ctgggccaca gcatctggtg ggggcctcga gctgcttcta gtcataatgg aaggtgaagg       240
gtgtaaagat catgtgacaa gggaggaaag aagagaagga aggaggtgct ggttctttct       300
atcaaccaat tcgcaagaga actaatagag aaagaactca cttagccctg tgggaacaca       360
ttaatctatt cataagggat ctggctgtat gatacaaaca cctcccatta ggccccacct       420
ccaaattgta tcccattggg gatcaaattt caaaaagaga tttggaagga acaaacaaac       480
catatctaag ccatagtaaa aggaatggct tttcaaaggt aaaatttact aagtgtatta       540
atattttacc aatttccagc caggagagta tgaatgttgc attattacat tgctttgaaa       600
caaagcatta ktcttaattc agaagtttaa attcagatgt taacgttgca tatttaataa       660
tgcacaacca gtactaaaat cctcattgaa atgacaaata attttatttc gaatccctta       720
tagaggttca caatgtttta acaatgtagt tttgactaaa tagaagtagt caaaacctgt       780
cagattggaa atagtattta taaaacataa a                                      811
```

<210> 330
<211> 601
<212> DNA
<213> Homo sapiens

<400> 330

```
gctcatcaat tttgacttaa gaaaattcta gcaacattta tagattttgc caaaattcag        60
cttcttccca aatcaatcta taagaaggct cttccttaaa cataattttt atatctatga       120
actgcactag catttactat atatttttat cactctcacc attactggat aataaataaa       180
agctcattaa aagagttaac aaaacatatt tattttaggc atcctgaaaa aaagattcaa       240
ttttattatc atttctacaa taagtattga agaaaggaga atttaaatta cttcatatac       300
stgataaagg aaaacatatg caaggcaaat aaacatctta gatcatgaca tataaaataa       360
tagattatta ctaaagatta aaatactttc ttaagaatta aagcaattct aaaagcaata       420
gtaaataaca ttctttctag tgatcagaca ctggatacta tgtttgagat agacagtgaa       480
ttgggaatgt tgttttacag aagctcctac cttgcaagga caggcaagtt taaatgtcag       540
ctagaaaact atcttgagtt ttcagtaatg taagattttc ctattcaatt tcacacttta       600
a                                                                       601
```

<210> 331
<211> 601

<212> DNA
<213> Homo sapiens

<400> 331

```
agaaaattct agcaacattt atagattttg ccaaaattca gcttcttccc aaatcaatct      60
ataagaaggc tcttccttaa acataatttt tatatctatg aactgcacta gcatttacta     120
tatattttta tcactctcac cattactgga taataaataa aagctcatta aaagagttaa     180
caaaacatat ttattttagg catcctgaaa aaaagattca attttattat catttctaca     240
ataagtattg aagaaaggag aatttaaatt acttcatata cctgataaag gaaaacatat     300
rcaaggcaaa taaacatctt agatcatgac atataaaata atagattatt actaaagatt     360
aaaatacttt cttaagaatt aaagcaattc taaaagcaat agtaaataac attctttcta     420
gtgatcagac actggatact atgtttgaga tagacagtga attgggaatg ttgttttaca     480
gaagctccta ccttgcaagg acaggcaagt ttaaatgtca gctagaaaac tatcttgagt     540
tttcagtaat gtaagatttt cctattcaat ttcacacttt aaattttata tatatataaa     600
a                                                                     601
```

<210> 332
<211> 1110
<212> DNA
<213> Homo sapiens

<400> 332

```
tgtagaagtt cttatcactt cctggccttt tggctaagat caagtgtgaa atgtagaagt      60
tcctctaagc tttacttccc tcaaaaacta gttttatctt gtcagcagga ttcacttaaa     120
aagacaaatt cagattatga atttttttct tttttacagg gtctgctctg ttgcccaggc     180
tggagtgcag aggcacaatc tcggctcact gcagcctccg cctcctgggt tcaagcaatt     240
ctcttgcctc agcctcccga gtaactggga ttacaggcat gtgccaccac ccagctaatt     300
tttgtatttt tagtagagat ggggtttcac cacattggtc aggctggtct cgaactgctg     360
gcctcaagtg atccacttgc ctcggcctcc caaagtgcag agattacagg tgtgagccac     420
cgtgcccagc ctcataaccg tttcaactac tttttcactt gacaagcaga tgtgaagtta     480
```

acaaagtcac ccatatttga aataaagata gtatattcct ggggyaggca gaggcagttg 540

aggatcatga aataactatg ttggcatagt tatttaggtg ttgatactgt tattatgcca 600

ttgaaagtta aacagagaac cctctgggta catgttttat accaatgcac actatcttat 660

tagtccctct cataatgtgc agtcatcatt actgttacgg gttgaggtgt ccccatcctc 720

tatgggacac ctctatgttg aagtctcaga ttccctagaa tctcagaatg tgaccttgtt 780

tggaaacaga tttgctacag acgcaattag ttgagatgcg cttatatggg taggtcctaa 840

ttcagtgact ggtgtcctta aaaaaatgga aatgtacaca cggtggtaga catgcataga 900

gggaagagag atggagaaaa tggtcaccta caagccaaag acaggggtct ggagcagatc 960

cttccctcac agccctcaga aggaaccaat cttgccaata ccttgatttt ggacttccac 1020

ctccagaact ataacacatt tctgttcttc aagcaatttg tagccatttg ttacagctaa 1080

tacaatcaca catagaaatg acttgtaaat 1110

<210> 333
<211> 691
<212> DNA
<213> Homo sapiens

<400> 333

taaaacatgt acccagaggg ttctctgttt aactttcaat ggcataataa cagtatcaac 60

acctaaataa ctatgccaac atagttattt catgatcctc aactgcctct gcctacccca 120

ggaatatact atctttattt caaatatggg tgactttgtt aacttcacat ctgcttgtca 180

agtgaaaaag tagttgaaac rgttatgagg ctgggcacgg tggctcacac ctgtaatctc 240

tgcactttgg gaggccgagg caagtggatc acttgaggcc agcagttcga gaccagcctg 300

accaatgtgg tgaaacccca tctctactaa aaatacaaaa attagctggg tggtggcaca 360

tgcctgtaat cccagttact cgggaggctg aggcaagaga attgcttgaa cccaggaggc 420

ggaggctgca gtgagccgag attgtgcctc tgcactccag cctgggcaac agagcagacc 480

ctgtaaaaaa gaaaaaaatt cataatctga atttgtcttt ttaagtgaat cctgctgaca 540

agataaaact agtttttgag ggaagtaaag cttagaggaa cttctacatt tcacacttga 600

tcttagccaa aaggccagga agtgataaga acttctacat tttaagttat tcacaagata 660

actattaatg aacctgaaat agtttgtaaa g 691

<210> 334
<211> 640
<212> DNA
<213> Homo sapiens

<400> 334

```
aaacctttttt cctgttttac tattactaaa ggtggcacaa cagcaacctc aacaactttg       60
caccatgcca acactgatgt ttacacccag cacagcattt ttggtctcta tttttattct      120
cctctgaatg taatgaggat tcctagatgg ctagccaatt cgaatattta aggcaactga      180
aagttagaat gtttctgaaa catagtgttg ttgccagaga gtacgaaagt tttcaagaat      240
atcgggcaat tctgaaagta caaagaagcc agattaaatg aaataacact ggcgaagttt      300
tagcaaggtg actctcatat aatgatcatt atcattacca cagttaaaag aaaagagttg      360
tttatgaaag gccatgtgtc tgcaatgaaa ctcaaaagag aaaagttaac aggtgcaara      420
ggtagtttta ttataaaagg agggtaggca acaagaatat gtttaatttt tcttcctttt      480
catgagtaag gacaagagtt tcatatatgt gaatattttt atttaatttt aagtagaaat      540
ctgtttttaa aatatgggta tatgcttatt tgtgtaagtg taagaaacag aagtaagtac      600
agcaaaccag aaataggcca aacactcctg agcataattt                            640
```

<210> 335
<211> 919
<212> DNA
<213> Homo sapiens

<400> 335

```
tacacccagc acagcatttt tggtctctat ttttattctc ctctgaatgt aatgaggatt      60
cctagatggc tagccaattc gaatatttaa ggcaactgaa agttagaatg tttctgaaac     120
atagtgttgt tgccagagag tacgaaagtt ttcaagaata tcgggcaatt ctgaaagtac     180
aaagaagcca gattaaatga aataacactg gcgaagtttt agcaaggtga ctctcatata     240
atgatcatta tcattaccac agttaaaaga aaagagttgt ttatgaaagg ccatgtgtct     300
gcaatgaaac tcaaaagaga aaagttaaca ggtgcaaaag gtagttttat tataaaagga     360
gggtaggcaa caagaatatg tttaattttt cttccttttc atgagtaagg acaagagtkt     420
catatatgtg aatattttta tttaatttta agtagaaatc tgtttttaaa atatgggtat     480
atgcttattt gtgtaagtgt aagaaacaga agtaagtaca gcaaaccaga aataggccaa     540
acactcctga gcataatttt acttggtaga ttattcctga aacttaagga atcatctttg     600
aactcttttc ctcacttgac ttccaggatt caccatgcac ttgtgatttt cctttcattt     660
cactctccgt tcctcctcag tctttttttc tcccccaggt ctttttttgtt catcttaaac    720
tctaaatttt agaatatccc aggggtctgc cttcggcctt ctcttttata tctacactgg     780
cctcatacat aatcttaacc aagtcattat tttaaatacc tacaatatac tgaaaacttc     840
taaatttgta ttttaattct tgacttcttc catacagtct agatttgtat gtccataggc     900
tgacatcatt ggctgatac                                                  919
```

<210> 336
<211> 1001
<212> DNA
<213> Homo sapiens

<400> 336

ttactaaata ttctccaaca aatatatact tagtatatac tattagtgat gcatgctttc 60
aaatatttgg actatatcaa tgaatgaaac aaaaaattat ttgcccttaa ggagcttaga 120
ttctaacaga tggattcaga tgatttttat gccttatttc gtaggtttaa aagagcaatg 180
gggaaaaggg aagaagagag ggattgaaaa tattgagaag gttgggagac ttagcaattt 240
taagtaaggt agtgagggta ggttttattg gcaaagtgat ttttcagcag agactgggaa 300
agatgaacgt ggtatcctgg aggaaagcct cccaggcaga gttaagctgc taacaaaagt 360
gcccttaggc tggagtgggc ttgtttgatt aaggaacaaa gaggtcagca tggttgcact 420
agagagaaaa aatcagatgg cgtaaggaga tgaaatcaga aagatacgag gctaggcaaa 480
ggggtactct atgtaatgaa yatgacctgg cagtactgac atctcctgag ggactgttag 540
aagtgcagac tcttgtatct ttctcaagt ctatgaaatc tagacttcat tttaacaaga 600
tgacccgata tttacataca cattaaagtt ccagaagcac tgatataaca cattgtaaga 660
tcgcacagga cttcaattct ttttctggtt tttagaggca gtcctttggg gtgttttgtg 720
tagagtataa tgacctgaaa tatctaggat cactctagct actatcttga ggaaagagtg 780
caataaggcg gaacagttca gaggcaatgg tggtcttcta aatgaaagac acacagcact 840
caaaccaggc agttgaggag ggatgggaag aagttgtcaa attctagaca tattttaaag 900
gtagtgtcca gagaatttcc ttagatgcgt aggaacatgg aggataggac atagggtgga 960
aataaacgaa ataaagaaac tgaagctgat tctgacattt t 1001

<210> 337
<211> 1576
<212> DNA
<213> Homo sapiens

<400> 337

ataccttta agtgacatcc tagtgaatct ccatttgtca cgagacctca agctttccag 60
ttctggcaca aagtgattac tcataccatc acttcaaaat gatgattatc ttcatttatt 120
ttagttatat tgaacaaaat atacatttaa aaaatctaat tactaaatat tctccaacaa 180
atatatactt agtatatact attagtgatg catgctttca aatatttgga ctatatcaat 240
gaatgaaaca aaaaattatt tgcccttaag gagcttagat tctaacagat ggattcagat 300
gatttttatg ccttatttcg taggtttaaa agagcaatgg ggaaaaggga agaagagagg 360
gattgaaaat attgagaagg ttgggagact tagcaatttt aagtaaggta gtgagggtag 420
gttttattgg caaagtgatt tttcagcaga gactgggaaa gatgaacgtg gtatcctgga 480
ggaaagcctc ccaggcagag ttaagctgct aacaaaagtg cccttaggct ggagtgggct 540
tgtttgatta aggaacaaag aggtcagcat ggttgcacta gagagaaaaa atcagatggc 600

gtaaggagat gaaatcagaa agatacgagg ctaggcaaag gggtactcta tgtaatgaac 660

atgacctggc agtactgaca tctcctgagg gactgttaga agtgcagact cttgtatctt 720

ttctcaartc tatgaaatct agacttcatt ttaacaagat gacccgatat ttacatacac 780

attaaagttc cagaagcact gatataacac attgtaagat cgcacaggac ttcaattctt 840

tttctggttt ttagaggcag tcctttgggg tgttttgtgt agagtataat gacctgaaat 900

atctaggatc actctagcta ctatcttgag gaaagagtgc aataaggcgg aacagttcag 960

aggcaatggt ggtcttctaa atgaaagaca cacagcactc aaaccaggca gttgaggagg 1020

gatgggaaga agttgtcaaa ttctagacat attttaaagg tagtgtccag agaatttcct 1080

tagatgcgta ggaacatgga ggataggaca tagggtggaa ataaacgaaa taaagaaact 1140

gaagctgatt ctgacatttt agacctaaaa tctcaactaa aagttgccaa gatgggaaaa 1200

actaggtgca tcttgtttgg tgagtggaaa tcagccttgt gaattaagac ttaaactgat 1260

gtctttaatc ccgtagaaat accatgaagg cagtagaaga tggctaaaga gaggtctaga 1320

ctgtaggtac aaatttaaaa gtcacttgca tttggatgct taaagtcagg atattgtgaa 1380

gtcaacagag gaataaataa atgcagagag gggaaagaaa aggcccatag actgagccat 1440

tgtctggttt atttacatat tagtatatat tttcttaaag atgtttgcta tataataatg 1500

agttacctaa agtgtgactt ttctaaattt atggggaatt ttctacattg tgttatggca 1560

ctactaaaaa taataa 1576

<210> 338
<211> 1275
<212> DNA
<213> Homo sapiens

<400> 338

gtaaaactaa ttataattaa aatcaaaata tttactgaac ctacttactc ctataatttg 60

cgttgctggt taaaacccag ctataaaaat tttgatcaaa aatttttatt ttgtaaatga 120

tctgacacag cataaatgtt aatcacattt ctttatttta tttgcagatt aatttgagta 180

atttgaaaaa ttattaatgt tacttaatta ctctcaacac cttacagtgt ctcctgtaag 240

cactattggt gatactgaat ttaagttaca tttaacaact atcagaaaat agtttttaaa 300

gtaaaaatta tgatttggag tttaccaact aaatcttgtt agctttcact gcctctattg 360

agaagagcag cagttcttat cttcctcctt tttcttcttt aattaacaag agattatttg 420

tatcatagcc ataaaatcag ttcaggtatt acatgaacga cacccctgac tgcaatggtg 480

tagtttattg tattagtcca ttttcatgct gctgataaag acatacataa gactgggtaa 540

tttataaaga aatagaagtt taacggactc acagttccat gtggctgggg aagcctcaca 600

atcatgatcg aaggcaaaag gcacatctta catggcaaca ggcaagagag aatgagagcc 660

aagtgaaagg agaaacccct tataaaacct tcagacctca tgagacttat tcactaccac 720

aagaacagta tgtgagaaac agtcccatga tccagttatc tcccactggg tccctcccac 780

cacacaaggg aattatggga actgcaattc aagatgaaat gtgggtggaa gcacaacgga 840

actatatcat gatcaaagca ttattgtttt ctctgataag ctgatctaga aagtgctgct 900

tgtgatcagc tttggtgacc atgatcagtg aaatggttaa ggaaatctac agattttgta 960

ggtttgtgcc ttgacagacg accggtatct gtttctcttt tcatgatgaa gtatctaaca 1020

aagctctgtc caaaattttg aatttctcgt taaawgcatc atgattatag aacagaggtt 1080

acaatcaatt attcagtcac acaatcactc tcatcagtca ttaaggtgca tacctggtgt 1140

tccagttatt cagtgtggta taacaaacta cctggaactt aatggcttga aatagtcacc 1200

attacattat gattgtccat tctctgcatc aataattagg atttggcaaa gagggaatgg 1260

tttgtttaca gacag 1275

<210> 339
<211> 1275
<212> DNA
<213> Homo sapiens

<400> 339

```
gtaaaactaa ttataattaa aatcaaaata tttactgaac ctacttactc ctataatttg      60
cgttgctggt taaaacccag ctataaaaat tttgatcaaa aatttttatt ttgtaaatga     120
tctgacacag cataaatgtt aatcacattt ctttatttta tttgcagatt aatttgagta     180
atttgaaaaa ttattaatgt tacttaatta ctctcaacac cttacagtgt ctcctgtaag     240
cactattggt gatactgaat ttaagttaca tttaacaact atcagaaaat agtttttaaa     300
gtaaaaatta tgatttggag tttaccaact aaatcttgtt agctttcact gcctctattg     360
agaagagcag cagttcttat cttcctcctt tttcttcttt aattaacaag agattatttg     420
tatcatagcc ataaaatcag ttcaggtatt acatgaacga cacccctgac tgcaatggtg     480
tagtttattg tattagtcca ttttcatgct gctgataaag acatacataa gactgggtaa     540
tttataaaga aatagaagtt taacggactc acagttccat gtggctgggg aagcctcaca     600
atcatgatcg aaggcaaaag gcacatctta catggcaaca ggcaagagag aatgagagcc     660
aagtgaaagg agaaacccct tataaaacct tcagacctca tgagacttat tcactaccac     720
aagaacagta tgtgagaaac agtcccatga tccagttatc tcccactggg tccctcccac     780
cacacaaggg aattatggga actgcaattc aagatgaaat gtgggtggaa gcacaacgga     840
actatatcat gatcaaagca ttattgtttt ctctgataag ctgatctaga aagtgctgct     900
tgtgatcagc tttggtgacc atgatcagtg aaatggttaa ggaaatctac agattttgta     960
ggtttgtgcc ttgacagacg accggtatct gtttctcttt tcatgatgaa gtatctaaca    1020
aagctctgtc caaaattttg aatttctcgt taaatgcatc atgattatag aacagaggtt    1080
acaatcaatt attcagtcac acaatcactc tcatcagtca ttaaggtgcr tacctggtgt    1140
tccagttatt cagtgtggta taacaaacta cctggaactt aatggcttga aatagtcacc    1200
attacattat gattgtccat tctctgcatc aataattagg atttggcaaa gagggaatgg    1260
tttgtttaca gacag                                                     1275
```

<210> 340
<211> 1001
<212> DNA
<213> Homo sapiens

<400> 340

```
gaaacaaaaa attgcttttt atatattgat atttttgcac ggatttctta ggattttcta      60
tgtacatgac catgtcatct gcaaatgaaa tagttttatt tctttatcaa tccggatgaa     120
tttattaaaa ttatcttgcc taatttccca aatagggcct ccatgttgaa cataagtggt     180
ggcaagggtg atctgttgct aatctcagtg gatgatattc agtgttttac aatgatcttc     240
gacagctctg gctgttaaat tatcatagtc tgtatggcct aaacaaacaa aatacttatg     300
attatggggg aggctgggat atccaagatc aagttgctgg caggtctagc aacctgccac     360
tgggaagccc tgcttcccag ttttcagatg gccaccttct tatagtatct tcaccaaaga     420
tagggcagag agagcaagca agctctctac cttctcatat aagggcacta atcccaccat     480
gaaggcgcca ctgtcatgac stgattatgt cacaaagacc ccggggcaaa tattaccact     540
gtgaggagta cagttttagc atgtgaattt tggaagaaca caaacattta gtacagagtg     600
actattaagt atgttattaa ctatggagtt tttgtaggca ttttttaaca cattgagaaa     660
gtttcctcta ttcctacttt tgttgagaag tttttatgat gacaaggcat tacattttat     720
ccaatgactt ttctgtgtgt attgagatga ctgatttgtt ctgccaattt aaatccattg     780
ttgattctct ctaggatttt ttttatttca gttattaaat ttttcaacag gagaattact     840
gtcttgttct tttttttgta atttctgtcc ccttactggt attccatatt taataaggca     900
tcataatagt actcttcttt agtttcttaa agatggtttt ctttagtttt taacatattt     960
atgtctattt agaagtcttt gttaagtctg acatctgagc t                        1001
```

<210> 341
<211> 1001
<212> DNA
<213> Homo sapiens

<400> 341

```
ggatttctta ggattttcta tgtacatgac catgtcatct gcaaatgaaa tagttttatt      60
tctttatcaa tccggatgaa tttattaaaa ttatcttgcc taatttccca aatagggcct     120
ccatgttgaa cataagtggt ggcaagggtg atctgttgct aatctcagtg gatgatattc     180
```

agtgttttac aatgatcttc gacagctctg gctgttaaat tatcatagtc tgtatggcct 240

aaacaaacaa aatacttatg attatggggg aggctgggat atccaagatc aagttgctgg 300

caggtctagc aacctgccac tgggaagccc tgcttcccag ttttcagatg gccaccttct 360

tatagtatct tcaccaaaga tagggcagag agagcaagca agctctctac cttctcatat 420

aagggcacta atcccaccat gaaggcgcca ctgtcatgac ctgattatgt cacaaagacc 480

ccggggcaaa tattaccact stgaggagta cagttttagc atgtgaattt tggaagaaca 540

caaacattta gtacagagtg actattaagt atgttattaa ctatggagtt tttgtaggca 600

ttttttaaca cattgagaaa gtttcctcta ttcctacttt tgttgagaag tttttatgat 660

gacaaggcat tacattttat ccaatgactt ttctgtgtgt attgagatga ctgatttgtt 720

ctgccaattt aaatccattg ttgattctct ctaggatttt ttttatttca gttattaaat 780

ttttcaacag gagaattact gtcttgttct tttttttgta atttctgtcc ccttactggt 840

attccatatt taataaggca tcataatagt actcttcttt agtttcttaa agatggtttt 900

ctttagtttt taacatattt atgtctattt agaagtcttt gttaagtctg acatctgagc 960

tctctcaaag tttctgctga tttttttttt cctatgtttg g 1001

<210> 342
<211> 701
<212> DNA
<213> Homo sapiens

<400> 342

ggaaaccctg gcctcttgat cacactttcc tggagtttag tcccctctgc aatatgtacc 60

tgggagtcat aagaaatgcc agttacaaaa acttcctgta cagatatcct agcactcaac 120

tggaaaccgg ggagagtcac aattctgtct ttccagccat atgtaactga aatggagatc 180

ttttcaccct gagccagggg tgatgggaaa gggagctggt catggctcaa tgtttagcct 240

tttcttggtc ttcaagattt catagacatt cttaaataca tgtttctttc aatgaagttt 300

gcccttagga caattcacag ctacattagg tactttttaa ataatacttt tgaccatccg 360

tggttatttc attgaagaaa atctatagag cacctcagcc atcattccag aagtgactat 420

cctcctcagt aatggttctt attctaattt taaatatcat tgatgtagaa cattctattt 480

cactattcct tcattttatt rttatgggaa attatataca gttctccaga tttttaaagc 540

cttgctaaca tgttttaagt cacacaaata ttcttctgtg ggaaaatgac agtaatttag 600

tgtgcaacaa ttatatagaa ctatttttca aacttataaa cgaagtgaaa ttctaaataa 660

aatcatttat caaacacaaa aatttgagcc agaataagga a 701

<210> 343

<211> 701
<212> DNA
<213> Homo sapiens

<400> 343

```
aatgccagtt acaaaaactt cctgtacaga tatcctagca ctcaactgga aaccggggag      60
agtcacaatt ctgtctttcc agccatatgt aactgaaatg gagatctttt caccctgagc     120
caggggtgat gggaaaggga gctggtcatg gctcaatgtt tagccttttc ttggtcttca     180
agatttcata gacattctta aatacatgtt tctttcaatg aagtttgccc ttaggacaat     240
tcacagctac attaggtact ttttaaataa tacttttgac catccgtggt tatttcattg     300
aagaaaatct atagagcacc tcagccatca ttccagaagt gactatcctc ctcagtaatg     360
gttcttattc taattttaaa tatcattgat gtagaacatt ctatttcact attccttcat     420
tttattatta tgggaaatta tatacagttc tccagatttt taaagccttg ctaacatgtt     480
ttaagtcaca caaatattct yctgtgggaa aatgacagta atttagtgtg caacaattat     540
atagaactat ttttcaaact tataaacgaa gtgaaattct aaataaaatc atttatcaaa     600
cacaaaaatt tgagccagaa taaggaatgt aaattacaat ttaaacacag attataaact     660
atcttacttt taaaatgtta aaattcctaa cttgtttgaa a                         701
```

<210> 344
<211> 768
<212> DNA
<213> Homo sapiens

<400> 344

```
ctaaaatcta ccattatatg atatccttcc caatacataa attaaaaaaa aaaacactgt        60
agaggaaaaa gcaatatttt gaaatgatat gcttttcttt gtttgtcttc aaacaattac       120
atcttcatca taatggttgt attagtctgt ttttacactg ctataaagaa ttgcctgaga       180
ctgagtaaca tataaagaaa aaagttttaa ttgaccacag tttcacaggc ttaataggaa       240
gcatgactgg gaaacttaga atcatggcag aagaggaagg ggaagcaagg atcttcttca       300
catggtagca ggagagagag cacaaagggg gacacgctac acactttcaa acaacgagat       360
ctcctgagaa ctctatcggg agaacagcaa gagggaagtt cacccctatg attcaatcag       420
ctcccaccgg gcttctcccc tgacacatga ggaattacaa ttggatgaga gatttgggtg       480
gggacacaca gacaaaccat atcaactgtc atggacttaa acaattgtct ttgaattgtc       540
tttttcata cttttatttg catctttyca ctaaaaagat gacacaaagt aatcctagtt        600
tacattttt accatgtaat tccatattac tttttcctga aagttactta tttttaaatc       660
tcaaagctct tcatacttat ggtttgatct gcacttacaa ctggatctca gaaagattga       720
attctcccat cataccaagt tcatgtctct cactcttaat atttgttc                    768
```

<210> 345
<211> 701
<212> DNA
<213> Homo sapiens

<400> 345

```
aaatgatatg cttttctttg tttgtcttca aacaattaca tcttcatcat aatggttgta        60
ttagtctgtt tttacactgc tataaagaat tgcctgagac tgagtaacat ataaagaaaa       120
aagttttaat tgaccacagt ttcacaggct taataggaag catgactggg aaacttagaa       180
tcatggcaga agaggaaggg gaagcaagga tcttcttcac atggtagcag gagagagagc       240
acaaaggggg acacgctaca cactttcaaa caacgagatc tcctgagaac tctatcggga       300
gaacagcaag agggaagttc acccctatga ttcaatcagc tcccaccggg cttctcccct       360
gacacatgag gaattacaat tggatgagag atttgggtgg ggacacacag acaaaccata       420
tcaactgtca tggacttaaa caattgtctt tgaattgtct tttttcatac ttttatttgc       480
atcttttcac taaaaagatg rcacaaagta atcctagttt acatttttta ccatgtaatt       540
ccatattact ttttcctgaa agttacttat ttttaaatct caaagctctt catacttatg       600
gtttgatctg cacttacaac tggatctcag aaagattgaa ttctcccatc ataccaagtt       660
catgtctctc actcttaata tttgttccca agacaacaat t                           701
```

<210> 346
<211> 6758
<212> DNA

<213> Homo sapiens

<400> 346

```
agagtgggcc attgttctga ctagtctggg gctccccaaa gaactggtat ctgtctcacc      60
tgactcagaa caatgataag gctgtagatc tttttggaag tctatgaaaa caggcacaat     120
gaaggcagca tgttagagat ataattccac aggaagatgc caggtaaaac aaaagagaaa     180
aagcaggaac aagctgatta ggaaatttgt gatgactaaa agtatataca caagcccaaa     240
taagatactc caaagatgtt tgataggttc tagatctcta gatatactgc tcaatgaaag     300
tgtccccctg aacaaagcca gtctgcaaag actgggtgag atgatttttt ttaaatgtca     360
agtctcagca acaacaaaaa tgacaagaca tgcacagaag caagaaaata taacacaatc     420
aaagaaaaaa aagccacaga aatcagtcct agagaaaacy gatctatgag ctgcctgaca     480
ataattataa aataactatc ataaaaatgc ccagtgagat ataagaaaac acagacaact     540
aaatgaatca ggaaaatgat gcatgaacaa aatgggcata tcaacagaga tggaaatgac     600
aaagataaac aaacagaaat tttggagctt aaaaatacag taagtaaagt gaataattca     660
ctaaaaatat tcaatagcag actagatcag gcagaagaaa atatcaatga acttgaagac     720
agatcatcaa gtcagaggaa caacagcaac aaaaaagaat gaaaaaagtg aagacagcct     780
aagggactta ggagtcagta ccaaggaaat caatatatac gttatagatg tatcagaaga     840
```

aaaagggaga aaaatgaaaa gaaagcatat ttgaaaaaat aatagctgaa gaattctcaa 900
tttcaaagag agaaattgat atacaaattc aagaagttca aaagactcta gccataataa 960
atctaaagag actcacacta agacatatta tcatcaaact gtcaaaatca aagacaaaga 1020
attgtgaaat ctgccaagga aaagtgactc atcacacata agagatataa cataagattg 1080
tcacaggatt tctgaacaga cactttgcag gtcagaggga agtagggtga catattccag 1140
gtgctgaaag aagaaaacac cctgccaacc aagaatatgg catccagaaa aactttccta 1200
gaagaatgaa ggagaaattt agactttccc aaataaacaa aagctgaggg agttcattac 1260
taccagacct gctctgcaaa atgctaaaga gaaaccttca ggtgaaacaa aaagatgcta 1320
gacagtaaca caaaaccact cataaataac ttcttcagta aaaataatac atcgacaaat 1380
atggtaacct gtattaatac tggtgcacaa attcactttc aaattttata aataagaatt 1440
taaaggatga aaacatctaa aactaactat aaatctatat aatgaatata caatatataa 1500
aaaaatttgt gatcacaata acataaaatg ggggaggtag agctgtatag gggtagagct 1560
tttgtatgca attgaaatta ccatcagttt aaactgaact gttataacat taagatgttt 1620
tatgtaattg caatggtaac tatattctat agaatatatt aaaaagaaaa agaaaatagg 1680
aagggaatca aagcatgtcc ttgtaaaaaa gtcaatgaaa gcaaaagaaa ggcagaaaga 1740
gtgaaaagga ggaataaaaa gttataagac ataaaaaaaa tgaaaatagt aatagtcctg 1800
ccatatcagt aattacatta aatataaatg gattaaactc cctaatcaaa tcatagattg 1860
gtttgcaaga actaacttta caattaaaga cacacagctg acggtgaagg gagaaaaaaa 1920
acttccatgc agtgaccaaa atagaggagg gtggctgtat tactgtcaga caaaataaaa 1980
tttaagtcaa aaactgttac aagagtaaaa gaagggcatt atacagttaa aaaagtaaat 2040
tcgccaggca gacacaacaa ttataaatat caatacataa aaataagagc tcctaaatat 2100
atgcagcaaa cagacataat tgaagaaaga aataaatagc taaaatggta gaagacttta 2160
atacccccac ttacaataat gtataaaata acaagacaga atgtaaataa aaatgtagag 2220
aatttgagca acactgtaga ccaattggac ctaataaata tactcagaat aatccatcca 2280
accaaagcag aaacagaata tacattcttt tcaagtacac atttgacatt ctctgggatt 2340
aactacatgt tatgcaacaa acaagtctca acaatgttta aaagtctgat attacacaaa 2400
gtattgtttc tgatgacgat ggaaagaacc tagaagccaa tagcaaaaag aaaatagaaa 2460
atccacacat atgtggaaat taaactacat gcaattaagc aaagggccaa agaagaagaa 2520
gaaaaaagaa aacaccgtga aacaaataaa aacaaaaata cagcatatga aaatgcatgg 2580
gatgcagcaa aagtgatggt aagagaaatg tttatagtta taaatgcaaa ccttaaaaaa 2640
gaagaaagaa aacaaaaata ctcaaattaa caactttaca agtcaagaag gtagagaaaa 2700

aagaacaaac tataccaaaa gctaacacag aaagaaaaga ataaagatta aaaacaaaaa 2760
caatttaaaa aatagcagaa ctaaaagttg gttctttgaa aagatcaaca gaattgacaa 2820
tttcttagct acattaagaa aaatacaaga ctcaaataac acaaatcagt ggtgaaaggg 2880
ggtattataa ctgatgccac agaaatacaa aaggatcata agggactact acaaattgta 2940
tgacaacaaa ttgagtaacc taggatacct tgataaattc caaaaaatgc acaatatact 3000
gaatcatgaa tacatgaccc ttataaatca agactaaatc ataaagaaat agaaaatatc 3060
aacagaccaa taattagtaa ggagaataaa ctagtaatca gaaacctccc aacaaagaaa 3120
agcttaggac caaatggctt tactggagaa ttctaccaac cattaaaagg ataattaaga 3180
ccaatcttcc tcaaactttt aaaacaaatg ttaaagagga ggaaactctt tcaatctcat 3240
tcataaggtc agcattatcc ttataccaaa accagacaaa gacactatta aaaaaactta 3300
gaccaatatc cctgatgaat ttcgatgcaa gaatcctcag caaaatacta tcaaacaatt 3360
caacagcata cttaaatgat tatatgctgt aatcaagatg catttattct ttgaatgcaa 3420
gtgtaattca acacataaaa ttcaatcaat gtaatacacc acattaacag aatgagagac 3480
aaaaaccaca taattatatc aactgatgca gaaaaaaatc tgacacagtt caacaccttt 3540
tgtgataaaa acactcaaca aactaggaaa agaaggaaac aactttaaca catcatatgc 3600
tcactgatga aaatctacaa gttctttata aaagatcagg aacaagacaa taatctgcat 3660
tgttaccact tctattatac gtagtattgg aagttctaat cagagcaaat taggcaagaa 3720
aaataaataa aaggcatcca aagtggaaag gaagtaaaat aatctctttt tacagatgat 3780
ataaccttag aattagaaaa tcctaaaaat ttcacatacc aagaaaaagc gtgttaaaat 3840
taataagtaa attcagcaag ttgactgata caaaatcaac acagaaagct cagttgtgtg 3900
tctgtgtgtc tcatacacta acaatgaaca atctgaaaag gagattaaga aaacaatttc 3960
atttacaata gcatcaggaa aaaaaataaa tacttaggaa caaacttaac caaggggttg 4020
gaattcctgt atactgaaaa ctacaaatat tgccaaaaga aaataaagga gacacaaata 4080
agtgatatgt ttttaatatg tccacccaaa gtgatcttca gattcaatga aatccctatc 4140
aaagttataa tggcattttt ctgcaggaat gtaaaaattt atcctaaaat tcatatagaa 4200
tctctaggta ccctgagggc caaacaattt tgagaaaaaa aaaagaacaa aattggagga 4260
ctcacacttc cagattacaa gaatatttac aaattacata tttacaaaaa aaattacaaa 4320
gccacaataa tcaaaacaac gtgggatttg cataaaggca gatatataga ccagtggaat 4380
agtattgaga gtccagaaat aaacccttag gtatatcatc aaatgacatt tgacaaagtg 4440
ctggtaccac tcaatgggaa tgggacaatt tgttcaacaa atagagcaaa gaaaactaaa 4500
catccatgtg caaaagaata aatctggacc cttatattac actatagaca aaattaattc 4560
aaaatggatt aaagatctaa atgaaagatc taaaactata aactcctag gagaaaacag 4620

aggaaaaatt tcatgctaat ttggcaacat tttgtgatgt gacaccaaaa gcagagtcaa 4680
taaaagcaaa aattagacag atggaaatcc atcatagttt ataacttttg gtcattaaag 4740
aacagtcaac agagtgaaaa ggcaatctat aaaatggggg aaaaacagaa aatatgtgca 4800
aatcacagat atctgatagg ggattcatat ccagaataaa taaagaactc ctatatctca 4860
acaacaaaaa atctaatcca atcaaaaaat gggccaaggg agtgaagata catttctcca 4920
aagatgttat acaaatggcc aggaagcata tgaaaagatg ttcaatgtca ctaatcatca 4980
gagaaatgca aatcaaaacc acagtgcaat atcacttcac attcattaga atggcttctg 5040
tcatgaacaa cagaaaataa caagtgttga tgagtgtgta gagaaattga gacctttata 5100
taattttggc agaaattcaa aatggtgcaa ccactataaa aaatgatatg gaggtcctca 5160
aaaaattaaa aatagaacta ccatatgatc cacaatccca cctctgggta catattcaaa 5220
agaattgaaa gcagggtgtt gaagatatat ttgcacactc tttatagcag cactgttcac 5280
aatagccaag agatgaaagt aacccaaagg ttcatgaagc aatgaataaa caaaatatat 5340
tatgtacata gagtaaaata ctgtgcagct ttaaagagaa aggaaatctt atactatgct 5400
acaacatgaa tggaacttta gggcattata gtaagtaaaa taagccagtt ttttttaaag 5460
gacaaataaa cactatacga ttctacttaa gtatttaatg ttgtcaaatt tataaatata 5520
gaatgtagaa tagtggttac cctgagctgg gggaaagggg caaaggggaa ttgttatttt 5580
aatgggtata gtttcagttc tgcaaaatga aaaggttctg gaaatctgtt tcacaatgtt 5640
gtaaatataa ttactctgaa attgtacact taaaaatggt taagatgaca aatagagttg 5700
tgatgtcttc ttttgttatt atatagaaaa actttttcat atgataatag tctttgtttt 5760
taagctgact ttgctgatat taatataatc cttccatttt tctttaaaat gctatatgct 5820
ttcacataat tttgctttac gttgatgtat ttatacataa ggtgggtttc ttatagatac 5880
cacgttgtgt gtcttttttta tctaagttga tagacttgcc ttttgttagg gtatttaaat 5940
aatttatatt taatgtaatt attgatatag ttgagtgtgt tgatttttgt tttctatttg 6000
ctccatctgt tgttggttct cattattcct ctgtttctac cttcttttgt actaattatt 6060
atattttatt atttttcatc tcaactgttg gcttattagc cacattgctt ttaaaatttt 6120
taatgattgc tctagggttt ataataaaca aaatgttagc attttctacc atcaaatatt 6180
tttacactat tcatgtatac ttcaatttct ttcttcccat cctttgaact atatcttcat 6240
acattttact ctacatttgt tataactcag tgctttgaaa gtcaattatt tttgtctttg 6300
acagtcaatg atttttaaag agtttaacag tgaaaaaaaa tggctttcat ctttttccat 6360
tagatttcat actccttctg cctgaagaat ttcttttaat agaccttgta ctgcgggtct 6420
caggcaagaa attctctcag cctttgttgg tttgaaaaac tgcttattac acctttgttt 6480

```
ttgaaagata ttttcactag gtatagaagt ctgggttgac agttctcatt gtttgtcaca      6540
gcatttttaa gatgcccatt caattgtctt gtcttgtata attttggatt agtctggtgt      6600
atttcttacc tttgttcctc tctgtgcaat gcttcaacca tcccacttca ggctgccttt      6660
aagatgtttt cttttccctt aatctttagt ttttagctgg ttgacagtga cgcatctaag      6720
tgtagtgtat gaggttgctt ttattgtcac tgttgttg                              6758
```

<210> 347
<211> 6758
<212> DNA
<213> Homo sapiens

<400> 347

agagtgggcc attgttctga ctagtctggg gctccccaaa gaactggtat ctgtctcacc 60
tgactcagaa caatgataag gctgtagatc tttttggaag tctatgaaaa caggcacaat 120
gaaggcagca tgttagagat ataattccac aggaagatgc caggtaaaac aaaagagaaa 180
aagcaggaac aagctgatta ggaaatttgt gatgactaaa agtatataca caagcccaaa 240
taagatactc caaagatgtt tgataggttc tagatctcta gatatactgc tcaatgaaag 300
tgtccccctg aacaaagcca gtctgcaaag actgggtgag atgatttttt ttaaatgtca 360
agtctcagca acaacaaaaa tgacaagaca tgcacagaag caagaaaata taacacaatc 420
aaagaaaaaa aagccacaga aatcagtcct agagaaaact gatctatgag ctgcctgama 480
ataattataa aataactatc ataaaaatgc ccagtgagat ataagaaaac acagacaact 540
aaatgaatca ggaaaatgat gcatgaacaa aatgggcata tcaacagaga tggaaatgac 600
aaagataaac aaacagaaat tttggagctt aaaaatacag taagtaaagt gaataattca 660
ctaaaaatat tcaatagcag actagatcag gcagaagaaa atatcaatga acttgaagac 720
agatcatcaa gtcagaggaa caacagcaac aaaaaagaat gaaaaaagtg aagacagcct 780
aagggactta ggagtcagta ccaaggaaat caatatatac gttatagatg tatcagaaga 840
aaaagggaga aaaatgaaaa gaaagcatat ttgaaaaaat aatagctgaa gaattctcaa 900
tttcaaagag agaaattgat atacaaattc aagaagttca aaagactcta gccataataa 960
atctaaagag actcacacta agacatatta tcatcaaact gtcaaaatca aagacaaaga 1020
attgtgaaat ctgccaagga aaagtgactc atcacacata agagatataa cataagattg 1080
tcacaggatt tctgaacaga cactttgcag gtcagaggga agtagggtga catattccag 1140
gtgctgaaag aagaaaacac cctgccaacc aagaatatgg catccagaaa aactttccta 1200
gaagaatgaa ggagaaattt agactttccc aaataaacaa aagctgaggg agttcattac 1260
taccagacct gctctgcaaa atgctaaaga gaaaccttca ggtgaaacaa aaagatgcta 1320
gacagtaaca caaaaccact cataaataac ttcttcagta aaaataatac atcgacaaat 1380

atggtaacct gtattaatac tggtgcacaa attcactttc aaattttata aataagaatt 1440
taaaggatga aaacatctaa aactaactat aaatctatat aatgaatata caatatataa 1500
aaaaatttgt gatcacaata acataaaatg ggggaggtag agctgtatag gggtagagct 1560
tttgtatgca attgaaatta ccatcagttt aaactgaact gttataacat taagatgttt 1620
tatgtaattg caatggtaac tatattctat agaatatatt aaaaagaaaa agaaaatagg 1680
aagggaatca aagcatgtcc ttgtaaaaaa gtcaatgaaa gcaaaagaaa ggcagaaaga 1740
gtgaaaagga ggaataaaaa gttataagac ataaaaaaaa tgaaaatagt aatagtcctg 1800
ccatatcagt aattacatta aatataaatg gattaaactc cctaatcaaa tcatagattg 1860
gtttgcaaga actaacttta caattaaaga cacacagctg acggtgaagg gagaaaaaaa 1920
acttccatgc agtgaccaaa atagaggagg gtggctgtat tactgtcaga caaaataaaa 1980
tttaagtcaa aaactgttac aagagtaaaa gaagggcatt atacagttaa aaaagtaaat 2040
tcgccaggca gacacaacaa ttataaatat caatacataa aaataagagc tcctaaatat 2100
atgcagcaaa cagacataat tgaagaaaga aataaatagc taaaatggta gaagacttta 2160
atacccccac ttacaataat gtataaaata acaagacaga atgtaaataa aaatgtagag 2220
aatttgagca acactgtaga ccaattggac ctaataaata tactcagaat aatccatcca 2280
accaaagcag aaacagaata tacattcttt tcaagtacac atttgacatt ctctgggatt 2340
aactacatgt tatgcaacaa acaagtctca acaatgttta aaagtctgat attacacaaa 2400
gtattgtttc tgatgacgat ggaaagaacc tagaagccaa tagcaaaaag aaaatagaaa 2460
atccacacat atgtggaaat taaactacat gcaattaagc aaagggccaa agaagaagaa 2520
gaaaaaagaa aacaccgtga aacaaataaa aacaaaaata cagcatatga aaatgcatgg 2580
gatgcagcaa aagtgatggt aagagaaatg tttatagtta taaatgcaaa ccttaaaaaa 2640
gaagaaagaa aacaaaaata ctcaaattaa caactttaca agtcaagaag gtagagaaaa 2700
aagaacaaac tataccaaaa gctaacacag aaagaaaaga ataaagatta aaaacaaaaa 2760
caatttaaaa aatagcagaa ctaaaagttg gttctttgaa aagatcaaca gaattgacaa 2820
tttcttagct acattaagaa aaatacaaga ctcaaataac acaaatcagt ggtgaaaggg 2880
ggtattataa ctgatgccac agaaatacaa aaggatcata agggactact acaaattgta 2940
tgacaacaaa ttgagtaacc taggatacct tgataaattc caaaaaatgc acaatatact 3000
gaatcatgaa tacatgaccc ttataaatca agactaaatc ataaagaaat agaaaatatc 3060
aacagaccaa taattagtaa ggagaataaa ctagtaatca gaaacctccc aacaaagaaa 3120
agcttaggac caaatggctt tactggagaa ttctaccaac cattaaaagg ataattaaga 3180
ccaatcttcc tcaaactttt aaaacaaatg ttaaagagga ggaaactctt tcaatctcat 3240
tcataaggtc agcattatcc ttataccaaa accagacaaa gacactatta aaaaaactta 3300

gaccaatatc cctgatgaat ttcgatgcaa gaatcctcag caaaatacta tcaaacaatt 3360
caacagcata cttaaatgat tatatgctgt aatcaagatg catttattct ttgaatgcaa 3420
gtgtaattca acacataaaa ttcaatcaat gtaatacacc acattaacag aatgagagac 3480
aaaaaccaca taattatatc aactgatgca gaaaaaaatc tgacacagtt caacaccttt 3540
tgtgataaaa acactcaaca aactaggaaa agaaggaaac aactttaaca catcatatgc 3600
tcactgatga aaatctacaa gttctttata aaagatcagg aacaagacaa taatctgcat 3660
tgttaccact tctattatac gtagtattgg aagttctaat cagagcaaat taggcaagaa 3720
aaataaataa aaggcatcca aagtggaaag gaagtaaaat aatctctttt tacagatgat 3780
ataaccttag aattagaaaa tcctaaaaat ttcacatacc aagaaaaagc gtgttaaaat 3840
taataagtaa attcagcaag ttgactgata caaaatcaac acagaaagct cagttgtgtg 3900
tctgtgtgtc tcatacacta acaatgaaca atctgaaaag gagattaaga aaacaatttc 3960
atttacaata gcatcaggaa aaaaaataaa tacttaggaa caaacttaac caaggggttg 4020
gaattcctgt atactgaaaa ctacaaatat tgccaaaaga aaataaagga gacacaaata 4080
agtgatatgt ttttaatatg tccacccaaa gtgatcttca gattcaatga aatccctatc 4140
aaagttataa tggcattttt ctgcaggaat gtaaaaattt atcctaaaat tcatatagaa 4200
tctctaggta ccctgagggc caaacaattt tgagaaaaaa aaaagaacaa aattggagga 4260
ctcacacttc cagattacaa gaatatttac aaattacata tttacaaaaa aaattacaaa 4320
gccacaataa tcaaaacaac gtgggatttg cataaaggca gatatataga ccagtggaat 4380
agtattgaga gtccagaaat aaacccttag gtatatcatc aaatgacatt tgacaaagtg 4440
ctggtaccac tcaatgggaa tgggacaatt tgttcaacaa atagagcaaa gaaaactaaa 4500
catccatgtg caaaagaata aatctggacc cttatattac actatagaca aaattaattc 4560
aaaatggatt aaagatctaa atgaaagatc taaaactata aaactcctag gagaaaacag 4620
aggaaaaatt tcatgctaat ttggcaacat tttgtgatgt gacaccaaaa gcagagtcaa 4680
taaaagcaaa aattagacag atggaaatcc atcatagttt ataacttttg gtcattaaag 4740
aacagtcaac agagtgaaaa ggcaatctat aaaatggggg aaaaacagaa aatatgtgca 4800
aatcacagat atctgatagg ggattcatat ccagaataaa taaagaactc ctatatctca 4860
acaacaaaaa atctaatcca atcaaaaaat gggccaaggg agtgaagata catttctcca 4920
aagatgttat acaaatggcc aggaagcata tgaaaagatg ttcaatgtca ctaatcatca 4980
gagaaatgca aatcaaaacc acagtgcaat atcacttcac attcattaga atggcttctg 5040
tcatgaacaa cagaaaataa caagtgttga tgagtgtgta gagaaattga gacctttata 5100
taattttggc agaaattcaa aatggtgcaa ccactataaa aaatgatatg gaggtcctca 5160

aaaaattaaa aatagaacta ccatatgatc cacaatccca cctctgggta catattcaaa 5220

agaattgaaa gcagggtgtt gaagatatat ttgcacactc tttatagcag cactgttcac 5280

aatagccaag agatgaaagt aacccaaagg ttcatgaagc aatgaataaa caaaatatat 5340

tatgtacata gagtaaaata ctgtgcagct ttaaagagaa aggaaatctt atactatgct 5400

acaacatgaa tggaacttta gggcattata gtaagtaaaa taagccagtt ttttttaaag 5460

gacaaataaa cactatacga ttctacttaa gtatttaatg ttgtcaaatt tataaatata 5520

gaatgtagaa tagtggttac cctgagctgg gggaaagggg caaaggggaa ttgttatttt 5580

aatgggtata gtttcagttc tgcaaaatga aaaggttctg gaaatctgtt tcacaatgtt 5640

gtaaatataa ttactctgaa attgtacact taaaaatggt taagatgaca aatagagttg 5700

tgatgtcttc ttttgttatt atatagaaaa actttttcat atgataatag tctttgtttt 5760

taagctgact ttgctgatat taatataatc cttccatttt tctttaaaat gctatatgct 5820

ttcacataat tttgctttac gttgatgtat ttatacataa ggtgggtttc ttatagatac 5880

cacgttgtgt gtcttttta tctaagttga tagacttgcc ttttgttagg gtatttaaat 5940

aatttatatt taatgtaatt attgatatag ttgagtgtgt tgatttttgt tttctatttg 6000

ctccatctgt tgttggttct cattattcct ctgtttctac cttcttttgt actaattatt 6060

atattttatt atttttcatc tcaactgttg gcttattagc cacattgctt ttaaaatttt 6120

taatgattgc tctagggttt ataataaaca aaatgttagc attttctacc atcaaatatt 6180

tttacactat tcatgtatac ttcaatttct ttcttcccat cctttgaact atatcttcat 6240

acattttact ctacatttgt tataactcag tgctttgaaa gtcaattatt tttgtctttg 6300

acagtcaatg atttttaaag agtttaacag tgaaaaaaaa tggctttcat ctttttccat 6360

tagatttcat actccttctg cctgaagaat ttcttttaat agaccttgta ctgcgggtct 6420

caggcaagaa attctctcag cctttgttgg tttgaaaaac tgcttattac acctttgttt 6480

ttgaaagata ttttcactag gtatagaagt ctgggttgac agttctcatt gtttgtcaca 6540

gcatttttaa gatgcccatt caattgtctt gtcttgtata attttggatt agtctggtgt 6600

atttcttacc tttgttcctc tctgtgcaat gcttcaacca tcccacttca ggctgccttt 6660

aagatgtttt cttttccctt aatctttagt ttttagctgg ttgacagtga cgcatctaag 6720

tgtagtgtat gaggttgctt ttattgtcac tgttgttg 6758

<210> 348
<211> 501
<212> DNA
<213> Homo sapiens

<400> 348

```
gaccatgtta tgacatttta gtgcttgcta agcagtaaat actgacttac tttcctgcta       60

cactcttcag agcagaaaga gaaatctaca aaaagggcaa tgtagttggg atccaccaca      120

gccttgagac tgggccatgt ttctacagct tacccacatt ttacccccac tttctctgag      180

aaacaatgca aactggagaa caaggtcaga gaagttatct tggatggtag aagagaagaa      240

aggagaagaa rggataagca gaaaatcaaa aagggcataa aaaaattact ggggaaaata      300

attcttagtc actcaccatt tcttatgttt gtgaaaacag aaacgaggag caagtgttgt      360

tgtaagaatt gttcttgccc ctccccctcc accacccaca tctgtcaagc tatccctgtt      420

tcactgtttc ctctgcactc tctattaact tctttgtcct cctcttttct tttcctacag      480

caaagacttt ttgtcatgtt t                                                 501
```

<210> 349
<211> 501
<212> DNA
<213> Homo sapiens

<400> 349

```
tgacttactt tcctgctaca ctcttcagag cagaaagaga aatctacaaa aagggcaatg       60

tagttgggat ccaccacagc cttgagactg ggccatgttt ctacagctta cccacatttt      120

acccccactt tctctgagaa acaatgcaaa ctggagaaca aggtcagaga agttatcttg      180

gatggtagaa gagaagaaag gagaagaaag gataagcaga aaatcaaaaa gggcataaaa      240

aaattactgg rgaaaataat tcttagtcac tcaccatttc ttatgtttgt gaaaacagaa      300

acgaggagca agtgttgttg taagaattgt tcttgcccct ccccctccac cacccacatc      360

tgtcaagcta tccctgtttc actgtttcct ctgcactctc tattaacttc tttgtcctcc      420

tcttttcttt tcctacagca aagacttttt gtcatgtttt gtttcttttt ctattgtttc      480

tttccctttt ctaatccttg a                                                 501
```

<210> 350
<211> 1148
<212> DNA
<213> Homo sapiens

<400> 350

```
tatgagattt aatgttaaga aataaaatgt aggatctaaa acgtaatcta tagcataatc      60
tcaaaaatgg tttagaaatg acataataat acagacattt gtgggtggta ggattatgca     120
tatttttata tatttttaaa tatatttttc aaaagcttcc tataaagaat gtaattcttt     180
cccaattcca aatctagctt aaacataatt ttacaaaaat tattctctca gaatgtaaac     240
tagtaccacc tctatggaaa acattatgga gatttcctaa agagttaaaa gtagatctac     300
catttgatcc agcaatctta atactgggta tctacccgga ggaaaagaag tcattgtatg     360
aaaaagacac ttgtacacat atgtttacag gaccacaatt cacaaatgca aagatgcaga     420
accaacctaa gtggccastg actaatgaga ggataaagaa gatgtggcat atatatatca     480

gggactacta ctcagccatt acaaggaaca aaataatgtc ttttgcaaca acttggatag     540
agctggaggc cattattcta agtaaagtaa ttcaggaatt ggaaaaccaa aaaccgtatg     600
ttctctctta taagtgggaa ctaagttagg aataagcaaa ggcacacaga gggacatatt     660
ggactttaga gactcacgag gaggagggta ataggggact agggattaaa agaaaaacta     720
gacattaggt acaaggtacc ctacttaagt gcactaaaat ctcagaattc accactacgt     780
aattcaacta agtaacaaga aaccacttgt accccaaaag ctactgaaat aaaaattatt     840
ctctcaaaaa ttttaagccc taaacttcag ttcctattgt ttatatttac taagaaaaac     900
aacagaaaac actgttttaa aaatggtgga tttttttaag gttaaaggta tataagacag     960
ctgcctaagg aaacgcagat acccctgtac cttgttgttg ttgttgtttt tcactttttt    1020
aaaaaacata gagatgggat ctccttatgc tgcccaggct tgtctcaaac tcctgagctc    1080
aagcaatcct ctgacctcag actctcaaag ttttgggact acaggcgaca gtcaccatgc    1140
cagccaat                                                             1148
```

<210> 351
<211> 1148
<212> DNA
<213> Homo sapiens

<400> 351

```
tatgagattt aatgttaaga aataaaatgt aggatctaaa acgtaatcta tagcataatc      60
tcaaaaatgg tttagaaatg acataataat acagacattt gtgggtggta ggattatgca     120
tatttttata tatttttaaa tatatttttc aaaagcttcc tataaagaat gtaattcttt     180
cccaattcca aatctagctt aaacataatt ttacaaaaat tattctctca gaatgtaaac     240
tagtaccacc tctatggaaa acattatgga gatttcctaa agagttaaaa gtagatctac     300
catttgatcc agcaatctta atactgggta tctacccgga ggaaaagaag tcattgtatg     360
aaaaagacac ttgtacacat atgtttacag gaccacaatt cacaaatgca aagatgcaga     420
accaacctaa gtggccactg actaatgaga ggataaagaa gatgtggcat atatayatca     480
gggactacta ctcagccatt acaaggaaca aaataatgtc ttttgcaaca acttggatag     540
agctggaggc cattattcta agtaaagtaa ttcaggaatt ggaaaaccaa aaaccgtatg     600
ttctctctta taagtgggaa ctaagttagg aataagcaaa ggcacacaga gggacatatt     660
ggactttaga gactcacgag gaggagggta ataggggact agggattaaa agaaaaacta     720
gacattaggt acaaggtacc ctacttaagt gcactaaaat ctcagaattc accactacgt     780
aattcaacta agtaacaaga aaccacttgt accccaaaag ctactgaaat aaaaattatt     840
ctctcaaaaa ttttaagccc taaacttcag ttcctattgt ttatatttac taagaaaaac     900
aacagaaaac actgttttaa aaatggtgga tttttttaag gttaaaggta tataagacag     960
ctgcctaagg aaacgcagat acccctgtac cttgttgttg ttgttgtttt tcactttttt    1020
aaaaaacata gagatgggat ctccttatgc tgcccaggct tgtctcaaac tcctgagctc    1080
aagcaatcct ctgacctcag actctcaaag ttttgggact acaggcgaca gtcaccatgc    1140
cagccaat                                                             1148
```

<210> 352
<211> 1148
<212> DNA
<213> Homo sapiens

<400> 352

```
tatgagattt aatgttaaga aataaaatgt aggatctaaa acgtaatcta tagcataatc      60
tcaaaaatgg tttagaaatg acataataat acagacattt gtgggtggta ggattatgca     120
tatttttata tatttttaaa tatatttttc aaaagcttcc tataaagaat gtaattcttt     180
cccaattcca aatctagctt aaacataatt ttacaaaaat tattctctca gaatgtaaac     240
tagtaccacc tctatggaaa acattatgga gatttcctaa agagttaaaa gtagatctac     300
catttgatcc agcaatctta atactgggta tctacccgga ggaaaagaag tcattgtatg     360
aaaaagacac ttgtacacat atgtttacag gaccacaatt cacaaatgca aagatgcaga     420
accaacctaa gtggccactg actaatgaga ggataaagaa gatgtggcat atatayatca     480
gggactacta ctcagccatt acaaggaaca aaataatgtc ttttgcaaca acttggatag     540
agctggaggc cattattcta agtaaagtaa ttcaggaatt ggaaaaccaa aaaccgtatg     600
ttctctctta taagtgggaa ctaagttagg aataagcaaa ggcacacaga gggacatatt     660
ggactttaga gactcacgag gaggagggta ataggggact agggattaaa agaaaaacta     720
gacattaggt acaaggtacc ctacttaagt gcactaaaat ctcagaattc accactacgt     780
aattcaacta agtaacaaga aaccacttgt accccaaaag ctactgaaat aaaaattatt     840
ctctcaaaaa ttttaagccc taaacttcag ttcctattgt ttatatttac taagaaaaac     900
aacagaaaac actgttttaa aaatggtgga tttttttaag gttaaaggta tataagacag     960
ctgcctaagg aaacgcagat accccctgtac cttgttgttg ttgttgtttt tcactttttt    1020
aaaaaacata gagatgggat ctccttatgc tgcccaggct tgtctcaaac tcctgagctc    1080
aagcaatcct ctgacctcag actctcaaag ttttgggact acaggcgaca gtcaccatgc    1140
cagccaat                                                             1148
```

<210> 353
<211> 1148
<212> DNA
<213> Homo sapiens

<400> 353

```
tatgagattt aatgttaaga aataaaatgt aggatctaaa acgtaatcta tagcataatc      60
tcaaaaatgg tttagaaatg acataataat acagacattt gtgggtggta ggattatgca     120
tatttttata tatttttaaa tatatttttc aaaagcttcc tataaagaat gtaattcttt     180
cccaattcca aatctagctt aaacataatt ttacaaaaat tattctctca gaatgtaaac     240
tagtaccacc tctatggaaa acattatgga gatttcctaa agagttaaaa gtagatctac     300
catttgatcc agcaatctta atactgggta tctacccgga ggaaaagaag tcattgtatg     360
aaaaagacac ttgtacacat atgtttacag gaccacaatt cacaaatgca aagatgcaga     420
accaacctaa gtggccactg actaatgaga ggataaagaa gatgtggcat atatatatca     480
gggactactr ctcagccatt acaaggaaca aaataatgtc ttttgcaaca acttggatag     540
agctggaggc cattattcta agtaaagtaa ttcaggaatt ggaaaaccaa aaaccgtatg     600
ttctctctta taagtgggaa ctaagttagg aataagcaaa ggcacacaga gggacatatt     660
ggactttaga gactcacgag gaggagggta ataggggact agggattaaa agaaaaacta     720
gacattaggt acaaggtacc ctacttaagt gcactaaaat ctcagaattc accactacgt     780
aattcaacta agtaacaaga aaccacttgt accccaaaag ctactgaaat aaaaattatt     840
ctctcaaaaa ttttaagccc taaacttcag ttcctattgt ttatatttac taagaaaaac     900
aacagaaaac actgttttaa aaatggtgga ttttttaag gttaaaggta tataagacag      960
ctgcctaagg aaacgcagat acccctgtac cttgttgttg ttgttgtttt tcactttttt    1020
aaaaaacata gagatgggat ctccttatgc tgcccaggct tgtctcaaac tcctgagctc    1080
aagcaatcct ctgacctcag actctcaaag ttttgggact acaggcgaca gtcaccatgc    1140
cagccaat                                                             1148
```

<210> 354
<211> 611
<212> DNA
<213> Homo sapiens

<400> 354

caaaacctca accttccaga taagtctaag ggtgagaact tcacacaaga tgaataagaa 60
ccaatttctt ccagggcgat gttgaacctg gaaatgaaag ccaatctctc ttggaaggcc 120
tggtttgtag aaatgtcagt ctttgtttca agctgtggga gaatgagaag caagacttta 180
gggaaagagg aataaaatag atgtgcagaa ataacagagt gagaaagtct tcagggtgtc 240
gctagcccta attgcaggca tccctgaatc ctagaccttg gattgcaaga gactccttaa 300
tatcttccca tgtccacatt tgcttcacat agtttgaatg tggcttctat tatatacaga 360
tacaagattc aaatccaacc tctaygatga ctggtcttgt gaataagcag aagaggcact 420
aacaatatga cgtgagggat tcagggaaga gcactttctt gagcacatat cttccctggt 480
ctgccagctg tagtttatga aattccacaa tgaggatgaa atggaatcac catttacaga 540
gtactctcca gatgtctaac cctaagctag gtaccttcaa aatattatct agtttagata 600
atcaaccctt t 611

<210> 355
<211> 601
<212> DNA
<213> Homo sapiens

<400> 355

ttctctagtc caaagggttg attatctaaa ctagataata ttttgaaggt acctagctta 60
gggttagaca tctggagagt actctgtaaa tggtgattcc atttcatcct cattgtggaa 120
tttcataaac tacagctggc agaccaggga agatatgtgc tcaagaaagt gctcttccct 180
gaatccctca cgtcatattg ttagtgcctc ttctgcttat tcacaagacc agtcatcata 240
gaggttggat ttgaatcttg tatctgtata taatagaagc cacattcaaa ctatgtgaag 300
yaaatgtgga catgggaaga tattaaggag tctcttgcaa tccaaggtct aggattcagg 360
gatgcctgca attagggcta gcgacaccct gaagactttc tcactctgtt atttctgcac 420
atctatttta ttcctctttc cctaaagtct tgcttctcat tctcccacag cttgaaacaa 480
agactgacat ttctacaaac caggccttcc aagagagatt ggctttcatt tccaggttca 540
acatcgccct ggaagaaatt ggttcttatt catcttgtgt gaagttctca cccttagact 600
t 601

<210> 356
<211> 527
<212> DNA
<213> Homo sapiens

<400> 356

```
gctctagaat atggcattcc agaagtggga tgctacaaat agtctcattg agagtcaact        60
tgcacaatgt atcgtcctac ccttacatca atttctgaaa caacttctct ttgcacttcc       120
cctatagtta catgcataat aaattctgac aactcttatg aagtcatgga ataactttct       180
tcttatgttt cctatcaatg tcattagccc tttatcttgt ttgagtttcc atcagcaatg       240
ttttcaagtc ccaagatcat tcatgtatcc acaagcaatg atacgccaga tttggacaaa       300
taatactgaa tactatctta ttttcactgc catgatcaag gcagtgtgga ttgctgccaa       360
gtccaagaga agtgaggtca gcagctgcaa gccacctccg tcatttagaa aagcttcatg       420
atgtagtgtg tcgtttcgat gtgacactgt ctcacagagt taaaatgatg tgmaaggaac       480
tgttcaatgg aaatttagaa atttctcttt ttctcaattt tagtgta                     527
```

<210> 357
<211> 601
<212> DNA
<213> Homo sapiens

<400> 357

```
gaacaagatt ttcctgcttt taaaaatact acattaaagc tgaaaattta ggccaaaatt        60
ttcaagtggt aatagttaca ggcaattcat ctttctggtc agaaaagggt gttactgcag       120
ctatttctgc ctgaaactgg gtggcactac tacttttttt tttttttttt taactgagca       180
gacattttcc ttacactaaa attgagaaaa agagaaattt ctaaatttcc attgaacagt       240
tccttgcaca tcattttaac tctgtgagac agtgtcacat cgaaacgaca cactacatca       300
ygaagctttt ctaaatgacg gaggtggctt gcagctgctg acctcacttc tcttggactt       360
ggcagcaatc cacactgcct tgatcatggc agtgaaaata agatagtatt cagtattatt       420
tgtccaaatc tggcgtatca ttgcttgtgg atacatgaat gatcttggga cttgaaaaca       480
ttgctgatgg aaactcaaac aagataaagg gctaatgaca ttgataggaa acataagaag       540
aaagttattc catgacttca taagagttgt cagaatttat tatgcatgta actacagggg       600
a                                                                       601
```

<210> 358
<211> 1001
<212> DNA
<213> Homo sapiens

<400> 358

```
gcttaatacc tgagtgatgg aatattctgt tcaacaaacc cctctgacat aggtttgcct      60
atataataaa cctgttcatg tactcctgaa cctaaaagtt taaaaaagat tatgtagaaa     120
acccaaagga atctataaaa agtctactag agctagagtg attttaacaa gatttcaata     180
cacaaattca aatgtctttc tatatattaa tgacaatcaa caataaaatt ttaaaacatt     240
attaaagtat aatgaaaata tcaactgttt agggagaaat gtaacaagaa tggtgaagga     300
cctatacact aaaaagcttc aatatgttgt tgagattaac tgaagaaggt ctaaatagat     360
tttttttca tgtctcggaa gacttaatat gtgaagatac caattcttcc ccaaatgatc      420
aacaggtgaa atgcaatccc aatcaaaatc ccagcaatta ttttaagggg gaaattggca     480
atctgattct aaaattcata yggaaaaaaa caatggagtt agaataacta aaacaagtcc     540
gaaaaagaaa aagaaatgga ggactaatgc tacctgattt caagtcttat cgtataaatc     600
tacatcaata aaggacaagt tggtattggg ttaaagatag ataaatacat cagtggaata     660
gaatattgaa tccagaataa atccacacat atatggataa aaataccaga caattcagtg     720
gagatggttt tgtttttaca acaaatgtta ctggaacaaa ttgatatatg tattagtcag     780
atatggctgc cataacaaag aaccacaaac aggtggttta aataatggaa ataaatttcc     840
tcagaattct ggagtatgga agcccaagat caagttgctg ggaggattcg tttcttctga     900

gtgtctcttt ttttgatgac agatgactat cttttaccaa tgtcttcact tggttttccc     960
tctgtgtgtg cctaggtcct attctccaat tcctataagg a                        1001
```

<210> 359
<211> 1001
<212> DNA
<213> Homo sapiens

<400> 359

ctaaaagttt aaaaaagatt atgtagaaaa cccaaaggaa tctataaaaa gtctactaga 60

gctagagtga ttttaacaag atttcaatac acaaattcaa atgtctttct atatattaat 120

gacaatcaac aataaaattt taaaacatta ttaaagtata atgaaaatat caactgttta 180

gggagaaatg taacaagaat ggtgaaggac ctatacacta aaaagcttca atatgttgtt 240

gagattaact gaagaaggtc taaatagatt tttttttcat gtctcggaag acttaatatg 300

tgaagatacc aattcttccc caaatgatca acaggtgaaa tgcaatccca atcaaaatcc 360

cagcaattat tttaaggggg aaattggcaa tctgattcta aaattcatat ggaaaaaaac 420

aatggagtta gaataactaa aacaagtccg aaaaagaaaa agaaatggag gactaatgct 480

acctgatttc aagtcttatc rtataaatct acatcaataa aggacaagtt ggtattgggt 540

taaagataga taaatacatc agtggaatag aatattgaat ccagaataaa tccacacata 600

tatggataaa aataccagac aattcagtgg agatggtttt gtttttacaa caaatgttac 660

tggaacaaat tgatatatgt attagtcaga tatggctgcc ataacaaaga accacaaaca 720

ggtggtttaa ataatggaaa taaatttcct cagaattctg gagtatggaa gcccaagatc 780

aagttgctgg gaggattcgt ttcttctgag tgtctctttt tttgatgaca gatgactatc 840

ttttaccaat gtcttcactt ggttttccct ctgtgtgtgc ctaggtccta ttctccaatt 900

cctataagga aaccagtcat attggattag ggcccactct aatggcccca ttttacttgc 960

attatctctt taaagacact atctccagat gtagccacac t 1001

<210> 360
<211> 1058
<212> DNA
<213> Homo sapiens

<400> 360

catgattagc tatgctactt tccactgctc ttagtatact gagaggcagc ataagtaaaa 60

ctaaaatatc tgaagatagc aatagactat ttaaagtaga agaagtatgc tatttttgtt 120

ttgttttcat ttcgaaggaa atatgcaaag gtttattgag tatttcagct tctcttacag 180

taggtttttt ttggattctt tctgtgtttg tctatgttga taaaacattg aaatgccata 240

tagctcaaag gtcattcact taagaaatct aagtactgat aacatcttag ccccgattct 300

tcataggcat tgttaagcct attataattt tggtwcagag agaaggtaaa ctatattcca 360

```
gacaggcata taaagcaatt tctcctataa ttggagttca cgaaaaattc acatatttct      420
ttttaatagt aactctcaca gcaagaacat atgtttgtaa ataatacatc acagaatctt      480
attggcagac aaggaaattc ctaaaatatt ttttactgcc acatcaatta agatatataa      540
aataccttat atagaagatg tttgcaccca ggccaaacaa atcaaacaag aatagaagca      600
ctgacagtct tatttcaaaa ttggtttaac ttgtatttac aggatattgt agtaccttat      660
aaagttgatt gctgattggc cgtcttttac agaattctgt cagattgtta ttatttcttg      720
taaagattga ttcaaacaaa taaaaattgt caggattgga tatgtcctat agtgaggtgt      780
agttatgtca catgagattt ttaattacaa agaaatggaa aataaaatga gaatagaatt      840
gagactcccc tgtcacctca caaatatgtt gaaatacaat gaaatttcca aagatgttaa      900
agcatataaa gttgaataat tcttattatg tattaaactt acagaaattt aatttcttta      960
ctttataaga ggtagtgaaa atataaaatt aattatgaag acagagtagt cttagtcaga     1020
catggcccta taaagcatat tcccattcgt tacatcaa                             1058
```

<210> 361
<211> 1058
<212> DNA
<213> Homo sapiens

<400> 361

catgattagc tatgctactt tccactgctc ttagtatact gagaggcagc ataagtaaaa 60
ctaaaatatc tgaagatagc aatagactat ttaaagtaga agaagtatgc tatttttgtt 120
ttgttttcat ttcgaaggaa atatgcaaag gtttattgag tatttcagct tctcttacag 180
taggtttttt ttggattctt tctgtgtttg tctatgttga taaaacattg aaatgccaya 240
tagctcaaag gtcattcact taagaaatct aagtactgat aacatcttag ccccgattct 300
tcataggcat tgttaagcct attataattt tggtacagag agaaggtaaa ctatattcca 360
gacaggcata taaagcaatt tctcctataa ttggagttca cgaaaaattc acatatttct 420
ttttaatagt aactctcaca gcaagaacat atgtttgtaa ataatacatc acagaatctt 480
attggcagac aaggaaattc ctaaaatatt ttttactgcc acatcaatta agatatataa 540
aataccttat atagaagatg tttgcaccca ggccaaacaa atcaaacaag aatagaagca 600
ctgacagtct tatttcaaaa ttggtttaac ttgtatttac aggatattgt agtaccttat 660
aaagttgatt gctgattggc cgtcttttac agaattctgt cagattgtta ttatttcttg 720
taaagattga ttcaaacaaa taaaaattgt caggattgga tatgtcctat agtgaggtgt 780
agttatgtca catgagattt ttaattacaa agaaatggaa aataaaatga gaatagaatt 840
gagactcccc tgtcacctca caaatatgtt gaaatacaat gaaatttcca aagatgttaa 900
agcatataaa gttgaataat tcttattatg tattaaactt acagaaattt aatttcttta 960

ctttataaga ggtagtgaaa atataaaatt aattatgaag acagagtagt cttagtcaga 1020
catggcccta taaagcatat tcccattcgt tacatcaa 1058

<210> 362
<211> 956
<212> DNA
<213> Homo sapiens

<400> 362

```
aaaacaagga acaaacaaac aaaaatgtta caaccgaaca acagactttt gagtcatgtt      60
tcaggccaag aggtgatgag ttactgtagt tgcttgagct ggttggtgaa atattacctg     120
gcaacaaaac tgaaatagaa ggtggcttag taaaatgcag attcagaatg agtgccttaa     180
ggttaaggca tataagacca aactgatttt ctttttcacg aggtcttcag gtaaggccat     240
tgtagaagat accttgtttg cgaacttcag taaattactt cacttgtctc atattttcat     300
tttcaggatg gaggcttgag attgaattgt agtgcaatta ggtaaatttt tacccatttt     360
aaatataata ttaaaatatt aattataaat taccttattt gaatctggaa taatatttat     420
tgcagggcat ataatctaag ctgtaaacgt cctgtyagaa gacaacatat tcatcttgct     480
aaggtataag ctatatgact ggcactgtgc tcaactcaga gtcattgaat gaacagtatt     540
tatttaatct atgaatgaga gcacttcaag tatacagaaa gatatctcaa aagattcagc     600
cttacattgc tcataacttc aatgacttag atgaaaacct cctgaacatt tttatcagtt     660
gtataggtac cccaaatcat aagggaatgt ttatcaatta gatgatgaaa tggggatgca     720
actacatcat ggcaggctaa agcaatagaa tgactttgac aagaggaaat tacatagagg     780
cacctgagtc tcctaaacca atttcaaagg tatgagaggg gggtgatata aataaatagt     840
tgatagatga aaaaactcag aagttatagt tgacagcaat tttaatataa tatgaaaaat     900
gtggttggac ttttagggaa aaaaacctaa taaaatctaa tggaaattag tggtcc         956
```

<210> 363
<211> 956
<212> DNA
<213> Homo sapiens

<400> 363

```
caaccgaaca acagactttt gagtcatgtt tcaggccaag aggtgatgag ttactgtagt      60
tgcttgagct ggttggtgaa atattacctg gcaacaaaac tgaaatagaa ggtggcttag     120
taaaatgcag attcagaatg agtgccttaa ggttaaggca tataagacca aactgatttt     180
ctttttcacg aggtcttcag gtaaggccat tgtagaagat accttgtttg cgaacttcag     240
taaattactt cacttgtctc atattttcat tttcaggatg gaggcttgag attgaattgt     300
agtgcaatta ggtaaatttt tacccatttt aaatataata ttaaaatatt aattataaat     360
```

```
taccttattt gaatctggaa taatatttat tgcagggcat ataatctaag ctgtaaacgt      420
cctgtcagaa gacaacatat tcatcttgct aaggtrtaag ctatatgact ggcactgtgc      480
tcaactcaga gtcattgaat gaacagtatt tatttaatct atgaatgaga gcacttcaag      540
tatacagaaa gatatctcaa aagattcagc cttacattgc tcataacttc aatgacttag      600
atgaaaacct cctgaacatt tttatcagtt gtataggtac cccaaatcat aagggaatgt      660
ttatcaatta gatgatgaaa tggggatgca actacatcat ggcaggctaa agcaatagaa      720
tgactttgac aagaggaaat tacatagagg cacctgagtc tcctaaacca atttcaaagg      780
tatgagaggg gggtgatata aataaatagt tgatagatga aaaaactcag aagttatagt      840
tgacagcaat tttaatataa tatgaaaaat gtggttggac ttttagggaa aaaaacctaa      900
taaaatctaa tggaaattag tggtccactc atttctccac ctaggatgtt aaaaat          956
```

<210> 364
<211> 601
<212> DNA
<213> Homo sapiens

<400> 364

```
gtaaaacaca tagatcgctg tatccttgtt cagtaagcta caacatactc gtatctcctg       60
aaatcctggg cttaaatcga ggtctcaaag gctttgtttt gttttgttgt atggttgtat      120
ggtgagtgtg tgtgtgtgtg tgtgtgtgtg tgtttattct cctgaaattc tcctcctcac      180
ttgacttaag ctaaaagata aacgtcctct tcctttcagc cacagatggt gatggataaa      240
ttgaatgtca ttcacattat tcccttaaaa taaactctct ccctcccctc tcccgtctca      300
wccttgtccc tttctttata taatgggtaa tgcgttaatg tcagcagaat agttttgggg      360
ccataatggc aagtatcacg tggatggttt agcattgttt ttagaatgct gtgaatttgg      420
gtatatgtga gttttgggga aagttttgca actatatgtt tgttaattaa atgaggacta      480
taaagtaata taaaattatg tttctggaac atattttgga agctataaag tcatctgtat      540
ttattatcca cagacataat gtcattgttc aggtcctgca accttcttat aatcaacata      600
c                                                                      601
```

<210> 365
<211> 601
<212> DNA
<213> Homo sapiens

<400> 365

```
agtaagctac aacatactcg tatctcctga aatcctgggc ttaaatcgag gtctcaaagg 60
ctttgttttg ttttgttgta tggttgtatg gtgagtgtgt gtgtgtgtgt gtgtgtgtgt 120
gtttattctc ctgaaattct cctcctcact tgacttaagc taaaagataa acgtcctctt 180
cctttcagcc acagatggtg atggataaat tgaatgtcat tcacattatt cccttaaaat 240
aaactctctc cctcccctct cccgtctcat ccttgtccct ttctttatat aatgggtaat 300
kcgttaatgt cagcagaata gttttggggc cataatggca agtatcacgt ggatggttta 360
gcattgtttt tagaatgctg tgaatttggg tatatgtgag ttttggggaa agttttgcaa 420
ctatatgttt gttaattaaa tgaggactat aaagtaatat aaaattatgt ttctggaaca 480
tattttggaa gctataaagt catctgtatt tattatccac agacataatg tcattgttca 540
ggtcctgcaa ccttcttata atcaacatac gtgggcccag ggattttatg tatcttcgcc 600
t 601
```

<210> 366
<211> 1079
<212> DNA
<213> Homo sapiens

<400> 366

gaatttatgg tctgatggag aagggaatca ttaaagttct atgtagtgag atatccccaa 60
ggggtgtatt aggcttacca ccactggaat ctggatagat gaagacagag tggcagggaa 120
gtcgtattaa ggttctgttt ctgctgggag ccacaggtcc tcaggaagca acaagtactg 180
ggcagattga tactgtagct rggctctagc tctatacctc tagaataaag gttacaaact 240
agcaacttga aagctaaacc tggcccacag atatgtttta tttggctctt acactgtttt 300
aaaaaatatt accaacattt aaaactggga agttttatga aaaaacccag acttctggat 360
tctgttgaaa aaaaaaatca gaagatctgg caatactgag ctgacattcc tatatgacaa 420
caattggctg gatctatgca gcttctctcc aaaaagcaaa gaatgtgttc ttgcttaaca 480
cagtccccac cactccctca tattctccaa tcctggacct gagcgtcatt tgctatgtat 540
cgccatttgc catgaagttt tacactctac agaaatataa tttttttgta gaagactatg 600
ctttaatcaa gatcaggata atataaagtg agatctgaaa gtggaaaaaa gataaatgtc 660
caacaatgat agactggatt aagaaaatgt ggcacatata caccgtggag tactatgcag 720
ccaaaaaaaa cgatgagttc atgtcctttg tagggacatg gatgaagctg gaaaccacca 780
ttctcagcaa actatcgcaa ggacaaaaaa ccaaacgccg catgttctca ctcataggtg 840
ggaattgaac aatgagaaca cttgggcaca ggaaggggaa catcacacac cgggccctgt 900
tgtggggtgg ggggaggagg gagggatagc atttggagat atacctaatg ttaaatgact 960
agtttctggg tgcagcacac catcatggca catgtataca tatgtaacta acctgcacat 1020
tgtgcacatg taccctaaaa cttaaagtat aatttttaaa aaaagatatt ttcttatct 1079

<210> 367
<211> 501
<212> DNA
<213> Homo sapiens

<400> 367

ataaattttc tcttccctca agaatttatg gtctgatgga gaagggaatc attaaagttc 60
tatgtagtga gatatcccca aggggtgtat taggcttacc accactggaa tctggataga 120
tgaagacaga gtggcaggga agtcgtatta aggttctgtt tctgctggga gccacaggtc 180
ctcaggaagc aacaagtact gggcagattg atactgtagc tgggctctag ctctatacct 240
ctagaataaa kgttacaaac tagcaacttg aaagctaaac ctggcccaca gatatgtttt 300
atttggctct tacactgttt taaaaaatat taccaacatt taaaactggg aagttttatg 360
aaaaaaccca gacttctgga ttctgttgaa aaaaaaaatc agaagatctg gcaatactga 420
gctgacattc ctatatgaca acaattggct ggatctatgc agcttctctc caaaaagcaa 480
agaatgtgtt cttgcttaac a 501

<210> 368
<211> 1001
<212> DNA
<213> Homo sapiens

<400> 368

```
tgaagaagcc gcctggcttc ttgtttcttc tcatagcaaa atgcaatgag aaagagataa      60
tttgagaaaa gaaccgttta aacaaaaaga aaccaagaca taatgatttt ggaaattctc     120
agtttattca gactgcaaaa gatattaaaa taaagaaact cagtaacagg gatagataat     180
ctaaagaaaa agcctaggac acggctgtag taaccttctg tttttatacc tcagcaattt     240
gctaatgcct caaaaagatc aaaagtactc aaatataaag ggctctttga agagattaga     300
tttcctcaat caaaccaaag agcatcgagg aagcttaagg ttactgtccc tcacatatct     360
cagcagaagg caaaaataga agactgatta tctaagaaag atctctgaaa gagtctcata     420
ttatggagtg aacccctgtg gcatacatgg gagacccact tggttcttga gaattttata     480
tcaggagaaa cactgtcagt ytgtattgaa aggaacagag aaaatacgaa attaaagaag     540
actattaaac ctccaaaatt ctggcaggaa agaagcttac acagctactc agttgcaaag     600
atctgccact tttcatatac atgaaaggac tcagaggagg aagccacagg tttagaagga     660
aaagctaaaa gcaacatcgt attagtcttg gatctaggaa cctaatttct ctagcagaat     720
ctagaaatgg cttgggacaa gtgattgttt ttttacctag gattttctcc ctcttgaaaa     780
caggactgtc tgtaactatt atcctatgcc tgccctacca tcatatttca gaaacaggta     840
acttatgttt tcactttcaa agattcacaa taaagagaaa ttgtacctca gaatggatta     900
taccagagct ttcctcatgc ataaattaaa taatttaggt tatgtgattt gaagcttttg     960
agtgggtgag gtgacatttt ggatgctgag ttggtgccgt a                        1001
```

<210> 369
<211> 1001
<212> DNA
<213> Homo sapiens

<400> 369

tcttctcata gcaaaatgca atgagaaaga gataatttga gaaaagaacc gtttaaacaa 60
aaagaaacca agacataatg attttggaaa ttctcagttt attcagactg caaaagatat 120
taaaataaag aaactcagta acagggatag ataatctaaa gaaaaagcct aggacacggc 180
tgtagtaacc ttctgttttt atacctcagc aatttgctaa tgcctcaaaa agatcaaaag 240
tactcaaata taaagggctc tttgaagaga ttagatttcc tcaatcaaac caaagagcat 300
cgaggaagct taaggttact gtccctcaca tatctcagca gaaggcaaaa atagaagact 360
gattatctaa gaaagatctc tgaaagagtc tcatattatg gagtgaaccc ctgtggcata 420
catgggagac ccacttggtt cttgagaatt ttatatcagg agaaacactg tcagtctgta 480
ttgaaaggaa cagagaaaat rcgaaattaa agaagactat taaacctcca aaattctggc 540
aggaaagaag cttacacagc tactcagttg caaagatctg ccacttttca tatacatgaa 600
aggactcaga ggaggaagcc acaggtttag aaggaaaagc taaaagcaac atcgtattag 660
tcttggatct aggaacctaa tttctctagc agaatctaga aatggcttgg gacaagtgat 720
tgttttttta cctaggattt tctccctctt gaaaacagga ctgtctgtaa ctattatcct 780
atgcctgccc taccatcata tttcagaaac aggtaactta tgttttcact ttcaaagatt 840
cacaataaag agaaattgta cctcagaatg gattatacca gagctttcct catgcataaa 900
ttaaataatt taggttatgt gatttgaagc ttttgagtgg gtgaggtgac attttggatg 960
ctgagttggt gccgtagtga gtccagaatt ctgcggaact t 1001

<210> 370
<211> 601
<212> DNA
<213> Homo sapiens

<400> 370

ctctagactc ctcctgtatt ttaatttagc cacttttta gggcctacaa ttttagatct 60
ccacagggct cttgaaactt cttgaacctc atcagtaaca tgtccattag tggcatgacc 120
caagagttct agaacatcta ttcagcaagt gtgtatctgg taagtgaata ttccttctat 180
gtgttccctt ttgcatcaaa ctacacactg tcattcctcc tttatctcca aaagcttgaa 240
aattcctcac ttgtatctca ttctttctct cttagaaaac tgatcacctc tgatgaatta 300
raacggaatg accaagcttt gggagaggca aaagaatctc ggtgttaaag actcagagtt 360
taagaagcaa caaaaagatt atacagatgt gaatatgtga ccttcctcca ccagggcatg 420
ttgccttgga gtaagataat ctaagcacac acttcatagc ctgagaacaa ttttggaagt 480
ctttgcttta tggatattta cataaagcaa atatggatat ttacctaaag gctggaccaa 540

ggcctaattc ctctagagcc ccttgatcat gaacaccatt cctgtcatga ttcttaaggt 600

c 601

<210> 371
<211> 601
<212> DNA
<213> Homo sapiens

<400> 371

acaagctcca gccatggacg caattccttc tagaagcaaa atttatctct agactcctcc 60

tgtattttaa tttagccact tttttagggc ctacaatttt agatctccac agggctcttg 120

aaacttcttg aacctcatca gtaacatgtc cattagtggc atgacccaag agttctagaa 180

catctattca gcaagtgtgt atctggtaag tgaatattcc ttctatgtgt tcccttttgc 240

atcaaactac acactgtcat tcctccttta tctccaaaag cttgaaaatt cctcacttgt 300

rtctcattct ttctctctta gaaaactgat cacctctgat gaattagaac ggaatgacca 360

agctttggga gaggcaaaag aatctcggtg ttaaagactc agagtttaag aagcaacaaa 420

aagattatac agatgtgaat atgtgacctt cctccaccag ggcatgttgc cttggagtaa 480

gataatctaa gcacacactt catagcctga gaacaatttt ggaagtcttt gctttatgga 540

tatttacata aagcaaatat ggatatttac ctaaaggctg gaccaaggcc taattcctct 600

a 601

<210> 372
<211> 701
<212> DNA
<213> Homo sapiens

<400> 372

```
gaagatgcac tctaatgttt tttcccagaa gctctgtagg tttagctttt acctttctgg      60
gtttgttttg ttttgttttt tgagatggag tcccactcgt gtcacccagg ctggagtaca     120
atggtgcaat ctcggttcac tgcaacctcc acctcccggg ttcaagcaat tcccctgtct     180
ccacctctcg agtagctggg atgggaggcg cctgccacca tacctggcta attttcatat     240
ttttagtaaa gatagggttt caccatgtta gccaggctgg tctcgaactc ctgacctcaa     300
gtgatccacc cgcctcagct tcccaaagtg ctgggattac aggcgtgagc cactgcgccc     360
agccctagct tttggtcta tgattcctcc caaattaatt tctgtgaacc attaccttaa     420
gatgttgaga tttaatgtcc agaatctcat ttgttcacct ttgaaaatta agaaaccctg     480
gcacagtgtt gactggagcc wcttacctta atagaaaata aagctcacat atatccataa     540
tgaaaagcag agaccagcac aaccatagtc acctgacagt tttaaaatcc aaggccagga     600
tcttctcaac tcaggcccac tcacttactc cacaacatac ttcttctttc ctcagcatct     660
actacttgtg ctgggacctt ggtcttccca ttgttcatgt c                         701
```

<210> 373
<211> 701
<212> DNA
<213> Homo sapiens

<400> 373

```
agatggagtc ccactcgtgt cacccaggct ggagtacaat ggtgcaatct cggttcactg      60
caacctccac ctcccgggtt caagcaattc ccctgtctcc acctctcgag tagctgggat     120
gggaggcgcc tgccaccata cctggctaat tttcatattt ttagtaaaga tagggtttca     180
ccatgttagc caggctggtc tcgaactcct gacctcaagt gatccacccg cctcagcttc     240
ccaaagtgct gggattacag gcgtgagcca ctgcgcccag ccctagcttt ttggtctatg     300
attcctccca aattaatttc tgtgaaccat taccttaaga tgttgagatt taatgtccag     360
aatctcattt gttcaccttt gaaaattaag aaaccctggc acagtgttga ctggagccac     420
ttaccttaat agaaaataaa gctcacatat atccataatg aaaagcagag accagcacaa     480
ccatagtcac ctgacagttt waaaatccaa ggccaggatc ttctcaactc aggcccactc     540
acttactcca caacatactt cttctttcct cagcatctac tacttgtgct gggaccttgg     600
tcttcccatt gttcatgtca ttcttttcct cacagttccc attcttttct ccctgaaata     660
aagaaatttc aaaatatacc atgtttcatg aaaaagacaa a                         701
```

<210> 374
<211> 701
<212> DNA
<213> Homo sapiens

<400> 374

```
gatttccacc ctcaggtgat ggggatggtt gaacatccaa cacctgaaac aggacagacg      60
atattgacag tacttgttag ttgcatataa tcacagacca gtggaaacag atgaaccaca     120
cagggccaca gcggggtttc actggggaac agagtgaaca atcaggaggt gtgggaggca     180
ggtttagtag tttaaagagg ttgaggtgtc cccctggatc ccatgggagg atcacattgg     240
ctcatttgaa ttatcatacg gactggcagg gaactgaaat cttctactca gggataagca     300
gaaactgtcc ctggtttcct tgataaaaag ggttgtttga taggggacct tatccatggg     360
aggaaagtga ggagggaaat ttgtggctaa gccattcaag gccctcccag ttttactaga     420
tgtcaaggca gcacacgtaa tattgggact taattttagc cacataacta ataaatttgt     480
aagtatgtgc aacggctcac rcttgcttcc agaatggcac ctaaaaaaca gatttacctc     540
tccccaaatt cagatatgga attaaatgta atgtcaggaa aattgtctaa gagttggaaa     600
tgggaaaaaa atgttctttt ggtggagtta tggactccag aggttatcag attctattga     660
ataacgtact tttgattgta tttgtaacaa ttaggctatt t                         701
```

<210> 375
<211> 1001
<212> DNA
<213> Homo sapiens

<400> 375

```
gcatataatc acagaccagt ggaaacagat gaaccacaca gggccacagc ggggtttcac        60
tggggaacag agtgaacaat caggaggtgt gggaggcagg tttagtagtt taaagaggtt       120
gaggtgtccc cctggatccc atgggaggat cacattggct catttgaatt atcatacgga       180
ctggcaggga actgaaatct tctactcagg gataagcaga aactgtccct ggtttccttg       240
ataaaaaggg ttgtttgata ggggacctta tccatgggag gaaagtgagg agggaaattt       300
gtggctaagc cattcaaggc cctcccagtt ttactagatg tcaaggcagc acacgtaata       360
ttgggactta attttagcca cataactaat aaatttgtaa gtatgtgcaa cggctcacac       420
ttgcttccag aatggcacct aaaaaacaga tttacctctc cccaaattca gatatggaat       480
taaatgtaat gtcaggaaaa ytgtctaaga gttggaaatg ggaaaaaaat gttcttttgg       540
tggagttatg gactccagag gttatcagat tctattgaat aacgtacttt tgattgtatt       600
tgtaacaatt aggctatttg tgaactcggt aggggtagaa atcgagttgt agaaaatgga       660
tggtaatgca agtgattttt gaccatatca atgcaaatga attctgttgg tagaaatatt       720
catttccaca ctgtagatga ccctaaacat atgtcattac attatatttt attgccttat       780
agactattaa ccaattttga atcatacagt agcaaattta tttcagcatt cttgtgtgta       840
tgtgtttata tatacacgtg catatgtatt taagatatat aattgtatat tcttcaaatt       900
cttctttgaa caggtttgaa cctcttatta gtttcctcat taaggaattt aataagacct       960
ttaatgcatg tttgtatttt catgagagtc attattttac c                          1001
```

<210> 376
<211> 695
<212> DNA
<213> Homo sapiens

<400> 376

```
tgctccttca ttagtgcaat ggaacagcaa atcaggatac tttcacagtt ctcttaagtg        60
agcctagaag tggggagctg cttgttcaca aacttgaagc ctgaatatgt taatattctt       120
tcagtggccg gacgcggtgg ctcatgcctg taatcccaac actttgggag gccgaggtag       180
gcagatcaac ctgaagtcag gagttcgagg ccagcctggc caacatggtg aaaccccacc       240
tgttggtctg tactaaaaat agaaaaatta gctgggcatg gtggcgcatg cctgtaatcc       300
cagctactca ggaggctgtg gcagaagaat cgcctgcacc tgggaggcag aggttgcttt       360
gagttgatat cgtgtcactg cactccagcc tgggcaacag agtgagatcc tttcagaaac       420
```

ctgctgtctg tatttggata caattaaaaa aaaaaaaaag atgagacagg caggtgcgaa 480

agaaataaaa gtcamaactg atccagttgg gaaactcaga attgacagtt acgtgtcctt 540

tcatttattg atattttgag attcacaggg gtttaaactt tattttcca agactgaata 600

gttcccacct cccttccata tataaaattt gagtagctgg ggagatttaa aagaggctcc 660

ccataaactc agaagttaaa agagacaagg gtccc 695

<210> 377
<211> 601
<212> DNA
<213> Homo sapiens

<400> 377

aaccccacct gttggtctgt actaaaaata gaaaaattag ctgggcatgg tggcgcatgc 60

ctgtaatccc agctactcag gaggctgtgg cagaagaatc gcctgcacct gggaggcaga 120

ggttgctttg agttgatatc gtgtcactgc actccagcct gggcaacaga gtgagatcct 180

ttcagaaacc tgctgtctgt atttggatac aattaaaaaa aaaaaaaaga tgagacaggc 240

aggtgcgaaa gaaataaaag tcacaactga tccagttggg aaactcagaa ttgacagtta 300

sgtgtccttt catttattga tattttgaga ttcacagggg tttaaacttt attcttccaa 360

gactgaatag ttcccacctc ccttccatat ataaaatttg agtagctggg gagatttaaa 420

agaggctccc cataaactca gaagttaaaa gagacaaggg tcccagtaaa tacaaaatga 480

ttggggttga ggaggcagat tttctgtcct cagtgaagtt tgttggttgg ttggttggtt 540

ggttggttaa ttggttggtt tttgagtcag ggtctcactt tgtcacccaa gctggagtgc 600

a 601

<210> 378
<211> 663
<212> DNA
<213> Homo sapiens

<400> 378

tgtagcaaca ggagggatga gacccaaagg tctgaaaagc cagtatttta agaagtcttg 60
gaaaatgtgg aggttgaaaa atctaacagg agtgcttgct tcagcagcaa tttagagtag 120
attagcatgg cctctgcgcc aggatgacat gcacattcct aaaagtgttc cgtgttttaa 180
aaaaaagaga gagacagaat ctaaggggat gtgtacattt gctagagcta ctataacaaa 240
gtaccagagg cagggtcact tcaacaacag aaatttattt ctcacagttc tggaggctag 300
acgtccaaga ttaaggtgtt gactgggttg aattcagccc ataacaggaa ataaggagtt 360
aaataaagca cttgcttcta ttgtttgtac ctaaacttaa cagaayacag taagtaacaa 420
gtcattggga tgcagaaaag aaaaaagaga gtgaaggaag gagagaaggt gaagggagaa 480
tggaagagag gaagggaggg aggaaagaaa agtttgatga atgattgcag tctaaactgg 540
ttcaaacaag agatcttgtt taattaagga attcatccca tctctgccta ttaggaggag 600
gaaaaagtct aaaatagaag atggtgaaag ttggatgacc ccaggcatta aggccattca 660
tct 663

<210> 379
<211> 662
<212> DNA
<213> Homo sapiens

<400> 379

ttaagaagtc ttggaaaatg tggaggttga aaaatctaac aggagtgctt gcttcagcag 60
caatttagag tagattagca tggcctctgc gccaggatga catgcacatt cctaaaagtg 120
ttccgtgttt taaaaaaaag agagagacag aatctaaggg gatgtgtaca tttgctagag 180
ctactataac aaagtaccag aggcagggtc acttcaacaa cagaaattta tttctcacag 240
ttctggaggc tagacgtcca agattaaggt gttgactggg ttgaattcag cccataacag 300
gaaataagga gttaaataaa gcacttgctt ctattgtttg tacctaaact taacagaaca 360
cagtaagtaa caagtcattg ggatgcagaa aagaaaaaag agagtgaagg aaggagaraa 420
ggtgaaggga gaatggaaga gaggaaggga gggaggaaag aaaagtttga tgaatgattg 480
cagtctaaac tggttcaaac aagagatctt gtttaattaa ggaattcatc ccatctctgc 540
ctattaggag gaggaaaaag tctaaaatag aagatggtga aagttggatg accccaggca 600
ttaaggccat tcatctttaa ctgttatgct tggatcatgc aaatgtgtct ggtagctaca 660
ag 662

<210> 380
<211> 615
<212> DNA
<213> Homo sapiens

<400> 380

```
ttccatacat tccttccaca ccattgccct taacctttca aattcctgct taaaactaat        60
cccattttta tggctgacct caccctgtat caaaaactcc gacatccctt tacgacagag       120
agcacaaact agtggtccaa aatgtcatgg gggtcttctc agagttgttt tttcaatcag       180
gaaatttcac ataaaaatat ggatttctga tttctctttt aaaaacagaa aaacgagcca       240
ccagtgggag cactgcaggt atctgtgtga gaccygtact tcacaactcc tgctttccct       300
ccataaagta gcttgcattt tccacattga ctttgcagtt ctttggtatc tgtattggtt       360
ttaagataat ttctactata tcacatatct cctcacagta caaagatatc attttctttc       420
ccttttcttt ttaaaaaatt tgtattttta atttttgtgg gtacacagta gatatttatg       480
gggcatatga ggtattttat aggcatataa tatgtactag ggtaagtggg gtattcatca       540

cctcaagcat ttatcctttc tttgtgtaaa atatagcatt ttctgaacac tatgaatact       600
taagtacaag gatca                                                        615
```

<210> 381
<211> 994
<212> DNA
<213> Homo sapiens

<400> 381

tcaaagtgta acaaatttcc tttcctcata aactagcaga cattctatcc cctcattatt 60

gtaacacatt tctaatatct ttctcaaatt gtcttcctgt attacaatgc actcaccttg 120

gcttagaatg tctgagacaa gaaaatctat tcaccattcc cacagatgac tccctcactc 180

tcctcccaag tcttccatac attccttcca caccattgcc cttaaccttt caaattcctg 240

cttaaaacta atcccatttt tatggctgac ctcaccctgt atcaaaaact ccgacatccc 300

tttacgacag agagcacaaa ctagtggtcc aaaatgtcat gggggtcttc tcagagttgt 360

tttttcaatc aggaaatttc acataaaaat atggatttct gatttctctt ttaaaaacag 420

aaaaacgagc caccagtggg agcactgcag gtatctgtgt gagacctgta cttcacaact 480

cctgctttcc ctccataaag yagcttgcat tttccacatt gactttgcag ttctttggta 540

tctgtattgg ttttaagata atttctacta tatcacatat ctcctcacag tacaaagata 600

tcattttctt tccctttttct ttttaaaaaa tttgtatttt taatttttgt gggtacacag 660

tagatattta tggggcatat gaggtatttt ataggcatat aatatgtact agggtaagtg 720

gggtattcat cacctcaagc atttatcctt tctttgtgta aaatatagca ttttctgaac 780

actatgaata cttaagtaca aggatcaagt cataggattt ggaattgatt tttaaaatat 840

gttgaccaaa gtgctcttat catcaaactt aacatcacta atgaaggatg aacatcccaa 900

atctgaaaat ccaaaatcca aaatgctcca taatctaaaa cttgttgagc accaacatga 960

tgcttaaagg aaatgctcct ggagcatttc agat 994

<210> 382
<211> 1001
<212> DNA
<213> Homo sapiens

<400> 382

ctatgagaaa tatttttaaa gtggttagga acaattcata gcactgacat gttatcagta 60

aaaatagaag aaaataaatt aatattatga aatattaatt atatttcatt aattatgtaa 120

tatgaattat gttttagctc aaatatttcc caagggacaa ttaagtaaat gaaaaataca 180

cacagattaa aataataaat agagaaggag atattaatga ggtacaaaaa gaaaaaatac 240

atgtaatcac atgaaatgct attatttgaa agattaacaa aacttgtaaa ctacctgcta 300

acttgatcaa agaaaaaaat cgagaaacca tatgcgcaat taatagtaag agggaaataa 360

```
acattgaaac agaagacatt tgaaatacca tataagactg ggtttcagag ctctatgtac      420
gtaaattgat aatgtcctgg agaagtgcag atgaccaaaa tggacacctt tcaacttaga      480
aatcataaac agattcattt ycttaaagtt aatgaaaaga attaacagac cctcctcaaa      540
aaagacatat atgcggccta caatcatatg aaaaaaagtt caacattact gttcattaga      600
gaaatgcaaa tcaaaaccac aatgagatac catctcacac cagtcagaat ggctattatt      660
aagaagtcaa aaaataaaag atgctggcga ggttgtggag aaaaaagaat gcttttatac      720
acttggtggg aatgtaaatt agttcagtca ttgtggaaga ctttgatgat tcctagaaga      780
cctaaataca gaactactat ttgacccaac aatcccatta ctgggtatat actcaaatga      840
ctataaatca ttctattata aagacacatg catggatatg ttcattacag cactatgcac      900
aatagcaaag acttggaatc aacatgaatg tccatcaatg atagactaga taaagaaaat      960
gtggtacaca tataccatgg aatactatgc agccataaaa a                          1001
```

<210> 383
<211> 1001
<212> DNA
<213> Homo sapiens

<400> 383

```
tcagtaaaaa tagaagaaaa taaattaata ttatgaaata ttaattatat ttcattaatt      60
atgtaatatg aattatgttt tagctcaaat atttcccaag ggacaattaa gtaaatgaaa     120
aatacacaca gattaaaata ataaatagag aaggagatat taatgaggta caaaaagaaa     180
aaatacatgt aatcacatga aatgctatta tttgaaagat taacaaaact tgtaaactac     240
ctgctaactt gatcaaagaa aaaaatcgag aaaccatatg cgcaattaat agtaagaggg     300
aaataaacat tgaaacagaa gacatttgaa ataccatata agactgggtt tcagagctct     360
atgtacgtaa attgataatg tcctggagaa gtgcagatga ccaaaatgga cacctttcaa     420
cttagaaatc ataaacagat tcatttcctt aaagttaatg aaaagaatta acagaccctc     480
ctcaaaaaag acatatatgc rgcctacaat catatgaaaa aaagttcaac attactgttc     540
attagagaaa tgcaaatcaa aaccacaatg agataccatc tcacaccagt cagaatggct     600
attattaaga agtcaaaaaa taaaagatgc tggcgaggtt gtggagaaaa aagaatgctt     660
ttatacactt ggtgggaatg taaattagtt cagtcattgt ggaagacttt gatgattcct     720
agaagaccta aatacagaac tactatttga cccaacaatc ccattactgg gtatatactc     780
aaatgactat aaatcattct attataaaga cacatgcatg gatatgttca ttacagcact     840
atgcacaata gcaaagactt ggaatcaaca tgaatgtcca tcaatgatag actagataaa     900
gaaaatgtgg tacacatata ccatggaata ctatgcagcc ataaaaatga aggagatcat     960
gccctttgca gggacacgaa tagaggtgga ggccattatc c                        1001
```

<210> 384
<211> 501
<212> DNA
<213> Homo sapiens

<400> 384

```
agttgcttga aagcaaagtt ctcgcagtag ctctctatct agaaggaggc attttattta      60
tgtaaggaag tcacctaaaa gaaaattcat ttgttatggt gtggctttaa gagttactta     120
cttttaatgg aatcccccag ataataataa attctgaaaa aaaaaaatca gaatcatggc     180
atgttaaaac tggatacatt cctagaaata gatggaaact gctcttgcaa aaagcttagc     240
acatgttaaa rcattttaga aacaatttgc caaagtttat ttagtctagt gatttcgaca     300
ggttaaatgg accctttgag atcttttttc ctcaagtaca aaggctcact tgcttaatga     360
acacagtccc agaaaagcag ggggctgaac cttggctcta ccatcttacc taagattcta     420
gagttagcaa agggtttcca caagcccaaa ttattatgtt taatcttttc aattatctgt     480
gaagcattag gttggtgcaa a                                               501
```

<210> 385
<211> 501
<212> DNA
<213> Homo sapiens

<400> 385

```
gaggcatttt atttatgtaa ggaagtcacc taaaagaaaa ttcatttgtt atggtgtggc      60
tttaagagtt acttactttt aatggaatcc cccagataat aataaattct gaaaaaaaaa     120
aatcagaatc atggcatgtt aaaactggat acattcctag aaatagatgg aaactgctct     180
tgcaaaaagc ttagcacatg ttaaagcatt ttagaaacaa tttgccaaag tttatttagt     240
ctagtgattt ygacaggtta aatggaccct ttgagatctt ttttcctcaa gtacaaaggc     300
tcacttgctt aatgaacaca gtcccagaaa agcagggggc tgaaccttgg ctctaccatc     360
ttacctaaga ttctagagtt agcaaagggt ttccacaagc ccaaattatt atgtttaatc     420
ttttcaatta tctgtgaagc attaggttgg tgcaaaagta actgcaggtt ttgacattaa     480
aactggcaaa aactgcaata a                                               501
```

<210> 386
<211> 703
<212> DNA
<213> Homo sapiens

<400> 386

```
gacaccagtt agcatattgt cgcgggggag aggggtggga aaggcgagag aacagcatgt      60
ggtccagagg ccatacccag atggaggctg cagtcagctc cccagtcaaa ggcaaagccc     120
aagtcaaagc catgcttccc tcttgcccac ctgctccaat gccacccaca gagagtgcgc     180
cacagctcac aggatgcagg tctggttgaa tcttaacaat aactttgtaa gggaggtgtc     240
attagctcca ttctcctggc aggaggatga ggctcaaggc agctaaaggc ttttgctgaa     300
catcaagtgg tgagccagga ctcaawgcca gatcttcttg tttccctgtt aggtgtatgt     360
agcacaactg gtatctgcag actatgctgc tggaagggct agccgtcact gttatcacag     420
cgactgctgc ctgagatatg ccaggtactg ctgcaagaag tttacaaata taagctcact     480
tgatcttcat aacatactac ctaggtacaa tcattatatt tatttgacag atacagagac     540
agaggggaca cagaaaggat tagtaacttg ccccaaacca cacagccagc aaggtgtaag     600
tgagcacctg cagtctagat gagacaccac tcaaaacgtc atttttctgg cagccccgtg     660
cagttaccac agtggtcacc ccagtggtca gctaaaggcc aag                       703
```

<210> 387
<211> 704

<212> DNA
<213> Homo sapiens

<400> 387

```
gcatattgtc gcgggggaga ggggtgggaa aggcgagaga acagcatgtg gtccagaggc      60
catacccaga tggaggctgc agtcagctcc ccagtcaaag gcaaagccca agtcaaagcc     120
atgcttccct cttgcccacc tgctccaatg ccacccacag agagtgcgcc acagctcaca     180
ggatgcaggt ctggttgaat cttaacaata actttgtaag ggaggtgtca ttagctccat     240
tctcctggca ggaggatgag gctcaaggca gctaaaggct tttgctgaac atcaagtggt     300
gagccaggac tcaatgccag atcttcttgt ttccctgtta ggtgtwtgta gcacaactgg     360
tatctgcaga ctatgctgct ggaagggcta gccgtcactg ttatcacagc gactgctgcc     420
tgagatatgc caggtactgc tgcaagaagt ttacaaatat aagctcactt gatcttcata     480
acatactacc taggtacaat cattatattt atttgacaga tacagagaca gaggggacac     540
agaaaggatt agtaacttgc cccaaaccac acagccagca aggtgtaagt gagcacctgc     600
agtctagatg agacaccact caaaacgtca tttttctggc agccccgtgc agttaccaca     660
gtggtcaccc cagtggtcag ctaaaggcca agcccaccgt ttct                      704
```

<210> 388
<211> 975
<212> DNA
<213> Homo sapiens

<400> 388

```
gacttaagac aaggggtct taatttgatt attttttct gttttatatg atttctatga      60
aaactacaac aaaataaagt taattctatt taagtgactt tttaatgaat tgcctttgtt     120
agaaaaaaaa ttaagtgttt ttgtctcact ctgtcaccca ggctggagca cagtggtgtg     180
```

```
atcatggctt actgcagcca tgacctcccg ggctcaggtg atcctcccac ctcagcttcc      240
caaatagatg ggactacagt tgtgtgccac aacgcctggc taatttttgt atttttttgt      300
agagacaggg tctcaccagg ttgcccaggc tgatcttgaa ctccttggct caagcgatcc      360
acccacctca gcctccctga gtgctgggat tacaggcatg agccagcgca cccagccaga      420
attacatttt tttaaatggt actgtcctag aaaatccagg atgtgcagtg atcaygtatg      480
aatgcatgga cctgcacaca caggagtgaa caaaagaccc acccctgcca ggtcaccact      540
catatctcac cccagcccac gctagctcac actcctcccc acacaccact gacctcatca      600
ttgctaggta cccacttgac ttctcaacag gttcaagaca attggccttc ctcgtctctt      660
ctagaaacac cctcttttct gggctttgtg taacacctgg tctttctccc ctctctggcc      720
acttctcagc ttttcttttt ctttctttct tttttttttt tttttttttg ccacttcctc      780
ttcctctaca tcaagcttgt ccaacccaca gcccaggaca gctttgaatg cagcctaaca      840
caaattcgta agctttctta aaacattatg agatgtgtgt gtgtgtgtgt gtgtgtgtgt      900
gtgtgtgtgt gtgtgtgtgt gtttagctca tcagctatcg ttattgttag tgtattttat      960
gtgtggccca agaca                                                       975
```

<210> 389
<211> 976
<212> DNA
<213> Homo sapiens

<400> 389

```
gactttttaa tgaattgcct ttgttagaaa aaaaattaag tgtttttgtc tcactctgtc        60
acccaggctg gagcacagtg gtgtgatcat ggcttactgc agccatgacc tcccgggctc       120
aggtgatcct cccacctcag cttcccaaat agatgggact acagttgtgt gccacaacgc       180
ctggctaatt tttgtatttt tttgtagaga cagggtctca ccaggttgcc caggctgatc       240
ttgaactcct tggctcaagc gatccaccca cctcagcctc cctgagtgct gggattacag       300
gcatgagcca gcgcacccag ccagaattac atttttttaa atggtactgt cctagaaaat       360
ccaggatgtg cagtgatcac gtatgaatgc atggacctgc acacacagga gtgaacaaaa       420
gacccacccc tgccaggtca ccactcatat ctcaccccag cccacgctag ctcacrctcc       480
tccccacaca ccactgacct catcattgct aggtacccac ttgacttctc aacaggttca       540
agacaattgg ccttcctcgt ctcttctaga aacaccctct tttctgggct ttgtgtaaca       600
cctggtcttt ctcccctctc tggccacttc tcagcttttc tttttctttc tttctttttt       660
tttttttttt ttttgccact tcctcttcct ctacatcaag cttgtccaac ccacagccca       720
ggacagcttt gaatgcagcc taacacaaat tcgtaagctt tcttaaaaca ttatgagatg       780
tgtgtgtgtg tgtgtgtgtg tgtgtgtgtg tgtgtgtgtg tgtgtgttta gctcatcagc       840
tatcgttatt gttagtgtat tttatgtgtg gcccaagaca tttcttcttc cagtgtggcc       900
cagggaagcc aaaagattgg acacccctgc tctacaacat ctcaatatag gccTTTTtca       960
tgtttcattc tagatt                                                       976
```

<210> 390
<211> 801
<212> DNA
<213> Homo sapiens

<400> 390

```
atccagacgg tgcccatact ccctgctctg tctagatggt gtccacattc cctgctccgt      60
ctagactgtg cccatattcg ctgctggctg caaatgcgag gagttgacag cagcctcccc     120
tttacaaggc aggaggtgcc actgttcgcc attgtctcca cctagggctt cacttgcttt     180
ctatctgcag acatcagagg gacccacatc tctctgttct gacacgctgt gtgttgatgg     240
cagagtttaa ttatccacat gcaatcttac tttccttatt cccaagtccg tggggctgcc     300
tcatcaaagc attgtaagaa ctgataacca tcttctagaa gtatcatagt gatattaaga     360
acacacatca cagatcatag taaatggctt taatttttta rcgaaatctc actactgcaa     420
atgcattgtt gtcctagcta atgaatgcat agagtattgc ctgcaaaata ataattgaga     480
ttctattttt aagaagctta gaacagtaca tggtgcatag caaagactct gtgtatgtga     540
agccagattt taaaatatgg taacaagtgt ctgaaaatat gtggctcaat ttgtctcccg     600
gttacttttc cctctccccc tttaaaatgt agaggaagga gaagaagaga taagaggttt     660
gtgagtgaag acaagggccc tttaaggcct gggaagacta acgccatagg gatctccctc     720
tgccttaaaa ggcacaggaa tcttagtggg gaaaaagaag tggtgataaa tagccagtcc     780
gtgtgcctgg aatatcaaag t                                               801
```

<210> 391
<211> 801
<212> DNA
<213> Homo sapiens

<400> 391

```
ccctgctccg tctagactgt gcccatattc gctgctggct gcaaatgcga ggagttgaca      60
gcagcctccc ctttacaagg caggaggtgc cactgttcgc cattgtctcc acctagggct     120
tcacttgctt tctatctgca gacatcagag ggacccacat ctctctgttc tgacacgctg     180
tgtgttgatg gcagagttta attatccaca tgcaatctta ctttccttat tcccaagtcc     240
gtggggctgc ctcatcaaag cattgtaaga actgataacc atcttctaga agtatcatag     300
tgatattaag aacacacatc acagatcata gtaaatggct ttaatttttt agcgaaatct     360
cactactgca aatgcattgt tgtcctagct aatgaatgca yagagtattg cctgcaaaat     420
aataattgag attctatttt taagaagctt agaacagtac atggtgcata gcaaagactc     480
```

```
tgtgtatgtg aagccagatt ttaaaatatg gtaacaagtg tctgaaaata tgtggctcaa      540
tttgtctccc ggttactttt ccctctcccc ctttaaaatg tagaggaagg agaagaagag      600
ataagaggtt tgtgagtgaa gacaagggcc ctttaaggcc tgggaagact aacgccatag      660
ggatctccct ctgccttaaa aggcacagga atcttagtgg ggaaaaagaa gtggtgataa      720
atagccagtc cgtgtgcctg gaatatcaaa gtcagtgcgt gccagggatc acactgcggg      780
tcacgtgcac tctgggtctc t                                                801
```

<210> 392
<211> 601
<212> DNA
<213> Homo sapiens

<400> 392

```
ttggcctggg gctgattcct ccaaagcaat gtgtctcttc gcagagtctc ttagagctgc       60
aaggcagtat gggatcatca gagaggatgc taggaagctt cagaaatgga ggtcctggta      120
gaaagggtcc tttggcgtgg cctctgaaga gtccaaatgt gggacaagac cctccgaaag      180
cggtggcctg gggagccaca ggtggggcag ccagcacgga agagggtggc tttgctacca      240
ttgggaaaac ttatcctcca catcctcatg aggcaaacac ctttcctacc ttaccgctcc      300
ycagtggcct ccctgttgcc ttcttattca agactaagac cctctagaat gttctttatc      360
ctgagtccag ctgattgtct atactaatat cagtacgggg tgtagatgag gacaaccagt      420
gtgcctggct gccaggcacc ccctccccaa accccaggag tttctggaac attccaactc      480
tgcttgaggg tatccatgca gcatctacta ctgtgagcag gtggtctgat ctgtggaaaa      540
cttctatgat tcacctgagg gtaactgccc tttgtgattt gaaagaatga tgctaacaga      600
a                                                                      601
```

<210> 393
<211> 601
<212> DNA
<213> Homo sapiens

<400> 393

```
gcagagtctc ttagagctgc aaggcagtat gggatcatca gagaggatgc taggaagctt        60
cagaaatgga ggtcctggta gaaagggtcc tttggcgtgg cctctgaaga gtccaaatgt       120
gggacaagac cctccgaaag cggtggcctg gggagccaca ggtggggcag ccagcacgga       180
agagggtggc tttgctacca ttgggaaaac ttatcctcca catcctcatg aggcaaacac       240
ctttcctacc ttaccgctcc tcagtggcct ccctgttgcc ttcttattca agactaagac       300
yctctagaat gttctttatc ctgagtccag ctgattgtct atactaatat cagtacgggg       360
tgtagatgag gacaaccagt gtgcctggct gccaggcacc ccctccccaa accccaggag       420
tttctggaac attccaactc tgcttgaggg tatccatgca gcatctacta ctgtgagcag       480
gtggtctgat ctgtggaaaa cttctatgat tcacctgagg gtaactgccc tttgtgattt       540
gaaagaatga tgctaacaga aagtgttgtc atttctgaac ttttctgaac tctgcagcga       600
g                                                                       601
```

<210> 394
<211> 1001
<212> DNA
<213> Homo sapiens

<400> 394

```
agatttggat ggggacacaa aaccaaacca tatcataggt taaattgtgt ctcccacccc        60
aaaaatgtgt atgttgaagt cctaaccttc agtactcaga atgtgacatt atttggaaat       120
agggtcattg cagatggagt tagttaagat gaggtcatta ggatgagtcc ctaatccaat       180
atgactggtg ctcttacaaa aaggggaagt ttggacacag agccatgcac atgggtggga       240
agaatcccaa atgaacggat aggcagaggg ttggagagat gcatcaacaa ggaacaccaa       300
agattgccag caacccccag aagctggggg agaggcctgg aacagattct ccctcacagc       360
ctgagaggaa ccaagctggc tgacaccttg atctcaggtt accggccttg agaactgaga       420
gaccctgggt ttctgttgtt taagcctctc agggtgcagc actttattat ggaagcctga       480
gctgactaat acaggtgtct ytatatctca ctgagggaaa gtgacaggaa agtaagaacc       540
atttatgtcc aagagtccag aggagtcaac cagattctgg gggaaaagaa ggtacaatgc       600
tggcctctcc atgcagccta gtccccaaca cttgtagggc ccagggcaag atctaaagca       660
ctctctcacc tatgcatcta tatgctgtaa ctcagataaa caaactatta aataatatat       720
gtgtcttgcc tctcaatctg acaattacac ctttataata gcaacatagg aaaataacta       780
aaactatggt ttttaggcaa ccaaatacca gcaaaatgta ataattccta ttattagata       840
tgtttaagtg ttctgctggt gggtcagcat ctttggtaga gtcataaaat taaaatgtac       900
ataattaatt aaatattata tgtttattcc ctaacattta tttctgtcat ttcttttttc       960
tttttttcag acagtctcac tcttttgccc aggccggagt g                          1001
```

<210> 395
<211> 1001
<212> DNA
<213> Homo sapiens

<400> 395

```
ttgtgtctcc caccccaaaa atgtgtatgt tgaagtccta accttcagta ctcagaatgt        60
gacattattt ggaaataggg tcattgcaga tggagttagt taagatgagg tcattaggat       120
gagtccctaa tccaatatga ctggtgctct tacaaaaagg ggaagtttgg acacagagcc       180
atgcacatgg gtgggaagaa tcccaaatga acggataggc agagggttgg agagatgcat       240
```

```
caacaaggaa caccaaagat tgccagcaac ccccagaagc tgggggagag gcctggaaca      300
gattctccct cacagcctga gaggaaccaa gctggctgac accttgatct caggttaccg      360
gccttgagaa ctgagagacc ctgggtttct gttgtttaag cctctcaggg tgcagcactt      420
tattatggaa gcctgagctg actaatacag gtgtctctat atctcactga gggaaagtga      480
caggaaagta agaaccattt rtgtccaaga gtccagagga gtcaaccaga ttctgggga       540
aaagaaggta caatgctggc ctctccatgc agcctagtcc ccaacacttg tagggcccag      600
ggcaagatct aaagcactct ctcacctatg catctatatg ctgtaactca gataaacaaa      660
ctattaaata atatatgtgt cttgcctctc aatctgacaa ttacaccttt ataatagcaa      720
cataggaaaa taactaaaac tatggttttt aggcaaccaa ataccagcaa aatgtaataa      780
ttcctattat tagatatgtt taagtgttct gctggtgggt cagcatcttt ggtagagtca      840
taaaattaaa atgtacataa ttaattaaat attatatgtt tattccctaa catttatttc      900
tgtcatttct tttttctttt tttcagacag tctcactctt ttgcccaggc cggagtgcag      960
tggcgtgatc tcagctcact gcaacctccg cctcccaggt t                          1001
```

<210> 396
<211> 1218
<212> DNA
<213> Homo sapiens

<400> 396

gataaagaaa ggtcatcctc aatttcaatt tactttatat attctttgag aggtaaccgt 60
gtcttatctc cccccaaaat tccttttaaa aggaaatttc caaagatgct ctattctgtg 120
aataaagcat tgtgccacag ccgagaggat ccagcaatga acatgagatt gcccttgatt 180
cataaggtct acaagctagt aaggatagag aacactttaa aataaaaaaa aatagttttt 240
ggtatattta tattgtgtat ttggtataat tgagttttct acattctcat atatgtattt 300
catattttga agaatatgca gaaaataatc aagcttccaa ataaacattt tttttaaga 360
actgcacaag tgagaattta ggagaacaga agatcagagg gctgcacrgg ctaaactaga 420
caatgagccc atgcaagtaa gttaagagga gaagcgggta agtatgcacc tgctttgtct 480
aggtgaccag caagcattta gcaatagtct tttcaaaaca acagctcctt atattgtcaa 540
atctcaagaa gtaatattta tggttaaaaa aatctcagac ccaacagaaa atccatgagg 600
gagatggttt tggaaacgca gaattttcag ctatgatatc cttttataaa caagcagata 660
ctttccccaa atataattca atgcctcagt ctacctcctg ctgaaaccac taacaccacc 720
actaaagctc gactatatgg gaaaatttag gtgtcacttt caaaatatgt cctagcataa 780
aggcaattaa aaaatgtaaa gcaccaaaga tgcaagagag acataaatga ataaaatatc 840
tggcacgaaa gttttcaaaa gcttgggaat ctgattcaaa aaaaaataaa atcagccaag 900

cagtgttagt aagttagcca atcaggtttc aagaaggcag aaagacaaaa tcaacatcac 960
cagcatttga caccgctact gggggaaaaa agggggatgg agttcgttta tggccttttt 1020
aaaaatgcca ttacttggac aagagtcata acagagaagc actgcttatt tcagttctgt 1080
taactgtaaa tatcagagcc aacacccaga aaaagttcac cattagccaa ttggttttgc 1140
ctggccaatt ggagatggta ataggcctgc tatggatgac attctttctg atataagttg 1200
tttcttgctt tttctccc 1218

<210> 397
<211> 1218
<212> DNA
<213> Homo sapiens

<400> 397

```
gataaagaaa ggtcatcctc aatttcaatt tactttatat attctttgag aggtaaccgt        60
gtcttatctc cccccaaaat tccttttaaa aggaaatttc caaagatgct ctattctgtg       120
aataaagcat tgtgccacag ccgagaggat ccagcaatga acatgagatt gcccttgatt       180
cataaggtct acaagctagt aaggatagag aacactttaa aataaaaaaa aatagttttt       240
ggtatattta tattgtgtat ttggtataat tgagttttct acattctcat atatgtattt       300
catattttga agaatatgca gaaaataatc aagcttccaa ataaacattt ttttttaaga       360
actgcacaag tgagaattta ggagaacaga agatcagagg gctgcacggg ctaaactaga       420
caatgagccc atgcaagtaa gttaagagga gaagcgggta agtatgcacc tgctttgtct       480
aggwgaccag caagcattta gcaatagtct tttcaaaaca acagctcctt atattgtcaa       540
atctcaagaa gtaatattta tggttaaaaa aatctcagac ccaacagaaa atccatgagg       600
gagatggttt tggaaacgca gaattttcag ctatgatatc cttttataaa caagcagata       660
ctttccccaa atataattca atgcctcagt ctacctcctg ctgaaaccac taacaccacc       720
actaaagctc gactatatgg gaaaatttag gtgtcacttt caaaatatgt cctagcataa       780
aggcaattaa aaaatgtaaa gcaccaaaga tgcaagagag acataaatga ataaaatatc       840
tggcacgaaa gttttcaaaa gcttgggaat ctgattcaaa aaaaaataaa atcagccaag       900
cagtgttagt aagttagcca atcaggtttc aagaaggcag aaagacaaaa tcaacatcac       960
cagcatttga caccgctact gggggaaaaa agggggatgg agttcgttta tggccttttt      1020
aaaaatgcca ttacttggac aagagtcata acagagaagc actgcttatt tcagttctgt      1080
taactgtaaa tatcagagcc aacacccaga aaaagttcac cattagccaa ttggttttgc      1140
ctggccaatt ggagatggta ataggcctgc tatggatgac attctttctg atataagttg      1200
tttcttgctt tttctccc                                                     1218
```

<210> 398
<211> 1072
<212> DNA
<213> Homo sapiens

<400> 398

```
cacttaaaag ctctggaaac ctacgagatt atctttaaaa tcgtggggac caaatggctg      60
gccaaggact tgtttctgta caggtgcgat tgcttctctg ctgtgttcct ttttattacc     120
caagtaaccg gtatttcagc tcacaagatg agaaaatgac aaacaggcaa aataagcgta     180
gggctgtgtg tgcaacagtt watcataaag ccatcaccag gagacgtcac tgggcgcctt     240
ctggagtcta tccgtcctaa ctttgctttc tttctttttt tttttaaatt taagttctag     300
ggtacatatg cacaacgtgc aggtttgtca cacatgtata catgtgccat gttggtgtgc     360
tgcacccatt aactcgtcat ttacattagg tgtatctcct agtgctatcc ctccccactc     420
ccccgacccc atgacaggcc ccagtgtgtg atgttcccct tcctgtgtcc aagtgttctc     480
attgttcaat ccccacctat gagtgagaac atgccatgtt tggttttttg tccttgcgat     540
agtttgctga gaatgatggt ttccagcttc atccatgtcc ctacaaagga catgaactca     600
tcctttttta tggctacata gtattccatg gtgtatatgt gccacatttt cttaatccag     660
tctatcatcg atggacattt gggttggttc caagtctttg ctattgtgac tagtgttgca     720
ataaatatac gtgtggatgt gtctttatag cagtttgatt tataatcctt tgggtatata     780
cccagtaacg ggatggctgg gtcaaatggt atttctagtt ctagatcctt gaggaatcgc     840
cacactgact tccacaatgg ttgaactagt taacagtccc accaacagtg tgaaagtgtt     900
cctatttctc cacatcctct ccagcacccc attttgactt tgctataagg gaactttagc     960
atctgaacgt gcggacagct tcattgctgg cttgttacgt aacagtgttt tgtgaccatc    1020
tcatgtcata cccacacatc gaaaccagca gtttaaatgg ccagctgttt gc            1072
```

<210> 399
<211> 1072
<212> DNA
<213> Homo sapiens

<400> 399

```
agattatctt taaaatcgtg gggaccaaat ggctggccaa ggacttgttt ctgtacaggt      60
gcgattgctt ctctgctgtg ttccttttta ttacccaagt aaccggtatt tcagctcaca     120
agatgagaaa atgacaaaca ggcaaaataa gcgtagggct gtgtgtgcaa cagtttatca     180
taaagccatc accaggagac rtcactgggc gccttctgga gtctatccgt cctaactttg     240
ctttctttct tttttttttt aaatttaagt tctagggtac atatgcacaa cgtgcaggtt     300
tgtcacacat gtatacatgt gccatgttgg tgtgctgcac ccattaactc gtcatttaca     360
ttaggtgtat ctcctagtgc tatccctccc cactcccccg accccatgac aggccccagt     420
```

```
gtgtgatgtt ccccttcctg tgtccaagtg ttctcattgt tcaatcccca cctatgagtg      480
agaacatgcc atgtttggtt ttttgtcctt gcgatagttt gctgagaatg atggtttcca      540
gcttcatcca tgtccctaca aaggacatga actcatcctt ttttatggct acatagtatt      600
ccatggtgta tatgtgccac attttcttaa tccagtctat catcgatgga catttgggtt      660
ggttccaagt ctttgctatt gtgactagtg ttgcaataaa tatacgtgtg gatgtgtctt      720
tatagcagtt tgatttataa tcctttgggt atatacccag taacgggatg gctgggtcaa      780
atggtatttc tagttctaga tccttgagga atcgccacac tgacttccac aatggttgaa      840
ctagttaaca gtcccaccaa cagtgtgaaa gtgttcctat ttctccacat cctctccagc      900
accccatttt gactttgcta taagggaact ttagcatctg aacgtgcgga cagcttcatt      960
gctggcttgt tacgtaacag tgttttgtga ccatctcatg tcatacccac acatcgaaac     1020
cagcagttta aatggccagc tgtttgcttg tgaaaactcc cctcggctgg ct             1072
```

<210> 400
<211> 948
<212> DNA
<213> Homo sapiens

<400> 400

```
aaatttctt  tgctgaagtg tcttttcaaa tttttgcctt ttaaaaaaat tgagttgtct       60
taatattgag tcgtaaggtt ctttatatat tctggctata tgtcctttgt cagatatatg      120
tcttgcaaat attttctccc agtctgtggc ttaccttttc catttttaaa ctgtgtttta      180
taaaaaaaag aagttttttt agatcaaagt ccattttaat catttttttct tttatagttc     240
atgcttttttg tgtctcattt aagaaatctt tccctactcc aatgtcacaa atatattctc     300
tgagaagctt aacagttttt gcaactaaat ttaggtctat gatccgtttt gacttaattt      360
ttccatatgg tgtcatgtaa cagttgagat ttttttccta tgcaggcaga tattcaatgg      420
ttcaagtacc atttattgaa atggctatct tttctccact gaatgacctt ggcactttta      480
tcaaacatca actggccaca yacaggtgag tctacttctg gacacttacc ctgttccatt      540
catctgtata tctctatcct tacaccaaca cgcatagtct tgaatactag ggcaagttaa      600
ttttaagatg tctcctggat atgtaaaaat tatatctgag ttgaactaca gtttatttat      660
atatccaggc agcaaataaa tgtgagaatc tggaggtgag ggaagagatc agagatacca      720
ccttggaaac catcaattta gagatgattc ttaaggcagg ggactaaggg acactctgta      780
ggacacagac atagagaagg gaaggggctg cggcctgaac accccacctg catgctcact      840
cacatacttt cgtcggcctg tgttaacgaa gtgctgggtc tccccagcct ctctcatctg      900
taagcagtgc caacaacgtc caacacagtt ccatccaatt tggatctg                   948
```

<210> 401
<211> 920
<212> DNA
<213> Homo sapiens

<400> 401

```
aatttttgcc ttttaaaaaa attgagttgt cttaatattg agtcgtaagg ttctttatat      60
attctggcta tatgtccttt gtcagatata tgtcttgcaa atattttctc ccagtctgtg     120
gcttaccttt tccattttta aactgtgttt tataaaaaaa agaagttttt ttagatcaaa     180
gtccatttta atcatttttt cttttatagt tcatgctttt tgtgtctcat ttaagaaatc     240
tttccctact ccaatgtcac aaatatattc tctgagaagc ttaacagttt ttgcaactaa     300
atttaggtct atgatccgtt ttgacttaat ttttccatat ggtgtcatgt aacagttgag     360
atttttttcc tatgcaggca gatattcaat ggttcaagta ccatttattg aaatggctat     420
cttttctcca ctgaatgacc ttggcacttt tatcaaacat caactggcca cacacaggtg     480
agtctacttc tggacactta ycctgttcca ttcatctgta tatctctatc cttacaccaa     540
cacgcatagt cttgaatact agggcaagtt aattttaaga tgtctcctgg atatgtaaaa     600
attatatctg agttgaacta cagtttattt atatatccag gcagcaaata aatgtgagaa     660
tctggaggtg agggaagaga tcagagatac caccttggaa accatcaatt tagagatgat     720
tcttaaggca ggggactaag ggacactctg taggacacag acatagagaa gggaaggggc     780
tgcggcctga acaccccacc tgcatgctca ctcacatact ttcgtcggcc tgtgttaacg     840
aagtgctggg tctccccagc ctctctcatc tgtaagcagt gccaacaacg tccaacacag     900
ttccatccaa tttggatctg                                                 920
```

<210> 402
<211> 701
<212> DNA
<213> Homo sapiens

<400> 402

tgtgctgctt ccattccata ggcacctgat cctaagtgtt aaccaatccc agaactctcc 60
ccttatttct tgctgcatgt tttgaattga tgtgataaac aatgtgattc gagcgtctta 120
actcagccta tgagcctctc tattctgtga ctgctggaat aggctgcttg gccatgttct 180
tggaagctac caccatatca rggtaatttc ccacacaaca ttccagcccc tgctttcccc 240
tctggcctta tctagggcca ttccccaact caggtgaatg cagactccaa atgtactgag 300
ctgtgtgcag gggccaggtg caaatgcttt ctgtgcatct gcacatgctg ttctacctgg 360
gaagtccttt cctcctttca cctattttta ccttaaacct cagacatcat ctaccctgga 420
aagtccttcc tgacctcacg catctaagta ggtccccccc ataatcccta tccatgcctt 480
ctatagtact taacatggtg acctttaatt gttcatttac ttagctctct gctctcccac 540
actgtgaact ccttacaaac agggaatgtc atctctgaat gaatctttca tctccatgta 600
acacatgcct ccaaccctac ctagcacaca atctggcata taacaggcac tcaataaacc 660
ttcaatgaat gccttgatca agtacaagga acataagcaa a 701

<210> 403
<211> 701
<212> DNA
<213> Homo sapiens

<400> 403

ttaaccaatc ccagaactct ccccttattt cttgctgcat gttttgaatt gatgtgataa 60
acaatgtgat tcgagcgtct taactcagcc tatgagcctc tctattctgt gactgctgga 120
ataggctgct tggccatgtt cttggaagct accaccatat caggtaatt tcccacacaa 180
cattccagcc cctgctttcc yctctggcct tatctagggc cattccccaa ctcaggtgaa 240
tgcagactcc aaatgtactg agctgtgtgc aggggccagg tgcaaatgct ttctgtgcat 300
ctgcacatgc tgttctacct gggaagtcct ttcctccttt cacctatttt taccttaaac 360
ctcagacatc atctaccctg gaaagtcctt cctgacctca cgcatctaag taggtccccc 420
ccataatccc tatccatgcc ttctatagta cttaacatgg tgacctttaa ttgttcattt 480
acttagctct ctgctctccc acactgtgaa ctccttacaa acagggaatg tcatctctga 540
atgaatcttt catctccatg taacacatgc ctccaaccct acctagcaca caatctggca 600
tataacaggc actcaataaa ccttcaatga atgccttgat caagtacaag gaacataagc 660
aaatttcctg tggaaaaaaa gaattgtatt aagttctttg g 701

<210> 404
<211> 601
<212> DNA
<213> Homo sapiens

<400> 404

```
atgttcactt acacatcttt ctttcactta attgaatcct ttatttttgt cttagaatct      60
tctgaatatt gaaaacagag aactatactg gaagaacata gtgtattaag actcatggag     120
agggagatgt gatactgtgt cactgaggtc gttccagtca taggagaaat gttaccactg     180
gattgaggtc tggtacattt taaaagatga tttaattcta tgatatgtgt tcaacttgca     240
ctaggatagt ttttactttc acctttgttc catgcaccgc gcaaatacct gggaaccctt     300
rttgcccaac tcaagagcca gagtcctctg tcatcatttt gcctctctcc taagtgacag     360
gactgagtgc agacttggtg tttgtgggtg aggcatgtgg actgacaggc aggcttcagt     420
ttatttagcg agtgtgagcc ctggcaggaa gattctcttt ctctgcttgc caggttgagg     480
aggcctcatt aagcagtttg aacttgtggt tttggcgtgt ctagtcctgg tgcaggtggc     540
ttggtatcct cacaggcatt tctttggcct caccccttggg gtgactgttc acttgtgttt     600

g                                                                     601
```

<210> 405
<211> 601
<212> DNA
<213> Homo sapiens

<400> 405

```
tcttctgaat attgaaaaca gagaactata ctggaagaac atagtgtatt aagactcatg      60
gagagggaga tgtgatactg tgtcactgag gtcgttccag tcataggaga aatgttacca     120
ctggattgag gtctggtaca ttttaaaaga tgatttaatt ctatgatatg tgttcaactt     180
gcactaggat agtttttact ttcacctttg ttccatgcac cgcgcaaata cctgggaacc     240
cttgttgccc aactcaagag ccagagtcct ctgtcatcat tttgcctctc tcctaagtga     300
saggactgag tgcagacttg gtgtttgtgg gtgaggcatg tggactgaca ggcaggcttc     360
agtttattta gcgagtgtga gccctggcag gaagattctc tttctctgct tgccaggttg     420
aggaggcctc attaagcagt ttgaacttgt ggttttggcg tgtctagtcc tggtgcaggt     480
ggcttggtat cctcacaggc atttctttgg cctcaccctt ggggtgactg ttcacttgtg     540
tttgagcggc tgggactcag taggttcact ggagtaggta tttctttaga gccactggcg     600
g                                                                     601
```

<210> 406
<211> 701
<212> DNA
<213> Homo sapiens

<400> 406

```
cagctccttg gcaagcctgc tccttcccca gcaaatggaa acaccattct gaacacctgg      60
gcattgtctc tgatgtccct tttcatctcc ctactctcac acaatccagc tgcctctctg     120
ccttccacgg atattaagaa cgtccaccat ctcctgagtc caagcccttc tcactcacct     180
ctttcttgaa ctaatttctt yctgtttttt tccagtcctc ccttctgttc atgtctctcc     240
tctgcacact tccattttct ggttcagaaa atgtcaccgt cccagtcaca cttgccttat     300
ggctgttgtg tcataaatac agttgacact tgaacaacat gggtttgaac tgcatggatt     360
cacttataca catatttttt caatacaaat atatttaaaa attttggaga tttgcaacaa     420
tttgaaaaaa cttgcagatg aacagcatag catagaaata ttgaaaaatt aagaaaaagg     480
tatgtcatga atgcataaaa catatgcaga tactagtcta ttttaacctt tactgccata     540
aaatatacac aaatctatta taaaaggtta aagtttatca aagcttatgc acacaaacac     600
ttatagacca tatagggagc cattcagtag agagaaatgt aagcgaacgt aaaggtgtgc     660
tatttaatca caactgcata cacactgtac cactgcacta a                         701
```

<210> 407
<211> 701
<212> DNA
<213> Homo sapiens

<400> 407

```
gggcattgtc tctgatgtcc cttttcatct ccctactctc acacaatcca gctgcctctc      60
tgccttccac ggatattaag aacgtccacc atctcctgag tccaagccct tctcactcac     120
ctctttcttg aactaatttc tttctgtttt tttccagtcc tcccttctgt tcatgtctct     180
cctctgcaca cttccatttt stggttcaga aaatgtcacc gtcccagtca cacttgcctt     240
atggctgttg tgtcataaat acagttgaca cttgaacaac atgggtttga actgcatgga     300
ttcacttata cacatatttt ttcaatacaa atatatttaa aaattttgga gatttgcaac     360
aatttgaaaa aacttgcaga tgaacagcat agcatagaaa tattgaaaaa ttaagaaaaa     420
ggtatgtcat gaatgcataa aacatatgca gatactagtc tattttaacc tttactgcca     480
taaaatatac acaaatctat tataaaaggt taaagtttat caaagcttat gcacacaaac     540
acttatagac catataggga gccattcagt agagagaaat gtaagcgaac gtaaaggtgt     600
gctatttaat cacaactgca tacacactgt accactgcac taatttcaga gccacctcct     660
gttgtgattg tggtgagccc aagtgttgtg aggatctgct t                         701
```

<210> 408
<211> 501
<212> DNA
<213> Homo sapiens

<400> 408

```
caggtagggt aagcaaatga acacaaattc aaactcggaa ttcaaaacca gcctctgtgt      60
attcctgagg accatactgt ctgctaagtg tagagaaagg cacatcctgg ttcaacagca     120
gagaaagcaa acaggaggca ctttctgtga gtcatctcca ccacagggcc ctctcttttg     180
tgatccagcg atacttgttc acagtcaaag cccaggaaga gtggaaagat taacctttgt     240
gagccaaacc rtgtgacact tgattacttg acagaactaa tccttctgtc ctgatgacag     300
aaattcaact acacaggtac atgcaagcta atatctgttg taatgcctcc cagtttctct     360
ggagaattcc ttagtttcct ggacatctct gaaatgcaaa gttttggcaa cgagtctctg     420
aattaacctc tgaaaatctc acccagccaa gatggccttc ttgagaagac tgaagaacat     480
ggttggtttc aggctgagct g                                               501
```

<210> 409
<211> 604
<212> DNA
<213> Homo sapiens

<400> 409
cactttctgt gagtcatctc caccacaggg ccctctcttt tgtgatccag cgatacttgt 60

```
tcacagtcaa agcccaggaa gagtggaaag attaaccttt gtgagccaaa ccgtgtgaca     120
cttgattact tgacagaact aatccttctg tcctgatgac agaamttcaa ctacacaggt     180
acatgcaagc taatatctgt tgtaatgcct cccagtttct ctggagaatt ccttagtttc     240
ctggacatct ctgaaatgca aagttttggc aacgagtctc tgaattaacc tctgaaaatc     300
tcacccagcc aagatggcct tcttgagaag actgaagaac atggttggtt tcaggctgag     360
ctggaagtgg tttacctccc aggagaggtt ccccacagtg gtgtttaagg catggggtgg     420
accaacacca ggaagactca gacatcacac cacccacctt caactcagtc acatccacct     480
acattttctg aaaacaaaag gcagtctccc caaaaagcac tgagactctt gtgtaggtaa     540
tctgagcaga caccaacttc ccagggcttc cttttatcca ggagagcttg gctgttcttt     600
ttaa                                                                  604
```

<210> 410
<211> 701
<212> DNA
<213> Homo sapiens

<400> 410

ctccttccgc catggttgta agtttcctga cgcctcccag tcatgcttcc cgtacagcct 60

gcagaactgc gagtcaatga aatccctttt ctccacaaat tacccagtct caggtagttc 120

cttacagcag cgtgggaaca gactcaagag ctgaagcaag caaggccgtt agcaaggagc 180

gggctgggga gagcactcca ggcagaggga acagccaggg ccagggcctt gagacagacg 240

tgagccagga tatctgagga acagcagaga agccagtgtg gccgcagcta aatgaggaac 300

aatgtgtgag ttccctgggg cggccaaaac aaacaccacg gacgggggcc ttcaaccaca 360

gacaccgatt tcctcacagc tctggaggcg aaaagtccaa gaaaactgca cggagtatct 420

atgaggccct gatggagacc tgacctggtc cacacccatg gcctggcaag ctagatgggg 480

tgaattttca cctgccacag ycgcaagtca aagccaccgg cttctctctt ctccctccca 540

ttgctcctga cagccagggt taatattttg cctcatgtaa acagggaggc atccacccga 600

gaatctcccc tcagcccaca taagctctgc agagagggct gtgttgctcc agttcccacc 660

tggacatgag cactttgaag ggcagcttcc ctcccggggt c 701

<210> 411
<211> 612
<212> DNA
<213> Homo sapiens

<400> 411

gggctgggga gagcactcca ggcagaggga acagccaggg ccagggcctt gagacagacg 60

tgagccagga tatctgagga acagcagaga agccagtgtg gccgcagcta aatgaggaac 120

aatgtgtgag ttccctgggg cggccaaaac aaacaccacg gacgggggcc ttcaaccaca 180

gacaccgatt tcctcacagc tctggaggcg aaaagtccaa gaaaactgca cggagtatct 240

atgaggccct gatggagacc tgacctggtc cacacccatg gcctggcaag ctagatgggg 300

tgaattttca cctgccacag tcgcaagtca aagccaccgg cttctctctt ctccctccca 360

ttgctcctga cagccagggt taatattttg cctcatgtaa acagggaggc ayccacccga 420

gaatctcccc tcagcccaca taagctctgc agagagggct gtgttgctcc agttcccacc 480

tggacatgag cactttgaag ggcagcttcc ctcccggggt ctggctgagc tcagggtagg 540

cgtcagtctg catggattgg atggaggaag gctgtgcgtg gcaggagatg acactgccct 600

tgggctgtgt gg 612

<210> 412
<211> 1001
<212> DNA
<213> Homo sapiens

<400> 412

```
ttggggaagg aagcactggg gggaaggaag cactgggctt gggacagggc tgggcgctgc      60
ctcttcactg gaccatgaca aggttgttac ctcaccaagg agaggtgcaa aaagcttagg     120
ggcttggatt tctagatttc agtgccaact atgccactta ctggctttat ccttggggaa     180
tttatctact ctgtgaccct cagttttttt atcttaatta ttaatacata cctcataatg     240
tgactgtgag gattcactta ataatatatg gaaaaccata gaatagtgcc cagcatctag     300
gaagtgccac agccccccttc agaagctagt gaaacctgca gaccactttt cagagtgata     360
ttattatttt tttctaggtt tactgagtta taattgaaaa aataaaaatg gaatatagat     420
gtacaacatg aagctctgat gcatatatcc attgtgaaat gatgaccaca atcaagctaa     480
ttaatgttat ctatcacttc wcatagttca acctttttttt gtggtgagag tactgaagat     540
ctactctctt agcaattttc aaatctaaaa tacattatta ttaacacagt cactgtgccg     600
tacgttagct ctgaggacct tattcatttt atacctaaaa gtctgtatcc tttaaccaac     660
ctctcctaat ttcccactgt catccctact gccacctctg gtaaccagcc ttctgctctg     720
tttctgagtc caaccttctt agattccaca tatgagtgag atcatgctgt gcagtgtttg     780
tttttctgtg tctggcttgc tttcacttag cataatgtcc tccaggtcca cccatgttgt     840
tgcaaatggc agaatcttct tcttgttaaa gactgaataa tatccctgtg tgtgcgtgca     900
tgtgtgtgtg tgtttgtgtg tgtgtgtgta tcacattttc ttcatccatt catccatcaa     960
tggacactaa gcactaaggt tgattccgta tcttggctat t                        1001
```

<210> 413
<211> 2480
<212> DNA
<213> Homo sapiens

<400> 413

aacattactt ggggaaggaa gcactggggg gaaggaagca ctgggcttgg gacagggctg 60
ggcgctgcct cttcactgga ccatgacaag gttgttacct caccaaggag aggtgcaaaa 120
agcttagggg cttggatttc tagatttcag tgccaactat gccacttact ggctttatcc 180
ttggggaatt tatctactct gtgaccctca gtttttttat cttaattatt aatacatacc 240
tcataatgtg actgtgagga ttcacttaat aatatatgga aaaccataga atagtgccca 300
gcatctagga agtgccacag ccccccttcag aagctagtga aacctgcaga ccacttttca 360
gagtgatatt attatttttt tctaggttta ctgagttata attgaaaaaa taaaaatgga 420
atatagatgt acaacatgaa gctctgatgc atatatccat tgtgaaatga tgaccacaat 480
caagctaatt aatgttatct atcacttcac atagttcaac cttttttgt ggtgagagta 540
ctgaagatct actctcttag caattttcaa atctaaaata cattattatt aacacagtca 600
ctgtgccrta cgttagctct gaggacctta ttcattttat acctaaaagt ctgtatcctt 660
taaccaacct ctcctaattt cccactgtca tccctactgc cacctctggt aaccagcctt 720
ctgctctgtt tctgagtcca accttcttag attccacata tgagtgagat catgctgtgc 780
agtgtttgtt tttctgtgtc tggcttgctt tcacttagca taatgtcctc caggtccacc 840
catgttgttg caaatggcag aatcttcttc ttgttaaaga ctgaataata tccctgtgtg 900
tgcgtgcatg tgtgtgtgtg tttgtgtgtg tgtgtgtatc acattttctt catccattca 960
tccatcaatg gacactaagc actaaggttg attccgtatc ttggctattg tgaataatgc 1020
tgcaataaac atatgagtcc agatacctct tcaagatact gatttcattt cctttaaata 1080
tatgcccaga agtgggattg ctggatcata tggtagttct atatttagta tcttgaggaa 1140
tttccatact gtttttcata atgattgtag caatctatat tcccatcaac agtgtacaag 1200
ggttccattt tctacatggc cttaccaacg tttgttatca cttatctttt tgataataga 1260
tattctagca ggtgtgaggt ggtatctcat tgtggtttta atttgcattt tcctgatgat 1320
tagtggtgta gagcatcttt tcatattccc attggtaatt cgtatatctt cctttgagaa 1380
atatttattc agatcttttg cccattgtta gctgagttat atgtgagttg gttttggttt 1440
gttgttgttt tttgtttttg ctattgagct gagttccttg tatattttgg atattaaatc 1500
cttctcagct gtatggttga cagatacatt cttgcattct gtaagttgca tctgtaggtt 1560
gcaacagagt ctctttactc tgttgattgc ttgctttact gtgtgaaagc ttttttagct 1620
tgatgtaatt gtgtttgtct atttttgctt ttgttgcttg tacttttagt gtcatatcca 1680
aaaagttatt gcccagacca gtgtcatccc ctatgttttc ttctagtaat tttaaagttt 1740
caggtcttat gtctatgtct ttaatccatt ttgagttaat ttttgtgtag ggtttaagat 1800

aagaatccaa ttttattttt attttttgta tatggatatc caatttcccc aacaccattt 1860

attgaaaatt ctatcctttc tttgttgtgt attaacatca gaataatatt tttaaataca 1920

taaaattcag aagatgacaa aggaaaccaa ttacattgaa atgcatacag agttataatt 1980

ctgaaagagc aatatatgtg cctctttgta aacacatcat atatcaaact gcagtgaccg 2040

ttctaacaac tattgcaatt tcaaagtcat gttgagtagg aggagtactt tgagattctg 2100

aaacaacgtt cttgtgctat gaaatatcca tgattttgat tggtgatggt atcccaggtc 2160

ttgttaatgc tgctgtaatc tgttgcttcc attccatagt tgaataaaat gcttgatatc 2220

tgttggaaat tagtaaaaat aaaaacgtat ttttttccat ccaagttcat tctcagaccc 2280

tgaagagtca cttctctgga ttctgcagca aagttcccag ctggggcagc aagatttagg 2340

caattgaaaa gaacatacac cttgttctca gtggcaaacc acatggaaag ctttaaatgt 2400

cagagaagaa ttctgccatt ttgctgactt ttttgtagtt ctcctaataa acaagtgtta 2460

agtgacaagc ttttcagagg 2480

<210> 414
<211> 601
<212> DNA
<213> Homo sapiens

<400> 414

cccccccccc ccccgcagat ctcaggtggg catttttgaa cttaactaga taacaaaaca 60

cagctaagac aagtcctttt ctccagcaaa gatggcaatg ctctaataac tctgagcata 120

ttaaagattc tccaagactc tagcctctgc tgcaaaaaca catacaaata cctactacta 180

ctgctgctgt gatgatgatg atgacagcaa tagtgagaat attttaaata tgccaggcac 240

ggtggcaact gctttccaaa tattatcata tttaatctga tcattgccct atgaggtagg 300

ragtattctg attcccattt tataaataag gaacccgagg cttagagagc atcggtgact 360

tgttcaaggt cacccacagc tgtcaagtga cagaacttcg ataaaaatcc agactccttt 420

aatggagtat ggagggaggt cagaaaacat aggaagtaag ggattgtgat tgacaatgtg 480

tccttgcaaa gggacaggtt aagagacaca agggcagctg tctgaggtgt gccattcacc 540

agcttcagga gagaagtggc aggctacctc cagctatcca gccctatcca gccaaggaag 600

c 601

<210> 415
<211> 601
<212> DNA
<213> Homo sapiens

<400> 415

caaaacacag ctaagacaag tccttttctc cagcaaagat ggcaatgctc taataactct 60

gagcatatta aagattctcc aagactctag cctctgctgc aaaaacacat acaaatacct 120

actactactg ctgctgtgat gatgatgatg acagcaatag tgagaatatt ttaaatatgc 180

caggcacggt ggcaactgct ttccaaatat tatcatattt aatctgatca ttgccctatg 240

aggtagggag tattctgatt cccattttat aaataaggaa cccgaggctt agagagcatc 300

rgtgacttgt tcaaggtcac ccacagctgt caagtgacag aacttcgata aaaatccaga 360

ctcctttaat ggagtatgga gggaggtcag aaaacatagg aagtaaggga ttgtgattga 420

caatgtgtcc ttgcaaaggg acaggttaag agacacaagg gcagctgtct gaggtgtgcc 480

attcaccagc ttcaggagag aagtggcagg ctacctccag ctatccagcc ctatccagcc 540

aaggaagctt gggagacatg ttagttcccg ccttcatttc catcagcaac ctcaaagcca 600

c 601

<210> 416
<211> 5823
<212> DNA
<213> Homo sapiens

<400> 416

```
tatttcaggc tttcttcttt ctatggataa gaaagctcct caggtggcaa caaaggccat     60
ttctttggaa gcaggcatgg catgtgacga aaaaaagaca tctcagaaaa gagccaagaa    120
taagactgga gagccactgt cagagaacag aaactgggct taatcaagga acatctcttg    180
ttcccagagt aggaggctgg caatattttc tcactgaaat ttcagaattg ttatggacca    240
gtgactgctc tatgtgttca atttgttccc ttttcaaatg gaagcattta ttgcagacga    300
cctgcctctg tcccaccatt gtgtattagg tttgtagagy gtagacaact tgccttttta    360
gtttgtaggt ttctgtatca agagaagatg tgtgtgggcc taacctagat tacaggatcc    420
tggacttcaa gtctgatata atgactggat gagactttga ctgtcctaga attgggatga    480
acatattttg ccggtgggag ggcgtgagta attgcggtta gagggcagac tgtccctcac    540
acctattcct tttcatggtg ccttcccaaa ctgcctctgg aggtggccac acaaatggct    600
ttggccattg tgaccatggg aaacttgatg cagaggctgg aaaaagcact tgcatgtttc    660
tgtctcctct cttgttcctc tacaatcaca agaaatgtct aggcaggtct gagcaggccc    720
aggctcatct gccatggaag aagaatggca catggaagag ggtcacattg tcccaaccaa    780
gacgatccta gaccagccag gccccagttc atggttcaag acacatgaac atagttgcac    840
gaaccaagat tagttgtgta tggcccagac tagcagcagc acccatccaa cctacagact    900
ctgagaaata aatactagtt gtcttaagct tccaagtttc agtgtgagca ttaggtagta    960
acagttaatg aataagacag ataatcattt tatctgtctg gatacttata caatgatttc   1020
tattttttat tgatacataa tattttacat attgctgggg tacatgtgac attttgctac   1080
atacatagaa tgtgtaatga tccagtcagg atatctgagg tgtccatcac tttgagaatt   1140
```

tctcacttct gtgtgttggg aacaattcaa gtcgtctctt ctagttattt taaaatatac 1200

aatacattgt taactgtagt ctttttttatt gaatgacagg acttgtacct tttatctaac 1260

tgtatgtttg tatctattaa gctagttctc tttatccctg cccccctccta cccactcact 1320

cttcccaacc tctaacatgt atcatcctat tctatatctc catgagatca acttctttag 1380

ctcccacata tgagcaaaaa catatgatgt ttgtctttct gtgcccggtt tatttcactt 1440

atgacctcca tttccatcca tgttactata aatgacagga tttcattctt tttgtggcca 1500

aacagtattt cattgtgtat atatactaca ttttctttat ccattcatcc attgatgaac 1560

acttacgttg attccatatc tttgctattg tgaatggtgc tgcaataaac atgcacgtgc 1620

agttatccct ttgatacact gatttatttt cctttggata aatacccagt agtgagattg 1680

ctggatcata cggtagttct acttttagtt tttgagacat ttccatactt ttccagtgtt 1740

tgtattaatt tacattccca tcaacaatgt ataagatttc cctttcctcc acatcctcac 1800

cagcatctgc tattttttgt ctttttaata atagtcattc taactggggt gagaggatat 1860

ctcgctatgg ttttgatttg catttccctg atatttaatg atattgagca tttcttcata 1920

taacctattg gccatttgtg tgtctttttt tttttttttt ttttttttga gaattgtcta 1980

ctcattttg gctttttaaa agatttattt tttgttgttg ttgagtttag tgcatatcct 2040

ggatattagt ctcttatctg atgaagagtt tgccaatatt ttctcccatt caacaggttg 2100

tctcttcatt ctgttgactg tttcctttgc tgtgcagaag cactttatat acagtcccat 2160

ttgtctattt tttagtagtc tatgcattta aggtctcagc cacaaaatct ttgcctagac 2220

cagtcctaaa gtgtttcccc tatattttct tctagtagtt ttattgtttc atgtcttata 2280

tttaagtcta taatccattg tgagttgatt tttgtatatg gtgagatagg ggccttgttt 2340

cattcctctg catatagata tttaattttc tcagcaccat ttattgaagg tgtccttccc 2400

tattgtatgt tcttggtgcc tttgtcaaaa ttcagttggc tataaatatg tgaatttatt 2460

tctgggttct ctatgtggtt ccattagtct atgtgtctat ttttatacca atatcatgct 2520

gttttgatta ccatagcctt gtaatatatt ttgaagtcag gtagtgtgat gcctccagct 2580

ttgttctttt tgctcaggat tgctgtgcat actctggctt tttggttaca tacaaatttc 2640

aggattttttg tatttctgtg aaaaatggca ttagtatttt gataggaatt gcactggatc 2700

tgtatattgt cctggacaac atggtcattt taacaatatt aattcttcta atctatgagt 2760

atgagacgtc ttcccacttg tttgtgtcct cttcaatttc tttcattggt gtttcataat 2820

ctcccttcta caggcctttc acctccttgg ttaaattaat tcctaggtat ttttttgtag 2880

ctactgtaaa tgggactgcc ttctttctca gctagttcat ttttggtgca tagaaaccct 2940

atttttgtat gttcattttc tatcctgcaa cattaccaaa tttgcttatc agctttaagt 3000

gtgtattttg ctttgcttgt agagtcttct ggtttctcta aatgtaagac gatgtcatct 3060
gcaaacgggg acaatttgac ttcctcttaa aaatctgtat gccttttatt cctttctctt 3120
gcctgattgc tctggctcta cctccagtac tatactgaat aaaagtggta aaagtgagca 3180
tccttccttg tcttgctcta gttcttagag gaaatacttt cagtttttcc ccactcagta 3240
tgatgttagc tgtgggtcat atatagcctt tattatgtta agatatgttt cttctgtacc 3300
tggtttgttg acagcttttt atcataaaag gatgtagaat tttatcaaat gtttttctg 3360
catctgttga gataatcata tggtttttgt cattccttct actgttgtga tgtatcatgt 3420
ttattgattt gtgtatgtta aaccatcctt gtgtccttgg tataaattat acttggtcat 3480
ggtgtattat ctttttggca tcctgtcgaa ttgtttgcta gctttttgtt ttgttctttt 3540
tgagaatttt tatgtctagg ttccttagaa acactggcct gtagttctct ttttgtgtgt 3600
gtgtccttgt ctagtttggt gtcagggaaa tggtggtctt gtagaatgag ttgtttttc 3660
tttgattttt ttgcaagagt ttgaggagaa tgggtattag ttcttcttta tgtggttggt 3720
caaattggca gtgaattcat tcagtcatga gcttttcttt ttttgggagg gttctcatta 3780
ctgagttaat cacactgctc attactgatc tgttcagatt ttctatttct tctggaatct 3840
cagtagttgt atgtttccag caatttatcc atttcctcta ggttttctag tttggtagta 3900
tatagctatt cataatagtc tctgatgatc ttttgtattt ctgtgatatc agttgtaatg 3960
tctttttcat ttcctatttt atttgggtct tttcttgttt agtctagcaa ggggtttatc 4020
tattttatct ttttgaagaa ccaacttttt gtttcattga ccctttctac gtctttagtc 4080
tttatttcat ttagatttgc tctgaacttt actatgtctt tccttctaat tttgggtttg 4140
gtttgttctt ttctagttcc ttgaggtgca tcattgaatt gtttctttga tatctatcta 4200
ctcatttgat gtaggtgttt attgctatac actctcccct cctagagctc cttttgttgt 4260
gtcccatagg tcttggtatg ttgtttctat tttcatttgt ttcaaacatt ttatttccat 4320
attaattttt atcattcagg aggagcatat tatttaattc ccatgtattt gtatagtttc 4380
caaagttcct cttatttcta tttttactcc attgtggtct gagaagatac ttcatatgat 4440
ttcaattttt aaaaatttgt caagacttgt tttttgtcct aacatatggt ctatcctgga 4500
gaatgttcca tgtgctgatg agaaaaatgt gtactcagca gttgttgagt aacatgttct 4560
acaaatatct gttagatcca tttggtctaa agtctagttt aaatccaatg agtttttgtt 4620
aattttgtct agatgatcat gatctgagac tgaggtgaag tccccaacaa ttatcgtgtt 4680
ggagtctacc tctcttttta aatctagaaa tatttgcttt ataaatccgg gtggtctagt 4740
gttgggtgca tatatttagt tgttatttcc tcattagatt gatctcttta ctattatata 4800
ataactgttt actgcttctg gcataaagtc tgttttatgt aagtacagcc attcctgctt 4860
gagtttagta ccatgttgac aaagggatgc atagagagtt ggtaaagcat gatttctggg 4920

tgtctgtgtg aaggtgtttt gagaagagtt tagcatgagt ctgtggagtg agtgggaaga 4980
ttctccctca atgtcagcag gaaccatcca tccactgggg gcccaggtag aaaaagatga 5040
agaaatggtg aattctctct ctctcctgga gctgggtcac ccttcttctg cccttgaaca 5100
ggacatcaca actccaggct ctccagcctt tggactccaa gactgacacc agtgcccctc 5160
cccaattacc ccaggccctc aggcctttgg cctaggattg agacttacac catcagcttc 5220
cctggttctg aggcttctgg acttgcactg ggccatacta ccagcatccc agggtctcca 5280
gcttgcagag agcctgttgt gggacttttc agcctccata atcaagtaag ccaatttccc 5340
tggtatctat atagatatac aatcatgttt tgcttaccag cctgaaaaat gtatcgctag 5400
atgagtctgt cattgcataa acatcatagt gtacttacac aaacctagat tctatagcct 5460
actacacacc tagtctataa acatgtacag catgttactg tactgaatat tgtaggcaac 5520
tgtaacacaa tggtgaatat ttgcatattt aaacatatct tatcattaaa aagatacagt 5580
aaacataagg tataaaagac aaaaaccggc acacctatat agggcactta ccataaatgc 5640
agcttgcagg actagaagtc actcagggtg agtcagtgag cgaacgtgaa ggcctaggtt 5700
attactgtcc actacggtag actttatcaa cactgtacac aggctacact aaatttattt 5760
tttaaaaatt tgctctccaa taataaatta atcttcgcat cctttttttg ttgttcactg 5820
tgg 5823

<210> 417
<211> 5823
<212> DNA
<213> Homo sapiens

<400> 417

```
tatttcaggc tttcttcttt ctatggataa gaaagctcct caggtggcaa caaaggccat      60
ttctttggaa gcaggcatgg catgtgacga aaaaaagaca tctcagaaaa gagccaagaa     120
taagactgga gagccactgt cagagaacag aaactgggct taatcaagga acatctcttg     180
ttcccagagt aggaggctgg caatattttc tcactgaaat ttcagaattg ttatggacca     240
gtgactgctc tatgtgttca atttgttccc ttttcaaatg gaagcattta ttgcagacga     300
cctgcctctg tcccaccatt gtgtattagg tttgtagagt gtagacaact tgccttttta     360
gtttgtaggt ttctgtatca agagaagatg tgtgtrggcc taacctagat tacaggatcc     420
tggacttcaa gtctgatata atgactggat gagactttga ctgtcctaga attgggatga     480
acatattttg ccggtgggag ggcgtgagta attgcggtta gagggcagac tgtccctcac     540
acctattcct tttcatggtg ccttcccaaa ctgcctctgg aggtggccac acaaatggct     600
ttggccattg tgaccatggg aaacttgatg cagaggctgg aaaaagcact tgcatgtttc     660
tgtctcctct cttgttcctc tacaatcaca agaaatgtct aggcaggtct gagcaggccc     720
```

aggctcatct gccatggaag aagaatggca catggaagag ggtcacattg tcccaaccaa 780
gacgatccta gaccagccag gcccccagttc atggttcaag acacatgaac atagttgcac 840
gaaccaagat tagttgtgta tggcccagac tagcagcagc acccatccaa cctacagact 900
ctgagaaata aatactagtt gtcttaagct tccaagtttc agtgtgagca ttaggtagta 960
acagttaatg aataagacag ataatcattt tatctgtctg gatacttata caatgatttc 1020
tatttttttat tgatacataa tattttacat attgctgggg tacatgtgac attttgctac 1080
atacatagaa tgtgtaatga tccagtcagg atatctgagg tgtccatcac tttgagaatt 1140
tctcacttct gtgtgttggg aacaattcaa gtcgtctctt ctagttattt taaaatatac 1200
aatacattgt taactgtagt ctttttttatt gaatgacagg acttgtacct tttatctaac 1260
tgtatgtttg tatctattaa gctagttctc tttatccctg cccccctccta cccactcact 1320
cttcccaacc tctaacatgt atcatcctat tctatatctc catgagatca acttctttag 1380
ctcccacata tgagcaaaaa catatgatgt ttgtctttct gtgcccggtt tatttcactt 1440
atgacctcca tttccatcca tgttactata aatgacagga tttcattctt tttgtggcca 1500
aacagtattt cattgtgtat atatactaca ttttctttat ccattcatcc attgatgaac 1560
acttacgttg attccatatc tttgctattg tgaatggtgc tgcaataaac atgcacgtgc 1620
agttatccct ttgatacact gatttatttt cctttggata aataccccagt agtgagattg 1680
ctggatcata cggtagttct acttttagtt tttgagacat ttccatactt ttccagtgtt 1740
tgtattaatt tacattccca tcaacaatgt ataagatttc cctttcctcc acatcctcac 1800
cagcatctgc tatttttttgt ctttttaata atagtcattc taactggggt gagaggatat 1860
ctcgctatgg ttttgatttg catttccctg atatttaatg atattgagca tttcttcata 1920
taacctattg gccatttgtg tgtctttttt tttttttttt tttttttttga gaattgtcta 1980
ctcatttttg gctttttaaa agatttattt tttgttgttg ttgagtttag tgcatatcct 2040
ggatattagt ctcttatctg atgaagagtt tgccaatatt ttctcccatt caacaggttg 2100
tctcttcatt ctgttgactg tttcctttgc tgtgcagaag cactttatat acagtcccat 2160
ttgtctattt tttagtagtc tatgcattta aggtctcagc cacaaaatct ttgcctagac 2220
cagtcctaaa gtgtttcccc tatattttct tctagtagtt ttattgtttc atgtcttata 2280
tttaagtcta taatccattg tgagttgatt tttgtatatg gtgagatagg ggccttgttt 2340
cattcctctg catatagata tttaattttc tcagcaccat ttattgaagg tgtccttccc 2400
tattgtatgt tcttggtgcc tttgtcaaaa ttcagttggc tataaatatg tgaatttatt 2460
tctgggttct ctatgtggtt ccattagtct atgtgtctat ttttatacca atatcatgct 2520
gttttgatta ccatagcctt gtaatatatt ttgaagtcag gtagtgtgat gcctccagct 2580

ttgttctttt tgctcaggat tgctgtgcat actctggctt tttggttaca tacaaatttc 2640
aggatttttg tatttctgtg aaaaatggca ttagtatttt gataggaatt gcactggatc 2700
tgtatattgt cctggacaac atggtcattt taacaatatt aattcttcta atctatgagt 2760
atgagacgtc ttcccacttg tttgtgtcct cttcaatttc tttcattggt gtttcataat 2820
ctcccttcta caggcctttc acctccttgg ttaaattaat tcctaggtat ttttttgtag 2880
ctactgtaaa tgggactgcc ttctttctca gctagttcat ttttggtgca tagaaaccct 2940
atttttgtat gttcattttc tatcctgcaa cattaccaaa tttgcttatc agctttaagt 3000
gtgtattttg ctttgcttgt agagtcttct ggtttctcta aatgtaagac gatgtcatct 3060
gcaaacgggg acaatttgac ttcctcttaa aatctgtat gccttttatt cctttctctt 3120
gcctgattgc tctggctcta cctccagtac tatactgaat aaaagtggta aaagtgagca 3180
tccttccttg tcttgctcta gttcttagag gaaatacttt cagtttttcc ccactcagta 3240
tgatgttagc tgtgggtcat atatagcctt tattatgtta agatatgttt cttctgtacc 3300
tggtttgttg acagcttttt atcataaaag gatgtagaat tttatcaaat gttttttctg 3360
catctgttga gataatcata tggtttttgt cattccttct actgttgtga tgtatcatgt 3420
ttattgattt gtgtatgtta aaccatcctt gtgtccttgg tataaattat acttggtcat 3480
ggtgtattat ctttttggca tcctgtcgaa ttgtttgcta gctttttgtt ttgttctttt 3540
tgagaatttt tatgtctagg ttccttagaa acactggcct gtagttctct ttttgtgtgt 3600
gtgtccttgt ctagtttggt gtcagggaaa tggtggtctt gtagaatgag ttgtttttc 3660
tttgattttt ttgcaagagt ttgaggagaa tgggtattag ttcttcttta tgtggttggt 3720
caaattggca gtgaattcat tcagtcatga gcttttcttt ttttgggagg gttctcatta 3780
ctgagttaat cacactgctc attactgatc tgttcagatt ttctatttct tctggaatct 3840
cagtagttgt atgtttccag caatttatcc atttcctcta ggttttctag tttggtagta 3900
tatagctatt cataatagtc tctgatgatc ttttgtattt ctgtgatatc agttgtaatg 3960
tctttttcat ttcctatttt atttgggtct tttcttgttt agtctagcaa ggggtttatc 4020
tattttatct ttttgaagaa ccaacttttt gtttcattga ccctttctac gtctttagtc 4080
tttatttcat ttagatttgc tctgaacttt actatgtctt tccttctaat tttgggtttg 4140
gtttgttctt ttctagttcc ttgaggtgca tcattgaatt gtttctttga tatctatcta 4200
ctcatttgat gtaggtgttt attgctatac actctcccct cctagagctc cttttgttgt 4260
gtcccatagg tcttggtatg ttgtttctat tttcatttgt ttcaaacatt ttatttccat 4320
attaattttt atcattcagg aggagcatat tatttaattc ccatgtattt gtatagtttc 4380
caaagttcct cttatttcta tttttactcc attgtggtct gagaagatac ttcatatgat 4440
ttcaattttt aaaaatttgt caagacttgt tttttgtcct aacatatggt ctatcctgga 4500

gaatgttcca tgtgctgatg agaaaaatgt gtactcagca gttgttgagt aacatgttct 4560

acaaatatct gttagatcca tttggtctaa agtctagttt aaatccaatg agtttttgtt 4620

aattttgtct agatgatcat gatctgagac tgaggtgaag tccccaacaa ttatcgtgtt 4680

ggagtctacc tctcttttta aatctagaaa tatttgcttt ataaatccgg gtggtctagt 4740

gttgggtgca tatatttagt tgttatttcc tcattagatt gatctcttta ctattatata 4800

ataactgttt actgcttctg gcataaagtc tgttttatgt aagtacagcc attcctgctt 4860

gagtttagta ccatgttgac aaagggatgc atagagagtt ggtaaagcat gatttctggg 4920

tgtctgtgtg aaggtgtttt gagaagagtt tagcatgagt ctgtggagtg agtgggaaga 4980

ttctccctca atgtcagcag gaaccatcca tccactgggg gcccaggtag aaaaagatga 5040

agaaatggtg aattctctct ctctcctgga gctgggtcac ccttcttctg cccttgaaca 5100

ggacatcaca actccaggct ctccagcctt tggactccaa gactgacacc agtgcccctc 5160

cccaattacc ccaggccctc aggcctttgg cctaggattg agacttacac catcagcttc 5220

cctggttctg aggcttctgg acttgcactg ggccatacta ccagcatccc agggtctcca 5280

gcttgcagag agcctgttgt gggacttttc agcctccata atcaagtaag ccaatttccc 5340

tggtatctat atagatatac aatcatgttt tgcttaccag cctgaaaaat gtatcgctag 5400

atgagtctgt cattgcataa acatcatagt gtacttacac aaacctagat tctatagcct 5460

actacacacc tagtctataa acatgtacag catgttactg tactgaatat tgtaggcaac 5520

tgtaacacaa tggtgaatat ttgcatattt aaacatatct tatcattaaa aagatacagt 5580

aaacataagg tataaaagac aaaaaccggc acacctatat agggcactta ccataaatgc 5640

agcttgcagg actagaagtc actcagggtg agtcagtgag cgaacgtgaa ggcctaggtt 5700

attactgtcc actacggtag actttatcaa cactgtacac aggctacact aaatttattt 5760

tttaaaaatt tgctctccaa taataaatta atcttcgcat ccttttttttg ttgttcactg 5820

tgg 5823

<210> 418
<211> 707
<212> DNA
<213> Homo sapiens

<400> 418

aacggtgtca gctggagtga actcctgtgt gtgcaaggcc tgggtctcct ggtcagacta 60
ctttctatgg gaaaggcata gtgtatagtc tatatactat acataggggt gctgggagga 120
actggggttt tcacagccag ctttggtttt cattaggttt gtttagtttc cattgcttca 180
ggggtgttag ttttgtgttc mcaactagat tataaactcc tcttgcattc ctgatggcag 240
tgacttgaag gcatttattt gaagaataat agacatacag aaaggggcgc atgtcataaa 300
ggtacagctg gacgactttt cacaaagtga gcacatttgt atgatcgatg ttgagaccaa 360
gagcattcag tggacaactc ctttccagtt actccacccc actcccagtg accatcattc 420
tgacttctaa ctgtgtagac atgttttgct tgttttgtac tttacaaaca tatctactct 480
attttaggtg gctagacaat gtgttttaca atgctggcca tgacagtgtt tgaaagaata 540
aaatggaatc aaatagaatg ggcagtatca gagtgtgttg cctgcctaag aaatgttttg 600
tgacattttg gctttgggtc tatttacaca ttaaatctaa gagcaccaga atgtggtgtc 660
aaaatgtgtt tggggatgaa gatattctaa agtcctgtag taagcaa 707

<210> 419
<211> 712
<212> DNA
<213> Homo sapiens

<400> 419

cagactactt tctatgggaa aggcatagtg tatagtctat atactataca taggggtgct 60
gggaggaact ggggttttca cagccagctt tggttttcat taggtttgtt tagtttccat 120
tgcttcaggg gtgttagttt tgtgttccca actagattat aaactcctct tgcattcctg 180
atggcagtga cttgaaggca tttatttgaa gaataataga catacagaaa ggggcrcatg 240
tcataaaggt acagctggac gacttttcac aaagtgagca catttgtatg atcgatgttg 300
agaccaagag cattcagtgg acaactcctt tccagttact ccaccccact cccagtgacc 360
atcattctga cttctaactg tgtagacatg ttttgcttgt tttgtacttt acaaacatat 420
ctactctatt ttaggtggct agacaatgtg ttttacaatg ctggccatga cagtgtttga 480
aagaataaaa tggaatcaaa tagaatgggc agtatcagag tgtgttgcct gcctaagaaa 540
tgttttgtga cattttggct ttgggtctat ttacacatta aatctaagag caccagaatg 600
tggtgtcaaa atgtgtttgg ggatgaagat attctaaagt cctgtagtaa gcaatgcaaa 660
acgttctgga ggtgtttatt aaacatttgt ttgtagaatg gagaggaaga ca 712

<210> 420
<211> 1210
<212> DNA
<213> Homo sapiens

<400> 420

```
aaagcagcac tgctctgcat tcagccttgc tacgtctcct tcagatgggc gcactagata      60
ctgagtgatg atcatgcctt gtctaggatc tcaccaagac agttcatgaa agagacagtg     120
cagctcatgg aggagatggt gcagctcaca gagaggatgg tgccatcatg gaaagcatgg     180
ggcagtcatg gagatgacgg rgtagctcat ggagaatata atgccatcat ggaaggcata     240
gtgcagtcat ggagatgatg gtgcagctca tggagaagat ggtgccatca tggaaggcat     300
ggtgcaatca tggagtagac agtgcagctg ggccaagatt ctccctgact aagctcttct     360
caggcacctc tgagccgtcg tcttaactag gcctccagct tggcttgtga aaactgcaga     420
ctctcagcac aaatgatttg cctcctacat taagagactt aaataaacac ttgcatggct     480
gtgtttattt aaacagctca aggctgtgtc cctgggatga caatgactcc agcccctaaa     540
attcctgctt gtgaaagctc attgctgaca gaaggatcta ccatttgttc cagccaacac     600
ctggtggcag gcagataggc cctgagcccc atttaagagc agttccttta gaaagcttgc     660
aattgtaaat cttttctctg cccatttgag atgtaaatct tctaccacct agaactgtct     720
tctcaaggac ctgtgagctg actcactgaa atgcaaacat tcagggagat aactccactc     780
ctgtccccat gcgacggcga ggccctgact ttggtgggca ccttgctctt atttgcacca     840
ccacctcctg tcctaaagac atgagacgtt tgtctctcct ctggataagt gcctattaac     900
caacccaggt gtcctggtca catgaaccag tccagcctag cacctggcac tgcctttccc     960
tcagcacact ccagtctgta aaagtctcct tatggttgtt ttggcaaagt tgagcttagt    1020
taatgctaga ccccttctct actgcaatag ttactgctga ataaagtcta tccttaccac    1080
tttaactagt gttgggcttt gtttctcttt cataagctca tggagaagac aatgcagttc    1140
catcaagttt ctggctctta cactgctaac agtcagctct ggggtccctg agagggacag    1200
actcacacca                                                          1210
```

<210> 421
<211> 1194
<212> DNA
<213> Homo sapiens

<400> 421

```
gcattcagcc ttgctacgtc tccttcagat gggcgcacta gatactgagt gatgatcatg      60
ccttgtctag gatctcacca agacagttca tgaaagagac agtgcagctc atggaggaga     120
tggtgcagct cacagagagg atggtgccat catggaaagc atggggcagt catggagatg     180
acggagtagc tcatggagaa kataatgcca tcatggaagg catagtgcag tcatggagat     240
gatggtgcag ctcatggaga agatggtgcc atcatggaag gcatggtgca atcatggagt     300
agacagtgca gctgggccaa gattctccct gactaagctc ttctcaggca cctctgagcc     360
gtcgtcttaa ctaggcctcc agcttggctt gtgaaaactg cagactctca gcacaaatga     420
tttgcctcct acattaagag acttaaataa acacttgcat ggctgtgttt atttaaacag     480
ctcaaggctg tgtccctggg atgacaatga ctccagcccc taaaattcct gcttgtgaaa     540
gctcattgct gacagaagga tctaccattt gttccagcca acacctggtg gcaggcagat     600
aggccctgag ccccatttaa gagcagttcc tttagaaagc ttgcaattgt aaatcttttc     660
tctgcccatt tgagatgtaa atcttctacc acctagaact gtcttctcaa ggacctgtga     720
gctgactcac tgaaatgcaa acattcaggg agataactcc actcctgtcc ccatgcgacg     780
gcgaggccct gactttggtg ggcaccttgc tcttatttgc accaccacct cctgtcctaa     840
agacatgaga cgtttgtctc tcctctggat aagtgcctat taaccaaccc aggtgtcctg     900
gtcacatgaa ccagtccagc ctagcacctg gcactgcctt tccctcagca cactccagtc     960
tgtaaaagtc tccttatggt tgttttggca aagttgagct tagttaatgc tagacccctt    1020
ctctactgca atagttactg ctgaataaag tctatcctta ccactttaac tagtgttggg    1080
ctttgtttct ctttcataag ctcatggaga agacaatgca gttccatcaa gtttctggct    1140
cttacactgc taacagtcag ctctggggtc cctgagaggg acagactcac acca          1194
```

<210> 422
<211> 1194
<212> DNA
<213> Homo sapiens

<400> 422

gcattcagcc ttgctacgtc tccttcagat gggcgcacta gatactgagt gatgatcatg 60
ccttgtctag gatctcacca agacagttca tgaaagagac agtgcagctc atggaggaga 120
tggtgcagct cacagagagg atggtgccat catggaaagc atggggcagt catggagatg 180
acggagtagc tcatggagaa kataatgcca tcatggaagg catagtgcag tcatggagat 240
gatggtgcag ctcatggaga agatggtgcc atcatggaag gcatggtgca atcatggagt 300
agacagtgca gctgggccaa gattctccct gactaagctc ttctcaggca cctctgagcc 360
gtcgtcttaa ctaggcctcc agcttggctt gtgaaaactg cagactctca gcacaaatga 420
tttgcctcct acattaagag acttaaataa acacttgcat ggctgtgttt atttaaacag 480
ctcaaggctg tgtccctggg atgacaatga ctccagcccc taaaattcct gcttgtgaaa 540
gctcattgct gacagaagga tctaccattt gttccagcca acacctggtg gcaggcagat 600
aggccctgag ccccatttaa gagcagttcc tttagaaagc ttgcaattgt aaatcttttc 660
tctgcccatt tgagatgtaa atcttctacc acctagaact gtcttctcaa ggacctgtga 720
gctgactcac tgaaatgcaa acattcaggg agataactcc actcctgtcc ccatgcgacg 780
gcgaggccct gactttggtg ggcaccttgc tcttatttgc accaccacct cctgtcctaa 840
agacatgaga cgtttgtctc tcctctggat aagtgcctat taaccaaccc aggtgtcctg 900
gtcacatgaa ccagtccagc ctagcacctg gcactgcctt tccctcagca cactccagtc 960
tgtaaaagtc tccttatggt tgttttggca aagttgagct tagttaatgc tagacccctt 1020
ctctactgca atagttactg ctgaataaag tctatcctta ccactttaac tagtgttggg 1080
ctttgtttct ctttcataag ctcatggaga agacaatgca gttccatcaa gtttctggct 1140
cttacactgc taacagtcag ctctggggtc cctgagaggg acagactcac acca 1194

<210> 423
<211> 1118
<212> DNA
<213> Homo sapiens

<400> 423

accaagacag ttcatgaaag agacagtgca gctcatggag gagatggtgc agctcacaga 60

gaggatggtg ccatcatgga aagcatgggg cagtcatgga gatgacggag tagctcatgg 120

agaagataat gccatcatgg aaggcatagt gcagtcatgg agatgatggt gcagctcatg 180

gagaagatgg tgccatcatg raaggcatgg tgcaatcatg gagtagacag tgcagctggg 240

ccaagattct ccctgactaa gctcttctca ggcacctctg agccgtcgtc ttaactaggc 300

ctccagcttg gcttgtgaaa actgcagact ctcagcacaa atgatttgcc tcctacatta 360

agagacttaa ataaacactt gcatggctgt gtttatttaa acagctcaag gctgtgtccc 420

tgggatgaca atgactccag cccctaaaat tcctgcttgt gaaagctcat tgctgacaga 480

aggatctacc atttgttcca gccaacacct ggtggcaggc agataggccc tgagccccat 540

ttaagagcag ttcctttaga aagcttgcaa ttgtaaatct tttctctgcc catttgagat 600

gtaaatcttc taccacctag aactgtcttc tcaaggacct gtgagctgac tcactgaaat 660

gcaaacattc agggagataa ctccactcct gtccccatgc gacggcgagg ccctgacttt 720

ggtgggcacc ttgctcttat ttgcaccacc acctcctgtc ctaaagacat gagacgtttg 780

tctctcctct ggataagtgc ctattaacca acccaggtgt cctggtcaca tgaaccagtc 840

cagcctagca cctggcactg cctttccctc agcacactcc agtctgtaaa agtctcctta 900

tggttgtttt ggcaaagttg agcttagtta atgctagacc ccttctctac tgcaatagtt 960

actgctgaat aaagtctatc cttaccactt taactagtgt tgggctttgt ttctctttca 1020

taagctcatg gagaagacaa tgcagttcca tcaagtttct ggctcttaca ctgctaacag 1080

tcagctctgg ggtccctgag agggacagac tcacacca 1118

<210> 424
<211> 601
<212> DNA
<213> Homo sapiens

<400> 424

gtaggggcac tgtctatact ggctgcactc tggccagtgc tgtcccaacg ctgacccctc 60

tggaagctaa tctggcttat aatgaggatg ctttctttag aggggactct ccatgcacag 120

cagaaaatcc caatggagtg gttcttccct atgtccccaa gggactggga atattctttc 180

agtaacaatg gcccattggg ggaagaagga tgaaagtggg gtgagagacg tgaaatttgg 240

agaggtccct caaagattgt gatgtgcctc tcttgttcca atcacaggac aggggtataa 300

yggctttcct ttgaaacacg gggatgaatt taactattca cttcccaggt agattcatca 360

```
gggtctagag cttcagctaa cagcatgagg aagattccaa atgtgccccc atcagcatag      420
gaactgggta tgttgagtct atggtctcat aaaaccagaa gaaggacaag ggattgtggc      480
tccaggcttg ggagcacctt ttccttacca tgggctacag tatttattta gggtaaagga      540
aggaaactcc tgaggtgcta tggggtgcca gcaatttgga gcatcagtaa ttcaatgtcc      600
c                                                                      601
```

<210> 425
<211> 601
<212> DNA
<213> Homo sapiens

<400> 425

```
acgctgaccc ctctggaagc taatctggct tataatgagg atgctttctt tagaggggac       60
tctccatgca cagcagaaaa tcccaatgga gtggttcttc cctatgtccc caagggactg      120
ggaatattct ttcagtaaca atggcccatt gggggaagaa ggatgaaagt ggggtgagag      180
acgtgaaatt tggagaggtc cctcaaagat tgtgatgtgc ctctcttgtt ccaatcacag      240
gacaggggta taacggcttt cctttgaaac acggggatga atttaactat tcacttccca      300
rgtagattca tcagggtcta gagcttcagc taacagcatg aggaagattc caaatgtgcc      360
cccatcagca taggaactgg gtatgttgag tctatggtct cataaaacca gaagaaggac      420
aagggattgt ggctccaggc ttgggagcac cttttcctta ccatgggcta cagtatttat      480
ttagggtaaa ggaaggaaac tcctgaggtg ctatggggtg ccagcaattt ggagcatcag      540
taattcaatg tcccttcagc catgtgtatt caactcctgc tgtgggtgtg gacttggtgc      600
a                                                                      601
```

<210> 426
<211> 701
<212> DNA
<213> Homo sapiens

<400> 426

```
ttcctgggca tcgtcatatt ctgtaaaaca aggaagctca gcccagtgtg ttctaacatg        60
acctcctttc tacatcctta ggtgttgtta tgcgtgaatc acgtcccccc aaaagacatg       120
ttcatgtcct aaccccccagg acctcagaat gtgtgatctg gtttggaaat aaggtcatca       180
cagatgaaat tagctaagac aaggtcatat tggaataggg ttggccctta atccactgtg       240
actggtgtcc ttttaagaag aggacacaga cacaggaggg gagagggcca tgggatgatg       300
caggtggaga ctggagtgct acagctgcaa gcaaatacat ttctgtgctg tgaagccacc       360
catttggtgg tactacgtta aaacagctct aggaaattaa tacagatgtt gcctgtattt       420
ttgtttctca tattactact cattgtttta atgatgactg ttttattcat taagttgaaa       480
gctcctaaag cagagggacc rtatttttat gtcccaactc tccttaaggc cttgcctatg       540
atagcacatc tcttcaatag aattgtccta actttaacag agacaacttg ggttatttaa       600
tatggagaac aaagggttaa gctggtgcca gatgggtttc attttctcta aatctggaac       660
caaaggcagc aagtctatgg ggtggacgga gttcttagct c                           701
```

<210> 427
<211> 701
<212> DNA
<213> Homo sapiens

<400> 427

```
caaggaagct cagcccagtg tgttctaaca tgacctcctt tctacatcct taggtgttgt        60
tatgcgtgaa tcacgtcccc ccaaaagaca tgttcatgtc ctaaccccca ggacctcaga       120
atgtgtgatc tggtttggaa ataaggtcat cacagatgaa attagctaag acaaggtcat       180
attggaatag ggttggccct taatccactg tgactggtgt ccttttaaga agaggacaca       240
gacacaggag gggagagggc catgggatga tgcaggtgga gactggagtg ctacagctgc       300
aagcaaatac atttctgtgc tgtgaagcca cccatttggt ggtactacgt taaaacagct       360
ctaggaaatt aatacagatg ttgcctgtat ttttgtttct catattacta ctcattgttt       420
taatgatgac tgttttattc attaagttga aagctcctaa agcagaggga ccatattttt       480
atgtcccaac tctccttaag sccttgccta tgatagcaca tctcttcaat agaattgtcc       540
taactttaac agagacaact tgggttattt aatatggaga acaaagggtt aagctggtgc       600
cagatgggtt tcattttctc taaatctgga accaaaggca gcaagtctat ggggtggacg       660
gagttcttag ctcaaccctt tggtgaggta agaagaagga t                           701
```